# EUROPEAN PATENT SPECIFICATION

(11) **EP 2 480 549 B1**
(45) Date of publication and mention of the grant of the patent: **08.04.2015**
(21) Application number: 10769036.4
(22) Date of filing: 24.09.2010
(51) Int. Cl.: C07D 471/14, C07D 487/04, C07D 487/14, C07D 519/00, A61K 31/4985, A61P 35/00, A61P 37/06, A61P 29/00, A61P 31/12, A61P 25/00, A61P 9/00, A61P 11/00, A61P 19/00, A61P 3/00, A61P 25/28

(54) **Fused imidazo[3,2-d]pyrazines as PI3 kinase inhibitors**
Kondensierte Imidazo[3,2-d]pyrazine als PI3 Kinase Inhibitoren
Imidazo[3,2-d]pyrazines fusionnees utilisees comme inhibiteurs de kinase PI3

(30) Priority: 24.09.2009 EP 09380153
(43) Date of publication of application: 01.08.2012
(73) Proprietor: Fundación Centro Nacional De Investigaciones Oncológicas Carlos III, 28029 Madrid (ES)
(72) Inventor: PASTOR FERNÁNDEZ, Joaquín, E-28029 Madrid (ES); MARTÍNEZ GONZÁLEZ, Sonia, E-28029 Madrid (ES); ALVAREZ ESCOBAR, Rosa María, E-28029 Madrid (ES); RODRÍGUEZ ARISTEGUI, Sonsoles, E-28029 Madrid (ES); GONZÁLES CANTALAPIEDRA, Esther, E-28029 Madrid (ES); HERNÁNDEZ HIGUERAS, Ana Isabel, E-28029 Madrid (ES); VARELA BUSTO, Carmen, E-28029 Madrid (ES)
(74) Representative: Potter Clarkson LLP
(86) International application number: PCT/GB2010/001804
(87) International publication number: WO 2011/036461

(56) References cited:
- EP-A1- 1 277 738
- WO-A1-2008/077631
- HAYAKAWA ET AL: "Synthesis and biological evaluation of pyrido[3',2':4,5]furo[3,2-d]py rimidine derivatives as novel PI3 kinase p110alpha inhibitors", BIOORGANIC & MEDICINAL CHEMISTRY LETTERS, PERGAMON, ELSEVIER SCIENCE, GB, vol. 17, no. 9, 3 April 2007 (2007-04-03), pages 2438-2442, XP022015320, ISSN: 0960-894X

## Description

### Field of the Invention

This invention relates to novel pharmaceutically-useful compounds, which compounds are useful as inhibitors of protein or lipid kinases (such as inhibitors of the phosphoinositide 3'OH kinase (PI3 kinase) family, particularly the PI3K class I sub-type. The compounds may also be useful as inhibitors of the mammalian target of rapamycin (mTOR)). The compounds are of potential utility in the treatment of diseases such as cancer. The invention also relates to the use of such compounds as medicaments, to the use of such compounds for *in vitro, in situ* and *in vivo* diagnosis or treatment of mammalian cells (or associated pathological conditions), to pharmaceutical compositions containing them, and to synthetic routes for their production.

### Background of the Invention

The malfunctioning of protein kinases (PKs) is the hallmark of numerous diseases. A large share of the oncogenes and proto-oncogenes involved in human cancers code for PKs. The enhanced activities of PKs are also implicated in many non-malignant diseases, such as benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis. PKs are also implicated in inflammatory conditions and in the multiplication of viruses and parasites. PKs may also play a major role in the pathogenesis and development of neurodegenerative disorders.

For a general reference to PKs malfunctioning or disregulation see, for instance, Current Opinion in Chemical Biology 1999, 3, 459 - 465.

Phosphatidylinositol 3-kinases (PI3Ks) are a family of lipid and serine/threonine kinases that catalyze the phosphorylation of the membrane lipid phosphatidylinositol (PI) on the 3'-OH of the inositol ring to produce phosphoinositol-3-phosphate (PIP), phosphoinositol-3,4-diphosphate (PIP₂) and phosphoinositol-3,4,5-triphosphate (PIP₃), which act as recruitment sites for various intracellular signalling proteins, which in turn form signalling complexes to relay extracellular signals to the cytoplasmic face of the plasma membrane. These 3'-phosphoinositide subtypes function as second messengers in intracellular signal transduction pathways (see e.g. Trends Biochem. Sci 22 87,267-72 (1997) by Vanhaesebroeck *et al*.; Chem. Rev. 101 (8), 2365-80 (2001) by Leslie et al (2001); Annu. Rev. Cell. Dev. Boil. 17, 615-75 (2001) by Katso *et al*; and Cell. Mol. Life Sci. 59 (5), 761-79 (2002) by Toker et al).

Multiple PI3K isoforms categorized by their catalytic subunits, their regulation by corresponding regulatory subunits, expression patterns and signalling specific funtions (p110α, β, δ, γ) perform this enzymatic reaction (Exp. Cell. Res. 25 (1),. 239-54 (1999) by Vanhaesebroeck and Katso et al., 2001, above).

The closely related isoforms p110α and β are ubiquitously expressed, while δ and γ are more specifically expressed in the haematopoietic cell system, smooth muscle cells, myocytes and endothelial cells (see e.g. Trends Biochem. Sci. 22 (7),. 267-72 (1997) by Vanhaesebroeck et al). Their expression might also be regulated in an inducible manner depending on the cellular, tissue type and stimuli as well as disease context. Inductibility of protein expression includes synthesis of protein as well as protein stabilization that is in part regulated by association with regulatory subunits.

Eight mammalian PI3Ks have been identified so far, including four class I PI3Ks. Class Ia includes PI3Kα, PI3Kβ and PI3Kδ. All of the class Ia enzymes are heterodimeric complexes comprising a catalytic subunit (p110α, p110β or p110δ) associated with an SH2 domain containing p85 adapter subunit. Class Ia PI3Ks are activated through tyrosine kinase signalling and are involved in cell proliferation and survival. PI3Kα and PI3Kβ have also been implicated in tumorigenesis in a variety of human cancers. Thus, pharmacological inhibitors of PI3Kα and PI3Kβ are useful for treating various types of cancer.

PI3Kγ, the only member of the Class Ib PI3Ks, consists of a catalytic subunit p110γ, which is associated with a p110 regulatory subunit. PI3Kγ is regulated by G protein coupled receptors (GPCRs) via association with βγ subunits of heterotrimeric G proteins. PI3Kγ is expressed primarily in hematopoietic cells and cardiomyocytes and is involved in inflammation and mast cell function. Thus, pharmacological inhibitors of PI3Kγ are useful for treating a variety of inflammatory diseases, allergies and cardiovascular diseases.

These observations show that deregulation of phosphoinositol-3-kinase and the upstream and downstream components of this signalling pathway is one of the most common deregulations associated with human cancers and proliferative diseases (see e.g. Parsons et al., Nature 436:792 (2005); Hennessey et al., Nature Rev. Drug Discovery 4: 988-1004 (2005).

The mammalian target of rapamycin (mTOR) also known as FK506 binding protein 12-rapamycin associated protein 1 (FRAP1) is a protein which in humans is encoded by the *FRAP1* gene. mTOR is a serine/threonine protein kinase that regulates cell growth, cell proliferation, cell motility, cell survival, protein synthesis, and transcription. The inhibition of mTORs are believed to be useful for treating various diseases/conditions, such as cancer (for example, as described in Easton et al. (2006). "mTOR and cancer therapy". Oncogene 25 (48): 6436-46).

The listing or discussion of an apparently prior-published document in this specification should not necessarily be taken as an acknowledgement that the document is part of the state of the art or is common general knowledge.

Patent applications EP1277738 and WO 2008/077631 and Hayakawa et al., Bioorganic and Medicinal Chemistry Letters, 17 (2007), pages 2438-2442 disclose tricyclic compounds as PI3 kinase inhibitors.

International patent applications WO 2009/040552 and WO 2009/060197 disclose imidazolodiathiazoles and imidazopyridazines for use as kinase inhibitors and/or for use in the treatment of e.g. cancer. These applications do not disclose or suggest imidazopyrazines.

Unpublished international patent application number PCT/GB2010/000773 discloses imidazopyrazines for use as kinase inhibitors and/or for use in the treatment of e.g. cancer. However, this document does not mention or suggest imidazopyrazines that are further fused to a third ring to form a tricyclic structure.

### Disclosure of the Invention

According to the invention, there is now provided a compound of formula I, 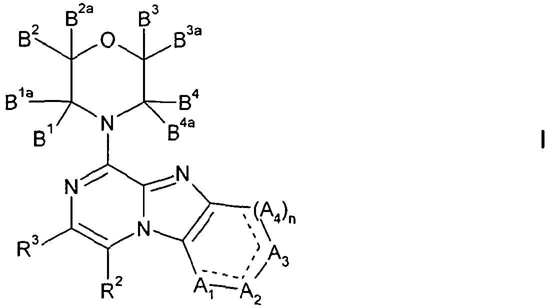 wherein:
n represents 0, 1 or 2;
A₁, A₂, A₃ and each A₄ (if present) independently represents -C(R⁴)R⁵-, -N(R⁶)-, -C(O)-, -O-, -S-, -S(O)- or -S(O)₂-;
the dotted lines represent the presence of an optional double bond, which may be present between A₁ and A₂, A₂ and A₃, A₃ and A₄ (if the latter is present, i.e. when n does not represent 0) and/or between two A₄ groups (if present, i.e. when n represents 2), provided that the A₁ to A₄-containing ring is not aromatic;
each B¹, B^{1a}, B², B^{2a}, B³, B^{3a}, B⁴ and B^{4a} independently represent hydrogen or a substituent selected from halo, -C(=Y)-R^{10a}, -C(=Y)-OR^{10a}, -C(=Y)N(R^{10a})R^{11a}, -S(O)₂N(R^{10a})R^{11a}, C₁₋₁₂ alkyl, heterocycloalkyl (which latter two groups are optionally substituted by one or more substituents selected from =O and E¹), aryl and/or heteroaryl (which latter two groups are optionally substituted by one or more substituents selected from E²); or
any two B¹, B^{1a}, B², B^{2a}, B³, B^{3a}, B⁴ and B^{4a} substituents that are attached to the same carbon atom (i.e. B¹ and B^{1a}; B² and B^{2a} ; B³ and B^{3a}; and/or B⁴ and B^{4a}) may together form a =O group;
or, any two B¹, B^{1a}, B², B^{2a}, B³, B^{3a}, B⁴ and B^{4a} substituents may be linked together to form a further 3- to 12- membered (e.g. 3- to 6-membered) ring, optionally containing (in addition to the atom(s) of the morpholine ring) one or more (e.g. two or, preferably, one) heteroatom(s) (preferably selected from sulfur, oxygen and nitrogen), which ring optionally contains one or more (e.g. one to three) double bonds, and which ring is itself optionally substituted by one or more substituents selected from halo, =O and C₁₋₃ alkyl optionally substituted by one or more fluoro atoms;
R² represents hydrogen or a substituent selected from halo, -CN, -OR^{10b}, -N(R^{10b})R^{11b}, -C(O)N(R^{10b})R^{11b}, C₁₋₁₂ (e.g. C₁₋₆) alkyl and heterocycloalkyl (e.g. a 3- to 7-membered heterocycloalkyl), which latter two groups are optionally substituted by one or more substituents selected from E³ and =O;
R³ represents aryl or heteroaryl (both of which are optionally substituted by one or more substituents selected from E⁴);
R⁴ and R⁵ independently represent, on each occasion when used herein, hydrogen, halo, -OR^{10c}, -N(R^{10d})R^{11d}, -N(R^{10e})-C(O)-R^{10f}, -C(O)R^{10g}, -C(O)OR^{10h}, -C(O)N(R¹⁰ⁱ)R¹¹ⁱ, -N(R^{10j})-C(O)OR^{10k}, -N(R^{10m})-C(O)-N(R¹⁰ⁿ)R¹¹ⁿ, -N[-C(O)-T¹-R^{10p}]-C(O)-T²-R^{10q}, C₁₋₁₂ alkyl, heterocycloalkyl (which latter two groups are optionally substituted by one or more substituents selected from E⁵ and =O), aryl or heteroaryl (which latter two groups are optionally substituted by one or more substituents selected from E⁶ and =O); or
R⁴ and R⁵ may be linked together to form a 3- to 6-membered ring, optionally containing one or more (e.g. two or, preferably, one) heteroatom(s) (preferably selected from sulfur, oxygen and nitrogen), which ring optionally contains one or more (e.g. one or two) double bonds, and which ring is itself optionally substituted by one or more substituents selected from halo, =O and C₁₋₃ alkyl optionally substituted by one or more fluoro atoms;
T¹ and T² independently represent a single bond, -N(R^{10x})- or -O-;
R⁶ represents hydrogen, -C(O)-R^{10r}, -C(O)-OR^{10s}, -C(O)-N(R^{10t})R^{11t}, -S(O)₂R^{10u}, C₁₋₁₂ alkyl, heterocycloalkyl (which latter two groups are optionally substituted by one or more substituents selected from E⁷ and =O), aryl or heteroaryl (which latter two groups are optionally substituted by one or more substituents selected from E⁸ and =O);
each R^{10a} R^{11a}, R^{10b}, R^{11b}, R^{10c}, R^{10d}, R^{11d}, R^{10e}, R^{10f}, R^{10g}, R^{10h}, R¹⁰ⁱ, R¹¹ⁱ, R^{10j}, R^{10k}, R^{10m}, R¹⁰ⁿ, R¹¹ⁿ, R^{10p}, R^{10q}, R^{10r}, R^{10s}, R^{10t}, R^{11t}, R^{10u} and R^{10x} independently represent, on each occasion when used herein, hydrogen, C₁₋₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹); or
any relevant pair of R^{10a} and R^{11a} (for example, when attached to the same atom, adjacent atom (i.e. 1,2-relationship) or to atoms that are two atom atoms apart, i.e. in a 1,3-relationship) and/or any pair of R^{10b} and R^{11b}, R^{10d} and R^{11d}, R¹⁰ⁱ and R¹¹ⁱ, R¹⁰ⁿ and R¹¹ⁿ, and R^{10t} and R^{11t} may be linked together to form (e.g. along with the requisite nitrogen atom to which they may be attached) a 4- to 20- (e.g. 4- to 12-) membered ring, optionally containing one or more heteroatoms (for example, in addition to those that may already be present, e.g. (a) heteroatom(s) selected from oxygen, nitrogen and sulfur), optionally containing one or more unsaturations (preferably, double bonds), and which ring is optionally substituted by one or more substituents selected from =O, =S, =N(R²⁰) and E¹²;
each E¹, E², E³, E⁴, E⁵, E⁶, E⁷, E⁸, E¹⁰, E¹¹ and E¹² independently represents, on each occasion when used herein:
   (i) Q⁴;
   (ii) C₁₋₁₂ alkyl optionally substituted by one or more substituents selected from =O and Q⁵; or
any two E¹, E², E³, E⁴, E⁵, E⁶, E⁷, E⁸, E¹⁰, E¹¹ or E¹² groups, for example on C₁₋₁₂ alkyl groups, e.g. when they are attached to the same or adjacent carbon atoms, may be linked together to form a 3- to 12-membered ring, optionally containing one or more (e.g. one to three) unsaturations (preferably, double bonds), and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -OC(=Y)-R²⁰, -OC(=Y)-OR²⁰, -OC(=Y)N(R²⁰)R²¹, -OS(O)₂OR²⁰, -OP(=Y)(OR²⁰)(OR²¹), -OP(OR²⁰)(OR²¹), -N(R²²)C(=Y)R²¹, -N(R²²)C(=Y)OR²¹, -N(R²²)C(=Y)N(R²⁰)R²¹, -NR²²S(O)₂R²⁰, -NR²²S(O)₂N(R²⁰)R²¹, -S(O)₂N(R²⁰)R²¹, -SC(=Y)R²⁰, -S(O)₂R²⁰, -SR²⁰, -S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
each Y independently represents, on each occasion when used herein, =O, =S, =NR²³ or =N-CN;
each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or
any relevant pair of R²⁰, R²¹ and R²², may (for example, when attached to the same atom, adjacent atom (i.e. 1,2-relationship) or to atoms that are two atom atoms apart, i.e. in a 1,3-relationship) be linked together to form (e.g. along with the requisite nitrogen atom to which they may be attached) a 4- to 20- (e.g. 4- to 12-) membered ring, optionally containing one or more heteroatoms (for example, in addition to those that may already be present, e.g. (a) heteroatom(s) selected from oxygen, nitrogen and sulfur), optionally containing one or more unsaturations (preferably, double bonds), and which ring is optionally substituted by one or more substituents selected from J⁶ and =O;
each J¹, J², J³, J⁴, J⁵ and J⁶ independently represents, on each occasion when used herein:
   (i) Q⁷;
   (ii) C₁₋₆ alkyl or heterocycloalkyl, both of which are optionally substituted by one or more substituents selected from =O and Q⁸;
each Q⁷ and Q⁸ independently represents, on each occasion when used herein: halo, -N(R⁵⁰)R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹, -NR⁵²S(O)₂R⁵⁰, -S(O)₂R⁵⁰, -SR⁵⁰, -S(O)R⁵⁰ or C₁₋₆ alkyl optionally substituted by one or more fluoro atoms;
each Y^{a} independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or
any relevant pair of R⁵⁰, R⁵¹ and R⁵² may (for example when attached to the same or adjacent atoms) be linked together to form, a 3- to 8-membered ring, optionally containing one or more heteroatoms (for example, in addition to those that may already be present, heteroatoms selected from oxygen, nitrogen and sulfur), optionally containing one or more unsaturations (preferably, double bonds), and which ring is optionally substituted by one or more substituents selected from =O and C₁₋₃ alkyl;
R⁶⁰, R⁶¹ and R⁶² independently represent hydrogen or C₁₋₆ alkyl optionally substituted by one or more fluoro atoms;
or a pharmaceutically acceptable ester, amide, solvate or salt thereof,
which compounds, esters, amides, solvates and salts are referred to hereinafter as "the compounds of the invention".

Pharmaceutically-acceptable salts include acid addition salts and base addition salts. Such salts may be formed by conventional means, for example by reaction of a free acid or a free base form of a compound of formula I with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin.

By "pharmaceutically acceptable ester, amide, solvate or salt thereof", we include salts of pharmaceutically acceptable esters or amides, and solvates of pharmaceutically acceptable esters, amides or salts. For instance, pharmaceutically acceptable esters and amides such as those defined herein may be mentioned, as well as pharmaceutically acceptable solvates or salts.

Pharmaceutically acceptable esters and amides of the compounds of the invention are also included within the scope of the invention. Pharmaceutically acceptable esters and amides of compounds of the invention may be formed from corresponding compounds that have an appropriate group, for example an acid group, converted to the appropriate ester or amide. For example, pharmaceutically acceptable esters (of carboxylic acids of compounds of the invention) that may be mentioned include optionally substituted C₁₋₆ alkyl, C₅₋₁₀ aryl and/or C₅₋₁₀ aryl-C₁₋₆ alkyl- esters. Pharmaceutically acceptable amides (of carboxylic acids of compounds of the invention) that may be mentioned include those of the formula -C(O)N(R^{z1})R^{z2}, in which R^{z1} and R^{z2} independently represent optionally substituted C₁₋₆ alkyl, C₅₋₁₀ aryl, or C₅₋₁₀ aryl-C₁₋₆ alkylene-. Preferably, C₁₋₆ alkyl groups that may be mentioned in the context of such pharmaceutically acceptable esters and amides are not cyclic, e.g. linear and/or branched.

Further compounds of the invention that may be mentioned include carbamate, carboxamido or ureido derivatives, e.g. such derivatives of existing amino functional groups.

Prodrugs of compounds of the invention are also described.

The term "prodrug" of a relevant compound of the invention includes any compound that, following oral or parenteral administration, is metabolised *in vivo* to form that compound in an experimentally-detectable amount, and within a predetermined time (e.g. within a dosing interval of between 6 and 24 hours (i.e. once to four times daily)). For the avoidance of doubt, the term "parenteral" administration includes all forms of administration other than oral administration.

Prodrugs of compounds of the invention may be prepared by modifying functional groups present on the compound in such a way that the modifications are cleaved, *in vivo* when such prodrug is administered to a mammalian subject. The modifications typically are achieved by synthesising the parent compound with a prodrug substituent. Prodrugs include compounds of the invention wherein a hydroxyl, amino, sulfhydryl, carboxy or carbonyl group in a compound of the invention is bonded to any group that may be cleaved *in vivo* to regenerate the free hydroxyl, amino, sulfhydryl, carboxy or carbonyl group, respectively.

Examples of prodrugs include, but are not limited to, esters and carbamates of hydroxy functional groups, esters groups of carboxyl functional groups, N-acyl derivatives and N-Mannich bases. General information on prodrugs may be found e.g. in Bundegaard, H. "Design of Prodrugs" p. I-92, Elesevier, New York-Oxford (1985).

Compounds of the invention may contain double bonds and may thus exist as E (*entgegen*) and Z *(zusammen)* geometric isomers about each individual double bond. Positional isomers may also be embraced by the compounds of the invention. All such isomers (e.g. if a compound of the invention incorporates a double bond or a fused ring, the cis- and trans- forms, are embraced) and mixtures thereof are included within the scope of the invention (e.g. single positional isomers and mixtures of positional isomers may be included within the scope of the invention).

Compounds of the invention may also exhibit tautomerism. All tautomeric forms (or tautomers) and mixtures thereof are included within the scope of the invention. The term "tautomer" or "tautomeric form" refers to structural isomers of different energies which are interconvertible *via* a low energy barrier. For example, proton tautomers (also known as prototropic tautomers) include interconversions *via* migration of a proton, such as keto-enol and imine-enamine isomerisations. Valence tautomers include interconversions by reorganisation of some of the bonding electrons.

Compounds of the invention may also contain one or more asymmetric carbon atoms and may therefore exhibit optical and/or diastereoisomerism. Diastereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation or epimerisation (i.e. a 'chiral pool' method), by reaction of the appropriate starting material with a chiral auxiliary' which can subsequently be removed at a suitable stage, by derivatisation (i.e. a resolution, including a dynamic resolution), for example with a homochiral acid followed by separation of the diastereomeric derivatives by conventional means such as chromatography, or by reaction with an appropriate chiral reagent or chiral catalyst all under conditions known to the skilled person.

All stereoisomers (including but not limited to diastereoisomers, enantiomers and atropisomers) and mixtures thereof (e.g. racemic mixtures) are included within the scope of the invention.

In the structures shown herein, where the stereochemistry of any particular chiral atom is not specified, then all stereoisomers are contemplated and included as the compounds of the invention. Where stereochemistry is specified by a solid wedge or dashed line representing a particular configuration, then that stereoisomer is so specified and defined.

The compounds of the present invention may exist in unsolvated as well as solvated forms with pharmaceutically acceptable solvents such as water, ethanol, and the like, and it is intended that the invention embrace both solvated and unsolvated forms.

The present invention also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature). All isotopes of any particular atom or element as specified herein are contemplated within the scope of the compounds of the invention. Exemplary isotopes that can be incorporated into compounds of the invention include isotopes of hydrogen, carbon, nitrogen, oxygen, phosphorus, sulfur, fluorine, chlorine and iodine, such as ²H, ³H, ¹¹C, ¹³C, ¹⁴C, ¹³N, ¹⁵O, ¹⁷O, ¹⁸O, ³²P, ³³P, ³⁵S, ¹⁸F, ³⁶Cl, ¹²³I, and ¹²⁵I. Certain isotopically-labeled compounds of the present invention (e.g., those labeled with ³H and ¹⁴C) are useful in compound and for substrate tissue distribution assays. Tritiated (³H) and carbon-I4 (¹⁴C) isotopes are useful for their ease of preparation and detectability. Further, substitution with heavier isotopes such as deuterium (i.e., ²H may afford certain therapeutic advantages resulting from greater metabolic stability (e.g., increased *in vivo* half-life or reduced dosage requirements) and hence may be preferred in some circumstances. Positron emitting isotopes such as ¹⁵O, ¹³N, ¹¹C and ¹⁸F are useful for positron emission tomography (PET) studies to examine substrate receptor occupancy. Isotopically labeled compounds of the present invention can generally be prepared by following procedures analogous to those disclosed in the Scheme 1 and/or in the Examples herein below, by substituting an isotopically labeled reagent for a non-isotopically labeled reagent.

Unless otherwise specified, C_{1-q} alkyl groups (where q is the upper limit of the range) defined herein may be straight-chain or, when there is a sufficient number (i.e. a minimum of two or three, as appropriate) of carbon atoms, be branched-chain, and/or cyclic (so forming a C_{3-q}-cycloalkyl group). Such cycloalkyl groups may be monocyclic or bicyclic and may further be bridged. Further, when there is a sufficient number (i.e. a minimum of four) of carbon atoms, such groups may also be part cyclic. Such alkyl groups may also be saturated or, when there is a sufficient number (i.e. a minimum of two) of carbon atoms, be unsaturated (forming, for example, a C_{2-q} alkenyl or a C_{2-q} alkynyl group).

Unless otherwise stated, the term C_{1-q} alkylene (where q is the upper limit of the range) defined herein may be straight-chain or, when there is a sufficient number of carbon atoms, be saturated or unsaturated (so forming, for example, an alkenylene or alkynylene linker group). However, such C_{1-q} alkylene groups may not be branched.

C_{3-q} cycloalkyl groups (where q is the upper limit of the range) that may be specifically mentioned may be monocyclic or bicyclic alkyl groups, which cycloalkyl groups may further be bridged (so forming, for example, fused ring systems such as three fused cycloalkyl groups). Such cycloalkyl groups may be saturated or unsaturated containing one or more double bonds (forming for example a cycloalkenyl group). Substituents may be attached at any point on the cycloalkyl group. Further, where there is a sufficient number (i.e. a minimum of four) such cycloalkyl groups may also be part cyclic.

The term "halo", when used herein, preferably includes fluoro, chloro, bromo and iodo.

Heterocycloalkyl groups that may be mentioned include non-aromatic monocyclic and bicyclic heterocycloalkyl groups in which at least one (e.g. one to four) of the atoms in the ring system is other than carbon (i.e. a heteroatom), and in which the total number of atoms in the ring system is between 3 and 20 (e.g. between three and ten, e.g between 3 and 8, such as 5- to 8-). Such heterocycloalkyl groups may also be bridged. Further, such heterocycloalkyl groups may be saturated or unsaturated containing one or more double and/or triple bonds, forming for example a C_{2-q} heterocycloalkenyl (where q is the upper limit of the range) group. C_{2-q} heterocycloalkyl groups that may be mentioned include 7-azabicyclo[2.2.1]heptanyl, 6-azabicyclo[3.1.1]heptanyl, 6-azabicyclo[3.2.1]-octanyl, 8-azabicyclo-[3.2.1]octanyl, aziridinyl, azetidinyl, dihydropyranyl, dihydropyridyl, dihydropyrrolyl (including 2,5-dihydropyrrolyl), dioxolanyl (including 1,3-dioxolanyl), dioxanyl (including 1,3-dioxanyl and 1,4-dioxanyl), dithianyl (including 1,4-dithianyl), dithiolanyl (including 1,3-dithiolanyl), imidazolidinyl, imidazolinyl, morpholinyl, 7-oxabicyclo[2.2.1]heptanyl, 6-oxabicyclo-[3.2.1]octanyl, oxetanyl, oxiranyl, piperazinyl, piperidinyl, pyranyl, pyrazolidinyl, pyrrolidinonyl, pyrrolidinyl, pyrrolinyl, quinuclidinyl, sulfolanyl, 3-sulfolenyl, tetrahydropyranyl, tetrahydrofuranyl, tetrahydropyridyl (such as 1,2,3,4-tetrahydropyridyl and 1,2,3,6-tetrahydropyridyl), thietanyl, thiiranyl, thiolanyl, thiomorpholinyl, trithianyl (including 1,3,5-trithianyl), tropanyl and the like. Substituents on heterocycloalkyl groups may, where appropriate, be located on any atom in the ring system including a heteroatom. The point of attachment of heterocycloalkyl groups may be *via* any atom in the ring system including (where appropriate) a heteroatom (such as a nitrogen atom), or an atom on any fused carbocyclic ring that may be present as part of the ring system. Heterocycloalkyl groups may also be in the *N*- or S- oxidised form. Heterocycloalkyl mentioned herein may be stated to be specifically monocyclic or bicyclic.

For the avoidance of doubt, the term "bicyclic" (e.g. when employed in the context of heterocycloalkyl groups) refers to groups in which the second ring of a two-ring system is formed between two adjacent atoms of the first ring. The term "bridged" (e.g. when employed in the context of cycloalkyl or heterocycloalkyl groups) refers to monocyclic or bicyclic groups in which two non-adjacent atoms are linked by either an alkylene or heteroalkylene chain (as appropriate).

Aryl groups that may be mentioned include C₆₋₂₀, such as C₆₋₁₂ (e.g. C₆₋₁₀) aryl groups. Such groups may be monocyclic, bicyclic or tricyclic and have between 6 and 12 (e.g. 6 and 10) ring carbon atoms, in which at least one ring is aromatic. C₆₋₁₀ aryl groups include phenyl, naphthyl and the like, such as 1,2,3,4-tetrahydro-naphthyl. The point of attachment of aryl groups may be *via* any atom of the ring system. For example, when the aryl group is polycyclic the point of attachment may be *via* atom including an atom of a non-aromatic ring. However, when aryl groups are polycyclic (e.g. bicyclic or tricyclic), they are preferably linked to the rest of the molecule *via* an aromatic ring. Aryl groups may be substituted on any non-aromatic rings by a =O moiety (but are preferably not so).

Unless otherwise specified, the term "heteroaryl" when used herein refers to an aromatic group containing one or more heteroatom(s) (e.g. one to four heteroatoms) preferably selected from N, O and S. Heteroaryl groups include those which have between 5 and 20 members (e.g. between 5 and 10) and may be monocyclic, bicyclic or tricyclic, provided that at least one of the rings is aromatic (so forming, for example, a mono-, bi-, or tricyclic heteroaromatic group). When the heteroaryl group is polycyclic the point of attachment may be *via* atom including an atom of a non-aromatic ring. However, when heteroaryl groups are polycyclic (e.g. bicyclic or tricyclic), they are preferably linked to the rest of the molecule *via* an aromatic ring. Heteroaryl groups that may be mentioned include 3,4-dihydro-1*H*-isoquinolinyl, 1,3-dihydroisoindolyl, 1,3-dihydroisoindolyl (e.g. 3,4-dihydro-1*H*-isoquinolin-2-yl, 1,3-dihydroisoindol-2-yl, 1,3-dihydroisoindol-2-yl; i.e. heteroaryl groups that are linked *via* a non-aromatic ring), or, preferably, acridinyl, benzimidazolyl, benzodioxanyl, benzodioxepinyl, benzodioxolyl (including 1,3-benzodioxolyl), benzofuranyl, benzofurazanyl, benzothiadiazolyl (including 2,1,3-benzothiadiazolyl), benzothiazolyl, benzoxadiazolyl (including 2,1,3-benzoxadiazolyl), benzoxazinyl (including 3,4-dihydro-2*H*-1,4-benzoxazinyl), benzoxazolyl, benzomorpholinyl, benzoselenadiazolyl (including 2,1,3-benzoselenadiazolyl), benzothienyl, carbazolyl, chromanyl, cinnolinyl, furanyl, imidazolyl, imidazo[1,2-a]pyridyl, indazolyl, indolinyl, indolyl, isobenzofuranyl, isochromanyl, isoindolinyl, isoindolyl, isoquinolinyl, isothiaziolyl, isothiochromanyl, isoxazolyl, naphthyridinyl (including 1,6-naphthyridinyl or, preferably, 1,5-naphthyridinyl and 1,8-naphthyridinyl), oxadiazolyl (including 1,2,3-oxadiazolyl, 1,2,4-oxadiazolyl and 1,3,4-oxadiazolyl), oxazolyl, phenazinyl, phenothiazinyl, phthalazinyl, pteridinyl, purinyl, pyranyl, pyrazinyl, pyrazolyl, pyridazinyl, pyridyl, pyrimidinyl, pyrrolyl, quinazolinyl, quinolinyl, quinolizinyl, quinoxalinyl, tetrahydroisoquinolinyl (including 1,2,3,4-tetrahydroisoquinolinyl and 5,6,7,8-tetrahydroisoquinolinyl), tetrahydroquinolinyl (including 1,2,3,4-tetrahydroquinolinyl and 5,6,7,8-tetrahydroquinolinyl), tetrazolyl, thiadiazolyl (including 1,2,3-thiadiazolyl, 1,2,4-thiadiazolyl and 1,3,4-thiadiazolyl), thiazolyl, thiochromanyl, thiophenetyl, thienyl, triazolyl (including 1,2,3-triazolyl, 1,2,4-triazolyl and 1,3,4-triazolyl) and the like. Substituents on heteroaryl groups may, where appropriate, be located on any atom in the ring system including a heteroatom. The point of attachment of heteroaryl groups may be *via* any atom in the ring system including (where appropriate) a heteroatom (such as a nitrogen atom), or an atom on any fused carbocyclic ring that may be present as part of the ring system. Heteroaryl groups may also be in the *N*- or S- oxidised form. Heteroaryl groups mentioned herein may be stated to be specifically monocyclic or bicyclic. When heteroaryl groups are polycyclic in which there is a non-aromatic ring present, then that non-aromatic ring may be substituted by one or more =O group.

It may be specifically stated that the heteroaryl group is monocyclic or bicyclic. In the case where it is specified that the heteroaryl is bicyclic, then it may consist of a five-, six- or seven-membered monocyclic ring (e.g. a monocyclic heteroaryl ring) fused with another a five-, six- or seven-membered ring (e.g. a monocyclic aryl or heteroaryl ring).

Heteroatoms that may be mentioned include phosphorus, silicon, boron and, preferably, oxygen, nitrogen and sulfur.

For the avoidance of doubt, where it is stated herein that a group (e.g. a C₁₋₁₂ alkyl group) may be substituted by one or more substituents (e.g. selected from E⁶), then those substituents (e.g. defined by E⁶) are independent of one another. That is, such groups may be substituted with the same substituent (e.g. defined by E⁶) or different substituents (defined by E⁶).

For the avoidance of doubt, in cases in which the identity of two or more substituents in a compound of the invention may be the same, the actual identities of the respective substituents are not in any way interdependent. For example, in the situation in which there is more than one e.g. B¹ to B⁴ or E¹ to E¹² (such as E⁶) substituent present, then those B¹ to B⁴ or E¹ to E¹² (e.g. E⁶) substituents may be the same or different. Further, in the case where there are e.g. B¹ to B⁴ or E¹ to E¹² (such as E⁶) substituents present, in which one represents -C(O)R^{10a} (or e.g. -OR²⁰, as appropriate) and the other represents -C(O)₂R^{10a} (or e.g. -C(O)₂R²⁰, as appropriate), then those R^{10a} or R²⁰ groups are not to be regarded as being interdependent. Also, when e.g. there are two -OR^{10a} substituents present, then those -OR^{10a} groups may be the same or different (i.e. each R^{10a} group may be the same or different).

For the avoidance of doubt, when a term such as "*E¹ to E¹²*" is employed herein, this will be understood by the skilled person to mean E¹, E², E³, E⁴, E⁵, E⁶, E⁷, E⁸, E⁹ (if present), E¹⁰, E¹¹ and E¹², inclusively. The term "*B¹ to B⁴*" as employed herein will be understood to mean B¹, B^{1a}, B², B^{2a}, B³, B^{3a}, B⁴ and B^{4a}, inclusively.

For the avoidance of doubt, compounds of formula I that are included within the scope of the invention include those of the following formulae: 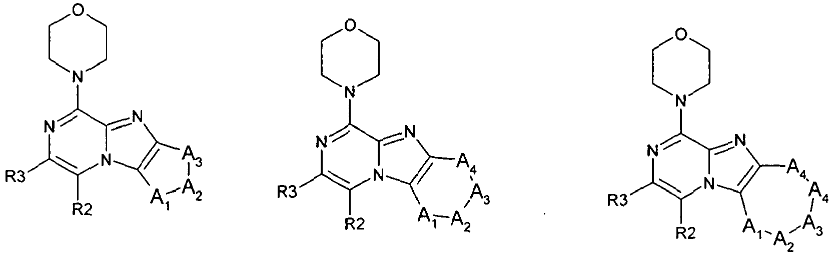 wherein the integers are as defined herein. For the avoidance of doubt the two A₄ substituents are independent of one another. Further, the morpholinyl group of the above formulae may be substituted as defined herein and the A₁ to A₄ containing ring may contain one or two double bonds, provided that the ring is not aromatic. The skilled person will appreciate that by "*A₁ to A₄-containing ring",* we mean the 5-, 6- or 7-membered ring containing the integers A₁, A₂, A₃, and optionally, one or two A₄ integers.

As stated herein, the dotted lines in the A₁ to A₄-containing ring represent the presence of an optional double bond. However, the A₁ to A₄-containing ring may not be aromatic. Hence, when the A₁ to A₄-containing ring is 5- or 6-membered (i.e. when n represents 0 or 1), then a maximum of one double bond may be present. When the A₁ to A₄-containing ring is 7-membered, then two double bonds may be present. The skilled person will also appreciate that the rules of valency should be adhered to. Hence, where for example any one of A₁ to A₄ represents e.g. -O- or -C(O)-, then a double bond may not be adjacent that A₁ to A₄ group. Similarly, a double bond may not be adjacent another double bond, etc.

All individual features (e.g. preferred features) mentioned herein may be taken in isolation or in combination with any other feature (including preferred feature) mentioned herein (hence, preferred features may be taken in conjunction with other preferred features, or independently of them).

The skilled person will appreciate that compounds of the invention that are the subject of this invention include those that are stable. That is, compounds of the invention include those that are sufficiently robust to survive isolation from e.g. a reaction mixture to a useful degree of purity.

Preferred compounds of the invention that may be mentioned include those in which:
R⁴ and R⁵ independently represent, on each occasion when used herein, hydrogen, halo, -OR^{10c}, -N(R^{10d})R^{11d}, -N(R^{10e})-C(O)-R^{10f}, -C(O)R^{10g}, -C(O)OR^{10h}, -C(O)N(R¹⁰ⁱ)R¹¹ⁱ, -N(R^{10j})-C(O)OR^{10k}, -N(R^{10m})-C(O)-N(R¹⁰ⁿ)R¹¹ⁿ, C₁₋₁₂ alkyl, heterocycloalkyl (which latter two groups are optionally substituted by one or more substituents selected from E⁵ and =O), aryl or heteroaryl (which latter two groups are optionally substituted by one or more substituents selected from E⁶ and =O); or
R⁴ and R⁵ are linked together as defined herein.

Exemplary embodiments of R³ include, but are not limited to: pyrrole, pyrazole, triazole, tetrazole, thiazole, isothiazole, oxazole, isoxazole, isoindole, 1,3-dihydro-indol-2-one, pyridine-2-one, pyridine, pyridine-3-ol, imidazole,1H-indazole, 1H-indole, indolin-2-one, 1-(indolin-1-yl)ethanone, pyrimidine, pyridazine, pyrazine and isatin groups. 1H-benzo[d][1,2,3]triazole, 1 H-pyrazolo [3,4-b]pyridine, 1H-pyrazolo[3,4-d]pyrimidine, 1 H-benzo[d]imidazole, 1 H-benzo[d]imidazol-2(3H)-one, 1 H-pyrazolo[3,4-c]pyridine, 1H-pyrazolo[4,3-d]pyrimidine, 5H-pyrrolo[3,2-d]pyrimidine, 2-amino-1H-purin-6(9H)-one, quinoline, quinazoline, quinoxaline, isoquinoline, isoquinolin-1(2H)-one, 3,4-dihydroisoquinolin-1(2H)-one, 3,4-dihydroquinolin-2(1 H)-one, quinazolin-2(1H)-one, quinoxalin-2(1H)-one, 1,8-napthyridine, pyrido[3,4-d]pyrimidine, and pyrido[3,2-b]pyrazine, 1,3-dihydro benzimidazolone, benzimidazole, benzothiazole and benzothiadiazole, groups. These groups may be unsubstituted or substituted.

Preferred compounds of the invention include those in which:
R³ represents optionally substituted aryl, monocyclic 5- or 6-membered heteroaryl or bicyclic 8-, 9- or 10-membered heteroaryl;
when R³ represents aryl (e.g. phenyl), then that group may be unsubstituted but is preferably substituted by at least one (e.g. two or, preferably, one) substituent(s) selected from E⁴;
when R³ represents monocyclic heteroaryl (e.g. a 5- or 6-membered heteroaryl group), then that group preferably contains 1, 2, 3 or 4 nitrogen atoms and, optionally 1 or 2 additional heteroatoms selected from oxygen and sulfur, and which heteroaryl group is optionally substituted by one or more substituents selected from E⁴;
when R³ represents bicyclic heteroaryl (e.g. a 8-, 9- or 10-membered heteroaryl group), then that group preferably consists of a 5- or 6-membered ring fused to another 5- or 6-membered ring (in which either one of those rings may contain one or more (e.g. four, or, preferably one to three) heteroatoms), in which the total number of heteroatoms is preferably one to four, and which ring is optionally substituted by one or more (e.g. two or, preferably, one) substituent(s) selected from E⁴ (and, if there is a non-aromatic ring present in the bicyclic heteroaryl group, then such a group may also be substituted by one or more (e.g. one) =O groups);
optional substituents (e.g. the first optional substituent) on the R³ group (e.g. when it represents aryl, such as phenyl) are preferably selected from -OR, -SR, -CH₂OR, CO₂R, CF₂OH, CH(CF₃)OH, C(CF₃)₂OH, -(CH₂)_{w}OR, -(CH₂)_{w}NR₂, -C(O)N(R)₂, -NR₂, -NRC(O)R, -NRC(O)NHR, -NRC(O)N(R)₂, -S(O)_{y}N(R)₂, -OC(O)R, OC(O)N(R)₂, -NRS(O)_{y}R, -NRC(O)N(R)₂, CN, halogen and -NO₂ (in which each R is independently selected from H, C₁-C₆ alkyl, C₃-C₁₀ cycloalkyl and a 5- to 12-membered aryl or heteroaryl group, the groups being unsubstituted or substituted (for example by one or more substituents as defined herein, e.g.
substituents on E⁴ moieties, e.g. =O, J², J³, J⁴ and/or J⁵), w is 0, 1 or 2 and y is 1 or 2);
when R³ represents aryl (e.g. phenyl), then that group is substituted by one or two substituents (e.g. by a first substituent as defined above, and, optionally a further substituent (or a further two substituents) preferably selected from halo, C₁₋₁₂ alkyl, CN, NO₂, OR^{d}, SR^{d}, NR^{d}₂, C(O)R^{d}, SOR^{d}, SO₂R^{d}, SO₂N(R)^{d}₂, NC(O)R^{d} and CO₂R^{d} (wherein each R^{d} is independently H or C₁-C₆ alkyl));
when R³ represents substituted aryl (e.g. phenyl), the the substituent may be situated at the 2-, 3-, 4-, 5- or 6- position of the phenyl ring (typically it is situated at position 3 or 4; particularly preferred are phenyl groups substituted by -OR^{d} (in which R^{d} is independently H or C₁-C₆ alkyl, e.g. methyl), e.g. -OH; in this embodiment the -OR^{d} group, or -OH group, is typically situated at the 3- or 4-position of the phenyl ring, so forming a 3-hydroxyphenyl or 4-hydroxyphenyl group or an isostere thereof, which is unsubstituted or substituted; an isostere as used herein is a functional group which possesses binding properties which are the same as, or similar to, the 3-hydroxyphenyl or 4- hydroxyphenyl group in the context of the compounds of the ivention; isosteres of 3-hydroxyphenyl and 4-hydroxyphenyl groups are encompassed within definitions (b) above for R⁵);
when R³ represents heteroaryl, it is unsubstituted or substituted (when substituted, it may be substituted by one or more substitutents selected from those listed in respect of substituents on R³, when R³ is a phenyl group; typically, the substituents are selected from OH and NH₂).

Further preferred compounds of the invention include those in which:
each R^{10a}, R^{11a}, R^{10b}, R^{11b}, R^{10c}, R^{10d}, R^{11d}, R^{10e}, R^{10f}, R^{10g}, R^{10h}, R¹⁰ⁱ, R¹¹ⁱ, R^{10j}, R^{10k}, R^{10m}, R¹⁰ⁿ, R¹¹ⁿ, R^{10p}, R^{10q}, R^{10r}, R^{10s}, R^{10t}, R^{11t}, R^{10u} and R^{10x} independently represent, on each occasion when used herein, hydrogen or C₁₋₁₂ (e.g. C₁₋₆) alkyl (which latter group is optionally substituted by one or more substituents selected from =O and E¹⁰); or
any relevant pair of R^{10a} and R^{11a} and/or any pair of R^{10b} and R^{11b}, R^{10d} and R^{11d}, R¹⁰ⁱ and R¹¹ⁱ, R¹⁰ⁿ and R¹¹ⁿ, and R^{10t} and R^{11t} may, when attached to the same nitrogen atom, be linked together to form (along with the requisite nitrogen atom to which they are attached) a 3- to 12- (e.g. 4- to 12-) membered ring, optionally containing one or more (e.g. one to three) double bonds, and which ring is optionally substituted by one or more substituents selected from E¹² and =O;
T¹ and T² independently represent a single bond;
each of E¹, E², E³, E⁴, E⁵, E⁶, E⁷, E⁸, E¹⁰, E¹¹ and E¹² independently represents, on each occasion when used herein, Q⁴ or C₁₋₁₆ alkyl (e.g. C₁₋₆, such as C₁₋₃) alkyl optionally substituted by one or more substituents selected from =O and Q⁵;
each Q⁴ and Q⁵ independently represent halo, -CN, -NO₂, -N(R²⁰)R²¹, -OR²⁰, -C(=Y)-R²⁰, -C(=Y)-OR²⁰, -C(=Y)N(R²⁰)R²¹, -N(R²²)C(=Y)R²¹, -N(R²²)C(=Y)OR²¹, -N(R²²)C(=Y)N(R²⁰)R²¹, -NR²²S(O)₂R²⁰, -NR²²S(O)₂N(R²⁰)R²¹, -S(O)₂N(R²⁰)R²¹, -S(O)₂R²⁰, -SR²⁰, -S(O)R²⁰ or C₁₋₆ alkyl optionally substituted by one or more fluoro atoms (and each Q⁵ more preferably represents halo, such as fluoro);
any two E¹, E², E³, E⁴, E⁵, E⁶, E⁷, E⁸, E¹⁰, E¹¹ or E¹² groups may be linked together, but are preferably not linked together;
each R²⁰, R²¹, R²² and R²³ independently represent, on each occasion when used herein, aryl (e.g. phenyl; preferably unsubstituted, but which may be substituted by one to three J⁵ groups) or, more preferably, hydrogen or C₁₋₆ (e.g. C₁₋₃) alkyl optionally substituted by one or more substituents selected from =O and J⁴; or
any pair of R²⁰ and R²¹, may, when attached to the same nitrogen atom, be linked together to form a 4- to 8-membered (e.g. 5- or 6-membered) ring, optionally containing one further heteroatom selected from nitrogen and oxygen, optionally containing one double bond, and which ring is optionally substituted by one or more substituents selected from J⁶ and =O;
each J¹, J², J³, J⁴, J⁵ and J⁶ independently represent C₁₋₆ alkyl (e.g. acyclic C₁₋₃ alkyl or, e.g. in the case of J⁴, C₃₋₅ cycloalkyl) optionally substituted by one or
more substituents selected from =O and Q⁸, or, more preferably, such groups independently represent a substituent selected from Q⁷;
each Q⁷ and Q⁸ independently represents a substituent selected from fluoro, -N(R⁵⁰)R⁵¹, -OR⁵⁰, -C(=Y^{a})-R⁵⁰, -C(=Y^{a})-OR⁵⁰, -C(=Y^{a})N(R⁵⁰)R⁵¹, -NR⁵²S(O)₂R⁵⁰, -S(O)₂R⁵⁰ or C₁₋₆ alkyl optionally substituted by one or more fluoro atoms;
each R⁵⁰, R⁵¹, R⁵² and R⁵³ substituent independently represents, on each occasion when used herein, hydrogen or C₁₋₆ (e.g. C₁₋₃) alkyl optionally substituted by one or more substituents selected from fluoro;
when any relevant pair of R⁵⁰, R⁵¹ and R⁵² are linked together, then those pairs that are attached to the same nitrogen atom may be linked together (i.e. any pair of R⁵⁰ and R⁵¹), and the ring so formed is preferably a 5- or 6-membered ring, optionally containing one further nitrogen or oxygen heteroatom, and which ring is optionally substituted by one or more substituents selected from =O and C₁₋₃ alkyl (e.g. methyl);
R⁶⁰, R⁶¹ and R⁶² independently represent hydrogen or C₁₋₃ (e.g. C₁₋₂) alkyl optionally substituted by one or more fluoro atoms.

Preferred optional substituents on R³ and the A₁ to A₄-containing ring (and, when they represent a substituent other than hydrogen on R⁴, R⁵ and R⁶ groups) include:
=O (e.g. in the case of alkyl, cycloalkyl or heterocycloalkyl groups);
-CN;
halo (e.g. fluoro, chloro or bromo);
C₁₋₄ alkyl, which alkyl group may be cyclic, part-cyclic, unsaturated or, preferably, linear or branched (e.g. C₁₋₄ alkyl (such as ethyl, n-propyl, isopropyl, *t*-butyl or, preferably, *n*-butyl or methyl), all of which are optionally substituted with one or more halo (e.g. fluoro) groups (so forming, for example, fluoromethyl, difluoromethyl or, preferably, trifluoromethyl) or substituted with an aryl, heteroaryl or heterocycloalkyl group (which themselves may be substituted with one or more -OR^{z1}, -C(O)R^{z2}, -C(O)OR^{z3}, -N(R^{z4})R^{z5}, -S(O)₂R^{z6}, -S(O)₂N(R^{z7})R^{z8};
-N(R^{z9})-C(O)-R^{z10}, -C(O)-N(R^{z11})R^{z12} and/or -N(R^{z9})-C(O)-N(R^{z10}) substituents; aryl (e.g. phenyl), if appropriate (e.g. when the substitutent is on an alkyl group, thereby forming e.g. a benzyl group);
-OR^{z1};
-C(O)R^{z2};
-C(O)OR^{z3};
-N(R^{z4})R^{z5};
-S(O)₂R^{z6};
-S(O)₂N(R^{z7})R^{z8};
-N(R^{z9})-C(O)-R^{z10};
-C(O)-N(R^{z11})R^{z12};
-N(R^{z9})-C(O)-N(R^{z10});
wherein each R^{z1} to R^{z12} independently represents, on each occasion when used herein, H or C₁₋₄ alkyl (e.g. ethyl, n-propyl, *t*-butyl or, preferably, n-butyl, methyl, isopropyl or cyclopropylmethyl (i.e. a part cyclic alkyl group)) optionally substituted by one or more halo (e.g. fluoro) groups (so forming e.g. a trifluoromethyl group). Further, any two R^{z} groups (e.g. R^{z4} and R^{z5}), when attached to the same nitrogen heteroatom may also be linked together to form a ring such as one hereinbefore defined in respect of corresponding linkage of R¹⁰ and R¹¹ or R^{10a} and R^{11a} groups.

Preferred compounds of the invention include those in which:
R² represents hydrogen or a substituent selected from -N(R^{10b})R^{11b} and, preferably, halo (e.g. chloro, bromo or iodo) and -CN;
B¹, B^{1a}, B², B^{2a}, B³, B^{3a}, B⁴ and B^{4a} independently represent hydrogen, C₁₋₆ (e.g. C₁₋₃) alkyl optionally substituted by one or more substituents selected from =O and E¹, any two of these together form a =O substituent on the morpholinyl ring, or, any two B¹, B^{1a}, B², B^{2a}, B³, B^{3a}, B⁴ and B^{4a} substituents when linked together, may form a linkage, for example between a B² or B^{2a} substituent and a B³ or B^{3a} substituent for a further ring, e.g. a five membered ring such as the one depicted below: 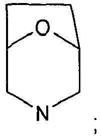
each E¹, E², E³, E⁴, E⁵, E⁶, E⁷, E⁸, E¹⁰, E¹¹ and E¹² independently represents C₁₋₁₂ alkyl optionally substituted by one or more substituents selected from =O and Q⁵, or, preferably (each E¹ to E¹² independently represent) Q⁴;
each R²⁰, R²¹, R²² and R²³ (e.g. each R²⁰ and R²¹) independently represents heteroaryl, preferably, aryl (e.g. phenyl) (which latter two groups are optionally substituted by one or more substituents selected from J⁵), or, more preferably, hydrogen or C₁₋₆ (e.g. C₁₋₄) alkyl optionally substituted by one or more substituents selected from =O and J⁴; or
any relevant pair of R²⁰, R²¹ and R²² (e.g. R²⁰ and R²¹) may (e.g. when both are attached to the same nitrogen atom) may be linked together to form a 3- to 8-(e.g. 4- to 8-) membered ring, optionally containing a further heteroatom, and optionally substituted by one or more substituents selected from =O and J⁶;
each J¹, J², J³, J⁴, J⁵ and J⁶ independently represent C₁₋₆ alkyl (e.g. C₁₋₃ acyclic alkyl or C₃₋₅ cycloalkyl) optionally substituted by one or more substituents selected from Q⁸, or, J¹ to J⁶ more preferably represent a substituent selected from Q⁷ ;
each Q⁷ and Q⁸ independently represent halo, -N(R⁵⁰)R⁵⁰, -OR⁵⁰, -C(=Y^{a})-OR⁵⁰, -C(=Y^{a})-R⁵⁰, -S(O)₂R⁵⁰ or C₁₋₃ alkyl optionally substituted by one or more fluoro atoms;
each R⁵⁰, R⁵¹, R⁵² and R⁵³ independently represents hydrogen or C₁₋₆ (e.g. C₁₋₄) alkyl optionally substituted by one or more fluoro atoms;
each R⁶⁰, R⁶¹ and R⁶² independently represents hydrogen or C₁₋₂ alkyl (e.g. methyl).

More preferred compounds of the invention include those in which:
R² represents hydrogen, chloro, bromo, iodo or -CN;
each R^{10a}, R^{11a} R^{10b}, R^{11b}, R^{10c}, R^{10d}, R^{11d}, R^{10e}, R^{10f}, R^{10g}, R^{10h}, R¹⁰ⁱ, R¹¹ⁱ, R^{10j}, R^{10k}, R^{10m}, R¹⁰ⁿ, R¹¹ⁿ, R^{10p}, R^{10q}, R^{10r}, R^{10s}, R^{10t}, R^{11t}, R^{10u} and R^{10x} independently represents hydrogen or C₁₋₄ (e.g. C₁₋₃) alkyl (e.g. ethyl) (however, R^{10u} is preferably not hydrogen), and which alkyl group may by substituted by one or more substituents selected from =O and E¹⁰ (but which alkyl group is more preferably unsubstituted); or
any relevant pair of R^{10a} and R^{11a} and/or any pair of R^{10b} and R^{11b}, R^{10d} and R^{11d}, R¹⁰ⁱ and R¹¹ⁱ, R¹⁰ⁿ and R¹¹ⁿ, and R^{10t} and R^{11t} may be linked together to form a 5-or, preferably, a 6-membered ring, optionally containing a further heteroatom (preferably selected from nitrogen and oxygen), which ring is preferably saturated (so forming, for example, a piperazinyl or morpholinyl group), and optionally substituted by one or more substituents selected from =O and E¹² (which E¹² substituent may be situated on a nitrogen heteroatom; and/or E¹² is preferably halo (e.g. fluoro) or C₁₋₃ alkyl optionally substituted by one or more fluoro atoms); R^{10k,} R^{10p}, R^{10q}, R^{10s} and R^{10u} independently represent C₁₋₁₂ alkyl optionally substituted by one or more substituents selected from E¹⁰;
each E¹, E², E³, E⁴, E⁵, E⁶, E⁷, E⁸, E¹⁰, E¹¹ and E¹² independently represents a substituent selected from Q⁴, or (e.g.) E⁴ may represent C₁₋₄ alkyl optionally substituted by one or more Q⁵ substituents;
Q⁴ and Q⁵ independently represent -N(R²²)-C(=Y)-N(R²⁰)R²¹ or, preferably, halo (e.g. fluoro), -OR²⁰, -N(R²⁰)R²¹, -C(=Y)OR²⁰, -C(=Y)N(R²⁰)R²¹, -NR²²S(O)₂R²⁰, heterocycloalkyl, aryl, heteroaryl (which latter three groups are optionally substituted with one or more substitutents selected from J² or J³, as appropriate) and/or C₁₋₆ alkyl (e.g. C₁₋₃ alkyl) optionally substituted by one or more fluoro atoms;
each Y represents, on each occasion when used herein, =S, or preferably =O; each R²⁰, R²¹, R²² and R²³ (e.g. each R²⁰ and R²¹) independently represents hydrogen or C₁₋₄ (e.g. C₁₋₃) alkyl (e.g. *tert*-butyl, ethyl, methyl or a part cyclic group such as cyclopropylmethyl) optionally substituted (but preferably unsubstituted) by one or more (e.g. one) J⁴ substituent(s); or
any relevant pair of R²⁰, R²¹ and R²² (e.g. R²⁰ and R²¹) may (e.g. when both are attached to the same nitrogen atom) be linked together to form a 5- or, preferably, a 6-membered ring, optionally containing a further heteroatom (preferably selected from nitrogen and oxygen), which ring is preferably saturated (so forming, for example, a piperazinyl or morpholinyl group), and optionally substituted by one or more substituents selected from =O and J⁶ (which J⁶ substituent may be situated on a nitrogen heteroatom);
R²² represents C₁₋₃ alkyl or, preferably, hydrogen;
each J¹, J², J³, J⁴, J⁵ and J⁶ independently represent a substituent selected from Q⁷, or J¹ to J⁶ (e.g. J⁴) represents C₁₋₆ alkyl (e.g. C₃₋₅ cycloalkyl);
each Q⁷ and Q⁸ independently represent -C(=Y^{a})-OR⁵⁰, -C(=Y^{a})-R⁵⁰, -S(O)₂R⁵⁰ or C₁₋₃ alkyl optionally substituted by one or more fluoro atoms;
each Y^{a} independently represents =S or, preferably, =O;
each R⁵⁰ independently represents C₁₋₄ alkyl (e.g. *tert*-butyl or methyl).

Preferred A₁ to A₄-containing rings of the compounds of the invention include those of the following formulae: 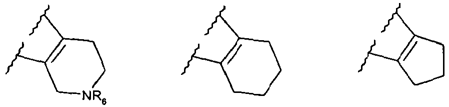 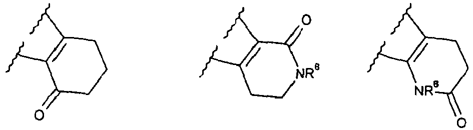 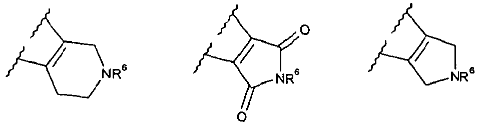 wherein, the carbon atoms may be unsubstituted or substituted by a substituent defined by R⁴ or R⁵, and R⁶ is as hereinbefore defined.

Preferred R³ groups of the compounds of the compounds of the invention include optionally substituted phenyl, indazolyl (e.g. 4-indazolyl), pyrimidinyl (e.g. 5-pyrimidinyl), azaindolyl (e.g. azaindol-5-yl), indolyl (e.g. 5-indolyl or 4-indolyl) and pyridyl (e.g. 3-pyridyl). Particularly preferred are optionally substituted pyrimidinyl (e.g. 5-pyrimidinyl, such as 2-NH₂-pyrimidin-5-yl).

More preferred compounds of the invention include those in which:
each R⁴ and R⁵ independently represent hydrogen, C₁₋₆ alkyl (optionally substituted as defined herein; but preferably unsubstituted), -OR^{10c}, -C(O)OR^{10h} or -C(O)N(R¹⁰ⁱ)R¹¹ⁱ (e.g. in which one of R¹⁰ⁱ and R¹¹ⁱ is hydrogen and the other is as herein defined);
each R⁶ (when/if present) independently represents hydrogen, -C(O)R^{10r}, -C(O)OR^{10s}, -C(O)N(R^{10t})R^{11t}, -S(O)₂R^{10u}, C₁₋₆ alkyl (optionally substituted by one or more (e.g. two or, preferably, one) substituents selected from E⁷), heterocycloalkyl (e.g. a 5- or 6-membered group preferably containing one or two heteroatoms; which is optionally substituted by one or more e.g. one, E⁷ substituent(s)) or aryl (e.g. phenyl; optionally substituted by one or more substituents selected from E⁸);
each R^{10a}, R^{11a}, R^{10b}, R^{11b}, R^{10c}, R^{10d}, R^{11d}, R^{10e}, R^{10f}, R^{10g}, R^{10h}, R¹⁰ⁱ, R¹¹ⁱ, R^{10j}, R^{10k}, R^{10m}, R¹⁰ⁿ, R¹¹ⁿ,R^{10p}, R^{10q}, R^{10r}, R^{10s}, R^{10t}, R^{11t}, R^{10u} and R^{10x} independently represent, on each occasion when used herein, hydrogen, C₁₋₆ alkyl (optionally substituted by one or more (e.g. one or two) substituents selected from E¹⁰), heterocycloalkyl (e.g. a 4- to 6-membered ring; which heterocycloalkyl group is optionally substituted by one or more (e.g. one or two) substituents selected from E¹⁰), aryl (optionally substituted by one or more (e.g. one or two) substituents selected from E¹¹) or heteroaryl (e.g. a 5- or preferably 6-membered group preferably containing two or one heteroatoms; which heteroaryl group is optionally substituted by one or more (e.g. one or two) substituents selected from E¹¹);
each E¹, E², E³, E⁴, E⁵, E⁶, E⁷, E⁸, E¹⁰, E¹¹ and E¹² independently represents a substituent selected from Q⁴ or C₁₋₆ alkyl optionally substituted by one or more Q⁵ substituents;
Q⁴ represents halo (e.g. chloro or fluoro), -CN, -OR²⁰, -N(R²⁰)R²¹, -C(=Y)OR²⁰, -C(=Y)-R²⁰, N(R²²)-S(O)₂R²⁰, -N(R²²)-C(=Y)-N(R²⁰)R²¹, -S(O)₂R²⁰, heterocycloalkyl (e.g. a 4- to 6-membered ring, containing preferably one heteroatom selected from nitrogen and oxygen; optionally substituted with two or, preferably, one substituent selected from J²), aryl (e.g. phenyl; optionally substituted with two or, preferably, one substituent selected from J³) or heteroaryl (e.g. a 5- or 6-membered monocyclic heteroaryl group preferably containing one or two heteroatoms preferably selected from nitrogen, oxygen and sulfur; which group may be substituted by one or more substituents selected from J³, but is preferably unsubstituted);
Q⁵ represents halo (e.g. fluoro);
Y represents =O;
R²⁰, R²¹, R²² and R²³ independently represent hydrogen or C₁₋₆ (e.g. C₁₋₄) alkyl optionally substituted by one or more (e.g. one) substituent(s) selected from J⁴; each J¹, J², J³, J⁴, J⁵ and J⁶ independently represent Q⁷ or C₁₋₆ alkyl optionally substituted by one or more Q⁸ groups;
Q⁷ represents halo (e.g. fluoro or chloro), -OR⁵⁰, -N(R⁵⁰)R⁵¹, -C(=Y^{a})-OR⁵⁰ or -S(O)₂R⁵⁰;
Q⁸ represents halo (e.g. fluoro);
Y^{a} represents =O;
R⁵⁰ represents hydrogen or C₁₋₆ (e.g. C₁₋₄) alkyl optionally substituted by one or more fluoro atoms.

Preferred compounds of the invention include those in which:
R² represents hydrogen or halo (e.g. chloro);
R³ represents aryl (e.g. phenyl) or heteroaryl (e.g. a 5- or 6-membered monocyclic heteroaryl group or a 9- or 10-membered bicyclic heteroaryl group; which groups may contain one to four, e.g 3 or, preferably, 1 or 2, heteroatoms preferably selected from nitrogen, oxygen and sulfur) both of which are optionally substituted by one or more (e.g. two, or, preferably, one) substituent(s) selected from E⁴ (e.g. halo, -N(H)-C(O)-N(H)-CH₃ and, preferably, -CF₃, -OH, -OCH₃ and/or -N(R²⁰)R²¹ (e.g. -NH₂ or -N(H)-CH₂-cyclopropyl));
each R⁴ and R⁵ independently represent -C(O)N(R¹⁰ⁱ)R¹¹ⁱ (e.g. in which one of R¹⁰ⁱ and R¹¹ⁱ is hydrogen and the other is as herein defined), or each R⁴ and R⁵ preferably (and independently) represent hydrogen, C₁₋₆ alkyl (optionally substituted as defined herein; but preferably unsubstituted), -OR^{10c} or -C(O)OR^{10h};
R⁴ and R⁵ may be linked, but are more preferably not linked together;
each R⁶ (when/if present) independently represents heterocycloalkyl (e.g. a 5- or 6-membered group preferably containing one or two heteroatoms, e.g. a pyrrolidinyl group, optionally substituted by one or more e.g. one, E⁷ substituent(s)), aryl (e.g. phenyl; optionally substituted by one or more substituents selected from E⁸) or, preferably, hydrogen, -C(O)R^{10r}, -C(O)OR^{10s}, -C(O)N(R^{10t})R^{11t}, -S(O)₂R^{10u} or C₁₋₆ alkyl (e.g. C₁₋₄ alkyl, such as isopropyl, ethyl or preferably methyl or butyl (e.g. s-butyl) or C₅₋₆ cycloalkyl, e.g. cyclohexyl) optionally substituted by one or more (e.g. two or, preferably, one) E⁷ substituents;
R^{10c} represents hydrogen;
R^{10r} represents aryl (optionally substituted by one or more substituents selected from E¹¹, e.g. R^{10r} represents phenyl or fluorophenyl), heteroaryl (e.g. a 5- or preferably 6-membered group preferably containing two or one heteroatoms, so forming e.g. a pyridyl group such as 3-pyridyl) or, preferably, C₁₋₃ alkyl (e.g. cyclopropyl or, preferably, ethyl or propyl, such as n-propyl or preferably isopropyl), which alkyl group may be substituted by one or more substituents selected from E¹⁰;
R^{10s} represents C₁₋₃ alkyl (e.g. methyl or, preferably, ethyl);
R^{10t} represents hydrogen;
R^{10u} represents aryl (optionally substituted by one or more substituents selected from E¹¹, e.g. R^{10u} represents phenyl, fluorophenyl or difluorophenyl), heterocycloalkyl (e.g. a 4- to 6-membered ring, in which the heteroatoms are preferably selected from nitrogen and oxygen, so forming e.g. tetrahydropyranyl or azetidinyl) or, preferably, C₁₋₆ alkyl (e.g. C₅₋₆ cycloalkyl or C₁₋₄, such as C₁₋₂, alkyl) (e.g. butyl (such as n-butyl, s-butyl or isobutyl), cyclopropyl, acyclic propyl (e.g. isopropyl or n-propyl), ethyl or, preferably, methyl), which C₁₋₆ alkyl group is preferably unsubstituted but may be substituted by one or more (e.g. one) substituent(s) selected from E¹⁰;
R^{10t} represents hydrogen or C₁₋₃ alkyl (e.g. ethyl);
R^{11t} represents aryl (optionally substituted by one or more substituents selected from E¹¹) or, preferably, C₁₋₆ (e.g. C₁₋₃) alkyl (e.g. C₅₋₆ cycloalkyl, methyl or preferably ethyl or propyl, such as n-propyl or, preferably, isopropyl) which group is preferably unsubstituted but which may be substituted by one or more (e.g. one) substituent selected from E¹⁰;
E⁴ represents Q⁴ (e.g. halo, -N(R²²)-C(=Y)-N(R²⁰)R²¹ or, preferably, -OR²⁰ and/or -N(R²⁰)R²¹) or C₁₋₆ (e.g. C₁₋₃, such as methyl) alkyl optionally substituted by one or more Q⁵ substituents (e.g. fluoro, so forming for example a trifluoromethyl group);
E⁷ represents C₁₋₆ (e.g. C₃₋₆) alkyl (such as C₃₋₆ cycloalkyl, e.g cyclohexyl, cyclopentyl or preferably cyclopropyl) or Q⁴ (e.g. -OR²⁰, -N(R²⁰)R²¹, -C(O)OR²⁰, -S(O)₂R²⁰, -N(R²²)-S(O)₂R²⁰, aryl, such as phenyl, or heteroaryl, such as a 5- or 6-membered heteroaryl group preferably containing one or two heteroatoms so forming e.g. thiazolyl, pyridazine or pyridyl, both of which aryl/heteroaryl groups are optionally substituted by one or more substituents selected from J³, or (Q⁴ may represent), heterocycloalkyl (e.g. piperidinyl) optionally substituted by one or more substituents selected from J²) (when E⁷ represents Q⁴, then Q⁴ more particularly represents -C(=Y)OR²⁰, heterocycloalkyl, aryl or heteroaryl);
E⁸ represents Q⁴ (in which Q⁴ is preferably halo, such as fluoro);
E¹⁰ represents Q⁴ (in which instance, Q⁴ is preferably fluoro, -CN, -OR²⁰, -C(=Y)OR²⁰ or phenyl optionally substituted with two or, preferably, one substituent(s) selected from J³);
E¹¹ represents C₁₋₄ (e.g. C₁₋₂) alkyl (optionally substituted by one or more Q⁵ groups, e.g. fluoro atoms, so forming e.g. -CF₃) or E¹¹ represents Q⁴, in which Q⁴ preferably represents halo (e.g. chloro or fluoro), -CN, -OR²⁰, -N(R²⁰)R²¹ or -C(=Y)-R²⁰;
Q⁴ represents halo (e.g. chloro or fluoro), -CN, -OR²⁰, -N(R²⁰)R²¹, -C(=Y)OR²⁰, -C(=Y)-R²⁰, N(R²²)-S(O)₂R²⁰, -N(R²²)-C(=Y)-N(R²⁰)R²¹, -S(O)₂R²⁰, heterocycloalkyl (e.g. a 4- to 6-membered ring, containing preferably one heteroatom selected from nitrogen and oxygen; optionally substituted with two or, preferably, one substituent selected from J²), aryl (e.g. phenyl; optionally substituted with two or, preferably, one substituent selected from J³) or heteroaryl (e.g. a 5- or 6-membered monocyclic heteroaryl group preferably containing one or two heteroatoms preferably selected from nitrogen, oxygen and sulfur; which group may be substituted by one or more substituents selected from J³, but is preferably unsubstituted);
Q⁵ represents halo (e.g. fluoro);
Y represents =O;
R²⁰ and R²¹ independently represent hydrogen, C₁₋₄ (e.g. C₁₋₃) alkyl (e.g. *tert-*butyl, cyclopropylmethyl, or preferably methyl or ethyl), which latter group is optionally substituted by one or more (e.g. one) substituent(s) selected from J⁴; R²² represents hydrogen;
when there is a -N(R²⁰)R²¹ moiety present, then one of R²⁰ and R²¹ represents hydrogen, and the other represents hydrogen, C₁₋₄ alkyl (e.g. cyclopropylmethyl, methyl or ethyl), which latter group is optionally substituted by one or more (e.g. one) substituent(s) selected from J⁴;
J² represents Q⁷ (in which Q⁷ is preferably -C(=Y^{a})-OR⁵⁰ or -S(O)₂R⁵⁰);
J³ represents C₁₋₃ (e.g. C₁₋₂) alkyl (e.g. methyl; which alkyl group is optionally substituted by one or more Q⁸ groups, e.g. fluoro atoms so forming e.g. a -CF₃ group) or Q⁷;
J⁴ represents C₁₋₆ alkyl, such as C₃₋₆ alkyl (especially C₃₋₆ cycloalkyl, such as cyclopropyl);
Q⁷ represents halo (e.g. fluoro or chloro), -OR⁵⁰, -C(=Y^{a})-OR⁵⁰ or -S(O)₂R⁵⁰;
Q⁸ represents halo (e.g. fluoro);
Y^{a} represents =O;
R⁵⁰ represents hydrogen or preferably C₁₋₄ (e.g. C₁₋₃) alkyl (e.g. *tert*-butyl or preferably methyl) optionally substituted by one or more fluoro atoms (so forming e.g. a -CF₃ moiety).

Particularly preferred compounds of the invention include those in which:
R² represents hydrogen or chloro;
R³ represents phenyl (e.g. urea-phenyl, (4-N(H)-C(O)-N(H)-CH₃)-phenyl) or, preferably, hydroxyphenyl (e.g. 3-hydroxyphenyl) or methoxyphenyl (e.g. 3-methoxyphenyl), indazolyl (e.g. 4-indazolyl), pyrimidinyl (e.g. 5-pyrimidinyl, such as 2-amino-5-pyrimidinyl (i.e. 2-[-N(R²⁰)(R²¹)]-pyrimidin-5-yl such as 2-NH₂-pyrimidin-5-yl or 2-[N(H)(CH₂-cyclopropyl]-pyrimidin-5-yl) or 2-methoxy-5-pyrimidinyl), azaindolyl (e.g. 7-azaindol-5-yl), indolyl (e.g. 5-indolyl or 4-indolyl, such as 5-fluoro-4-indolyl), pyridyl (e.g. 3-pyridyl, such as 6-NH₂-pyrid-3-yl, 5-OCH₃-pyrid-3-yl or 5-CF₃,6-NH₂-pyrid-3-yl);
A₁ represents -C(R⁴)R⁵-, -C(O)- or -N(R⁶)-;
A₂ represents -N(R⁶)-, -C(R⁴)R⁵- or -C(O)-;
A₃ represents -C(R⁴)R⁵-, -N(R⁶)- or -C(O)-;
one of A₂ and A₃ represents -C(R⁴)R⁵- and the other represents -C(R⁴)R⁵- or -N(R⁶)-;
n represents 0 or 1;
A₄ represents -C(R⁴)R⁵- or -C(O)-;
A² may represent -C(O)- when A¹ or A³ (e.g. A¹) represents -N(R⁶)-;
A³ may represent -C(O)- when e.g. n represents 0 and/or A² represents -N(R⁶)-; only a maximum of two of A₁, A₂, A₃ and, if present, A₄, represents -C(O)- (which, if there are two -C(O)- moieties, are preferably not adjacent to one another);
the dotted lines do not represent the presence of an optional double bond (i.e. the A₁ to A₄-containing ring does not contain a double bond, other than that double bond that is integral to the requisite imidazopyrazine of formula I);
B¹, B^{1a}, B², B^{2a}, B³, B^{3a}, B⁴ and B^{4a} independently represent hydrogen;
each R⁴ and R⁵ independently represent hydrogen, C₁₋₆ alkyl (e.g. C₁₋₃ alkyl such as methyl), -OR^{10c} (e.g. -OH) or -C(O)OR^{10q} (e.g. -C(O)OCH₂CH₃);
each R⁶ (when/if present) independently represents -C(O)-OCH₃, -S(O)₂-cyclopropyl, -C(O)-phenyl (e.g. -C(O)-[4-fluorophenyl]), -S(O)₂-phenyl (e.g. unsubstituted -S(O)₂-phenyl, -S(O)₂-[fluorophenyl] or -S(O)₂-[2,4-difluorophenyl]), -C(O)N(H)-CH₂CH₂CH₃, -S(O)₂-*n*-butyl, -S(O)₂-[4-tetrahydropyranyl], -S(O)₂-isopropyl, -S(O)₂-ethyl, propyl (e.g. isopropyl), -C(O)N(H)-CH₂-[phenyl] (e.g. -C(O)N(H)-CH₂-[fluorophenyl], -C(O)N(H)-CH₂-[methylphenyl] or -C(O)N(H)-CH₂-[methoxyphenyl]), -C(O)-N(H)-cyclopentyl, -C(O)-N(H)-cyclohexyl, -C(O)-N(H)-CH₂CH₂-OCH₃, -S(O)₂-cyclopentyl, -C(O)N(H)-CH₂-C(O)-O-CH₂CH₃, -C(O)-N(H)-phenyl (e.g. -C(O)N(H)-[fluorophenyl], -C(O)N(H)-[methoxy-phenyl], -C(O)N(H)-[aminophenyl], -C(O)N(H)-[acetyl-phenyl] (e.g. -C(O)N(H)-[3-C(O)CH₃-phenyl]), -C(O)N(H)-[methylphenyl], -C(O)N(H)-[4-chloro-3-trifluoromethylphenyl], -C(O)N(H)-[dichlorophenyl] or -C(O)N(H)-[cyanophenyl]), -C(O)-pyridyl (e.g. -C(O)-[3-pyridyl]), -S(O)₂-(CH₂)₃-CN, -CH₂-cyclopentyl, -CH₂-piperidinyl (e.g. -CH₂-[(1-C(O)O-*tert*-butyl)-4-piperidinyl], -CH₂-[(1-S(O)₂CH₃)-4-piperidinyl] or unsubstituted -CH₂-piperidinyl), -CH₂-pyridazine, -S(O)₂-(CH₂)₂-OCH₃, -C(O)-n-propyl, -C(O)-(CH₂)₂-OCH₃, pyrrolidinyl (e.g. 3-pyrrolidinyl), -S(O)₂-CH₂-C(H)(CH₃)₂, -S(O)₂-azetidinyl (e.g. -S(O)₂-[3-azetidinyl]), -CH₂-CH₂-OCH₃, -CH₂-CH₂-N(CH₃)₂, -CH₂CH₂-S(O)₂CH₃, -C(O)CF₃, -C(O)-cyclopropyl, phenyl (e.g. fluorophenyl), -CH₂CH₂-phenyl (e.g. -CH₂CH₂-[fluorophenyl]), -CH₂-CH₂-N(H)S(O)₂-CH₃ or, preferably, hydrogen, -C(O)CH₂CH₃, -CH₂-[pyridyl]- (e.g. -CH₂-[5-methyl,6-fluoro-3-pyridyl], -CH₂-[2-pyridyl], -CH₂-[3-pyridyl] or -CH₂-[4-pyridyl]), -CH₂-[thiazolyl] (e.g. -CH₂-[2-thiazolyl]), -CH₂-[furanyl] (e.g. -CH₂-[2-furanyl] or -CH₂-[3-furanyl]), -CH₂-[imidazolyl] (e.g. -CH₂-[2-imidazolyl]), -CH₂-[tetrahydropyranyl] (e.g. -CH₂-[4-tetrahydropyranyl]), -CH₂-[azetidinyl] (e.g. -CH₂-[3-azetidinyl]), -CH₂-[phenyl] (e.g. -CH₂-[fluorophenyl], -CH₂-[methoxyphenyl], -CH₂-[dimethoxyphenyl], -CH₂-[trifluoromethoxyphenyl], -CH₂-[3-CF₃,4-chlorophenyl], -CH₂-[trifluoromethyl-phenyl], -CH₂-[methylsulfonyl-phenyl] or, preferably, -CH₂-[methylsulfonyl-phenyl]), -CH₂-cyclohexyl, methyl, ethyl, butyl (e.g. s-butyl), cyclohexyl (e.g. 4-C(O)OCH₂CH₃-cyclohexyl or unsubstituted cyclohexyl), -CH₂-cyclopropyl, -C(O)N(H)CH₂CH₃, -C(O)N(H)CH(CH₃)₂, -S(O)₂CH₃, -C(O)CH₃ or -C(O)-CH(CH₃)₂.

Preferred compounds of the invention include those in which:
R² represents hydrogen or halo (e.g. chloro);
R³ represents aryl (e.g. phenyl) or heteroaryl (e.g. a 5- or 6-membered monocyclic heteroaryl group or a 9- or 10-membered bicyclic heteroaryl group; which groups may contain one to four, e.g 3 or, preferably, 1 or 2, heteroatoms preferably selected from nitrogen, oxygen and sulfur) both of which are optionally substituted by one or more (e.g. two, or, preferably, one) substituent(s) selected from E⁴ (e.g. -CF₃, -OH, -OCH₃ and/or -N(R²⁰)R²¹ (e.g. -NH₂ or -N(H)-CH₂-cyclopropyl));
each R⁴ and R⁵ independently represent -C(O)N(R¹⁰ⁱ)R¹¹ⁱ (e.g. in which one of R¹⁰ⁱ and R¹¹ⁱ is hydrogen and the other is as herein defined), or each R⁴ and R⁵ preferably (and independently) represent hydrogen, C₁₋₆ alkyl (optionally substituted as defined herein; but preferably unsubstituted), -OR^{10c} or -C(O)OR^{10h};
R⁴ and R⁵ may be linked, but are more preferably not linked together;
each R⁶ (when/if present) independently represents hydrogen, -C(O)R^{10r}, -C(O)OR^{10s}, -C(O)N(R^{10t})R^{11t}, -S(O)₂R^{10u} or C₁₋₆ (e.g. C₁₋₄, such as methyl or butyl (e.g. s-butyl) or C₅₋₆ cycloalkyl, e.g. cyclohexyl) alkyl optionally substituted by one or more (e.g. two or, preferably, one) E⁷ substituents;
R^{10c} represents hydrogen;
R^{10r} represents C₁₋₃ alkyl (e.g. ethyl or propyl, such as isopropyl);
R^{10s} represents C₁₋₃ alkyl (e.g. ethyl);
R^{10t} represents hydrogen;
R^{10u} represents C₁₋₃ (e.g. C₁₋₂) alkyl (e.g. methyl);
R^{10t} represents C₁₋₃ alkyl (e.g. ethyl);
R^{11t} represents C₁₋₃ alkyl (e.g. ethyl or propyl, such as isopropyl);
E⁴ represents Q⁴ (e.g. -OR²⁰ and/or -N(R²⁰)R²¹) or C₁₋₆ (e.g. C₁₋₃, such as methyl) alkyl optionally substituted by one or more Q⁵ substituents (e.g. fluoro, so forming for example a trifluoromethyl group);
E⁷ represents C₁₋₆ (e.g. C₃₋₆) alkyl (such as C₃₋₆ cycloalkyl, e.g cyclopropyl) or Q⁴ (e.g. -C(=Y)OR²⁰, heterocycloalkyl, aryl or heteroaryl);
Q⁴ represents -OR²⁰, -N(R²⁰)R²¹, -C(=Y)OR²⁰, heterocycloalkyl (e.g. a 4- to 6-membered ring, containing preferably one heteroatom selected from nitrogen and oxygen), aryl (e.g. phenyl; optionally substituted with two or, preferably, one substituent selected from J³) or heteroaryl (e.g. a 5- or 6-membered monocyclic heteroaryl group preferably containing one or two heteroatoms preferably selected from nitrogen, oxygen and sulfur; which group may be substituted, but is preferably unsubstituted);
Q⁵ represents halo (e.g. fluoro);
Y represents =O;
R²⁰ and R²¹ independently represent hydrogen, C₁₋₃ alkyl (e.g. methyl or ethyl), which latter group is optionally substituted by one or more (e.g. one) substituent(s) selected from J⁴;
when there is a -N(R²⁰)R²¹ moiety present, then one of R²⁰ and R²¹ represents hydrogen, and the other represents hydrogen, C₁₋₃ alkyl (e.g. methyl or ethyl), which latter group is optionally substituted by one or more (e.g. one) substituent(s) selected from J⁴;
J³ represents Q⁷;
J⁴ represents C₁₋₆ alkyl, such as C₃₋₆ alkyl (especially C₃₋₆ cycloalkyl, such as cyclopropyl);
Q⁷ represents -S(O)₂R⁵⁰;
R⁵⁰ represents C₁₋₃ alkyl (e.g. methyl).

Particularly preferred compounds of the invention include those in which:
R² represents hydrogen or chloro;
R³ represents hydroxyphenyl (e.g. 3-hydroxyphenyl), methoxyphenyl (e.g. 3-methoxyphenyl), indazolyl (e.g. 4-indazolyl), pyrimidinyl (e.g. 5-pyrimidinyl, such as 2-amino-5-pyrimidinyl (i.e. 2-[-N(R²⁰)(R²¹)]-pyrimidin-5-yl such as 2-NH₂-pyrimidin-5-yl or 2-[N(H)(CH₂-cyclopropyl]-pyrimidin-5-yl) or 2-methoxy-5-pyrimidinyl), azaindolyl (e.g. 7-azaindol-5-yl), indolyl (e.g. 5-indolyl or 4-indolyl, such as 5-fluoro-4-indolyl), pyridyl (e.g. 3-pyridyl, such as 6-NH₂-pyrid-3-yl, 5-OCH₃-pyrid-3-yl or 5-CF₃,6-NH₂-pyrid-3-yl);
A₁ represents -C(R⁴)R⁵-, -C(O)- or -N(R⁶)-;
A₂ represents -N(R⁶)-, -C(R⁴)R⁵- or -C(O)-;
A₃ represents -C(R⁴)R⁵-, -N(R⁶)- or -C(O)-;
one of A₂ and A₃ represents -C(R⁴)R⁵- and the other represents -C(R⁴)R⁵- or -N(R⁶)-;
n represents 0 or 1;
A₄ represents -C(R⁴)R⁵- or -C(O)-;
A² may represent -C(O)- when A¹ or A³ (e.g. A¹) represents -N(R⁶)-;
A³ may represent -C(O)- when e.g. n represents 0 and/or A² represents -N(R⁶)-;
only a maximum of two of A₁, A₂, A₃ and, if present, A₄, represents -C(O)- (which, if there are two -C(O)- moieties, are preferably not adjacent to one another);
the dotted lines do not represent the presence of an optional double bond (i.e. the A₁ to A₄-containing ring does not contain a double bond, other than that double bond that is integral to the requisite imidazopyrazine of formula I);
B¹, B^{1a}, B², B^{2a}, B³, B^{3a}, B⁴ and B^{4a} independently represent hydrogen;
each R⁴ and R⁵ independently represent hydrogen, C₁₋₆ alkyl (e.g. C₁₋₃ alkyl such as methyl), -OR^{10c} (e.g. -OH) or -C(O)OR^{10h} (e.g. -C(O)OCH₂CH₃);
each R⁶ (when/if present) independently represents hydrogen, -C(O)CH₂CH₃, -CH₂-[pyridyl]- (e.g. -CH₂-[3-pyridyl], -CH₂-[3-pyridyl] or -CH₂-[4-pyridyl]), -CH₂-[thiazolyl] (e.g. -CH₂-[2-thiazolyl]), -CH₂-[furanyl] (e.g. -CH₂-[2-furanyl] or -CH₂-[3-furanyl]), -CH₂-[imidazolyl] (e.g. -CH₂-[2-imidazolyl]), -CH₂-[tetrahydropyranyl] (e.g. -CH₂-[4-tetrahydropyranyl]), -CH₂-[azetidinyl] (e.g. -CH₂-[3-azetidinyl]), -CH₂-[phenyl] (e.g. -CH₂-[methylsulfonyl-phenyl]), -CH₂-cyclohexyl, methyl, ethyl, butyl (e.g. s-butyl), cyclohexyl (e.g. 4-C(O)OCH₂CH₃-cyclohexyl), -CH₂-cyclopropyl, -C(O)N(H)CH₂CH₃, -C(O)N(H)CH(CH₃)₂, -S(O)₂CH₃, -C(O)CH₃ or -C(O)-CH(CH₃)₂.

Particularly preferred compounds of the invention include those of the examples described hereinafter.

Compounds of the invention may be made in accordance with techniques that are well known to those skilled in the art, for example as described hereinafter.

According to a further aspect of the invention there is provided a process for the preparation of a compound of formula I which process comprises:
(i) reaction of a compound of formula II, 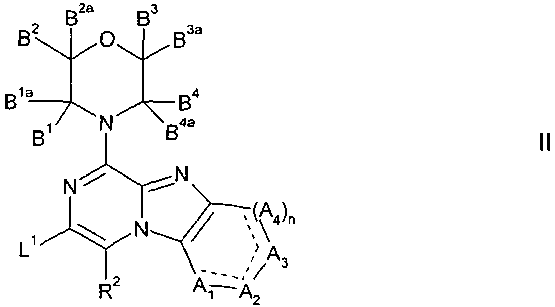 wherein L¹ represents a suitable leaving group, such as iodo, bromo, chloro or a sulfonate group (e.g. -OS(O)₂CF₃, -OS(O)₂CH₃ or -OS(O)₂PhMe), and A¹, A², A³, A⁴, n, the dotted lines, B¹, B^{1a}, B², B^{2a}, B³, B^{3a}, B⁴, B^{4a} and R² are as hereinbefore defined, with a compound of formula III,

   R³-L² III

   wherein L² represents a suitable group such as -B(OH)₂, -B(OR^{wx})₂ or -Sn(R^{wx})₃, in which each R^{wx} independently represents a C₁₋₆ alkyl group, or, in the case of -B(OR^{wx})₂, the respective R^{wx} groups may be linked together to form a 4- to 6-membered cyclic group (such as a 4,4,5,5-tetramethyl-1,3,2-dioxaborolan-2-yl group), thereby forming e.g. a pinacolato boronate ester group, (or L² may represent iodo, bromo or chloro, provided that L¹ and L² are mutually compatible) and R³ is as hereinbefore defined. The reaction may be performed, for example in the presence of a suitable catalyst system, e.g. a metal (or a salt or complex thereof) such as Pd, CuI, Pd/C, PdCl₂, Pd(OAc)₂, Pd(Ph₃P)₂Cl₂, Pd(Ph₃P)₄ (i.e. palladium tetrakistriphenylphosphine), Pd₂(dba)₃ and/or NiCl₂ (preferred catalysts include palladium) and a ligand such as PdCl₂(dppf).DCM, *t*-Bu₃P, (C₆H₁₁)₃P, Ph₃P, AsPh₃, P(*o*-Tol)₃, 1,2-bis(diphenylphosphino)ethane, 2,2'-bis(di-*tert*-butyl-phosphino)-1,1'-biphenyl, 2,2'-bis(dipheny)phosphino)-1,1'-bi-naphthyl, 1,1'-bis(diphenyl-phosphino-ferrocene), 1,3-bis(diphenylphosphino)propane, xantphos, or a mixture thereof (preferred ligands include PdCl₂(dppf).DCM), together with a suitable base such as, Na₂CO₃, K₃PO₄, Cs₂CO₃, NaOH, KOH, K₂CO₃, CsF, Et₃N, (*i*-Pr)₂NEt, *t*-BuONa or *t*-BuOK (or mixtures thereof; preferred bases include Na₂CO₃ and K₂CO₃) in a suitable solvent such as dioxane, toluene, ethanol, dimethylformamide, dimethoxyethane, ethylene glycol dimethyl ether, water, dimethylsulfoxide, acetonitrile, dimethylacetamide, *N*-methylpyrrolidinone, tetrahydrofuran or mixtures thereof (preferred solvents include dimethylformamide and dimethoxyethane). The reaction may be carried out for example at room temperature or above (e.g. at a high temperature such as at about the reflux temperature of the solvent system). Alternative reaction conditions include microwave irradiation conditions, for example at elevated temperature of about 130°C;
(ii) reaction of a compound of formula IV, 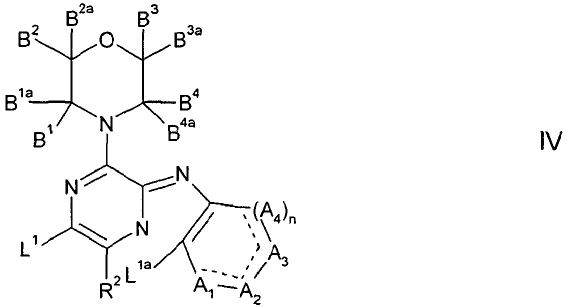 wherein L^{1a} represents a suitable leaving group, such as iodo, bromo, chloro or a sulfonate group (e.g. -OS(O)₂CF₃, -OS(O)₂CH₃ or -OS(O)₂PhMe), and, most preferably, L^{1a} represents bromo or chloro, and L¹, A¹, A², A³, A⁴, n, the dotted lines, B¹, B^{1a}, B², B^{2a}, B³, B^{3a}, B⁴, B^{4a} and R² are as hereinbefore defined, with a compound of formula III as hereinbefore defined, for example under reaction conditions known to those skilled in the art (e.g. such as those described hereinbefore in respect of process step (i) above). In this instance, the cyclisation to form the imidazopyrazine may occur as a distinct step, with the compound of formula II being formed from the intramolecular cyclisation of a compound of formula IV as as an intermediate; see also the synthesis of the compounds of formulae II and IV described hereinafter;
(iii) reaction of a compound of formula V, 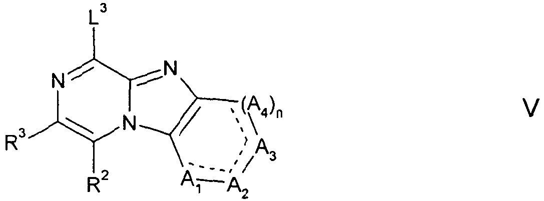 wherein L³ represents a suitable leaving group, such as one hereinbefore defined in respect of L¹, and A¹, A², A³, A⁴, n, the dotted lines R² and R³ as hereinbefore defined, with a compound of formula VI, 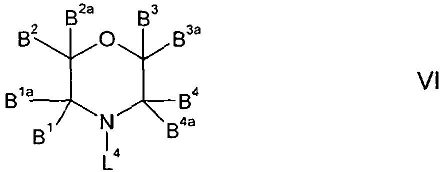 wherein L⁴ may represent hydrogen (so forming an amine group), and L¹, B¹, B^{1a}, B², B^{2a}, B³, B^{3a}, B⁴ and B^{4a} are as hereinbefore defined, and the reaction may be performed in the presence of an appropriate metal catalyst (or a salt or complex thereof) such as Cu, Cu(OAc)₂, CuI (or CuI/diamine complex), copper tris(triphenylphosphine)bromide, Pd(OAc)₂, tris(dibenzylideneacetone)-dipalladium(0) (Pd₂(dba)₃) or NiCl₂ and an optional additive such as Ph₃P, 2,2'-bis(diphenylphosphino)-1,1'-binaphthyl, xantphos, Nal or an appropriate crown ether such as 18-crown-6-benzene, in the presence of an appropriate base such as NaH, Et₃N, pyridine, *N*,*N*'-dimethylethylenediamine, Na₂CO₃, K₂CO₃, K₃PO₄, Cs₂CO₃, *t*-BuONa or *t*-BuOK (or a mixture thereof, optionally in the presence of 4A molecular sieves), in a suitable solvent (e.g. dichloromethane, dioxane, toluene, ethanol, isopropanol, dimethylformamide, ethylene glycol, ethylene glycol dimethyl ether, water, dimethylsulfoxide, acetonitrile, dimethylacetamide, *N*-methylpyrrolidinone, tetrahydrofuran or a mixture thereof). This reaction may be performed at elevated temperature or under microwave irradiation reaction conditions, for example as described in process step (i). The compound of formula V (e.g. in which L³ is chloro) may be prepared *in situ,* for example from a compound corresponding to a compound of formula V, but in which L³ represents -OC₁₋₃ alkyl (e.g. methoxy) by reaction in the presence of e.g. a chlorinating agent (such as POCl₃);
(iv) reaction of a compound of formula VII, 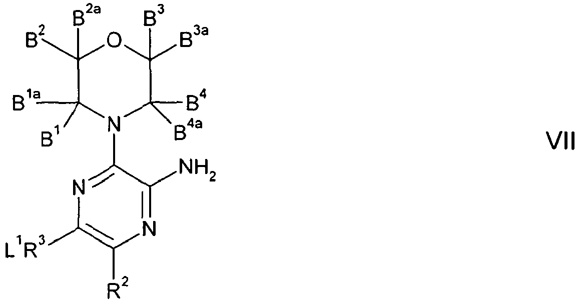 wherein L¹R³ represents either L¹ or R³, B¹, B^{1a}, B², B^{2a}, B³, B^{3a}, B⁴, B^{4a}, R², L¹ and R³ are as hereinbefore defined, with a compound of formula VIII, 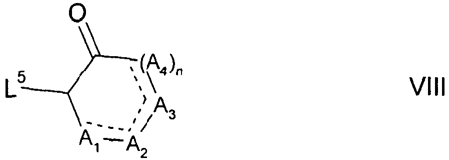 wherein L⁵ represents a suitable leaving group, such as one hereinbefore defined in respect of L¹ (and, especially, L⁵ represents iodo or, preferably, chloro or bromo), and A¹, A², A³, A⁴, n, and the dotted lines are as hereinbefore defined, under standard reaction conditions, for example in the presence of a suitable reaction solvent (such as DME and/or 2-propanol), or neat, i.e. in the absence of a solvent, at a convenient temperature, typically heating at 90°C, followed by (in the case of reaction with a compound of formula VII in which L¹R³ represents L¹), reaction with a compound of formula III as hereinbefore defined, for example under reaction conditions such as those described hereinbefore in respect of process step (i);
(v) for compounds of formula I in which R² represents halo (e.g. bromo, iodo or chloro), reaction of a corresponding compound of formula I, in which R² represents hydrogen, with a reagent that is a source of halide ions (a halogenating reagent). For instance, an electrophile that provides a source of iodide ions includes iodine, diiodoethane, diiodotetrachloroethane or, preferably, *N*-iodosuccinimide, a source of bromide ions includes *N*-bromosuccinimide and bromine, and a source of chloride ions includes *N*-chlorosuccinimide, chlorine and iodine monochloride, for instance in the presence of a suitable solvent, such as CHCl₃ or an alcohol (e.g. methanol), optionally in the presence of a suitable base, such as a weak inorganic base, e.g. sodium bicarbonate. Typically, the reaction maybe performed by heating at a convenient temperature, either by conventional heating under reflux or under microwave irradiation;
(vi) for compounds of formula I in which R² represents a substituent other than hydrogen, or halo (e.g. bromo, iodo or chloro), reaction of a corresponding compound of formula I, in which R² represents halo (e.g. bromo, chloro or iodo), with a compound of formula IX,

   R^{2a}-L⁷ IX

   wherein R^{2a} represents R² as hereinbefore described provided that it does not represent hydrogen or halo, and L⁷ represents a suitable leaving group such as one hereinbefore described in respect of L¹, L^{1a} or L² (see process step (i) or (ii) above; reaction conditions such as those mentioned above may also be employed). Alternatively, the skilled person will appreciate that different reagents and reaction steps may be employed, depending on the particular R^{2a} substituent required;
(vii) for certain compounds of formula I, reaction of a compound of formula X, 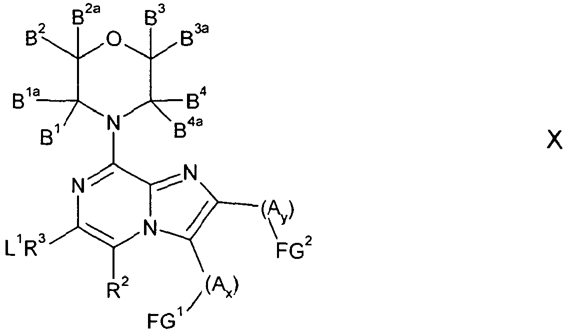 wherein (Aₓ) and (A_{y}) denotes the optional presence of the relevant A₁ to A₄ groups that are/may be present in the compound of formula I, and FG¹ and FG² independently represent mutually compatible functional groups, which may undergo an intramolecular reaction to form the requisite A₁ to A₄-containing ring of formula I (and L¹R³, B¹, B^{1a}, B², B^{2a}, B³, B^{3a}, B⁴, B^{4a} and R² are as hereinbefore defined). The skilled person will appreciate that the A₁ to A₄ groups in the compound of formula I formed, may be present either at the positions represented by (Aₓ) or (A_{y}) or may be an integral part of FG¹ and/or FG². By mutually compatible functional groups (FG¹ and FG²), we mean that such groups may be manipulated so as to promote an intramolecular reaction, for example, FG¹ may be -NH₂ and FG² may be -C(O)OH (or a derivative thereof; e.g. an ester), which functional groups may undergo an amide coupling reaction to form a -N(H)C(O)- linkage (and therefore a ring). In this instance, (Aₓ) is absent, the -N(H)-C(O)- linkage so formed represents the A₁ and A₂ moieties (i.e. these emanate from an integral part of FG¹ and FG²), and (A_{y}) represents A₃ and A₄, which together are -CH₂-CH₂-. Alternatively, FG¹ and FG² may independently represent leaving groups, such as those hereinbefore defined in respect of L¹. In this instance, the compound of formula X may be reacted with a nucleophile (such as one with more than one nucleophilic site e.g. ammonia), which may displace each of the leaving groups to form the requisite A₁ to A₄-containing ring of formula I (followed by, if necessary, reaction with a compound of formula III as hereinbefore defined). Transformation of the functional group(s) (FG¹ and/or FG²) following methodology very well known for any person skill in the art, would allow to obtain an intramolecular cyclisation to obtain a tricycle of formula I. Specifically, each of -(Aₓ)-FG¹ and -(A_{y})-FG² may represent -CH₂-Cl and such a compound may be reacted with ammonia to form a compound of formula I in which n represent 0 (hence, there is no A₄ present) and A₁ and A₃ each represent -CH₂- and A₂ represents -N(H)-;
(viii) for certain compounds of formula I, reaction of a compound of formula VII as hereinbefore defined, with a compound of formula XI, 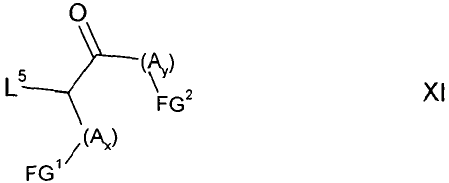 wherein (Aₓ), (A_{y}), FG¹, FG² and L⁵ are as hereinbefore defined, under reaction conditions known to those skilled in the art, for example, such as those described hereinbefore in respect of process step (iv), followed by those described in respect of process step (vii), followed by, if necessary (e.g. in the case where L¹R³ represents L¹), reaction with a compound of formula III as hereinbefore defined, for example under reaction conditions such as those described in respect of process step (i) above;
(ix) for compounds of formula I in which there is a -N(R⁶)- moiety present, in which R⁶ represents C₁₋₁₂ alkyl optionally substituted as hereinbefore defined (i.e. by one or more substituent(s) selected from E⁷ and =O), may be prepared by reaction of a corresponding compound of formula I in which R⁶ represents hydrogen, with a compound of formula XII,

   R^{6a}-C(O)H XII

   wherein R^{6a} represents C₁₋₁₁ alkyl optionally substituted by one or more substituent(s) selected from E⁷ and =O (but preferably not substituted with a =O substituent), under reductive amination reaction conditions, for example in a "one-pot" procedure in the presence of an appropriate reducing agent, such as sodium cyanoborohydride or, preferably, sodium triacetoxyborohydride, or alternatively in two distinct steps by a condensation reaction to form e.g. an enamine, followed by reduction under reaction conditions such as in the presence of NaBH₄ or the like;
(x) for compounds of formula I in which there is a -N(R⁶)- moiety present, in which R⁶ represents -C(O)N(H)R^{11t}, may be prepared by reaction of a corresponding compound of formula I in which R⁶ represents hydrogen, with a compound of formula XIII,

   R^{11t}-N=C=O XIII

   wherein R^{11t} is as hereinbefore defined, for example, under reaction conditions known to those skilled in the art, such as those described herein, e.g. in the presence of a suitable base such as an amine base (e.g. diisopropylamine or the like such as DIPEA) and a suitable solvent (e.g. acetonitrile or the like);
(xi) for compounds of formula I in which there is a -N(R⁶)- moiety present, in which R⁶ represents -C(O)R^{10r} or -S(O)₂R^{10u}, may be prepared by reaction of a corresponding compound of formula I in which R⁶ represents hydrogen, with a compound of formula XIV,

   G¹-L^{1b} XIV

   wherein G¹ represents either -C(O)R^{10r} or -S(O)₂R^{10u}, and L^{1b} (attached to the -C(O)- or -S(O)₂ moieties) represents a suitable leaving group such as iodo, bromo or, preferably, chloro, under reaction conditons known to those skilled in the art, for example at around room temperature or above in the presence of a suitable base (e.g. pyridine, triethylamine, dimethylaminopyridine, diisopropylamine, sodium hydroxide, a carbonate (e.g. Cs₂CO₃), another suitable amine base (e.g. DIPEA), or mixtures thereof), an appropriate solvent (e.g. acetonitrile, pyridine, dichloromethane, chloroform, tetrahydrofuran, dimethylformamide, triethylamine, dimethylsulfoxide, water or mixtures thereof) and, in the case of biphasic reaction conditions, optionally in the presence of a phase transfer catalyst.

Compound of formula X in which each of -(Aₓ)-FG¹ and -(A_{y})-FG² represent -CH₂-Cl may be prepared from corresponding compound in which -(Aₓ)-FG¹ and -(A_{y})-FG² each represent -CH₂-OH (by chlorination), which in turn may be prepared from compounds in which -(Aₓ)-FG¹ and -(A_{y})-FG² each represent -C(O)O-alkyl (e.g. -C(O)O-ethyl) (by an appropriate reduction reaction), which dicarboxylic acids may be prepared from a compound of formula VII as hereinbefore defined, with a compound of formula XI as hereinbefore defined but in which -(Aₓ)-FG¹ and -(A_{y})-FG² each represent -C(O)O-alkyl.

Other specific transformation steps (including those that may be employed in order to form compounds of formula I) that may be mentioned include:
(i) reductions of a carboxylic acid (or ester) to either an aldehyde or an alcohol, using appropriate reducing conditions (e.g. -C(O)OH (or an ester thereof), may be converted to a -C(O)H or -CH₂-OH group, using DIBAL and LiAlH₄, respectively (or similar chemoselective reducing agents);
(ii) reductions of an aldehyde (-C(O)H) group to an alcohol group (-CH₂OH), using appropriate reduction conditions such as those mentioned at point (i) above;
(iii) oxidations, for example of a moiety containing an alcohol group (e.g. -CH₂OH) to an aldehyde (e.g. -C(O)H) or of a -S- moiety to a -S(O)- or -S(O)₂- moiety (or the reverse reduction reaction), for example in the presence of a suitable oxidising agent, e.g. MnO₂ or mcpba or the like;
(iv) reductive amination of an aldehyde and an amine, under appropriate reaction conditions, for example in "one-pot" procedure in the presence of an appropriate reducing agent, such as sodium cyanoborohydride or, preferably, sodium triacetoxyborohydride, or the like;
(v) amide coupling reactions (e.g. the formation of an amide or sulfonamide), i.e. the formation of an amide from a carboxylic acid (or ester thereof) or by reaction of a sulfonyl chloride and an amine, for example when R² represents -C(O)OH (or an ester thereof), it may be converted to a -C(O)N(R^{10b})R^{11b} group (in which R^{10b} and R^{11b} are as hereinbefore defined, and may be linked together, e.g. as defined above), and which reaction may (e.g. when R² represents -C(O)OH) be performed in the presence of a suitable coupling reagent (e.g. 1,1'-carbonyldiimidazole, *N*,*N*'-dicyclohexylcarbodiimide, or the like) or, in the case when R² represents an ester (e.g. -C(O)OCH₃ or -C(O)OCH₂CH₃), in the presence of e.g. trimethylaluminium, or, alternatively the -C(O)OH group may first be activated to the corresponding acyl halide (e.g -C(O)Cl, by treatment with oxalyl chloride, thionyl chloride, phosphorous pentachloride, phosphorous oxychloride, or the like), and, in all cases, the relevant compound is reacted with a compound of formula HN(R^{10a})R^{11a} (in which R^{10a} and R^{11a} are as hereinbefore defined), under standard conditions known to those skilled in the art (e.g. optionally in the presence of a suitable solvent, suitable base and/or in an inert atmosphere);
(vi) conversion of a primary amide to a nitrile functional group, for example under dehydration reaction conditions, e.g. in the presence of POCl₃, or the like;
(vii) nucleophilic substitution (e.g. aromatic nucleophilic substitution) reactions, where any nucleophile replaces a leaving group, e.g. an amine may replace a -S(O)CH₃ leaving group, such reactions include "Mitsunobu"-type reactions (or variants thereof), i.e. in which a -OH is the leaving group, which is activated by treatment with e.g. iodine and triphenylphosphine);
(viii) transformation of a methoxy group to a hydroxy group, by reaction in the presence of an appropriate reagent, such as boron fluoride-dimethyl sulfide complex or BBr₃ (e.g. in the presence of a suitable solvent such as dichloromethane);
(ix) alkylation, acylation or sulfonylation reactions, which may be performed in the presence of base and solvent (such as those described hereinbefore);
(x) specific deprotection steps, such as deprotection of an *N*-Boc protecting group by reaction in the presence of an acid, or, a hydroxy group protected as a silyl ether (*e.g*. a *tert*-butyl-dimethylsilyl protecting group) may be deprotected by reaction with a source of fluoride ions, e.g. by employing the reagent tetrabutylammonium fluoride (TBAF). Further, a -O-C(O)-CH₃ may be converted to a -OH group by reaction with sodium methoxide in methanol, or similar hydrolysis reactions may be performed;
(xi) hydrogenations, e.g. of a double bond to a single bond for instance under standard hydrogenation reaction conditions, e.g. under a hydrogen atmosphere in the presence of a catalyst such as Pd/C;
(xii) Grignard reactions, e.g. the addition of a nucleophilic organometalic reagent, for instance the addition of MeMgCl to a carbonyl group;
(xiii) formation of a urea functional group by reaction of a isocyanate with an amine, e.g. when R⁵ represent phenyl substituted by -NH₂, this may be converted to a -N(H)-C(O)-N(R)R moiety;
(xiv) hydrolyses (e.g. conversion of an ester to a carboxylic acid), esterifications (e.g. conversion of a carboxylic acid to an ester), and trans-esterifications (e.g. conversion of a certain alkyl ester to a different alkyl ester).

Intermediate compounds described herein are either commercially available, are known in the literature, or may be obtained either by analogy with the processes described herein, or by conventional synthetic procedures, in accordance with standard techniques, from available starting materials using appropriate reagents and reaction conditions. Further, processes to prepare compounds of formula I may be described in the literature, for example in:
Werber,G. et al.; J. Heterocycl. Chem.; EN; 14; 1977; 823-827;
Andanappa K. Gadad et al. Bioorg. Med. Chem. 2004, 12, 5651-5659;
Paul Heinz et al. Monatshefte fur Chemie, 1977, 108, 665-680;
M.A. EI-Sherbeny et al. Boll. Chim. Farm. 1997, 136, 253-256;
Nicolaou, K. C.; Bulger, P. G.; Sarlah, D. Angew. Chem. Int. Ed. 2005, 44, 2-49;
Bretonnet et al. J. Med. Chem. 2007, 50, 1872 ;
Asunción Marin et al. Farmaco 1992, 47 (1), 63-75;
Severinsen, R. et al. Tetrahedron 2005, 61, 5565-5575;
Nicolaou, K. C.; Bulger, P. G.; Sarlah, D. Angew. Chem. Int. Ed. 2005, 44, 2-49;
M. Kuwahara et al., Chem. Pharm Bull., 1996, 44, 122;
Wipf, P.; Jung, J.-K. J. Org. Chem. 2000, 65(20), 6319-6337;
Shintani, R.; Okamoto, K. Org. Lett. 2005, 7 (21), 4757-4759;
Nicolaou, K. C.; Bulger, P. G.; Sarlah, D. Angew. Chem. Int. Ed. 2005, 44, 2-49;
J. Kobe et al., Tetrahedron, 1968, 24, 239 ;
P.F. Fabio, A.F. Lanzilotti and S.A. Lang, Journal of Labelled Compounds and Pharmaceuticals, 1978, 15, 407;
F.D. Bellamy and K. Ou, Tetrahedron Lett., 1985, 25, 839;
M. Kuwahara et al., Chem. Pharm Bull., 1996, 44, 122;
A.F. Abdel-Magid and C.A Maryanoff. Synthesis, 1990, 537;
M. Schlosser et al. Organometallics in Synthesis. A Manual, (M. Schlosser, Ed.), Wiley &Sons Ltd: Chichester, UK, 2002**,** and references cited therein;
L. Wengwei et al., Tetrahedron Lett., 2006, 47, 1941;
M. Plotkin et al. Tetrahedron Lett., 2000, 41, 2269;
Seyden-Penne, J. Reductions by the Alumino and Borohydrides, VCH, NY, 1991**;**
O. C. Dermer, Chem. Rev., 1934, 14, 385;
N. Defacqz, et al., Tetrahedron Lett., 2003, 44, 9111;
S.J. Gregson et al., J. Med. Chem., 2004, 47, 1161;
A. M. Abdel Magib, et al., J. Org. Chem., 1996, 61, 3849;
A.F. Abdel-Magid and C.A Maryanoff. Synthesis, 1990, 537;
T. Ikemoto and M. Wakimasu, Heterocycles, 2001, 55, 99;
E. Abignente et al., II Farmaco, 1990, 45, 1075;
T. Ikemoto et al., Tetrahedron, 2000, 56, 7915;
T. W. Greene and P. G. M. Wuts, Protective Groups in Organic Synthesis, Wiley, NY, 1999**;**
S. Y. Han and Y.-A. Kim. Tetrahedron, 2004, 60, 2447*;*
J. A. H. Lainton et al., J. Comb. Chem., 2003, 5, 400; or
Wiggins, J. M. Synth. Commun., 1988, 18, 741.

For example, intermediate compounds, and compounds of the invention may be prepared in accordance with the following scheme (the numbering of compounds in the following scheme is distinct from the numbering of the compounds elsewhere in the description).

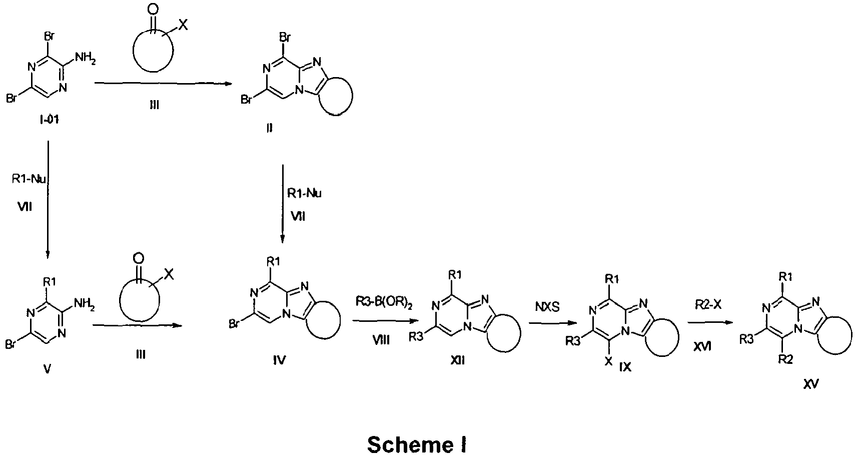

In the above scheme, R¹ represents the requisite morpholinyl group of formula I (which may be unsubstituted or is optionally substituted by a substituent defined by B¹, B^{1a}, B², B^{2a}, B³, B^{3a}, B⁴ or B^{4a}), and the circle represents the requisite A₁ to A₄-containing ring of formula I.

Compound I-01 was reacted with an intermediate (III) (which may be depicted as a compound of formula VIII as hereinbefore defined) where X represents a suitable leaving group (e.g. a halide), without solvent or in the presence of a suitable reaction solvent such as DME or 2-propanol, at a convenient temperature, typically heating at 90°C, to obtain compounds of formula (II).

Compounds of formula (II) can react with an intermediate (VII) of formula R¹-Nu, where R¹ represents the morpholinyl group hereinbefore defined and Nu represents the nucleophilic amino group of the morpholinyl group (i.e. R¹-Nu may represent a compound of formula VI as hereinbefore defined; and a corresponding compound of formula II hereinbefore defined may be formed, in which the morholinyl group is to be linked to the imidazopyrazine) in a suitable solvent such as DCM, dioxane at room temperature or by heating at a convenient temperature, for a period of time to ensure the completion of the reaction.

Further reaction (of intermediate (IV) in Scheme 1; or of a compound of formula II hereinbefore defined) may be with an intermediate (VIII) of formula R³-B(OR)₂, which R is H or C₁-C₆ alkyl or the two groups OR form, together with the boron atom to which they are attached a pinacolato boronate ester group, and where R³ is as defined before, in a suitable solvent such as DME or DMF, in the presence of a suitable base, such as an inorganic aqueous base Na₂CO₃ or K₂CO₃, in the presence of a metal catalyst, such as palladium, and a suitable ligand, such us PdCl₂(dppf).DCM, Pd(PPh₃)₄ by heating at a convenient temperature, such as 130°C under microwave irradiation or reflux temperature under traditional heating, for a period of time that allows the completion of reaction, to obtain compounds of formula (XII) (i.e. a certain compound of formula I of the invention).

Compound 1-01 can react with an intermediate (VII) of formula R¹-Nu (as hereinbefore defined), at a convenient temperature, such us 120°C, for a period of time that allows the completion of reaction, to afford compound (V).

Compound (V) can be reacted with an intermediate (III) (e.g. a compound of formula VIII as hereinbefore defined), under reaction conditions hereinbefore described to obtain compounds of formula (IV).

Compounds of formula (XII) can be reacted with a halogenating agent, such as N-bromosuccinimide, N-iodosuccininide, N-chlorosuccinimide or others, and X represents an halogen group such as Cl, Br or Iodine atom, in the presence of a suitable reaction solvent such as CHCl₃, typically heating at a convenient temperature, either by conventional heating under reflux or under microwave irradiation, for a period of time to ensure the completion of the reaction, to obtain compounds of formula (IX).

The halogen atom X of compounds of formula (IX) can be substituted *via* a coupling reaction with an intermediate (XVI) of formula R²-B(OR)₂, in which the -B(OR)₂ moiety is as hereinbefore defined, and R² is as hereinbefore defined, e.g. under reaction conditions hereinbefore described (e.g. the reaction of (IV) with (VIII)), for a period of time that allows the completion of reaction, to obtain compounds of formula XV.

The halogen atom X of compounds of formula (IX) can be substituted *via* coupling reaction of a CN group, by treatment with Zn(CN)₂, in a suitable solvent such as DMF, AcCN and in the presence of a Pd catalyst, such us Pd(PPh₃)₄ or PdCl₂(dppf)₂. Additionally an inorganic aqueous base can be added such as Na₂CO₃ aq. Heating at a convenient temperature, such as 130°C under microwave irradiation or reflux temperature under traditional heating, for a period of time that allows the completion of reaction, to obtain compounds of formula XV.

Compounds of formula (V) can react with an intermediate (VIII) of formula R³-B(OR)₂ as hereinbefore defined, e.g. under reaction conditions hereinbefore described (e.g. the reaction of (IV) with (VIII) to obtain (XII)), to obtain intermediate compounds.

Those skilled in the art will appreciate that other synthetic routes may be used to synthesise the compounds of the invention. Although specific starting materials and reagents are depicted in the Scheme 1 and discussed below, other starting materials and reagents can be easily substituted to provide a variety of derivatives and/or reaction conditions.

The substituents R², R³, B¹, B^{1a}, B², B^{2a}, B³, B^{3a}, B⁴, B^{4a}, A₁, A₂, A₃ and (A₄)ₙ in final compounds of the invention or relevant intermediates may be modified one or more times, after or during the processes described above by way of methods that are well known to those skilled in the art. Examples of such methods include substitutions, reductions, oxidations, alkylations, acylations, hydrolyses, esterifications, etherifications, halogenations or nitrations. Such reactions may result in the formation of a symmetric or asymmetric final compound of the invention or intermediate. The precursor groups can be changed to a different such group, or to the groups defined in formula I, at any time during the reaction sequence.

For example, when substituents in the compounds of the invention (e.g. represented by R², R³, B¹, B^{1a}, B², B^{2a}, B³, B^{3a}, B⁴, B^{4a}, A₁, A₂, A₃ and (A₄)ₙ) such as CO₂Et, CHO, CN and/or CH₂Cl, are present, these groups can be further derivatized to other fragments described (e.g. by those integers mentioned above) in compounds of the invention, following synthetic protocols very well know to the person skilled in the art and/or according to the experimental part described in the patent. Other specific transformation steps that may be mentioned include: the reduction of a nitro or azido group to an amino group; the hydrolysis of a nitrile group to a carboxylic acid group; and standard nucleophilic aromatic substitution reactions, for example in which an iodo-, preferably, fluoro-or bromo-phenyl group is converted into a cyanophenyl group by employing a source of cyanide ions (e.g. by reaction with a compound which is a source of cyano anions, e.g. sodium, copper (I), zinc or potassium cyanide, optionally in the presence of a palladium catalyst) as a reagent (alternatively, in this case, palladium catalysed cyanation reaction conditions may also be employed).

Other transformations that may be mentioned include: the conversion of a halo group (preferably iodo or bromo) to a 1-alkynyl group (e.g. by reaction with a 1-alkyne), which latter reaction may be performed in the presence of a suitable coupling catalyst (e.g. a palladium and/or a copper based catalyst) and a suitable base (e.g. a tri-(C₁₋₆ alkyl)amine such as triethylamine, tributylamine or ethyldiisopropylamine); the introduction of amino groups and hydroxy groups in accordance with standard conditions using reagents known to those skilled in the art; the conversion of an amino group to a halo, azido or a cyano group, for example *via* diazotisation (e.g. generated *in situ* by reaction with NaNO₂ and a strong acid, such as HCl or H₂SO₄, at low temperature such as at 0°C or below, e.g. at about -5°C) followed by reaction with the appropriate nucleophile e.g. a source of the relevant anions, for example by reaction in the presence of a halogen gas (e.g. bromine, iodine or chlorine), or a reagent that is a source of azido or cyanide anions, such as NaN₃ or NaCN; the conversion of -C(O)OH to a -NH₂ group, under Schmidt reaction conditions, or variants thereof, for example in the presence of HN₃ (which may be formed in by contacting NaN₃ with a strong acid such as H₂SO₄), or, for variants, by reaction with diphenyl phosphoryl azide ((PhO)₂P(O)N₃) in the presence of an alcohol, such as *tert*-butanol, which may result in the formation of a carbamate intermediate; the conversion of -C(O)NH₂ to -NH₂, for example under Hofmann rearrangement reaction conditions, for example in the presence of NaOBr (which may be formed by contacting NaOH and Br₂) which may result in the formation of a carbamate intermediate; the conversion of -C(O)N₃ (which compound itself may be prepared from the corresponding acyl hydrazide under standard diazotisation reaction conditions, e.g. in the presence of NaNO₂ and a strong acid such as H₂SO₄ or HCl) to -NH₂, for example under Curtius rearrangement reaction conditions, which may result in the formation of an intermediate isocyanate (or a carbamate if treated with an alcohol); the conversion of an alkyl carbamate to -NH₂, by hydrolysis, for example in the presence of water and base or under acidic conditions, or, when a benzyl carbamate intermediate is formed, under hydrogenation reaction conditions (e.g. catalytic hydrogenation reaction conditions in the presence of a precious metal catalyst such as Pd); halogenation of an aromatic ring, for example by an electrophilic aromatic substitution reaction in the presence of halogen atoms (e.g. chlorine, bromine, etc, or an equivalent source thereof) and, if necessary an appropriate catalyst/Lewis acid (e.g. AlCl₃ or FeCl₃).

Compounds of the invention bearing a carboxyester functional group may be converted into a variety of derivatives according to methods well known in the art to convert carboxyester groups into carboxamides, N-substituted carboxamides, N,N-disubstituted carboxamides, carboxylic acids, and the like. The operative conditions are those widely known in the art and may comprise, for instance in the conversion of a carboxyester group into a carboxamide group, the reaction with ammonia or ammonium hydroxide in the presence of a suitable solvent such as a lower alcohol, dimethylformamide or a mixture thereof; preferably the reaction is carried out with ammonium hydroxide in a methanol/dimethylformamide mixture, at a temperature ranging from about 50°C to about 100°C. Analogous operative conditions apply in the preparation of N-substituted or N,N-disubstituted carboxamides wherein a suitable primary or secondary amine is used in place of ammonia or ammonium hydroxide. Likewise, carboxyester groups may be converted into carboxylic acid derivatives through basic or acidic hydrolysis conditions, widely known in the art. Further, amino derivatives of compounds of the invention may easily be converted into the corresponding carbamate, carboxamido or ureido derivatives.

Compounds of the invention may be isolated from their reaction mixtures using conventional techniques (e.g. recrystallisations).

It will be appreciated by those skilled in the art that, in the processes described above and hereinafter, the functional groups of intermediate compounds may need to be protected by protecting groups.

The need for such protection will vary depending on the nature of the remote functionality and the conditions of the preparation methods (and the need can be readily determined by one skilled in the art). Suitable amino-protecting groups include acetyl, trifluoroacetyl, t-butoxycarbonyl (BOC), benzyloxycarbonyl (CBz), 9-fluorenylmethyleneoxycarbonyl (Fmoc) and 2,4,4-trimethylpentan-2-yl (which may be deprotected by reaction in the presence of an acid, e.g. HCl in water/alcohol (e.g. MeOH)) or the like. The need for such protection is readily determined by one skilled in the art.

The protection and deprotection of functional groups may take place before or after a reaction in the above-mentioned schemes.

Protecting groups may be removed in accordance with techniques that are well known to those skilled in the art and as described hereinafter. For example, protected compounds/intermediates described herein may be converted chemically to unprotected compounds using standard deprotection techniques.

The type of chemistry involved will dictate the need, and type, of protecting groups as well as the sequence for accomplishing the synthesis.

The use of protecting groups is fully described in *"*Protective Groups in Organic Synfhesis", 3rd edition, T.W. Greene & P.G.M. Wutz, Wiley-Interscience (1999).

### Medical and Pharmaceutical Uses

Compounds of the invention are indicated as pharmaceuticals. According to a further aspect of the invention there is provided a compound of the invention, as hereinbefore defined, for use as a pharmaceutical.

Compounds of the invention may inhibit protein or lipid kinases, such as a PI3 kinase (especially a class I PI3K), for example as may be shown in the tests described below (for example, the test for PI3Kα inhibition described below) and/or in tests known to the skilled person. The compounds of the invention may also inhibit mTOR. Thus, the compounds of the invention may be useful in the treatment of those disorders in an individual in which the inhibition of such protein or lipid kinases (e.g. PI3K, particularly class I PI3K, and/or mTOR) is desired and/or required (for instance compounds of the invention may inhibit PI3K, particularly class I PI3K and, optionally, may also inhibit mTOR).

The term "inhibit" may refer to any measurable reduction and/or prevention of catalytic kinase (e.g. PI3K, particularly class I PI3K, and/or mTOR) activity. The reduction and/or prevention of kinase activity may be measured by comparing the kinase activity in a sample containing a compound of the invention and an equivalent sample of kinase (e.g. PI3K, particularly class I PI3K, and/or mTOR) in the absence of a compound of the invention, as would be apparent to those skilled in the art. The measurable change may be objective (e.g. measurable by some test or marker, for example in an *in vitro* or *in vivo* assay or test, such as one described hereinafter, or otherwise another suitable assay or test known to those skilled in the art) or subjective (e.g. the subject gives an indication of or feels an effect).

Compounds of the invention may be found to exhibit 50% inhibition of a protein or lipid kinase (e.g. PI3K, such as class I PI3K, and/or mTOR) at a concentration of 100 µM or below (for example at a concentration of below 50 µM, or even below 10 µM, such as below 1 µM), when tested in an assay (or other test), for example as described hereinafter, or otherwise another suitable assay or test known to the skilled person.

Compounds of the invention are thus expected to be useful in the treatment of a disorder in which a protein or lipid kinase (e.g. PI3K, such as class I PI3K, and/or mTOR) is known to play a role and which are characterised by or associated with an overall elevated activity of that kinase (due to, for example, increased amount of the kinase or increased catalytic activity of the kinase). Hence, compounds of the invention are expected to be useful in the treatment of a disease/disorder arising from abnormal cell growth, function or behaviour associated with the protein or lipid kinase (e.g. PI3K, such as class I PI3K, and/or mTOR). Such conditions/disorders include cancer, immune disorders, cardiovascular diseases, viral infections, inflammation, metabolism/endocrine function disorders and neurological disorders.

The disorders/conditions that the compounds of the invention may be useful in treating hence includes cancer (such as lymphomas, solid tumours or a cancer as described hereinafter), obstructive airways diseases, allergic diseases, inflammatory diseases (such as asthma, allergy and Chrohn's disease), immunosuppression (such as transplantation rejection and autoimmune diseases), disorders commonly connected with organ transplantation, AIDS-related diseases and other associated diseases. Other associated diseases that may be mentioned (particularly due to the key role of kinases in the regulation of cellular proliferation) include other cell proliferative disorders and/or non-malignant diseases, such as benign prostate hyperplasia, familial adenomatosis, polyposis, neuro-fibromatosis, psoriasis, bone disorders, atherosclerosis, vascular smooth cell proliferation associated with atherosclerosis, pulmonary fibrosis, arthritis glomerulonephritis and post-surgical stenosis and restenosis. Other disease states that may be mentioned include cardiovascular disease, stroke, diabetes, hepatomegaly, Alzheimer's disease, cystic fibrosis, hormone-related diseases, immunodeficiency disorders, destructive bone disorders, infectious diseases, conditions associated with cell death, thrombin-induced platelet aggregation, chronic myelogenous leukaemia, liver disease, pathologic immune conditions involving T cell activation and CNS disorders.

As stated above, the compounds of the invention may be useful in the treatment of cancer. More, specifically, the compounds of the invention may therefore be useful in the treatment of a variety of cancer including, but not limited to: carcinoma such as cancer of the bladder, breast, colon, kidney, liver, lung (including non-small cell cancer and small cell lung cancer), esophagus, gall-bladder, ovary, pancreas, stomach, cervix, thyroid, prostate, skin, squamous cell carcinoma, testis, genitourinary tract, larynx, glioblastoma, neuroblastoma, keratoacanthoma, epidermoid carcinoma, large cell carcinoma, non-small cell lung carcinoma, small cell lung carcinoma, lung adenocarcinoma, bone, adenoma, adenocarcinoma, follicular carcinoma, undifferentiated carcinoma, papilliary carcinoma, seminona, melanoma, sarcoma, bladder carcinoma, liver carcinoma and biliary passages, kidney carcinoma, myeloid disorders, lymphoid disorders, hairy cells, buccal cavity and pharynx (oral), lip, tongue, mouth, pharynx, small intestine, colon-rectum, large intestine, rectum, brain and central nervous system, Hodgkin's and leukaemia; hematopoietic tumors of lymphoid lineage, including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma; hematopoietic tumors of myeloid lineage, including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannomas; and other tumors, including melanoma, seminoma, teratocarcinoma, osteosarcoma, xeroderma pigmentosum, keratoxanthoma, thyroid follicular cancer and Kaposi's sarcoma.

Further, the protein or lipid kinases (e.g. PI3K, such as class I PI3K, and/or mTOR) may also be implicated in the multiplication of viruses and parasites.

They may also play a major role in the pathogenesis and development of neurodegenerative disorders. Hence, compounds of the invention may also be useful in the treatment of viral conditions, parasitic conditions, as well as neurodegenerative disorders.

Compounds of the invention are indicated both in the therapeutic and/or prophylactic treatment of the above-mentioned conditions.

According to a further aspect of the present invention, there is provided a method of treatment of a disease (e.g. cancer or another disease as mentioned herein) which is associated with the inhibition of protein or lipid kinase (e.g. PI3K, such as class I PI3K, and/or mTOR) is desired and/or required (for example, a method of treatment of a disease/disorder arising from abnormal cell growth, function or behaviour associated with protein or lipid kinases, e.g. PI3K, such as class I PI3K, and/or mTOR), which method comprises administration of a therapeutically effective amount of a compound of the invention, as hereinbefore defined, to a patient suffering from, or susceptible to, such a condition.

"Patients" include mammalian (including human) patients. Hence, the method of treatment discussed above may include the treatment of a human or animal body.

The term "effective amount" refers to an amount of a compound, which confers a therapeutic effect on the treated patient. The effect may be objective (e.g. measurable by some test or marker) or subjective (e.g. the subject gives an indication of or feels an effect).

Compounds of the invention may be administered orally, intravenously, subcutaneously, buccally, rectally, dermally, nasally, tracheally, bronchially, sublingually, by any other parenteral route or *via* inhalation, in a pharmaceutically acceptable dosage form.

Compounds of the invention may be administered alone, but are preferably administered by way of known pharmaceutical formulations, including tablets, capsules or elixirs for oral administration, suppositories for rectal administration, sterile solutions or suspensions for parenteral or intramuscular administration, and the like. The type of pharmaceutical formulation may be selected with due regard to the intended route of administration and standard pharmaceutical practice. Such pharmaceutically acceptable carriers may be chemically inert to the active compounds and may have no detrimental side effects or toxicity under the conditions of use.

Such formulations may be prepared in accordance with standard and/or accepted pharmaceutical practice. Otherwise, the preparation of suitable formulations may be achieved non-inventively by the skilled person using routine techniques and/or in accordance with standard and/or accepted pharmaceutical practice.

According to a further aspect of the invention there is thus provided a pharmaceutical formulation including a compound of the invention, as hereinbefore defined, in admixture with a pharmaceutically acceptable adjuvant, diluent and/or carrier.

Depending on e.g. potency and physical characteristics of the compound of the invention (i.e. active ingredient), pharmaceutical formulations that may be mentioned include those in which the active ingredient is present in at least 1% (or at least 10%, at least 30% or at least 50%) by weight. That is, the ratio of active ingredient to the other components (i.e. the addition of adjuvant, diluent and carrier) of the pharmaceutical composition is at least 1:99 (or at least 10:90, at least 30:70 or at least 50:50) by weight.

The amount of compound of the invention in the formulation will depend on the severity of the condition, and on the patient, to be treated, as well as the compound(s) which is/are employed, but may be determined non-inventively by the skilled person.

The invention further provides a process for the preparation of a pharmaceutical formulation, as hereinbefore defined, which process comprises bringing into association a compound of the invention, as hereinbefore defined, or a pharmaceutically acceptable ester, amide, solvate or salt thereof with a pharmaceutically-acceptable adjuvant, diluent or carrier.

Compounds of the invention may also be combined with other therapeutic agents that are inhibitors of protein or lipid kinases (e.g. PI3K, such as class I PI3K, and/or mTOR) and/or useful in the treatment of a cancer and/or a proliferative disease. Compounds of the invention may also be combined with other therapies.

According to a further aspect of the invention, there is provided a combination product comprising:
(A) a compound of the invention, as hereinbefore defined; and
(B) another therapeutic agent that is useful in the treatment of cancer and/or a proliferative disease,
wherein each of components (A) and (B) is formulated in admixture with a pharmaceutically-acceptable adjuvant, diluent or carrier.

Such combination products provide for the administration of a compound of the invention in conjunction with the other therapeutic agent, and may thus be presented either as separate formulations, wherein at least one of those formulations comprises a compound of the invention, and at least one comprises the other therapeutic agent, or may be presented (i.e. formulated) as a combined preparation (i.e. presented as a single formulation including a compound of the invention and the other therapeutic agent).

Thus, there is further provided:
(1) a pharmaceutical formulation including a compound of the invention, as hereinbefore defined, another therapeutic agent that is useful in the treatment of cancer and/or a proliferative disease, and a pharmaceutically-acceptable adjuvant, diluent or carrier; and
(2) a kit of parts comprising components:
   (a) a pharmaceutical formulation including a compound of the invention, as hereinbefore defined, in admixture with a pharmaceutically-acceptable adjuvant, diluent or carrier; and
   (b) a pharmaceutical formulation including another therapeutic agent that is useful in the treatment of cancer and/or a proliferative disease in admixture with a pharmaceutically-acceptable adjuvant, diluent or carrier,
      which components (a) and (b) are each provided in a form that is suitable for administration in conjunction with the other.

The invention further provides a process for the preparation of a combination product as hereinbefore defined, which process comprises bringing into association a compound of the invention, as hereinbefore defined, or a pharmaceutically acceptable ester, amide, solvate or salt thereof with the other therapeutic agent that is useful in the treatment of cancer and/or a proliferative disease, and at least one pharmaceutically-acceptable adjuvant, diluent or carrier.

By "bringing into association", we mean that the two components are rendered suitable for administration in conjunction with each other.

Thus, in relation to the process for the preparation of a kit of parts as hereinbefore defined, by bringing the two components "into association with" each other, we include that the two components of the kit of parts may be:
(i) provided as separate formulations (i.e. independently of one another), which are subsequently brought together for use in conjunction with each other in combination therapy; or
(ii) packaged and presented together as separate components of a "combination pack" for use in conjunction with each other in combination therapy.

Depending on the disorder, and the patient, to be treated, as well as the route of administration, compounds of the invention may be administered at varying therapeutically effective doses to a patient in need thereof. However, the dose administered to a mammal, particularly a human, in the context of the present invention should be sufficient to effect a therapeutic response in the mammal over a reasonable timeframe. One skilled in the art will recognize that the selection of the exact dose and composition and the most appropriate delivery regimen will also be influenced by *inter alia* the pharmacological properties of the formulation, the nature and severity of the condition being treated, and the physical condition and mental acuity of the recipient, as well as the potency of the specific compound, the age, condition, body weight, sex and response of the patient to be treated, and the stage/severity of the disease.

Administration may be continuous or intermittent (e.g. by bolus injection). The dosage may also be determined by the timing and frequency of administration. In the case of oral or parenteral administration the dosage can vary from about 0.01 mg to about 1000 mg per day of a compound of the invention.

In any event, the medical practitioner, or other skilled person, will be able to determine routinely the actual dosage, which will be most suitable for an individual patient. The above-mentioned dosages are exemplary of the average case; there can, of course, be individual instances where higher or lower dosage ranges are merited, and such are within the scope of this invention.

Compounds of the invention may have the advantage that they are effective inhibitors of protein or lipid kinases (e.g. PI3K, such as class I PI3K, and/or mTOR).

Compounds of the invention may also have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties over, compounds known in the prior art, whether for use in the above-stated indications or otherwise.

### Examples/Biological Tests

Determination of the activity of PI3 kinase activity of compounds of the invention is possible by a number of direct and indirect detection methods. Certain exemplary compounds described herein were prepared, characterized, and tested for their PI3K binding activity and *in vitro* activity against tumor cells. The range of PI3K binding activities was less than 1 nM to about 10 µM (i.e. certain compounds of the examples/invention had PI3K binding activity IC₅₀ values of less than 10 nM). Compounds of the examples/invention had tumor cell-based activity IC₅₀ values less than 100 nM (see Table 4 below).

### PI3K activity assay

The kinase activity was measured by using the commercial ADP Hunter™ Plus assay available from DiscoveRₓ (#33-016), which is a homogeneous assay to measure the accumulation of ADP, a universal product of kinase activity. The enzyme, PI3K (p110α/p85α was purchased from Carna Biosciences (#07CBS-0402A). The assay was done following the manufacturer recommendations with slight modifications: Mainly the kinase buffer was replace by 50 mM HEPES, pH 7.5, 3 mM MgCl₂, 100 mM NaCl, 1 mM EGTA, 0.04% CHAPS, 2 mM TCEP and 0.01 mg/ml BGG. The PI3K was assayed in a titration experiment to determine the optimal protein concentration for the inhibition assay. To calculate the IC₅₀ of the ETP-compounds, serial 1:5 dilutions of the compounds were added to the enzyme at a fixed concentration (2.5 µg/ml. The enzyme was preincubated with the inhibitor and 30 µM PIP₂ substrate (P9763, Sigma) for 5 min and then ATP was added to a final 50 µM concentration. Reaction was carried out for 1 hour at 25°C. Reagent A and B were sequentially added to the wells and plates were incubated for 30 min at 37 °C. Fluorescence counts were read in a Victor instrument (Perkin Elmer) with the recommended settings (544 and 580 nm as excitation and emission wavelengths, respectively). Values were normalized against the control activity included for each enzyme (i.e., 100 % PI3 kinase activity, without compound). These values were plot against the inhibitor concentration and were fit to a sigmoid dose-response curve by using the Graphad software.

### Cellular Mode of Action

**Cell culture:** The cell lines were obtained from the American Type Culture Collection (ATCC). U2OS (human osteosarcoma) was cultured in Dulbecco's modified Eagle's medium (DMEM). PC3 (human prostate carcinoma), MCF7 (human breast cardinoma), HCT116 (human colon carcinoma), 768-0 (human neuroblastoma), U251 (human glyoblastoma) were grown in RPMI. All media were supplemented with 10% fetal bovine serum (FBS) (Sigma) and antibiotics-antimycotics. Cell were maintained in a humidified incubator at 37°C with 5% CO₂ and passaged when confluent using trypsin/EDTA.

**U2foxRELOC and U2nesRELOC assay:** The U2nesRELOC assay and the U2foxRELOC assay have been described previously (1, 2). Briefly, cells were seeded at a density of 1.0×10⁵ cells/ml into black-wall clear-bottom 96-well microplates (BD Biosciences) After incubation at 37°C with 5% CO₂ for 12 hours, 2µl of each test compound were transferred from the mother plates to the assay plates. Cells were incubated in the presence of the compounds for one hour. Then cells were fixed and the nucleus stained with DAPI (Invitrogen). Finally the plates were washed with 1X PBS twice and stored at 4°C before analysis. Compounds of the invention have a range of *in vitro* cell potency activities from about 1 nM to about 10 µM.

**Image acquirement and processing:** Assay plates were read on the BD Pathway™ 855 Bioimager equipped with a 488/10 nm EGFP excitation filter, a 380/10 nm DAPI excitation filter, a 515LP nm EGFP emission filter and a 435LP nm DAPI emission filter. Images were acquired in the DAPI and GFP channels of each well using 10x dry objective. The plates were exposed 0.066 ms (Gain 31) to acquire DAPI images and 0.55 ms (Gain 30) for GFP images.

**Data analysis:** The BD Pathway Bioimager outputs its data in standard text files. Data were imported into the data analysis software BD Image Data Explorer. The nuclear/cytoplasmic (Nuc/Cyt) ratios of fluorescence intensity were determined by dividing the fluorescence intensity of the nucleus by the cytoplasmic. A threshold ratio of greater than 1.8 was employed to define nuclear accumulation of fluorescent signal for each cell. Based on this procedure we calculated the percentage of cells per well displaying nuclear translocation or inhibition of nuclear export. Compounds that induced a nuclear accumulation of the fluorescent signal greater than 60% of that obtained from wells treated with 4nM LMB were considered as hits. In order to estimate the quality of the HCS assay, the Z' factor was calculated by the equation: Z' = 1 - [(3 × std. dev. of positive controls) + (3 × std. dev. of negative controls) / (mean of positive controls) - (mean of negative controls)].

### PI3K signalling

**AKT phosphorylation Inhibition.Western Blot Analysis:** Subconfluent cells were incubated under different conditions and washed twice with TBS prior to lysis. Lysis buffer was added containing 50 mM Tris HCl, 150 mM NaCl, 1% NP-40, 2mM Na₃VO₄, 100 mM NaF, 20 mM Na₄P₂O₇ and protease inhibitor cocktail (Roche Molecular Biochemicals). The proteins were resolved on 10% SDS-PAGE and transferred to nitrocellulose membrane (Schleicher & Schuell, Dassel, Germany). The membranes were incubated overnight at 4°C with antibodies specific for Akt, phospho-Ser-473-Akt (Cell Signaling Technology) and α-tubulin (Sigma), they were washed and then incubated with IRDye800 conjugated anti-mouse and Alexa Fluor 680 goat anti-rabbit IgG secondary antibodies. The bands were visualized using an Odyssey infrared imaging system (Li-Cor Biosciences). Compounds of the invention have a range of *in vitro* cell potency activities from about 1 nM to about 10 µM.

### Cytotoxicity assessment

The compounds were tested on 96-well trays. Cells growing in a flask were harvested just before they became confluent, counted using a haemocytometer and diluted down with media adjusting the concentration to the required number of cells per 0.2 ml (volume for each well). Cells were then seeded in 96-well trays at a density between 1000 and 4000 cells/well, depending of the cell size. Cells were left to plate down and grow for 24 hours before adding the drugs. Drugs were weighed out and diluted with DMSO to get them into solution to a concentration of 10mM. From here a "mother plate" with serial dilutions was prepared at 200X the final concentration in the culture. The final concentration of DMSO in the tissue culture media should not exceed 0.5%. The appropriate volume of the compound solution (usually 2 microlitres) was added automatically (Beckman FX 96 tip) to media to make it up to the final concentration for each drug. The medium was removed from the cells and replaced with 0.2 ml of medium dosed with drug. Each concentration was assayed in triplicate. Two sets of control wells were left on each plate, containing either medium without drug or medium with the same concentration of DMSO. A third control set was obtained with the cells untreated just before adding the drugs (seeding control, number of cells starting the culture). Cells were exposed to the drugs for 72 hours and then processed for MTT colorimetric read-out. Compounds of the invention have a range of *in vitro* cell potency activities from about 1 nM to about 10 µM.

### PI3K cellular activity, ELISA

Activity was measured as endogenous levels of phospho-AKT (Ser473) protein. Osteosarcoma U2OS cells were plated in 96 Poly-D-Lysine coating tissue culture plates (18.000 cells/well). After the treatment with serial dilutions of the compound during 3h, the cells were fixed directly in the wells with 4% paraformaldehide. After fixing, individual wells went through the same series of steps used for a conventional immunoblot: including blocking with 5% BSA, incubation with 1/1000 of primary antibody-AKT (Ser 74) in PBS containing 5% BSA at 4°C overnight (Cell Signalling), washing and incubation with second antibody HRP-anti-mouse IgG for 1h at RT (Amersham). After the addition of SuperSignal ELISA Femto maximum sensitivity chemiluminescent substrate (Pierce) the results were read in a luminescence plate reader (Victor).

### mTOR assay

Mammalian target of rapamycin (mTOR) was assayed by monitoring phosphorylation of GFP-4EBP using a homogeneous time-resolved fluorescence resonante energy transfer format and assay reagents from Invitrogen. In the presence of 10 µM ATP, 50 mM Hepes (pH 7.5), 0.01 % (v/v) Polysorbate 20, 10 mM MnCl₂, 1mM EGTA, and 2.5 mM DTT, the mTOR-mediated phosphorylation of 200 nM GFP-4E-BP1 was measured under initial rate conditions. After incubation at room temperature for 60 min, the reaction was terminated by addition of 10 mM EDTA, and phosphorylated GFP-4E-BP1 was detected with 2 nM Tb-anti-p4E-BP1 antibody before reading on a Perkin-Elmer Wallac 1420 Fluorescence Reader (exc 340; em 490/520).

Where compound names are given herein, they are typically generated with ChemDraw.

The invention is illustrated by way of the following examples, in which the following abbreviations (or chemical symbols) may be employed:
"dba" dibenzylidene acetone; "DCM" dichloromethane; "MeOH" methanol; "EtOH" ethanol; "THF" tetrahydrofuran; "DMF" dimethylformamide; "CHCl₃" chloroform; "DME" dimethoxyethane; "Et₂O" diethyl ether; "Hex" hexane; "EtOAc" ethyl acetate; "Pd(PPh₃)₄" tetrakis(triphenylphosphine)palladium; "KOAc" potassium acetate; "DIPEA" diisopropylethylamine; "Pd(PPh₃)₄" tetrakis(triphenylphosphine)-palladium; "Pd(dppf)Cl₂.DCM" 1,1'-bis(diphenylphosphino)ferrocenepalladium(II) dichloride, dichloromethane; "min." minutes; and "h." hours.

### Examples and Experimental

**Table 1 - Pyrazine Intermediates**

| | | | |
|---|---|---|---|
| | 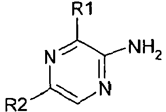 | | |

| **No.** | **Exp. Meth.** | **--R1** | **--R2** |
|---|---|---|---|
| **I-01** | **A-1** | --Br | --Br |
| **I-02** | **A-2** | 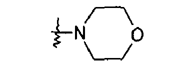 | --Br |

**Table 2 - Intermediates**

| | | | | |
|---|---|---|---|---|
| | 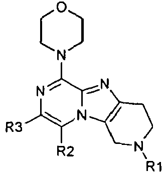 | | | |

| **No.** | **Exp. Meth.** | **--R1** | **--R2** | **--R3** |
|---|---|---|---|---|
| **I-03** | **A3** | --CO₂Et | --H | --Br |

**Table 3 -Final Products**

| | | | | |
|---|---|---|---|---|
| 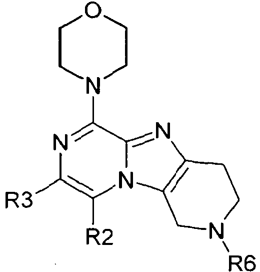 | | | | |

| **No.** | **Exp.** | --R6 | --R2 | --R3 |
|---|---|---|---|---|
| **2-01** | **B1** | --CO₂Et | --H | 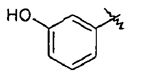 |
| **2-02** | **B2** | --H | --H | 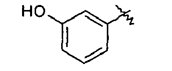 |
| **2-03** | **B1** | --CO₂Et | --H | 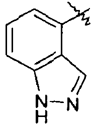 |
| **2-04** | **B2** | --H | --H | 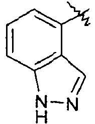 |
| **2-05** | **B3** | 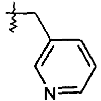 | --H | 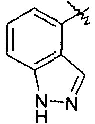 |
| **2-06** | **B3** | 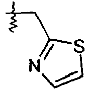 | --H | 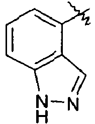 |
| **2-07** | **B3** | 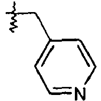 | --H | 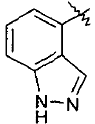 |
| **2-08** | **B3** | 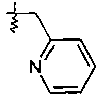 | --H | 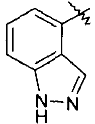 |
| **2-09** | **B3** | 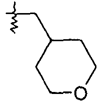 | --H | 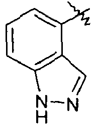 |
| **2-10** | **B3** | 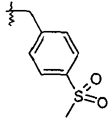 | --H | 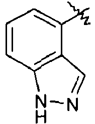 |
| **2-11** | **B3** | 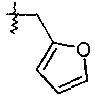 | --H | 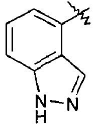 |
| **2-12** | **B3** | 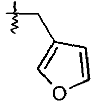 | --H | 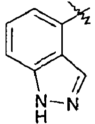 |
| **2-13** | **B3** | 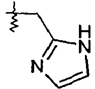 | --H | 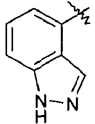 |
| **2-14** | **B3** | 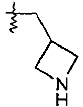 | --H | 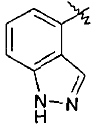 |
| **2-15** | **B3** | 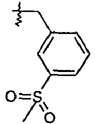 | --H | 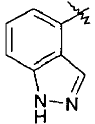 |
| **2-16** | **B1** | --CO₂Et | --H | 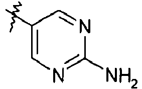 |
| **2-17** | **B2** | --H | --H | 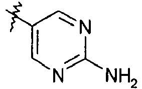 |
| **2-18** | **B3** | 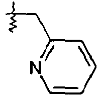 | --H | 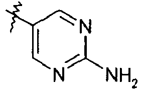 |
| **2-19** | **B1** | --CO₂Et | --H | 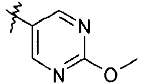 |
| **2-20** | **B1** | --CO₂Et | --H | 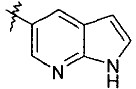 |
| **2-21** | **B1** | --CO₂Et | --H | 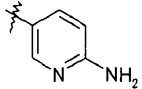 |
| **2-22** | **B1** | --CO₂Et | --H | 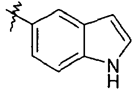 |
| **2-23** | **B1** | --CO₂Et | --H | 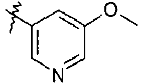 |
| **2-24** | **B1** | --CO₂Et | --H | 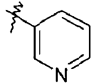 |
| **2-25** | **B1** | --CO₂Et | --H | 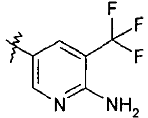 |
| **2-26** | **B1** | --CO₂Et | --H | 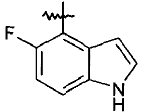 |
| **2-27** | **B4** | --CO₂Et | --Cl | 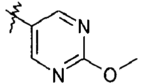 |
| **2-28** | **B4** | --CO₂Et | --Cl | 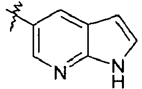 |
| **2-29** | **B4** | --CO₂Et | --Cl | 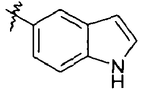 |
| **2-30** | **B4** | --CO₂Et | --Cl | 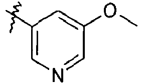 |
| **2-31** | **B4** | --CO₂Et | --Cl | 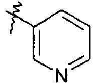 |
| **2-32** | **B4** | --CO₂Et | --Cl | 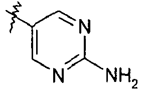 |
| **2-33** | **B4** | --CO₂Et | --Cl | 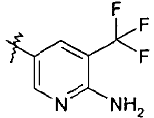 |
| **2-34** | **B4** | --H | --Cl | 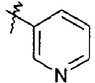 |
| **2-35** | **B3** | 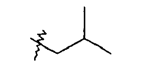 | --H | 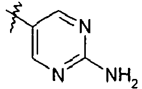 |
| **2-36** | **B3** | 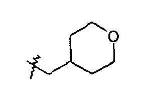 | --H | 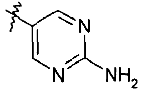 |
| **2-37** | **B3** | 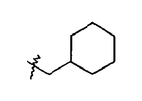 | --H | 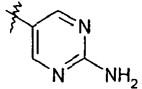 |
| **2-38** | **B5** | 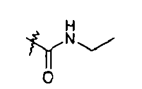 | --H | 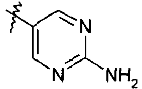 |
| **2-39** | **B6** | --SO₂Me | --H | 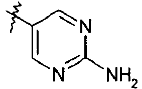 |
| **2-40** | **B3** | 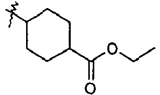 | --H | 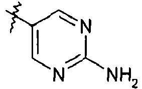 |
| **2-41** | **B1** | --CO₂Et | --H | 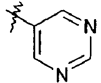 |
| **2-42** | **B7** | 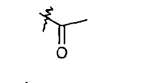 | --H | 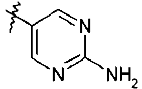 |
| **2-43** | **B7** | 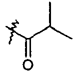 | --H | 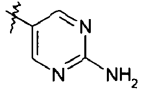 |
| **2-44** | **B5** | 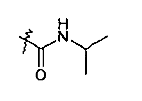 | --H | 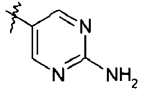 |
| **2-45** | **B3** | 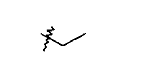 | --H | 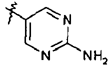 |
| **2-46** | **B3** | 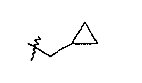 | --H | 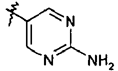 |
| **2-47** | **B3** | 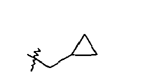 | --H | 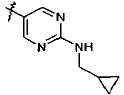 |
| **2-48** | **B8** | --CO2Me | --H | 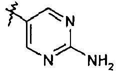 |
| **2-49** | **B4** | --SO2Me | --Cl | 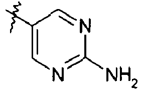 |
| **2-50** | **B6** | 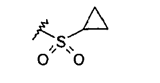 | --H | 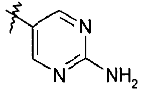 |
| **2-51** | **B7** | 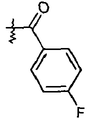 | --H | 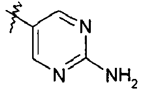 |
| **2-52** | **B6** | 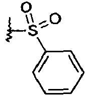 | --H | 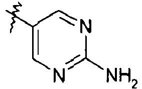 |
| **2-53** | **B5** | 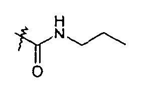 | --H | 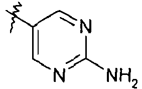 |
| **2-54** | **B4** | 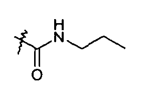 | --Cl | 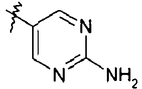 |
| **2-55** | **B6** | 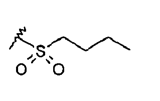 | --H | 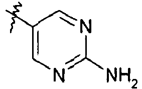 |
| **2-56** | **B6** | 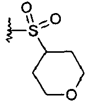 | --H | 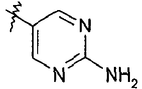 |
| **2-57** | **B4** | 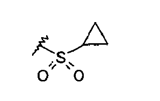 | --Cl | 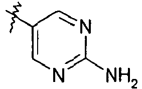 |
| **2-58** | **B4** | 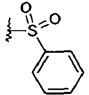 | --Cl | 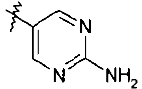 |
| **2-59** | **B6** | 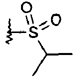 | --H | 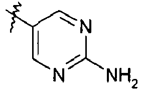 |
| **2-60** | **B6** | 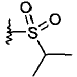 | --Cl | 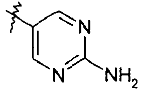 |
| **2-61** | **B6** | 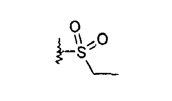 | --Cl | 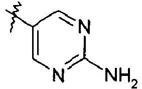 |
| **2-62** | **B3** | 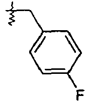 | --H | 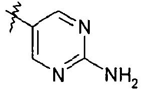 |
| **2-63** | **B3** | 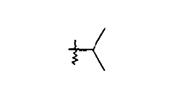 | --H | 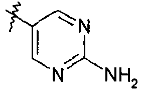 |
| **2-64** | **B6** | 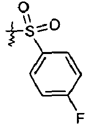 | --H | 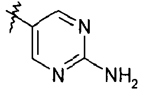 |
| **2-65** | **B5** | 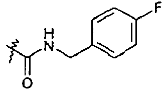 | --H | 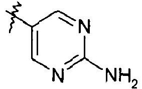 |
| **2-66** | **B5** | 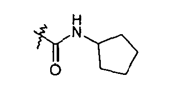 | --H | 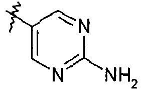 |
| **2-67** | **B5** | 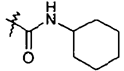 | --H | 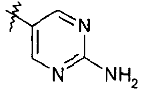 |
| **2-68** | **B5** | 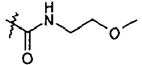 | --H | 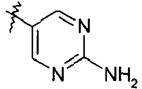 |
| **2-69** | **B6** | 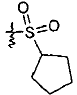 | --H | 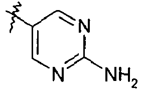 |
| **2-70** | **B5** | 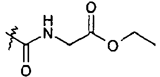 | --H | 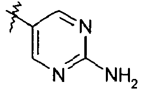 |
| **2-71** | **B5** | 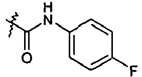 | --H | 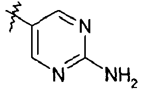 |
| **2-72** | **B5** | 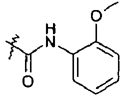 | --H | 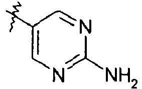 |
| **2-73** | **B6** | 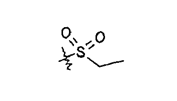 | --H | 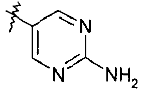 |
| **2-74** | **B5** | 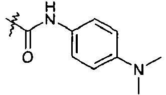 | --H | 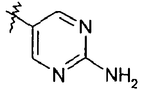 |
| **2-75** | **B5** | 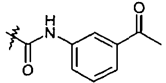 | --H | 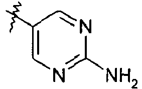 |
| **2-76** | **B7** | 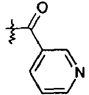 | --H | 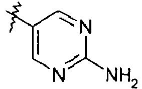 |
| **2-77** | **B6** | 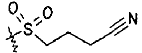 | --H | 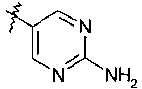 |
| **2-78** | **B6** | 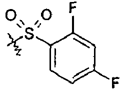 | --H | 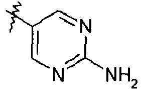 |
| **2-79** | **B5** | 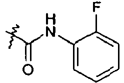 | --H | 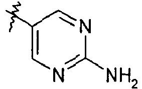 |
| **2-80** | **B3** | 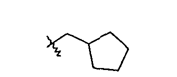 | --H | 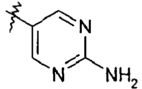 |
| **2-81** | **B3** | 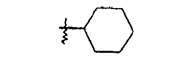 | --H | 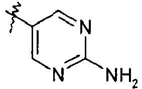 |
| **2-82** | **B3** | 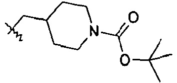 | --H | 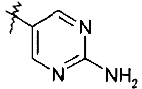 |
| **2-83** | **B5** | 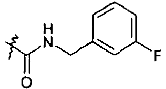 | --H | 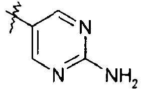 |
| **2-84** | **B3** | 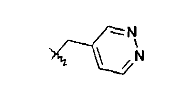 | --H | 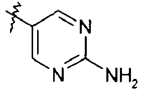 |
| **2-85** | **B6** | 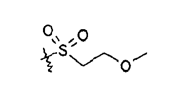 | --H | 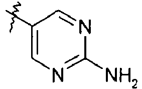 |
| **2-86** | **B7** | 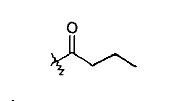 | --H | 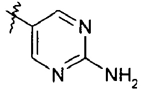 |
| **2-87** | **B7** | 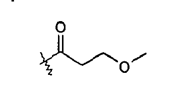 | --H | 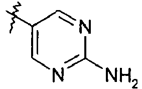 |
| **2-88** | **B3** | 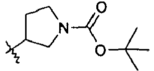 | --H | 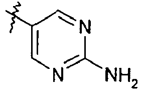 |
| **2-89** | **B9** | 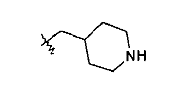 | --H | 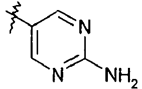 |
| **2-90** | **B5** | 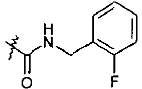 | --H | 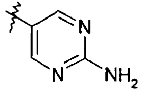 |
| **2-91** | **B5** | 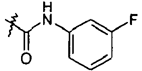 | --H | 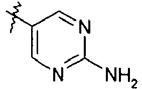 |
| **2-92** | **B3** | 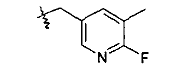 | --H | 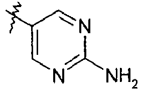 |
| **2-93** | **B5** | 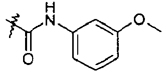 | --H | 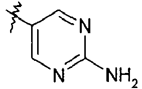 |
| **2-94** | **B5** | 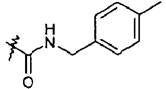 | --H | 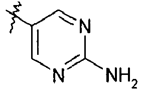 |
| **2-95** | **B3** | 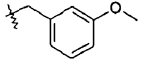 | --H | 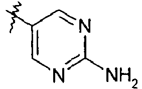 |
| **2-96** | **B5** | 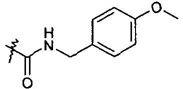 | --H | 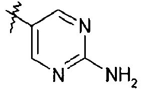 |
| **2-97** | **B5** | 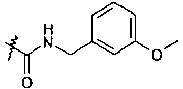 | --H | 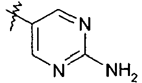 |
| **2-98** | **B5** | 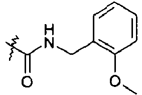 | --H | 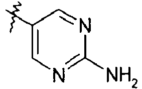 |
| **2-99** | **B3** | 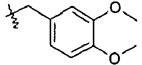 | --H | 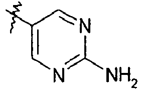 |
| **2-100** | **B3** | 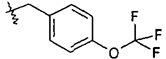 | --H | 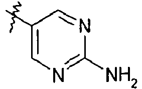 |
| **2-101** | **B5** | 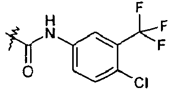 | --H | 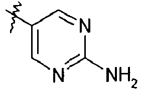 |
| **2-102** | **B5** | 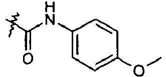 | --H | 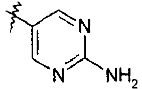 |
| **2-103** | **B9** | 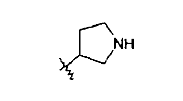 | --H | 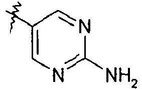 |
| **2-104** | **B3** | 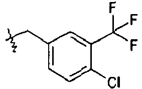 | --H | 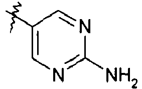 |
| **2-105** | **B5** | 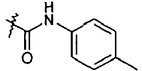 | --H | 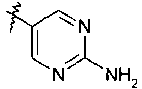 |
| **2-106** | **B3** | 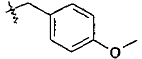 | --H | 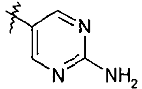 |
| **2-107** | **B3** | 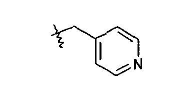 | --H | 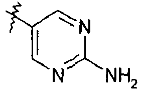 |
| **2-108** | **B3** | 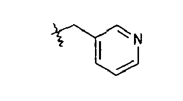 | --H | 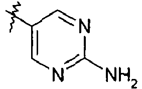 |
| **2-109** | **B6** | 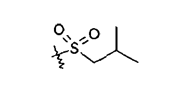 | --H | 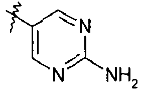 |
| **2-110** | **B9** | 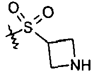 | --H | 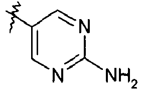 |
| **2-111** | **B3** | 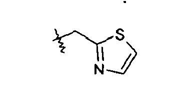 | --H | 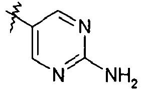 |
| **2-112** | **B5** | 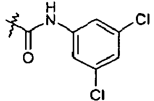 | --H | 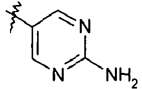 |
| **2-113** | **B5** | 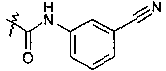 | --H | 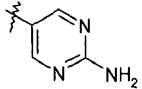 |
| **2-114** | **B3** | 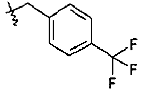 | --H | 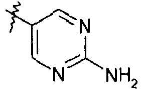 |
| **2-115** | **B3** | 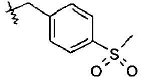 | --H | 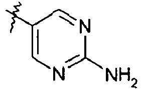 |
| **2-116** | **B3** | 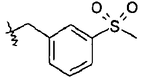 | --H | 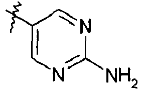 |
| **2-117** | **B3** | 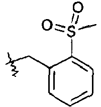 | --H | 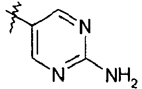 |

### Experimental part

### Preparation of the intermediates

### Method A1

### Preparation of intermediate I-01.

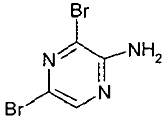

To a mixture of 2-amino pyrazine (50 g, 0.5 mol) in chloroform (1000 ml) cooled to 0°C was added pyridine (100 ml, 1.21 mol) and bromine (54 ml, 1.05 mmol) dropwise. The mixture was stirred at rt for 16h, then water was added. The organic phase was extracted, dried (MgSO₄), filtered and evaporated to obtain I-01, 48 g (Y: 36 %) of a yellow solid which was dried in vacuo.

### Method A2

### Preparation of intermediate I-02.

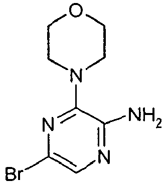

A solution of intermediate I-01 (15 g, 59.3 mmol) in morpholine (15 ml, 178 mmol) was heated at 120°C in a Parr reactor for 48h. A brown solid appears. The solid was suspended in DCM and washed with NaHCO₃ aq. sat (twice). The organic phase was dried (NaSO₄), filtered and evaporated to dryness to obtain I-02, 14.8 g of a brown solid (Y: 96 %).

### Method A3

### Preparation of intermediate I-03

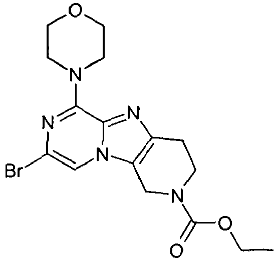

Three batches of 5-bromo-3-morpholin-4-yl-pyrazin-2-ylamine (I-02, 2 g, 7.7 mmol), ethyl-3-bromo-4-oxo-piperidine-1-carboxylate (2.4 g, 10 mmol) and small amount of DME (~ 0.3 mL) were heated in parallel at 120 °C for 4 h, until the reactants were consumed as determined by LCMS analysis. The three reaction mixtures were mixed and diluted with DCM (100 mL), washed with saturated aqueous solution of NaHCO₃ (2 x 200 mL) and brine (250 mL), dried over Na₂SO₄ and concentrated in vacuo rendering a dark residue. The black residue was purified in two portions by Biotage flash column chromatography (cartridge 40M) eluting with a solvent system of EtOAc/cyclohexane (30%-75% of EtOAc), yielding the required intermediate I-03 (2 g, 21%) as cream solid

### Preparation of final products

### Method B1

### Preparation of final product 2-01

A mixture of intermediate I-03 (240 mg, 0.585 mmol), 3-hydroxyphenylboronic acid (178 mg, 1.287 mmol), PdCl₂(dppf)·DCM (48 mg. 0.058 mmol) in DME (2 mL) and K₂CO₃ (0.9 mL of aqueous saturated solution, 1.5 mL/mmol) was heated at 130 °C under microwave irradiation for 10 min. The reaction mixture was cooled and filtered through a Celite pad washing with DCM. The filtrate was dried over Na₂SO₄ and concentrated under vacuum. The residue was purifired by flash chromatography (Isolute Si II 10 g) eluting with a gradient of MeOH/DCM from 0% to 10% of MeOH, yielding the required product but still with inpurities and it was precipitated from EtOAc/cyclohexane, affording the final product 2-01 (36 mg, 3-(3-Hydroxy-phenyl)-1-morpholin-4-yl-7,8-dihydro-5H-2,4a,6,9-tetraaza-fluorene-6-carboxylic acid ethyl ester, Y: 14%).

### Preparation of final product 2-03

A mixture of intermediate I-03 (300 mg, 0.731 mmol), indazole-4-boronic acid hydrochloride (220 mg, 1.1 mmol), PdCl₂(dppf)·DCM (6 mg. 0.007 mmol), aqueous saturated solution of Na₂CO₃ (1.1 mL, using 1.5 mL/mmol) in DME (5 mL) was heated at 130 °C under microwave irradiation for 10 min. The reaction mixture was diluted with DCM (50 mL), washed with saturated solution of NaHCO₃ (2 x 60 mL), brine (70 mL), dried over Na₂SO₄ and concentrated, yielding the crude product that was precipitated from EtOAc/cyclohexane, affording the required product (120 mg). It was further purified by flash column chromatography eluting with a gradient system of MeOH/DCM (from 0% to 4% of MeOH) yielding the final product 2-03 (3-(1H-Indazol-4-yl)-1-morpholin-4-yl-7,8-dihydro-5H-2,4a,6,9-tetraaza-fluorene-6-carboxylic acid ethyl ester, 30 mg, Y: 9%).

### Preparation of final product 2-16

A mixture of intermediate I-03 (500 mg, 1.219 mmol), 2-aminopyrimidine-5-boronic acid, pinacol ester (300 mg, 1.34 mmol), PdCl₂(dppf)·DCM (10 mg, 0.012 mmol), aqueous saturated solution of K₂CO₃ (2 mL) in DME (3 mL) was heated at 130 °C under microwave irradiation for 10 min. The reaction mixture was diluted with DCM (30 mL), washed with saturated solution of NaHCO₃ (2 x 30 mL), brine (30 mL), dried over Na₂SO₄ and concentrated, yielding the crude product. Part of it was used in next steps without further purification and another portion (50mg) was purified by flash column chromatography (Isolute Si II 10 g) eluting with a gradient solvent system of DCM/ MeOH (0% to 5% of MeOH) yielding a light brown solid that was triturated with MeOH and Et₂O leaving a white solid that was filtrated and rinsed with more MeOH, yielding the final product 2-16 (3-(2-aminopyrimidin-5-yl)-1-morpholin-4-yl-7,8-dihydro-5H-2,4a,6,9-tetraaza-fluorene-6-carboxylic acid ethyl ester, 18 mg).

### Method B2

### Preparation of final product 2-04

A reaction mixture composed of final product 2-03 (800 mg, 1.8 mmol), LiOH (900 mg, 21.4 mmol) in iPrOH/MeOH (1:1, 8 mL) was heated under microwave irradiation for 60 min at 160 °C. The reaction mixture was concentrated under high vacuum and the residue was redissolved in DCM (150 mL), washed with saturated solution of NaHCO₃ (2 x 100 mL), brine (100 mL), dried over Na₂SO₄ and concentrated, yielding the final product 2-04 (370 mg, 54%). A small part (50 mg) was purified by flash column chromatography (Isolute Si II 10 g) eluting with MeOH (7M of NH₃)/DCM (from 0% to 5%), yielding a light yellow solid that was repurified by semipreparative HPLC.

### Preparation of final product 2-17

Two reaction mixtures of final product 2-16 (650 mg, 1.53 mmol), LiOH (1.2 g, 27.6 mmol), i-PrOH/MeOH (1:1, 8 mL) and 0.5 mL of water each, were heated under microwave irradiation for 1 h and 30 min at 170°C. The two combined reaction mixtures were filtered off and the filtrate concentrated to yield a solid that was purified by flash column chromatography (Isolute Si II) eluting with a solvent system of MeOH/DCM (5% of MeOH) and then MeOH (7 M of NH3)/DCM (from 5% to 10% of MeOH), yielding the final product 2-17(650 mg, Y: 60%).

### Method B3

### Preparation of final product 2-12

A mixture of final product 2-04 (115 mg, 0.30 mmol), AcOH (25 µL, 0.36 mmol), 3-furaldehyde (30 µL, 0.33 mmol) in DCE (6 mL) was stirred at RT for 40 min. NaBH(OAc)₃ (100 mg, 0.43 mmol) was added and the resulting mixture was stirred for 3 h. The reaction mixture was quenched by adding 2N aqueous solution of KOH and extracted with EtOAc (2 x 25 mL). The combined organic layers were washed with brine (30 mL), dried over Na₂SO₄ and concentrated in vacuo. The crude product was purified by flash column chromatography (Isolute Si II, 10 g) eluting with a gradient of DCM/cyclohexane/MeOH (7N in NH₃) (25% of cyclohexane, from 0% to 10% of MeOH) and then repurified by preparative HPLC, yielding the final product 2-12 (30 mg, Y: 22%).

### Preparation of final product 2-36

Tetrahydro-pyran-4-carbaldehyde (32 µL, 0.284 mmol) and then NaCNBH₃ (18 mg, 0.284mmol) were added at RT to a suspension of final product 2-17 (50 mg, 0.142 mmol) in dry MeOH (1 mL). AcOH (26 µL, 0.426 mmol) was added and the mixture was stirred for 16h at RT. After evaporation of MeOH the residue was taken in DCM/MeOH. The white solid was collected affording pure final product 2-36 (32 mg, Y: 50%).

### Preparation of final product 2-63

A mixture of final product 2-17 (75 mg, 0.213 mmol), acetone (0.017 mL, 0.234 mmol) and AcOH (0.03 mL, 0.426 mmol) in DCE (3 mL) was stirred at rt for 1 h. NaBH(OAc)₃ (68 mg, 0.319 mmol) was added and the reaction mixture was stirred at rt for 80 h. A second addition of acetone (0.017 mL), NaBH(OAc)₃ (68 mg) and AcOH (0.03 mL) was done and the mixture was stirred at rt for 3 h and heated at 40°C for 3 days. KOH (2N) was added to the reaction mixture and it was extracted with DCM. The organic layer was washed with H₂O, dried (Na₂SO₄), filtered and evaporated. The residue was purified by column chromatography (Isolute/Flash, Sill, MeOH:DCM, 0:100 to 10:90) to give the desired final product 2-63 (50 mg, 60%) as a yellow solid.

### Method B4

### Preparation of final product 2-32

Final product 2-16 (30 mg, 0.071 mmol), NCS (9 mg, 0.071 mmol) in DCM (2 mL) was stirred at rt for 2 h (completion of reaction monitored by LC-MS). The mixture was diluted with DCM (30 mL), washed with saturated aqueous NaHCO₃ (3 x 30 mL), brine (40 mL), dried over Na₂SO₄ and concentrated under vacuum to render the crude product as a white solid. It was triturated with cyclohexane, filtered and washed with cyclohexane affording the pure final product 2-32 as a white solid (15 mg, Y: 46%).

### Method B5

### Preparation of final product 2-38

The final product 2-17 (50 mg, 0.142mmol) was suspended in dry acetonitrile (1.5 ml) and treated with ethyl isocyanate (13 µL, 0.170 mmol) and N,N-diisopropylethylamine (30 µL, 0.170 mmol). The reaction mixture was stirred at room temperature. After 2 hours the progress of the reaction was monitored by LC-MS. The solid formed was filtered off, washed with acetonitrile, diethylether and dried to afford pure final compound 2-38 (white solid, 42 mg, Y: 70%).

### Preparation of final product 2-68

A mixture of final product 2-17 (40 mg, 0.114 mmol), 1-isocyanato-2-methoxyethane (14 mg, 0.136 mmol) and DIPEA (0.024 mL, 0.136 mmol) in MeCN (1.3 mL) was stirred at rt for 16 h. More DIPEA (2 drops) and 1-isocyanato-2-methoxyethane (2 drops) were added and the reaction mixture was stirred at rt for 24 h. The solvent was evaporated and the residue was purified by column chromatography (Isolute/Flash, Sill, MeOH:DCM, 0:100 to 10:90) to give the desired final product 2-68 (29.82 mg, 58%) as a yellow solid.

### Preparation of final product 2-74

A mixture of Final Product 2-17 (40 mg, 0.114 mmol), 4-dimethylaminophenyl isocyanate (22 mg, 0.136 mmol) and DIPEA (0.024 mL, 0.136 mmol) in MeCN (1.3 mL) was stirred at rt for 16 h. The same amount of reagents was added and the mixture was stirred at rt for 3 days and then heated at 40°C for 20 h. The solvent was evaporated and the residue was purified by column chromatography (Isolute/Flash, Sill, MeOH:DCM, 0:100 to 10:90) to give the desired final product 2-74 (30 mg, 51%) as a beige solid.

### Preparation of final product 2-79

A mixture of Final Product 2-17 (40 mg, 0.114 mmol), 2-fluorophenyl isocyanate (0.015 mL, 0.136 mmol) and DIPEA (0.024 mL, 0.136 mmol) in MeCN (1.3 mL) was stirred at rt for 16 h. 2-Fluorophenyl isocyanate (0.015 mL) and DIPEA (0.024 mL) were added and the mixture was stirred at rt for 27 h and heated at 70°C for 26 h. More 2-fluorophenyl isocyanate (0.015 mL) and DIPEA (0.024 mL) were added and the reaction mixture was heated at 70°C for 26 h and under microwave irradiation at 130°C for 30 min. The solvent was evaporated and the residue was purified by column chromatography (Isolute/Flash, Sill, MeOH:DCM, 0:100 to 10:90) to give the desired final product 2-79 (20 mg, 36%) as a pale yellow solid.

### Method B6

### Preparation of final product 2-39

A mixture of final product 2-17 (60 mg, 0.17 mmol), methanesulfonyl chloride (20 µL, 0.25 mmol) and triethylamine (50 µL, 0.34 mmol) in acetonitrile was stirred at rt for 24 h until the reactants were consumed as determined by LCMS analysis. The mixture was concentrated in vacuo and the residue was redissolved in CHCl₃/iPrOH (1:1), washed with saturated aqueous solution of NaHCO₃ (2 x 40 mL), brine (50 mL), dried over Na₂SO₄ and concentrated in vacuo to render the crude product. It was purified by Biotage flash chromatography (25-S) eluting with a gradient of MeOH/DCM (from 0% to 15% of MeOH), yielding pure final product 2-39 (9 mg, Y: 12%)

### Preparation of final product 2-50

A mixture of final product 2-17 (100 mg, 0.284 mmol), cyclopropanesulfonyl chloride (120 mg, 0.851 mmol) and Cs₂CO₃ (370 mg, 1.135 mmol) in MeCN (4 mL) was heated in a sealed tube at 90°C for 48 h. More Cs₂CO₃ (200 mg), cyclopropanesulfonyl chloride (100 mg) and MeCN (1 mL) were added to the reaction mixture and it was heated at 90°C for 24 h and at RT for 3 days. More Cs₂CO₃ (200 mg) and cyclopropanesulfonyl chloride (0.1 g9 were added and the mixture was heated at 90°C for 24 h. The solvent was evaporated and the residue was purified by column chromatography (Isolute/Flash, Si II, MeOH:DCM, 0:100 to 2:98) to give the final product 2-50 (37 mg, 31%).

### Preparation of final product 2-52

A mixture of final product 2-17 (50 mg, 0.142 mmol), benzenesulfonyl chloride (0.072 mL, 0.568 mmol) and Cs₂CO₃ (277 mg, 0.851 mmol) in MeCN (3 mL) was heated under microwave irradiation at 130°C for 30 min. The solvent was evaporated and the residue was purified by column chromatography (Isolute/Flash, Sill, MeOH:DCM, 0:100 to 10:90) to give the final product 2-52 (60 mg, 86%).

### Preparation of final product 2-77

A mixture of Final Product 2-17 (40 mg, 0.114 mmol), 3-cyanopropane-1-sulfonyl chloride (76 mg, 0.454 mmol) and Cs₂CO₃ (222 mg, 0.681 mmol) in MeCN (2.4 mL) was heated under microwave irradiation at 130°C for 2 h. A second addition of reagents [Cs₂CO₃ (100 mg) and 3-cyanopropane-1-sulfonyl chloride (76 mg)] was done and the mixture was heated under microwave irradiation at 140°C for 2 h and at 150°C for 1 h. The solvent was evaporated and the residue was purified by column chromatography (Isolute/Flash, Sill, MeOH:DCM, 0:100 to 10:90) to give the Final Product 2-77 (18 mg, 33%) as a pale yellow solid.

### Method B7

### Preparation of final product 2-43

The final product 2-17 (50 mg, 0.142 mmol) was suspended in dry acetonitrile (1.4 ml) and treated with acetyl chloride (0.012 mL, 0.170 mmol) and N,N-diisopropylethylamine (0.030 mL, 0.170 mmol). The reaction mixture was stirred at room temperature for 16h. The white solid was filtered off, washed with acetonitrile, diethylether and dried to afford the crude product as white solid (64 mg). It was purified by flash column cromatography (twice) using DCM/MeOH 10:1 as eluent, rendering the pure final product 2-43 (white solid, 12 mg, Y: 21%).

### Preparation of final product 2-51

A mixture of final product 2-17 (75 mg, 0.213 mmol), 4-fluorobenzoyl chloride (40 mg, 0.255 mmol) and N,N-diisopropylethylamine (0.044 mL, 0.255 mmol) in DCM (2.5 mL) was stirred at rt for 16 h. The reaction mixture was diluted with DCM and washed with a saturated NaHCO₃ and brine. The organic layer was separated, dried (Na₂SO₄), filtered and concentrated. The residue was purified by automated column chromatography (Biotage, MeOH:DCM, 0:100 to 20:80) to give the final product 2-51 (62 mg, 61%).

### Preparation of final product 2-76

A mixture of Final Product 2-17 (75 mg, 0.213 mmol), nicotinoyl chloride hydrochloride (45 mg, 0.255 mmol) and DIPEA (0.074 mL, 0.426 mmol) in MeCN (2.5 mL) was stirred at rt for 24 h. More DIPEA (0.1 mL) and nicotinoyl chloride hydrochloride (40 mg) were added and the reaction mixture was stirred for 16 h. The mixture was diluted with DCM and washed with a saturated NaHCO₃ and brine. The organic layer was separated, dried (Na₂SO₄), filtered and concentrated. The residue was purified by column chromatography (Isolute/Flash, Sill, MeOH:DCM, 0:100 to 20:80) (twice) to give the desired Final Product 2-76 (30 mg, 31%) as a yellow solid.

### Preparation of final product 2-86

A mixture of Final Product 2-17 (40 mg, 0.114 mmol), butyryl chloride (0.014 mL, 0.136 mmol) and DIPEA (0.024 mL, 0.136 mmol) in MeCN (1.3 mL) was stirred at rt for 26 h and heated at 70°C for 46 h. Butyryl chloride (0.014 mL) and DIPEA (0.024 mL) were added and the mixture was heated at 70°C for 26 h. The solvent was evaporated and the residue was purified by column chromatography (Isolute/Flash, Sill, MeOH:DCM, 0:100 to 10:90) to give the Final Product (11 mg, 23%) as a pale yellow solid.

### Method B8

### Preparation of final product 2-48

To a suspension of the final product 2-16 (100 mg, 0.236 mmol) in MeOH:dioxane (1:1, 3 mL) was added NaH (17 mg, 0.71 mmol). The mixture was heated under microwave irradiation at 100 °C for 45 min. On cooling, sat. NaHCO₃ was carefully added and the mixture was extracted with iPrOH:CHCl₃ (1:1, 3 x 25 mL). The combined organic layers were washed with brine (40 mL), dried over Na₂SO₄, filtered and evaporated. The residue was purified by automated chromatography (Biotage, eluent: MeOH in DCM, from 0% to 20%) to give final product 2-48 (88 mg) which was triturated with MeOH and filtered to give final product 2-48 (25 mg, 26%).

### Method B9

### Preparation of final product 2-89

A mixture of Final Product 2-82 (15 mg, 0.027 mmol) and HCl (4M in dioxane, 0.07 mL, 0.27 mmol) in MeOH (1 mL) was stirred at rt for 16 h. The solvent was evaporated and the residue was triturated from a mixture of CHCl₃, MeOH and Et₂O to give the desired final product 2-89 (13 mg, 98%) as a yellow solid.

**Table 4.- Other tricycles compounds**

| **Cpd. Nr** | **Structure** |
|---|---|
| **5-01** | 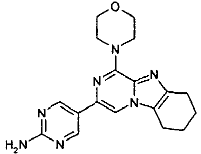 |
| **5-02** | 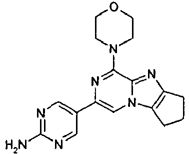 |
| **5-03** | 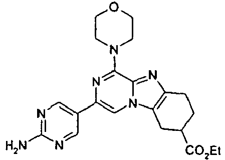 |
| **5-04** | 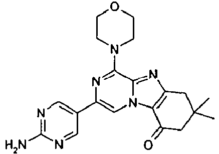 |
| **5-05** | 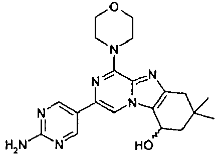 |
| **5-06** | 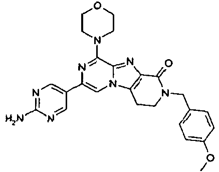 |
| **5-07** | 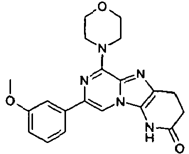 |
| **5-08** | 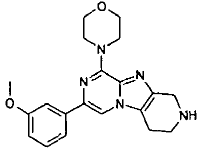 |
| **5-09** | 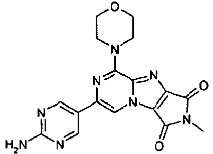 |
| **5-10** | 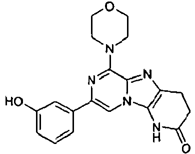 |
| **5-11** | 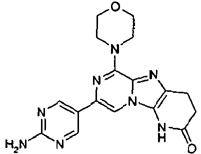 |
| **5-12** | 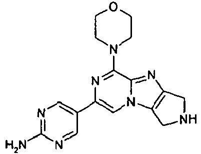 |

### Preparation of final products (and intermediates)

### Preparation of final product 5-01

A mixture of I-07 (104 mg, 0.36 mmol, 1 eq.), 2-aminopyrimidine-5-boronic acid, pinacol ester (95 mg, 0.432 mmol, 1.2 eq), and PdCl₂(dppf) (30 mg, 0.036 mmol, 0.1 eq.) in DME (10 mL) was added saturated aqueous solution of potassium carbonate (1.5 mL). The mixture was refluxed for 2 h and then for a further 4 h. Then, the solvent was removed in vacuo and the residue was triturated from MeCN/water to give a brown solid that was filtered off, and washed with water and diethyl ether. Purification of this solid twice by column chromatography (EtOAc/MeOH mixtures as eluent) did not afford desired compound in acceptable purity, therefore the residue was purified by semi-preparative HPLC (RP-C18 Gemini; 150x10 mm, 5 um; 30-70% B in 10 min, flow rate 6 mL/min; B: CH₃CN + 0.1% formic acid; A: H₂O + 0.1% formic acid) to give the desired product **5-01,** (10 mg , 9% yield).

### Preparation of intermediate I-07

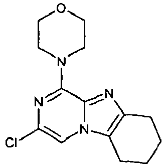

Three batches of 5-bromo-3-morpholinopyrazin-2-amine (100 mg, 0.39 mmol, 1 eq) and 2-chlorocyclohexanone (0.46 mL, 0.56 mmol, 1.2 eq) were heated for 1 h at 120°C under microwave irradiation. On cooling, crude reaction mixtures and were combined and the solvent was removed in vacuo. The residue was purified twice by column chromatography (hexane/EtOAc mixtures as eluent) to give intermediate **I-07** as a white solid (108 mg, 32% combined yield).

### Preparation of final product 5-02

To a mixture of I-04 (30 mg, 0.108 mmol) in 1,2-dimethoxyethane (1 mL), 2-aminopyrimidine-5-boronic acid (29 mg, 0.129 mmol), PdCl₂(dppf) (9 mg, 0.011 mmol) and a satured K₂CO₃ solution (0.3 mL) were added. The reaction mixture was heated at 120 °C for 2h. Then, water was added and it was extracted with dichloromethane, dried over Na₂SO₄ and evaporated to dryness. The resulting residue was purified by was purified by CCTLC in the Chromatotron (CH₂Cl₂:MeOH, 15:1), and then by HPLC to obtain 5mg of expected compound 5-**02.**

### Preparation of intermediate I-04

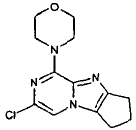

A solution of 5-bromo-3-morpholinopyrazin-2-amine (0.200 g, 0.772 mmol) in DME (1.5 mL), 2-chlorocyclopentanone (0.116 mL, 1.158 mmol) was added. The mixture was heated at 120 °C for 16 h, until the reactants were consumed as determined by LCMS analysis. The reaction mixtures were diluted with DCM (100 mL) mixed, washed with saturated aqueous solution of NaHCO₃, brine, dried over Na₂SO₄ and concentrated in vacuo. The dark residue was purified by biotage (cyclohex/EtOAc 1:1) to obtain 30 mg of desired compound **I-04** as a white solid, which was used in next reaction step without further purification.

### Preparation of final product 5-03

A mixture of I-05 (40 mg, 0.10 mmol, 1 eq), 2-aminopyrimidine-5-boronic acid, pinacol ester (27 mg, 0.12 mmol, 1.2 eq), and PdCl₂(dppf) (9 mg, 0.01 mmol, 0.1 eq) in DME (5 mL) was added saturated aqueous solution of potassium carbonate (0.5 mL). The mixture was refluxed for 10 h. The solvent was removed in vacuo and the residue was triturated from Acetonitrile/water to give a brown solid that was filtered off, and washed with water. This residue was purified by column chromatography (EtOAc/MeOH mixtures as eluent) to afford final product **5-03,** (7 mg, % yield) as a solid.

### Preparation of intermediate I-05

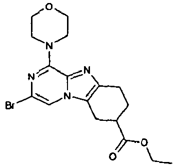

A mixture of 5-bromo-3-morpholinopyrazin-2-amine (150 mg, 0.70 mmol, 1 eq) and ethyl 3-bromo-4-oxocyclohexanecarboxylate (210 mg, 0.84 mmol, 1.2 eq) in dioxane (1.0 mL) was heated for 18 h at 120°C in a sealed tube. Two more batches were progressed. The crude mixtures from different batches were combined and the residue was purified by column chromatography (EtOAc as eluent) to give a brown oil that was repurified by column chromatography (hexanes/EtOAc 10:1) to give intermediate **I-05** as a yellow solid (42 mg, 6% combined yield).

### Preparation of intermediate I-06

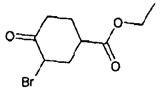

A solution of bromine (0.15 mL, 2.93 mmol, 1 eq) in diethyl ether (2 mL) was added dropwise to a solution of ethyl 4-oxocyclohexanecarboxylate (0.5 g, 2.93 mmol, 1 eq) in diethylether (20 mL) cooled to 0°C. The mixture was allowed to warm up to room temperature and then stirred for 1 h at this temperature. Saturated aqueous solution of NaHSO₃ (5 mL) was added, the phases separated and the aqueous layer was extracted with ether (2 x 25 mL). The combined organic layers were dried (MgSO₄) and the solvent removed in vacuo to give 3-bromo-4-oxo-cyclohexanecarboxylic acid ethyl ester as a brown oil (640 mg, 88%) as a mixture of diastereoisomers. The resulting product was used in next reaction step without further purification as intermediate **I-06.**

### Preparation of final product 5-04

Intermediate I-08 (200 mg,0.43 mmol), PdCl₂dppf (cat amount), K₂CO₃ (120 mg, 0.90 mmol) in DMF was stirred at 100 °C for 16 h. Excess of PdCl₂dppf was added and the heating continued for 4 h more. Upon reaction completion 2-aminopyrimidine-5-boronic acid, pinacol ester (125 mg, 0.56 mmol) was added. The resultant dark mixture was heated under microwave irradiation for 15 min at 130 °C. After, the reaction mixture was concentrated in vacuo, leaving a dark residue which was purified twice by Biotage flash column chromatography eluting with a gradient of MeOH/DCM (from 0% to 10%) to yield 30 mg of expected compound **5-04.**

### Preparation of intermediate I-08

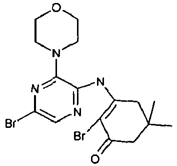

A mixture of 5-bromo-3-morpholin-4-yl-pyrazin-2-ylamine (1.4 g, 4.1 mmol) and 2-bromo-5,5-dimethylcyclohexane-1,3-dione (0.9 g, 6.1 mmol; intermediate I-09) in DME (4 mL) was heated at 120 °C for 16 h. The reaction mixture was cooled to rt, diluted with DCM (20 mL), washed with aqueous saturated NaHCO₃ (2 x 30 mL), brine (20 mL), dried over Na₂SO₄ and concentrated in vauco. The dark crude was purified by Biotage flash column chromatography, eluting with a solvent system of EtOAc/cyclohexane (from 20% to 75% on EtOAc), yielding the required product, intermediate **I-08,** as a pale yellow solid (430 mg, 22%).

### Preparation of intermediate I-09

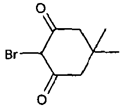

A mixture of 5,5-dimethylcyclohexane-1,3-dione (1.5 g, 10 mmol), NBS (1.9 g, 10.7 mmol) in acetonitrile (8 mL) was heated at 70 °C for 16 h. The reaction mixture was cooled to rt and concentrated in vacuo, leaving a yellow-white solid. The crude was dissolved in DCM, washed with water (4 x 30 mL), brine (40 mL) dried over Na₂SO₄ and concentrated, leaving a white solid as intermediate **I-09,** (1.150 g, 74%) that was used in next reaction step without further purification.

### Preparation of final product 5-05

To a suspension of final product **5-04** (30 mg, 0.07 mmol) in MeOH (1.5 mL) was added NaBH₄ (5 mg). The mixture was stirred at rt for 24 h. Excess of NaBH₄ was added upon reaction completion stirring for 16 h more. Some drops of water were added and the reaction mixture was concentrated in vacuo. The residue was purified by column chromatography eluting with a gradient of MeOH/DCM (from 0% to 5% on MeOH), yielding 3 mg of desired compound **5-05.**

### Preparation of final product 5-06

Intermediate I-10, 1-(4-methoxybenzyl)-3-(5-bromo-3-morpholinopyrazin-2-ylamino)-4-chloro-5,6-dihydropyridin-2(1H)-one (120 mg, 1 eq) was dissolved in DMF (2 mL) and PdCl₂(dppf) (20 mg, 0.1 eq) and K₂CO₃ (100 mg, 3 eq) were added. The reaction mixture was heated at 100 °C for 3 h. 2-Amino pyrimidine-5-boronic acid, pinacol ester (63 mg, 1.2 eq) was added and the reaction mixture was heated under microwave irradiation at 150 °C for 15 min. On cooling, the reaction mixture was purified by automated chromatography (Biotage, eluent: 5% to 15% MeOH in DCM) to give the expected product (40 mg, 26%) as a brown solid which was purified by HPLC to obtain the final product **5-06.**

### Preparation of intermediate I-10

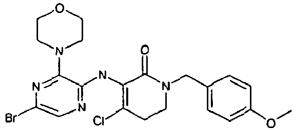

5-bromo-3-morpholin-4-yl-pyrazin-2-ylamine (100 mg, 1 eq) was suspended in DME (1 mL) and 1-(4-methoxybenzyl)-4-chloropiperidine-2,3-dione, **I**-**11**, (125 mg, 1.2 eq) was added. The reaction mixture was heated in a sealed tube at 90°C for 17 h. On cooling, the mixture was purified by automated chromatography (Biotage, eluent: 30% to 60% EtOAc in Cyclohexane) to give the expected product **I-10** (150 mg, 60% yield) which was used in next reaction step without further purification, as a brown solid.

### Preparation of intermediate I-11

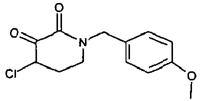

1-(4-Methoxybenzyl)piperidine-2,3-dione (0.2 g, 1 eq) was dissolved in EtOAc (8 mL) and NCS (114 mg, 1 eq) and Amberlyst-15 (240 mg, 1.2 eq) were added. The reaction mixture was stirred at 50 °C for 2 h. On cooling, EtOAc (35 mL) was added and the mixture was washed with NaHCO₃ sat. (2 x 40 mL). The organic layers were dried, filtered and evaporated to give a mixture of chlorinated products (170 mg) as a white solid. The mixture was used in the next reaction step without further purification.

### Preparation of final product 5-07

Intermediate I-12, Methyl 3-(3-amino-6-(3-methoxyphenyl)-8-morpholino-imidazo[1,2-a]pyrazin-2-yl)propanoate, (85 mg, 1 eq) was suspended in EtOH (2 mL) and TEA (0.3 mL, 10 eq) was added (pH~9-10). The reaction mixture was heated in a sealed tube at 80 °C for 4 h. On cooling, the mixture was filtered and rinsed with EtOH and DCM to give the expected product **5-07** (65 mg, 83% yield) as a beige solid.

### Preparation of intermediate I-12

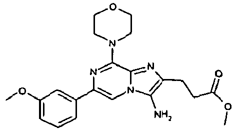

Intermediate I-13, Methyl 3-(3-(2,4,4-trimethylpentan-2-ylamino)-6-(3-methoxyphenyl)-8-morpholinoimidazo[1,2-a]pyrazin-2-yl)propanoate (110 mg, 1 eq) was dissolved in CHCl₃ (2 mL) and MeOH (2 mL) and HCl 37% in H₂O (1 mL) was added. The reaction mixture was stirred at rt for 3 h and then refluxed for 3 days. The mixture was evaporated. The corresponding residue **I-12,** was used in the next reaction step without further purification.

### Preparation of intermediate I-13

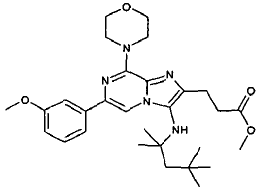

Intermediate I-14, Methyl 3-(3-(2,4,4-trimethylpentan-2-ylamino)-6-bromo-8-morpholinoimidazo[1,2-a]pyrazin-2-yl)propanoate (0.15 g, 1 eq) was suspended in DME (2 mL) and 3-methoxyphenylboronic acid (55 mg, 1.2 eq), PdCl₂(dppf) (25 mg, 0.1 eq), K₂CO₃ (125 mg, 3 eq) and H₂O (0.5 mL) were added. The reaction mixture was heated under microwave irradiation at 130 °C for 20 min. On cooling, the mixture was adsorbed in silica and purified by automated chromatography (Biotage, eluent: 20% to 40% EtOAc in Cyclohex.) to give the expected product **I-13** (110 mg, 70 % yield) as a yellow solid.

### Preparation of intermediate I-14

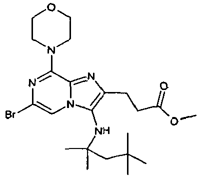

A mixture of 5-bromo-3-morpholin-4-yl-pyrazin-2-ylamine (0.3 g, 1 eq), methyl 4-oxobutanoate (182 µL, 1.5 eq), 1,1,3,3-tetramethylbutyl isocyanide (305 µL, 1.5 eq) and ZrCl₄ (54 mg, 0.2 eq) in PEG-400 (3 mL) was heated at 50 °C under open air for 20 h and 24 h more. On cooling, H₂O (60 mL) was added and the mixture was extracted with EtOAc (3 x 50 mL). The organics were dried, filtered and evaporated and the residue was purified by automated chromatography (Biotage, eluent: 20% to 40% EtOAc in Cyclohexane) to give the expected product **I-14** (155 mg, 26% yield) as a yellow oil.

### Preparation of final product 5-08

Intermediate I-15, 2-(2-(chloromethyl)-6-(3-methoxyphenyl)-8-morpholinoimidazo[1,2-a]pyrazin-3-yl)ethyl methanesulfonate (25 mg, 1 eq) was dissolved in Acetonitrile (2 mL) and NH₃ 33% in H₂O (2 mL) were added. The reaction mixture was stirred in a sealed tube at rt for 20 h and evaporated. The residue was purified by automated chromatography (Biotage, eluent: 5% to 30% MeOH in DCM) to give the expected product **5-08** (7 mg, 36% yield) as a white solid

### Preparation of intermediate I-15

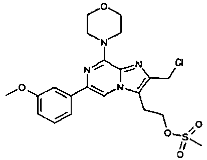

Intermediate I-16, 2-(2-(hydroxymethyl)-6-(3-methoxyphenyl)-8-morpholino-imidazo[1,2-a]pyrazin-3-yl)ethanol (30 mg, 1 eq) was suspended in DCM (2 mL) and TEA (44 µL, 4 eq) was added followed by Mesyl Chloride (18 µL, 3 eq). The reaction mixture was stirred at rt for 20 min and purified by automated chromatography (Biotage, eluent: 2% to 20% MeOH in DCM) to give 2-(2-(chloromethyl)-6-(3-methoxyphenyl)-8-morpholinoimidazo[1,2-a]pyrazin-3-yl)ethyl methanesulfonate as intermediate product **I-15** (27 mg, 72% yield) as a yellow solid.

### Preparation of intermediate I-16

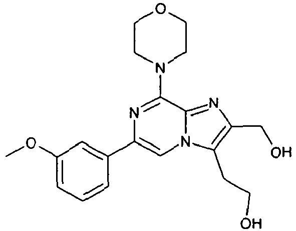

Intermediate I-17, Methyl 3-((methoxycarbonyl)methyl)-6-(3-methoxyphenyl)-8-morpholinoimidazo[1,2-a]pyrazine-2-carboxylate (60 mg, 1 eq) was dissolved in THF (7 mL) and LiAlH₄ 2M in THF (0.1 mL, 1.5 eq) was slowly added. The reaction mixture was stirred at rt for 30 min. Drops of MeOH were carefully added and the mixture was purified by automated chromatography (Biotage, eluent: 5% to 10% MeOH in DCM) to give the expected product, intermediate **I-16** (30 mg, 57% yield) as a white solid.

### Preparation of intermediate I-17

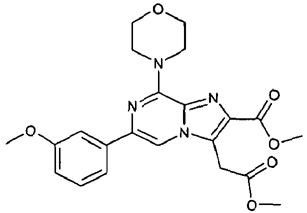

Intermediate I-18, methyl 3-((methoxycarbonyl)methyl)-6-bromo-8-morpholino-imidazo[1,2-a]pyrazine-2-carboxylate (0.92 g, 1 eq) was suspended in DME (6 mL) and 3-methoxyphenylboronic acid (406 mg, 1.2 eq), PdCl₂(dppf) (184 mg, 0.1 eq), K₂CO₃ (0.93 g, 3 eq) and H₂O (2 mL) were added. The reaction mixture was heated under microwave irradiation at 130 °C for 20 min. On cooling, the mixture was adsorbed in silica and purified by column chromatography (Biotage, eluent: 20% to 60% EtOAc in Cyclohexane) to give the expected product I-17 (410 mg, 41% yield) as a beige solid.

### Preparation of intermediate I-18

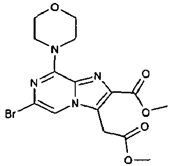

5-bromo-3-morpholin-4-yl-pyrazin-2-ylamine (5 g, 1 eq) was suspended in DME (30 mL) and dimethyl 3-bromo-2-oxopentanedioate (7.33 g, 1.5 eq) was added. The reaction mixture was heated in a sealed tube at 90°C for 27 h and then at 70 °C for 48 h. On cooling, the reaction mixture was filtered to give the expected product. This solid was dissolved in DCM (150 mL) and washed with HCl 2N (2 x 100 mL). The organic layer was dried, (Na₂SO₄), filtered and evaporated to give the expected product (1.9 g, 24% yield) as a beige solid. The filtrate of the reaction mixture was evaporated and the residue was purified by column chromatography (Biotage, eluent: 20% to 50% EtOAc in Cyclohexane) to give the expected product intermediate **I-18,** (0.77 g, 10% yield) as an orange solid.

### Preparation of intermediate I-19

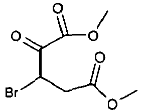

Dimethyl 2-oxoglutarate (4.16 mL, 1 eq) was added to a suspension of CuBr₂ (19.3 g, 3 eq) in EtOAc (300 mL) and CHCl₃ (150 mL). The reaction mixture was refluxed (90 °C) for 14 h. On cooling, the suspension was filtered through silica and rinsed with EtOAc. The filtrate was evaporated to give the expected product, **I-19** (7.4 g, 100% yield) as a green oil which was used in the next reaction step without further purification.

### Preparation of final product 5-09

Intermediate I-20 (100 mg, 1 eq) (Note 1) was dissolved in DMF (2 mL) and 2-aminopyrimidine-5-boronic acid, pinacol ester (72 mg, 1.2 eq), PdCl₂(dppf) (23 mg, 0.1 eq), K₂CO₃ (75 mg, 2 eq) were added. The reaction mixtures were heated under microwaveirradiation at 150 °C for 15 min. On cooling, the mixture was purified by column chromatography (Biotage, eluent: 5% to 20% MeOH in DCM) and then by HPLC to give the expected product **5-09** (4 mg) as a yellow solid.

### Preparation of intermediate I-20

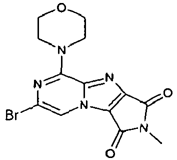

5-bromo-3-morpholin-4-yl-pyrazin-2-ylamine (300 mg, 1 eq) was suspended in EtOH (3 mL) and 2,3-dibromo-n-methylmaleimide (311 mg, 1 eq) was added. The reaction mixture was heated under microwave irradiation at 120 °C for 4 h. On cooling, the mixture was filtered to give a yellow solid (50 mg,) which was heated at 150 °C for 15 min under open air. On cooling, the mixture was purified by automated chromatography (Biotage, eluent: 2% to 10% MeOH in DCM) to obtain the expected product **I-20** (22 mg) as a yellow solid.

### Preparation of final product 5-10

Final product 5-07 (50 mg, 0.132 mmol) was suspended in DCM (2 mL) and Boron fluoride-dimethyl sulfide complex (0.139 mL, 1.32 mmol) was added. The reaction mixture was stirred at rt for 20 h. NaHCO₃ sat. (30 mL) was added and the mixture was extracted with CHCl₃:iPrOH (1:1; 2 x 35 mL). The organics were dried, filtered and evaporated. The residue was precipitated from DCM and filtered to give the expected product **5-10** (15 mg, 31%) as a beige solid.

### Preparation of final product 5-11

Intermediate I-21, methyl 3-(3-amino-6-(2-aminopyrimidin-5-yl)-8-morpholinoimidazo[1,2-a]pyrazin-2-yl)propanoate (210 mg, 0.527 mmol) was suspended in NaOMe 0.5 M in MeOH (16 mL, 7.91 mmol) (pH= 10-11) and the reaction mixture was refluxed for 18 h. On cooling, the mixture was evaporated and the residue was purified by automated chromatography (Biotage, eluent: 10% to 20% MeOH in DCM). The product obtained was precipitated with MeOH and filtered to give the expected product 5-11 (60 mg, 31%) as a white solid.

### Preparation of intermediate I-21

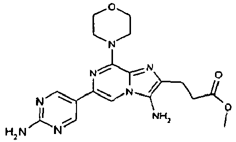

Intermediate I-22, methyl 3-(3-(2,4,4-trimethylpentan-2-ylamino)-6-(2-aminopyrimidin-5-yl)-8-morpholinoimidazo[1,2-a]pyrazin-2-yl)propanoate (270 mg, 0.529 mmol) was dissolved in CHCl₃:MeOH (1:1, 4 mL) and HCl 37% (1 mL) was added. The reaction mixture was refluxed for 2 h and evaporated. The residue obtained was used in the next reaction step without further purification.

### Preparation of intermediate I-22

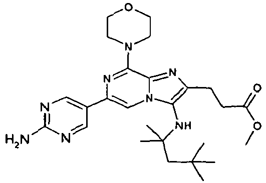

Intermediate I-14, methyl 3-(3-(2,4,4-trimethylpentan-2-ylamino)-6-bromo-8-morpholinoimidazo[1,2-alpyrazin-2-yl)propanoate (0.41 g, 0.826 mmol) was suspended in DME (4 mL) and 2-aminopyrimidine-5-boronic acid, pinacol ester (219 mg, 0.99 mmol), PdCl₂(dppf) (68 mg, 0.083 mmol), K₂CO₃ (342 mg, 2.48 mmol) and H₂O (1 mL) were added. The reaction mixture was heated under microwave irradiation at 130 °C for 20 min. On cooling, the mixture was adsorbed in silica and purified by automated chromatography (Biotage, eluent: 5% to 10% MeOH in DCM) to give the expected product **I**-**22** (270 mg, 64%) as a beige solid.

### Preparation of final product 5-12

Intermediate I-36 (29 mg, 0.104 mmol) was dissolved in DME (1 mL) and 2-aminopyrimidine-5-boronic acid, pinacol ester (28 mg, 0.124 mmol), PdCl₂(dppf) (9 mg, 0.01 mmol), K₂CO₃ (43 mg, 0.311 mmol) and H₂O (0.5 mL) were added. The reaction mixture was heated under microwave irradiation at 130 °C for 20 min and evaporated. The residue was purified by prep-HPLC to give the expected product 5-12 (1 mg, 3%) as a clear yellow solid.

### Preparation of intermediate I-36

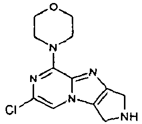

Intermediate I-37 (20 mg, 0.06 mmol) was dissolved in Acetonitrile (2 mL) and NH₃ 33% in H₂O (4 mL) was added. The reaction mixture was stirred in a sealed tube at RT for 16 h and evaporated. The residue was used in the next reaction step with no further treatment.

### Preparation of intermediate I-37

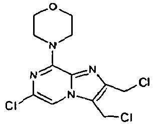

Intermediate I-38 (100 mg, 0.335 mmol) was suspended in DCM (3 mL) and TEA (187 µL, 1.34 mmol) was added followed by MsCl (78 µL, 1.00 mmol). The reaction mixture was stirred at RT for 1 h and purified by automated column chromatography (Biotage, MeOH:DCM, 2:98 to 10:90) to give a mixture of the expected product **I-37** (20 mg, 18%) as a yellow solid. It was used in the next reaction step without further treatment.

### Preparation of intermediate I-38

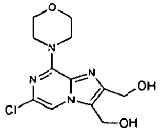

Intermediate I-39 (200 mg, 0.522 mmol) was dissolved in THF (10 mL) and LiAlH₄ 2M in THF (0.4 mL, 0.784 mmol) was slowly added. The reaction mixture was stirred at RT for 1 h. Drops of MeOH were carefully added and the mixture was purified by automated column chromatography (Biotage, MeOH:DCM, 5:95 to 15:85) to give the expected product **1-38** (100 mg, 64%) as a white solid.

### Preparation of intermediate I-39

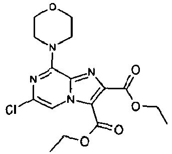

To a solution of Intermediate I-02 (3 g, 9.288 mmol) in 2-propanol (46 mL), 2-chloro-3-oxo-succinic acid diethyl ester (6.2 g, 27.865 mmol) was added. The reaction mixture was heated in a Parr reaction vessel at 90 °C for 2 days. The solvent was evaporated and the residue was purified by automated column chromatography (Biotage, EtOAc:Cyclohexane, 0:100 to 40:60). The product obtained was treated with Et₂O and filtered to give the expected product **I-39** (1.2 g, 34 %) as a white solid.

**Table 5 - Intermediates**

| | | | | |
|---|---|---|---|---|
| | 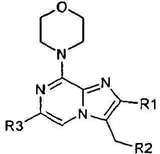 | | | |

| **No.** | **Exp. Meth.** | **--R1** | **--R2** | **--R3** |
|---|---|---|---|---|
| **I**-**23** | **C1** | --CO₂Me | --CO₂Me | --Cl |
| **I-24** | **C2** | --CH₂OH | --CH₂OH | --Cl |
| **I-25** | **C3** | --CH₂Cl | --CH₂OMs | --Cl |
| **I-26** | **C2** | --CH₂OH | --CH₂OH | --Br |
| **I-27** | **C3** | --CH₂Cl | --CH₂OMs | --Br |

**Table 6 - Intermediates**

| | | | | |
|---|---|---|---|---|
| | 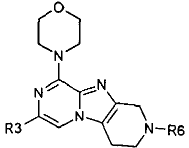 | | | |

| **No.** | **Exp. Meth.** | **--R3** | **--R6** | |
|---|---|---|---|---|
| **I-28** | **C4** | --Cl | --H | |
| **I-29** | **C4** | --Cl | --Me | |
| **I-30** | **C4** | --Cl | 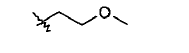 | |
| **I-31** | **C4** | --Br | 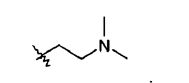 | |
| **I-32** | **C4** | --Br | 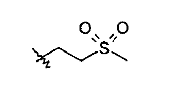 | |
| **I-33** | **C4** | --Br | 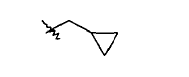 | |
| **I-34** | **C4** | --Br | 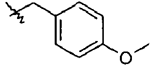 | |
| **I-35** | **C4** | --Br | 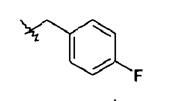 | |
| **I-36** | **C4** | --Br | 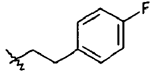 | |
| **I-37** | **C4** | --Br | 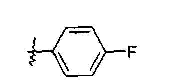 | |
| **I-38** | **C4** | --Br | 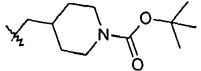 | |
| **I-39** | **C5** | 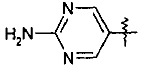 | 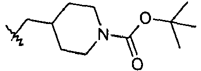 | |
| **I-40** | **C4** | --Br | 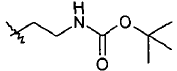 | |
| **I-41** | **C5** | 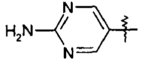 | 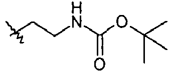 | |
| **I-42** | **C6** | 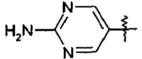 | 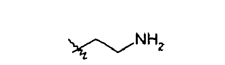 | |

**Table 7 -Final Products**

| | | | | |
|---|---|---|---|---|
| 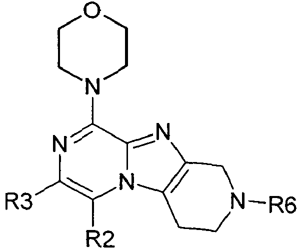 | | | | |

| **No.** | **Exp.** | --R6 | --R2 | --R3 |
|---|---|---|---|---|
| **6-01** | **D1** | --H | --H | 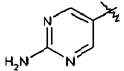 |
| **6-02** | **D1** | --Me | --H | 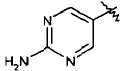 |
| **6-03** | **D1** | 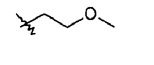 | --H | 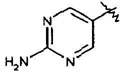 |
| **6-04** | **D1** | 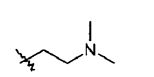 | --H | 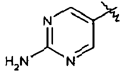 |
| **6-05** | **D1** | 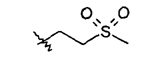 | --H | 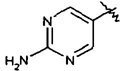 |
| **6-06** | **D1** | 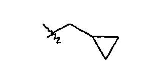 | --H | 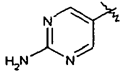 |
| **6-07** | **D1** | 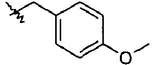 | --H | 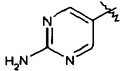 |
| **6-08** | **D1** | 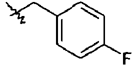 | --H | 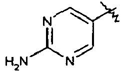 |
| **6-09** | **D2** | 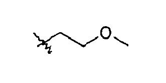 | --Cl | 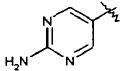 |
| **6-10** | **D2** | 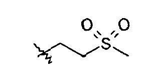 | --Cl | 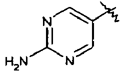 |
| **6-11** | **D2** | 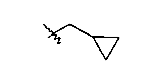 | --Cl | 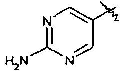 |
| **6-12** | **D2** | 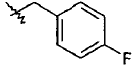 | --Cl | 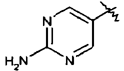 |
| **6-13** | **D3** | --SO₂Me | --H | 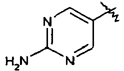 |
| **6-14** | **D4** | 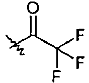 | --H | 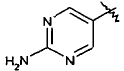 |
| **6-15** | **D3** | 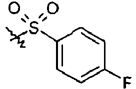 | --H | 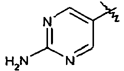 |
| **6-16** | **D3** | 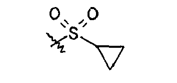 | --H | 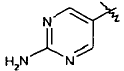 |
| **6-17** | **D3** | --SO₂Me | --Cl | 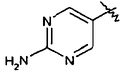 |
| **6-18** | **D5** | 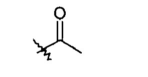 | --H | 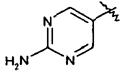 |
| **6-19** | **D6** | 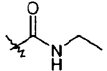 | --H | 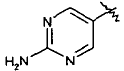 |
| **6-20** | **D3** | -SO₂Et | --H | 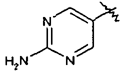 |
| **6-21** | **D6** | 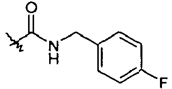 | --H | 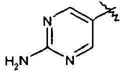 |
| **6-22** | **D6** | 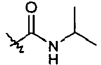 | --H | 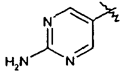 |
| **6-23** | **D5** | 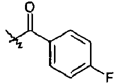 | --H | 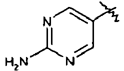 |
| **6-24** | **D5** | 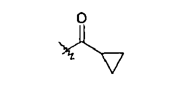 | --H | 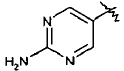 |
| **6-25** | **D6** | 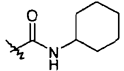 | --H | 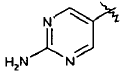 |
| **6-26** | **D5** | 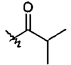 | --H | 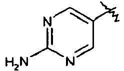 |
| **6-27** | **D1** | 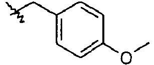 | --H | 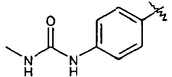 |
| **6-28** | **D3** | 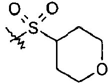 | --H | 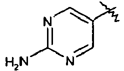 |
| **6-29** | **D3** | 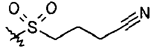 | --H | 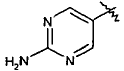 |
| **6-30** | **D6** | 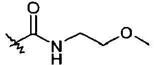 | --H | 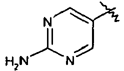 |
| **6-31** | **D2** | 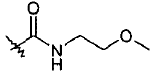 | --Cl | 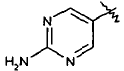 |
| **6-32** | **D6** | 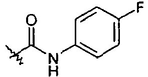 | --H | 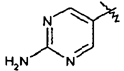 |
| **6-33** | **D6** | 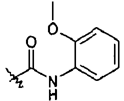 | --H | 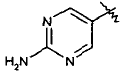 |
| **6-34** | **D6** | 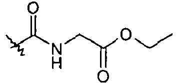 | --H | 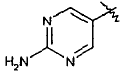 |
| **6-35** | **D5** | 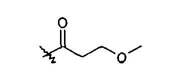 | --H | 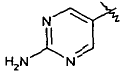 |
| **6-36** | **D6** | 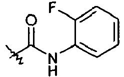 | --H | 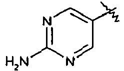 |
| **6-37** | **D6** | 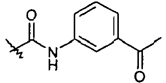 | --H | 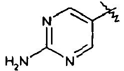 |
| **6-38** | **D7** | 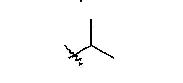 | --H | 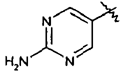 |
| **6-39** | **D6** | 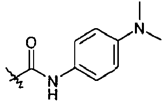 | --H | 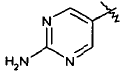 |
| **6-40** | **D1** | 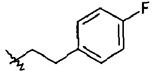 | --H | 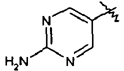 |
| **6-41** | **D1** | 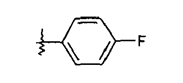 | --H | 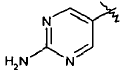 |
| **6-42** | **D8** | 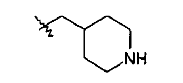 | --H | 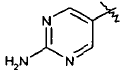 |
| **6-43** | **D3** | 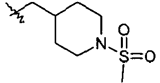 | --H | 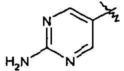 |
| **6-44** | **D6** | 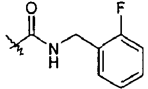 | --H | 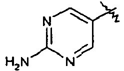 |
| **6-45** | **D6** | 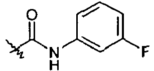 | --H | 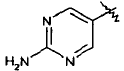 |
| **6-46** | **D6** | 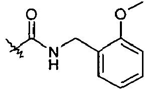 | --H | 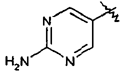 |
| **6-47** | **D3** | 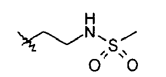 | --H | 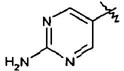 |

### Experimental part

### Preparation of the intermediates

### Method C1

### Preparation of intermediate I-23.

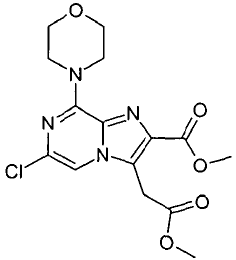

A mixture of Intermediate I-18, methyl 3-((methoxycarbonyl)methyl)-6-bromo-8-morpholinoimidazo[1,2-a]pyrazine-2-carboxylate, (2.5 g, 6.05 mmol) and KCI (10 g, 122 mmol) in Acetonitrile (40 mL) was heated at 90 °C for 3 days. The reaction mixture was cooled down, and evaporated. DCM (50 mL) was added and the mixture was washed with water (3 x 40 mL) and brine (40 mL). The organic layer was dried over Na₂SO₄, filtered and concentrated in vacuo to give the expected product **I**-**23** as a brown solid (1.7 g, 76%). It was used in the next reaction step without further purification.

### Method C2

### Preparation of intermediate I-24.

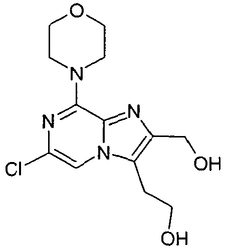

To a suspension of Intermediate I-23, methyl 3-((methoxycarbonyl)methyl)-6-chloro-8-morpholinoimidazo[1,2-a]pyrazine-2-carboxylate, (1.7 g, 4.6 mmol) in THF (20 mL) cooled to 0 °C, a 1M solution of LiAlH₄ in THF (7 mL) was added. The resulting dark mixture was stirred for 2 h and carefully quenched by addition of saturated solution of NH₄Cl. The mixture was filtered through a Celite pad and rinsed with DCM. The filtrate was extracted with DCM (3 x 40 mL). The combined organic layers were washed with brine, dried over Na₂SO₄ and concentrated in vacuo to give the expected product **I-24** (1.14 g, 79 %). It was used in the next reaction step without further purification.

### Method C3

### Preparation of intermediate I-25.

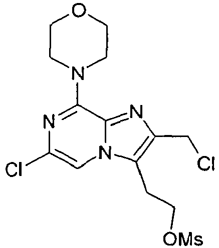

To a mixture of Intermediate I-24, 2-(6-chloro-2-(hydroxymethyl)-8-morpholinoimidazo[1,2-a]pyrazin-3-yl)ethanol, (1.2 g, 3.8 mmol) and TEA (1.6 mL, 11 mmol) in DCM (20 mL) at 0 °C was added dropwise CISO₂Me (0.6 mL, 7.6 mmol). The brown mixture was stirred at 0 °C for 30 min and at rt for 2 h. HCl 1 M (10 mL) was added and the mixture was extracted with DCM (3 x 30 mL). The combined organic layers were washed with water (40 mL) and brine (40 mL), dried over Na₂SO₄, filtered and evaporated. The residue, Intermediate **I**-**25**, (1.3 g, 83%) was used in the next reaction step without further purification.

### Method C4

### Preparation of intermediate I-28.

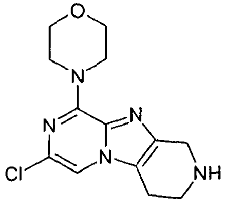

Intermediate I-25, 2-(6-chloro-2-(chloromethyl)-8-morpholinoimidazo[1,2-alpyrazin-3-yl)ethyl methanesulfonate, (300 mg, 0.73 mmol) was dissolved in Acetonitrile (3 mL) and ammonia (32% in H₂O) was added (35 mL). The reaction mixture was stirred at rt in a pressure tube for 18 h. The mixture was concentrated in vacuo to give the expected product, Intermediate **I-28,** as a brown-cream solid (400 mg). It was used in the next reaction step without further purification.

### Preparation of intermediate I-29.

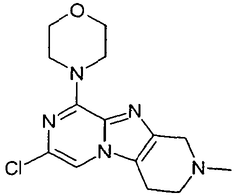

A mixture of Intermediate I-25, 2-(6-chloro-2-(chloromethyl)-8-morpholinoimidazo[1,2-a]pyrazin-3-yl)ethyl methanesulfonate, (150 mg, 0.36 mmol) and methylamine (2 M in MeOH, 1.1 mL, 2.2 mmol) in Acetonitrile (1.5 mL) was stirred at rt for 24 h. The brown mixture was acidified (pH∼1) by adding aqueous HCl 1 M and extracted with DCM (10 mL). The aqueous layer was basidified to pH>10 by addition of aqueous NaOH 5 M and extracted with DCM (3 x 10 mL). The combined organic layers were washed with brine (40 mL), dried over Na₂SO₄ and evaporated to give Intermediate **I-29** (90 mg) as a cream solid. It was used in the next reaction step without further purification.

### Preparation of intermediate I-36.

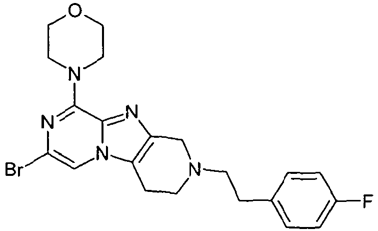

A mixture of Intermediate I-27 (185 mg, 0.4 mmol) and 4-fluorophenethylamine (215 µL, 1.6 mmol) in DCM (3 mL) was stirred at rt for 18 h.The reaction mixture was quenched by addition of HCl 1 M. The aqueous layer was basidified by addition of NaOH 0.5 M and re-extracted with DCM (3 x 10 mL). The combined organic layers were washed with brine, dried over Na₂SO₄ and concentrated in vacuo. The residue was used in the next experimental step without further purification.

### Preparation of intermediate I-38.

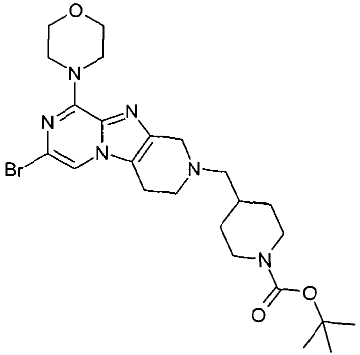

A mixture of Intermediate I-27 (200 mg, 0.44 mmol) and 1-Boc-4-(aminomethyl)piperidine hydrochloride (470 mg, 2.2 mmol) and Cs₂CO₃ (800 mg, 2.6 mmol) in ACN (4 mL) was stirred at rt for 18 h. The reaction mixture was quenched by addition of HCl 1 M. The aqueous layer was basidified by addition of NaOH 0.5 M and extracted with DCM (3 x 10 mL). The combined organic layers were washed with brine, dried over Na₂SO₄ and evaporated. The residue was used in the next experiment step without further purification.

### Method C5

### Preparation of intermediate I-39.

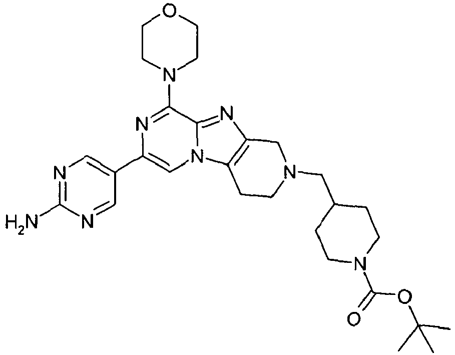

A mixture of Intermediate I-38 (500 mg, 0.93 mmol) with 2-aminopyrimidine-5-boronic acid, pinacol ester (230 mg, 1.03 mmol), PdCl₂dppf (cat.) and aq. sat. solution of K₂CO₃ (1.5 mL) in DME (3 mL) was heated under microwave irrardiration at 140 °C for 45 min. The reaction mixture was evaporated. The dark residue was suspended in DCM and washed with sat. NaHCO₃ (3 x 25 mL), brine (30 mL), dried over Na₂SO₄ and evaporated. The residue was purified by column chromatography (MeOH/DCM, 0:100 to 10:90) to give the Intermediate I-39 (70 mg, 30% over two steps from Intermediate I-38) as a beige solid. evaporated. The residue was used in the next experiment step without further purification.

### Method C6

### Preparation of intermediate I-42.

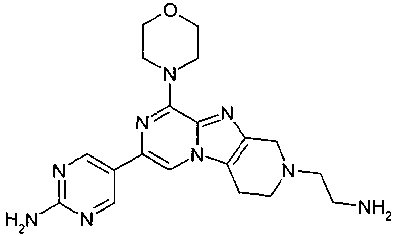

A suspension of Intermediate I-41 (100 mg, 0.2 mmol) in MeOH (5 mL) was stirred with amberlyst(r)-15 (1 g) at rt for 20 h. The mixture was filtered and the resin was suspended in NH3 (7 M in MeOH) (5 mL) and stirred for 1 h. The suspension was filtered and the filtrate was evaporated to give the Intermediate I-42 (60 mg, 76%) as cream solid. It was used in the next experiment step with no further treatment.

### Preparation of final products

### Method D1

### Preparation of final product 6-01

A mixture of intermediate I-28 (200 mg, 0.68 mmol), 2-aminopyrimidine-5-boronic acid, pinacol ester (175 mg, 0.8 mmol), PdCl₂(dppf) (cat.) and sat. K₂CO₃ (1 mL) in DME, was heated under microwave irradiation at 130 °C for 60 min. More PdCl₂dppf was added and the mixture was heated under the same conditions for 40 min. The mixture was filtered through a Celite pad, the filtrate was dried over Na₂SO₄ and evaporated. The residue was purified by flash column chromatography (Isolute Si II 10 g) (eluent: MeOH 7M NH₃/DCM, from 0% to 20%) to give Final Product 6-01 (40 mg, 17%) as a beige solid.

### Preparation of final product 6-27

A mixure of Intermediate I-34 (1.5 g, 3.27 mmol), 1-methyl-3-[4-(4,4,5,5-tetramethyl-1,3,2-dioxaborolan-2-yl)phenyl]urea (0.90 g, 3.27 mmol), PdCl₂dppf (0.271 g, 0.33 mmol) and K₂CO₃ sat. (3 mL) in DME (5 mL) was heated under microwave irradiation at 140 °C for 45 min. More reagents were added: 1-methyl-3-[4-(4,4,5,5-tetramethyl-1,3,2-dioxaborolan-2-yl)phenyl]urea (70 mg) and PdCl₂dppf (cat. amount) and the reaction mixture was heated under the same conditions for 40 min. The mixture was evaporated and the residue was suspended in DCM and washed with saturated aqueous NaHCO₃ (3 x 40 mL) and brine (40 mL), dried over Na₂SO₄, filtered and evaporated. The residue was purified by column chromatography (MeOH:DCM, 0:100 to 10:90) to give the desired final product 6-27 (130 mg, 12%) as a beige solid.

### Method D2

### Preparation of final product 6-09

A mixture of Final Product 6-03 (25 mg, 0.061 mmol) and NCS (8 mg, 0.061 mmol) in DCM (1 mL) was stirred at rt for 20 min. DCM (10 mL) was added and the mixture was washed with water (3 x 10 mL) and brine (10 mL), dried over Na₂SO₄ and evaporated. The residue was purified using a cromatotron (eluent: MeOH/DCM, from 0% to 15%) to yield Final Product 6-09 (12 mg, 44%).

### Method D3

### Preparation of final product 6-13

To a suspension of Final Product 6-01 (100 mg, 0.28 mmol) and Cs₂CO₃ (400 mg, 1.2 mmol) in Acetonitrile (5 mL) was added methanesulfonyl chloride (65 µL, 0.85 mmol). The reaction mixture was heated at 90 °C for 16 h but still starting material was observed. More methanesulfonyl chloride (65 µL, 0.85 mmol) and Cs₂CO₃ (200 mg, 0.6 mmol) were added and the mixture was heated at 90 °C for 6 h. The solvent was evaporated and the residue was suspended in DCM and washed with saturated NaHCO₃ (3 x 25 mL) and brine. The organic layer was dried over Na₂SO₄, filtered and evaporated. The aqueous layer also contained the required product and was extracted with a mixture of iPrOH/CHCl₃ (1:1, 4 x 25 mL). All the combined organic layers were dried over Na₂SO₄, filtered and concentrated. Both residues were mixed and purified by automated chromatography (Biotage, eluent: MeOH/DCM, from 5% to 10%) to give Final Product 6-13 (24 mg, 19%).

### Preparation of final product 6-15

A mixture of Final Product 6-01 (50 mg, 0.14 mmol), 4-fluorobenzenesulfonyl chloride (110 mg, 0.56 mmol) and Cs₂CO₃ (275 mg, 0.81 mmol) in Acetonitrile (2 mL) was heated under microwave irradiation at 130 °C for 30 min. The mixture was concentrated and the residue was purified by automated column chromatography (MeOH:DCM, 0:100 to 20:80) and by HPLC. The white solid obtained was triturated with MeOH, filtered and dried to render the Final Product 6-15 (5 mg, 7%).

### Preparation of final product 6-17

A mixture of 5-(4-Chloro-1-morpholin-4-yl-5,6,7,8-tetrahydro-2,4a,7,9-tetraaza-fluoren-3-yl)-pyrimidin-2-ylamine (30 mg, 0.078 mmol), methanesulfonyl chloride (25 µL, 0.3 mmol) and Cs₂CO₃ (150 mg, 0.46 mmol) in Acetonitrile (2 mL) was heated under microwave irradiation at 130 °C for 30 min. The reaction mixture was evaporated and the residue was purified by column chromatography (Isolute Si II 5 g; MeOH:DCM, 0:100 to 10:90). The solid obtained was triturated with MeOH (twice) and filtered to render the Final Product 6-17 (8 mg, 22%) as a white solid.

### Preparation of final product 6-28

A mixture of Final Product 6-01 (50 mg, 0.14 mmol), tetrahydro-pyran-4-sulfonyl chloride (65 mg, 0.35 mmol) and Cs₂CO₃ (162 mg, 0.50 mmol) in ACN (3 mL) was heated under microwave irradiation at 130 °C for 30 min and at 160 °C for 60 min. More reagents were added to the mixture [sulfonyl chloride (60 mg) and Cs₂CO₃ (100 mg)] and the mixture was heated at 160 °C for 2 h under microwave irradiation. A third addition of reagents was done [sulfonyl chloride (60 mg) and Cs₂CO₃ (100 mg)] and the mixture was heated at 160 °C for 1 h. On cooling, the mixture was evaporated and the residue was purified by column chromatography (Isolute Si II; MeOH:DCM, 0:100 to 10:90) and by prep-HPLC. The white solid was then triturated with MeOH and filtered to give the Final Product 6-28 (3 mg, 4%).

### Method D4

### Preparation of final product 6-14

A mixture of Final Product 6-07 (220 mg, 0.46 mmol) and TFA (2.5 mL) was heated under microwave irradiation at 120 °C for 1 h. The solvent was evaporated, DCM was added and the mixture was evaporated again. This process was repeated three times. 7 M NH₃ in MeOH was then added and the mixture was evaporated. This process was repeated twice. The residue was purified by column chromatography (Isolute Si II 10 g, eluent: 7 M NH₃ in MeOH:DCM, from 2% to 15%) to give Final Product 6-01 (55 mg, 37%) and Final Product 6-14 as a byproduct of this reaction (32 mg, 15%).

### Method D5

### Preparation of final product 6-18

A mixture of Final Product 6-01 (50 mg, 0.14 mmol), acetyl chloride (15 µL, 0.17 mmol) and DIPEA (50 µl, 0.28 mmol) in Acetonitrile (2 mL) was stirred at rt for 16 h. The mixture was diluted with DCM (10 mL) and washed with sat. aq. NaHCO₃ (3 x 15 mL) and brine (20 mL). The organic layer was dried over Na₂SO₄, filtered and evaporated. The residue was dissolved in MeOH (2 mL) and HCl (6 M, 1.5 mL) and stirred at rt for 6 h. The mixture was evaporated and the residue was suspended in DCM (30 mL) and washed with sat. aq. NaHCO₃ (3 x 25 mL) and brine (30 mL). The organic layer was dried over Na₂SO₄, filtered and evaporated. The residue was purified in a chromatroton (MeOH/DCM, 3:97) and triturated with MeOH twice to give the desired Final Product 6-18 (5 mg, 9%) as a white solid.

### Method D6

### Preparation of final product 6-19

A mixture of Final Product 6-01 (50 mg, 0.14 mmol), ethyl isocyanate (15 µL, 0.18 mmol) DIPEA (40 µl, 0.21 mmol) in Acetonitrile (2 mL) was stirred at rt for 16 h. More ethyl isocyanate (10 µL) was added stirring was continued for 6 h. The mixture was evaporated and the residue was purified by column chromatography (Isolute Si II, 10 g; MeOH:DCM, 0:100 to 5:95) to render the desired Final Product 6-19 (28 mg, 45%).

### Preparation of final product 6-39

A mixture of Final Product 6-01 (30 mg, 0.08 mmol), DIPEA (22 µL, 0.12 mmol) and 4-dimethylaminophenyl isocyanate (18 mg, 0.11 mmol) in ACN (4 mL) was stirred at rt for 18 h. More DIPEA (18 µL) and 4-dimethylaminophenyl isocyanate (10 mg) were added and mixture was stirred at rt for 4 h and heated at 60 °C for 24 h. More amounts of reagents [DIPEA (50 µL) and 4-dimethylaminophenyl isocyanate (35 mg)] were added and the mixture was heated at 60 °C for 24 h and under microwave irradiation at 120 °C for 90 min. The mixture was concentrated and the residue was purified by column chromatography (Isolute Si II, 10 g; MeOH/DCM, 0:100 to 5:95) to give the Final Product 6-39 (20 mg, 45%) as a white solid.

### Method D7

### Preparation of final product 6-38

A mixture of Final Product 6-01 (50 mg, 0.14 mmol), AcOH (15 µL, 0.18 mmol) and acetone (15 uL, 0.18 mmol) in DCE (4 mL) was stirred at rt for 45 min. Sodium triacetoxyborohydride (45 mg, 0.21 mmol) was added in one portion and the reaction mixture was stirred at rt for 2 h and heated at 40 °C for 18 h. AcOH (20 µL) and acetone (20 mL) were added and the mixture was heated at 80 °C for 5 h. More AcOH (50 µL), acetone (50 mL) and NaBH(OAc)₃ (50 mg) were added and the heating continued for 18 h. More reagents were added [AcOH (70 µL), acetone (70 mL) and NaBH(OAc)₃ (70 mg)] and the heating at 45 °C continued for 24 h. The mixture was cooled to rt, quenched with 2M KOH and extracted with EtOAc (3 x 25 mL). The combined organic layers were washed with brine, dried over Na₂SO₄ and concentrated in vacuo. The crude was purified by flash column chromatography (Isolute Si II, 10 g; DCM/NH₃ 7N in MeOH, 100:0 to 95:5) and by HPLC to give the Final Product 6-38 (10 mg, 18%) as a white solid.

### Method D8

### Preparation of final product 6-42

A mixture of Intermediate I-39 (70 mg, 0.127 mmol) and amberlyst(r) 15 (800 mg, 1.7 mmol) in MeOH (10 mL) was stirred at rt for 24 h. The mixture was filtered and the resin was suspended in 7 M NH₃ in MeOH and stirred at rt for 1 h. The mixture was filtered and the unbound process was repeated. The filtrates were evaporated and the residue was purified by column chromatography (Isolute Si II, 10 g) (DCM/MeOH (7 M NH₃), 100:0 to 85:15) to give the Final Product 6-42 (22 mg, 38%) as a white solid.

### General Procedure

The HPLC measurement was performed using a HP 1100 from Agilent Technologies comprising a pump (binary) with degasser, an autosampler, a column oven, a diode-array detector (DAD) and a column as specified in the respective methods below. Flow from the column was split to a MS spectrometer. The MS detector was configured with an electrospray ionization source or API/APCI. Nitrogen was used as the nebulizer gas. The source temperature was maintained at 150 °C. Data acquisition was performed with ChemStation LC/MSD quad, software.

### Method 1

Reversed phase HPLC was carried out on a RP-C18 Gemini column (150 x 4.6 mm, 5 um); 10 min. linear gradient of 50- 100% acetonitrile in water + 100 % acetonitrile in water 2 min ): 210 nm and 254 or DAD.

### Method 2

Reversed phase HPLC was carried out on a Gemini-NX C18 (100 x 2.0 mm; 5um), Solvent A: water with 0.1% formic acid; Solvent B: acetonitrile with 0.1% formic acid. Gradient: 5% of B to 100% of B within 8 min at 50 °C, DAD.

### Method 3

Reversed phase HPLC was carried out on a Gemini-NX C18 (100 x 2.0 mm; 5um), Solvent A: water with 0.1 % formic acid; Solvent B: acetonitrile with 0.1 % formic acid. Gradient: 5% of B to 40% of B within 8 min at 50 °C, DAD.

### Method 4

Reversed phase HPLC was carried out on a Gemini-NX C18 (100 x 2.0 mm; 5um), Solvent A: water with 0.1% formic acid; Solvent B: acetonitrile with 0.1% formic acid. Gradient: 0% of B to 30% of B within 8 min at 50 °C, DAD.

**Table 8: Analytical data and PI3Kα activity -** Rₜ means retention time (in minutes), [M+H]⁺ means the protonated mass of the compound, method refers to the method used for (LC)MS.

Biological activity in PI3Kα for certain examples is represented in Table 8 by semi-quantative results: IC50 >1µM (+), IC50 <500 nM (+++), 500 nM<IC50< 1 µM (++). Biological activity in PI3Kα and cellular p-AKT for certain examples is represented in Table 8 by quantitative results: IC50 (µM)

**Table 8**

| **Cpd Nr.** | **Rₜ** | **[M+1]⁺** | **Meth** | **PI3K α IC50** | **PI3K α IC50** | **p-AKT IC50** | **p-AKT ELISA** | **¹H NMR (300 MHz; δ in ppm, *J* in Hz)** |
|---|---|---|---|---|---|---|---|---|
| **2-01** | 6.73 | 424.2 | 2 | +++ | 0.207 | | | DMSO δ 9.41 (s, 1H), 8.35 (s, 1H), 7.51 (m, 2H), 7.22 (t, *J* = 8.1, 1H), 6.75 (m, 1H), 4.80 (s, 2H), 4.24 (m, 4H), 4.13 (q, *J* = 7.1, 2H), 3.77 (m, 6H), 2.79 (t, *J =* 5.2, 2H), 1.24 *(t, J* = 7.1, 3H). |
| **2-02** | 0.53 | 352.1 | 3 | +++ | 0.185 | | | DMSO δ 9.42 (s, 1H), 8.13 (s, 1H), 7.46 (m, 2H), 7.21 (t, *J* = 7.8, 1H), 6.74 (ddd, *J* = 7.9, 2.2, 0.6, 1H), 4.23 (m, 4H), 4.00 (s, 2H), 3.77 (m, 4H), 3.02 (t, *J* = 5.5, 2H), 2.67 (t, *J* = 5.1, 2H). |
| **2-03** | 6.60 | 448.2 | 2 | + | - | | | DMSO δ 13.16 (s, 1H), 8.59 (s, 1H), 8.36 (s, 1H), 7.69 *(d, J* = 6.9, 1H), 7.55 (d, *J* = 8.3, 1H), 7.42 (dd, *J* = 8.2, 7.3, 1H), 4.86 (s, 2H), 4.27 (m, 4H), 4.13 (q, *J* = 7.1, 2H), 3.81 (m, 6H), 2.82 *(t, J* = 5.2, 2H), 1.24 (t, *J=* 7.1, 3H)*.* |
| **2-04** | 0.43 | 376.1 | 3 | ++ | 0.755 | 0.056 | | MeOD δ 8.54 (d, *J* = 0.7, 1H), 7.92 (s, 1H), 7.54 (d, *J* = 7.1, 1H), 7.50 (d, *J* = 8.3, 1H), 7.39 (dd, *J* = 8.3, 7.2, 1H), 4.18 (m, 4H), 4.04 (s, 2H), 3.88 (m, 4H), 3.19 (t, *J* = 5.7, 2H), 2.85 (t, *J* = 5.6, 2H). |
| **2-05** | 4.55 | 467.2 | 3 | + | - | | | DMSO δ 13.15 (s, 1H), 8.61 (d, *J* = 1.6, 1H), 8.55 (s, 1H), 8.51 *(*dd, *J* = 4.8, 1.6, 1H), 8.20 (s, 1H), 7.82 (dt, *J* = 7.8, 1.9, 1H), 7.62 (d, *J* = 7.1, 1H), 7.54 (d, *J* = 8.3, 1H), 7.40 (m, 2H), 4.27 (m, 4H), 3.90 (s, 2H), 3.85 (s, 2H), 3.81 (m, 4H), 2.91 (t, *J* = 5.2, 2H), 2.82 (t, *J* = 4.9, 2H). |
| **2-06** | 3.91 | 473.2 | 2 | + | - | | | DMSO δ 13.15 (s, 1H), 8.56 (s, 1H), 8.20 (s, 1H), 7.76 *(d, J* = 3.2, 1H), 7.69 *(d, J* = 3.2, 1H), 7.63 (d, *J* = 7.1, 1H), 7.54 (d, *J* = 8.2, 1H), 7.39 (m, 1H), 4.27 (m, 4H), 4.16 (s, 2H), 4.05 (s, 2H), 3.81 (m, 4H), 3.01 (t, *J* = 4.9, 2H), 2.84 (m, 2H). |
| **2-07** | 4.73 | 467.2 | 3 | ++ | 0.852 | | | DMSO δ 13.16 (s, 1H), 8.55 (s, 3H), 8.18 (s, 1H), 7.61 (m, 1H), 7.53 *(d, J =* 8.3, 1H), 7.40 (m, 3H), 4.27 (m, 4H), 3.91 (s, 2H), 3.85 (s, 2H), 3.80 (m, 4H), 2.90 (t, *J* = 4.8, 2H), 2.84 (t, *J =* 4.8, 2H). |
| **2-08** | 4.66 | 467.2 | 3 | +++ | 0.439 | | | DMSO δ 13.17 (s, 1H), 8.55 *(*d, *J* = 0.6, 1H), 8.53 (m, 1H), 8.18 (s, 1H), 7.80 (td, *J* = 7.6, 1.8, 1H), 7.62 (d, *J* = 7.1, 1H), 7.54 (m, 2H), 7.39 *(*dd, *J* = 8.3, 7.2, 1H), 7.29 (ddd, *J* = 7.4, 4.9, 1.1, 1H), 4.27 (m, 4H), 3.95 (s, 2H), 3.94 (s, 2H), 3.80 (m, 4H), 2.94 *(t, J =* 5.3, 2H), 2.83 *(t, J* = 4.8, 2H). |
| **2-09** | 4.18 | 474.2 | 3 | + | - | | | DMSO δ 13.17 (s, 1H), 8.58 (s, 1H), 8.21 (s, 1H), 7.65 *(d, J* = 6.8, 1H), 7.55 (d, *J* = 8.3, 1H), 7.42 (dd, *J* = 8.2, 7.3, 1H), 4.26 (m, 4H), 3.82 (m, 8H), 3.34 (m, 4H), 2.82 (dd, *J* = 12.9, 4.3, 2H), 2.46 *(d, J* = 7.2, 2H), 1.94 (m, 1H), 1.67 (d, *J* = 11.5, 2H), 1.17 (dd, *J* = 20.4,11.7, 2H). |
| **2-10** | 5.54 | 544.2 | 3 | + | - | | | DMSO δ 13.17 (s, 1H), 8.55 (s, 1H), 8.19 (s, 1H), 7.92 *(d, J* = 8.3, 2H), 7.69 (d, *J* = 8.3, 2H), 7.62 (d, *J* = 6.9, 1H), 7.53 (d, *J* = 8.3, 1H), 7.39 (m, 1H), 4.27 (m, 4H), 3.93 (s, 4H), 3.80 (m, 4H), 3.21 (s, 3H), 2.91 (d, *J* = 4.8, 2H), 2.84 (d, *J* = 4.3, 2H). |
| **2-11** | 5.02 | 456.2 | 3 | + | 1.78 | | | DMSO δ 13.16 (s, 1H), 8.56 (s, 1H), 8.19 *(d, J =* 5.8, 1H), 7.63 (m, 2H), 7.54 (d, *J* = 8.3, 1H), 7.41 (dd, *J* = 8.2, 7.2, 1H), 6.44 (dd, *J* = 3.1, 1.8, 1H), 6.41 *(d, J =* 2.6, 1H), 4.26 (m, 4H), 3.89 (s, 2H), 3.84 (s, 2H), 3.80 (m, 4H), 2.90 (t, *J* = 5.3, 2H), 2.80 (m, 2H). |
| **2-12** | 4.62 | 456.2 | 3 | ++ | 0.708 | | | DMSO δ 13.17 (s, 1H), 8.55 (d, *J* = 0.7, 1H), 8.17 (s, 1H), 7.64 (m, 3H), 7.54 (d, *J* = 8.3, 1H), 7.40 (dd, *J* = 8.2, 7.3, 1H), 6.52 (m, 1H), 4.26 (m, 4H), 3.85 (s, 2H), 3.80 (m, 4H), 3.65 (s, 2H), 2.85 (m 2H), 2.79 (m, 2H). |
| **2-13** | 4.19 | 456.2 | 3 | + | - | | | DMSO δ 13.17 (s, 1H), 8.55 (d, *J* = 0.8, 1H), 8.18 (s, 1H), 7.64 (d, *J* = 7.1, 1H), 7.54 *(d, J* = 8.3, 1H), 7.40 (dd, *J* = 8.3, 7.2, 1H), 6.97 (s, 2H), 4.27 (m, 4H), 3.93 (s, 2H), 3.84 (s, 2H), 3.80 (m, 4H), 2.89 (t, *J* = 4.8, 2H), 2.81 (t, *J =* 4.7, 2H). |
| **2-14** | 2.59 | 445.2 | 3 | + | - | | | DMSO δ 8.56 (d, *J* = 0.7, 1H), 8.46 (s, 1H), 8.19 (s, 1H), 7.64 (d, *J* = 6.9, 1H), 7.56 (d, *J* = 8.3, 1H), 7.41 (dd, *J* = 8.2, 7.3, 1H), 4.26 (m, 4H), 3.97 (t, *J* = 9.1, 2H), 3.80 (m, 6H), 3.65 (dd, *J* = 9.2, 7.4, 2H), 3.11 (m, 1H), 2.83 (m, 6H). |
| **2-15** | 5.43 | 544.2 | 3 | + | 3.33 | | | DMSO δ 13.15 (s, 1H), 8.54 (s, 1H), 8.18 (s, 1H), 7.97 (s, 1H), 7.85 (d, *J =* 7.8, 1H), 7.77 *(d, J* = 7.7, 1H), 7.66 (d*, J* = 7.7, 1H), 7.61 *(d, J* = 6.8, 1H), 7.39 (m, 1H), 4.27 (m, 4H), 3.93 (s, 4H), 3.80 (m, 4H), 3.23 (s, 3H), 2.92 (m, 2H), 2.83 (m, 2H). |
| **2-16** | 7.17 | 425.2 | 3 | +++ | 0.067 | 0.128 | | DMSO δ 8.88 (s, 2H), 8.32 (s, 1H), 6.82 (s, 2H), 4.76 (s, 2H), 4.22 (m, 4H), 4.13 (q, *J* = 7.1, 2H), 3.77 (m, 6H), 2.78 (t, *J* = 5.0, 2H), 1.24 (t, *J* = 7.1, 3H). |
| **2-17** | 1.62 | 353.2 | 3 | +++ | - | | | DMSO δ 8.85 (s, 2H), 8.16 (s, 1H), 6.81 (s, 2H), 4.20 (m, 4H), 4.03 (s, 2H), 3.75 (m, 6H), 3.08 (t, *J* = 4.9, 2H), 2.72 (t, *J* = 5.0, 2H). |
| **2-18** | 2.43 | 444.2 | 2 | +++ | 0.069 | 0.245 | | DMSO δ 8.83 (s, 2H), 8.53 (d, *J* = 4.0, 1H), 8.16 (s, 1H), 7.80 (td, *J* = 7.7, 1.8, 1H), 7.54 (d, *J* = 7.8, 1H), 7.29 (dd, *J* = 6.4, 5.0, 1H), 6.80 (s, 2H), 4.22 (m, 4H), 3.93 (s, 2H), 3.84 (s, 2H), 3.76 (m, 4H), 2.95 *(t, J =* 5.2, 2H), 2.81 (t, *J* = 4.7, 2H). |
| **2-19** | 5.14 | 440.2 | 2 | ++ | 0.724 | | | DMSO δ 9.19 (s, 2H), 8.48 (s, 1H), 4.77 (s, 2H), 4.23 (t, *J* = 4.4, 4H), 4.14 (q, *J =* 7.1, 2H), 3.96 (s, 3H), 3.77 (m, 6H), 2.78 (t, *J* = 5.2, 2H), 1.24 (t, *J* = 7.1, 3H). |
| **2-20** | 4.82 | 448.2 | 2 | + | 1.05 | | | DMSO δ 11.71 (s, 1H), 8.96 *(d, J* = 2.0, 1H), 8.60 (d, *J* = 1.9, 1H), 8.46 (s, 1H), 7.49 (m, 1H), 6.51 (dd, *J* = 3.3, 1.7, 1H), 4.82 (s, 2H), 4.26 (t, *J* = 4.4, 4H), 4.14 (q, *J* = 7.1, 2H), 3.79 (m, 6H), 2.80 *(*t, *J* = 5.0, 2H), 1.24 *(t, J* = 7.1, 3H). |
| **2-21** | 4.85 | 424.2 | 3 | + | - | | | DMSO δ 8.63 (s, 1H), 8.23 (s, 1H), 8.03 (d, *J* = 6.8, 1H), 6.50 (d, *J* = 8.6, 1H), 6.09 (s, 2H), 4.77 (s, 2H), 4.21 (s, 4H), 4.13 (q, *J* = 7.0, 2H), 3.77 (m, 6H), 2.78 (s, 2H), 1.24 (t, *J* = 7.0, 3H). |
| **2-22** | 6.38 | **447.3** | 2 | + | - | | | DMSO δ 11.21 (s, 1H), 8.13 (s, 1H), 7.52 (d, *J* = 7.3, 1H), 7.42 (m, 2H), 7.16 (m, 1H), 6.97 (m, 1H), 4.83 (s, 2H), 4.25 (m, 4H), 4.13 (q, *J* = 7.1, 2H), 3.79 (m, 6H), 2.82 (t, *J* = 5.3, 2H), 1.23 (t, *J* = 7.1, 3H). |
| **2-23** | 4.47 | 439.3 | 2 | + | 2.7 | | | DMSO δ 8.89 (d, *J* = 1.6, 1H), 8.54 (s, 1H), 8.26 (d, *J* = 2.8, 1H), 7.93 (m, 1H), 4.80 (s, 2H), 4.23 (m, 4H), 4.14 (q, *J* = 7.1, 2H), 3.91 (s, 3H), 3.78 (m, 6H), 2.79 (t, *J* = 5.2, 2H), 1.24 (t, *J* = 7.1, 3H). |
| **2-24** | 3.74 | 409.2 | 2 | + | - | | | DMSO δ 9.28 (d, *J* = 1.7, 1H), 8.55 (dd, *J* = 4.8, 1.6, 2H), 8.42 (m, 1H), 7.47 (dd, *J* = 7.7, 4.5, 1H), 4.81 (s, 2H), 4.25 (m, 4H), 4.14 (q, *J* = 7.1, 2H), 3.78 (m, 6H), 2.80 (t, *J* = 5.4, 2H), 1.24 (t, J = 7.1, 3H). |
| **2-25** | 5.51 | 492.2 | 2 | + | - | | | DMSO δ 8.90 (d, *J* = 1.7, 1H), 8.41 (s, 1H), 8.35 (d, *J* = 1.8, 1H), 6.62 (s, 2H), 4.78 (s, 2H), 4.21 (m, 4H), 4.13 *(q, J* = 7.1, 2H), 3.76 (m, 6H), 2.78 (t, *J* = 5.0, 2H), 1.24 (t, *J* = 7.1, 3H). |
| **2-26** | 5.42 | 465.2 | 2 | + | - | | | DMSO δ 11.25 (s, 1H), 8.02 (d, *J* = 1.7, 1H), 7.42 (m, 2H), 7.02 (dd, *J* = 11.0, 8.8, 1H), 6.67 *(*d, *J* = 2.1, 1H), 4.76 (s, 2H), 4.20 (m, 4H), 4.11 (q, *J* = 7.1, 2H), 3.77 (m, 6H), 2.82 (t, J = 5.2, 2H), 1.22 (t, *J* = 7.1, 3H). |
| **2-27** | 5.63 | 474.2 | 2 | **++** | 0.603 | | | DMSO δ 8.94 (s, 2H), 5.17 (s, 2H), 4.16 (m, 4H), 4.10 (q, *J* = 7.1, 2H), 3.98 (s, 3H), 3.73 (m, 6H), 2.82 (t, *J* = 5.2, 2H), 1.22 (t, *J* = 7.1, 3H). |
| **2-28** | 5.38 | 482.2 | 2 | + | 1.07 | | | DMSO δ 11.80 (s, 1H), 8.51 (d, *J* = 2.0, 1H), 8.23 (d, *J* = 2.0, 1H), 7.53 (m, 1H), 6.52 (dd, J = 3.4, 1.7, 1H), 5.20 (s, 2H), 4.15 (m, 4H), 4.12 (q, *J* = 7.0, 2H), 3.74 (m, 6H), 2.83 (t, *J* = 5.1, 2H), 1.22 (t, *J* = 7.0, 3H). |
| **2-29** | 5.94 | 481.2 | 2 | + | - | | | DMSO δ 11.21 (s, 1H), 7.46 (m, 1H), 7.37 (m, 1H), 7.17 (d, *J* = 1.2, 1H), 7.15 (s, 1H), 6.36 (s, 1H), 5.19 (s, 2H), 4.12 (m, 6H), 3.74 (m, 6H), 2.84 (t, *J* = 5.4, 2H), 1.22 (t, *J* = 6.9, 3H). |
| **2-30** | 4.21 | 473.2 | 2 | + | - | | | DMSO δ 8.48 (d, *J* = 1.6, 1H), 8.33 *(d, J* = 2.8, 1H), 7.62 (dd, *J* = 2.6, 1.8, 1H), 5.18 (s, 2H), 4.12 (m, 6H), 3.88 (s, 3H), 3.73 *(t, J =* 5.2, 6H), 2.82 (s, 2H), 1.22 (t, J = 7.0, 3H). |
| **2-31** | 4.50 | 443.2 | 2 | + | - | | | DMSO δ 8.89 (d, *J* = 1.4, 1H), 8.61 (dd, *J* = 4.6, 1.1, 1H), 8.09 (m, 1H), 7.51 (dd, *J* = 7.8, 4.8, 1H), 5.18 (s, 2H), 4.15 (m, 4H), 4.12 (q, *J* = 7.0, 2H), 3.73 (m, 6H), 2.82 *(t, J* = 5.2, 2H), 1.22 (t, *J* = 7.0, 3H). |
| **2-32** | 4.61 | 459.2 | 2 | +++ | 0.084 | 0.078 | | DMSO δ 8.58 (s, 2H), 6.94 (s, 2H), 5.17 (s, 2H), 4.15 (m, 4H), 4.12 (q, *J* = 7.1, 2H), 3.73 (m, 6H), 2.81 (t, *J* = 5.4, 2H), 1.22 (t, *J* = 7.1, 3H). |
| **2-33** | 6.00 | 526.2 | 2 | **+** | 1.78 | | | DMSO δ 8.54 (d, *J* = 1.8, 1H), 8.02 *(d, J* = 2.0, 1H), 6.76 (s, 2H), 5.16 (s, 2H), 4.11 (m, 6H), 3.73 *(t, J =* 4.6, 6H), 2.80 (t, *J* = 5.1, 2H), 1.21 (t, *J* = 7.0, 3H). |
| **2-34** | 0.33 | 337.2 | 3 | +++ | 0.427 | | | DMSO δ 9.25 (d, *J* = 1.6, 1H), 8.54 (dd, *J* = 4.7, 1.6, 1H), 8.40 (m, 2H), 7.46 (ddd, *J* = 8.0, 4.8, 0.6, 1H), 4.25 (m, 4H), 4.03 (s, 2H), 3.77 (m, 4H), 3.05 (t, *J =* 5.5, 2H), 2.70 (t, *J* = 5.3, 2H). |
| **2-35** | 2.19 and 2.43 | 409.3 | 2 | **++** | 0.692 | | | DMSO δ 8.85 (s, 2H), 8.20 (s, 1H), 6.81 (s, 2H), 4.22 (m, 4H), 3.75 (m, 6H), 2.79 (dd, *J* = 14.3, 4.7, 4H), 2.35 (d, *J* = 7.3, 2H), 1.94 (dt, *J* = 14.0, 6.9, 1H), 0.92 (d, *J* = 6.5, 6H). |
| **2-36** | 0.38 | 451.3 | 2 | + | - | | | DMSO δ 8.85 (s, 2H), 8.19 (s, 1H), 6.82 (s, 2H), 4.22 (m, 4H), 3.86 (dd, J = 11.4, 2.6, 2H), 3.76 (m, 6H), 2.80 (dd, *J* = 19.3, 4.8, 4H), 2.45 *(d, J* = 7.2, 2H), 1.91 (m, 1H), 1.67 (d, J = 11.1, 2H), 1.19 (m, 2H) (2H under solvent signals). |
| **2-37** | 2.90 | 449.3 | 2 | +++ | 0.363 | | | DMSO δ 8.86 (s, 2H), 8.20 (s, 1H), 6.81 (s, 2H), 4.21 (m, 4H), 3.75 (m, 4H), 3.71 (s, 2H), 2.78 (dd, *J* = 13.3, 4.1, 4H), 2.40 (d, J = 7.0, 2H), 1.79 (d, *J* = 12.7, 2H), 1.67 (m, 4H), 1.24 (m, 3H), 0.89 (m, 2H). |
| **2-38** | 3.52 | 424.2 | 2 | **++** | 0.013 | | 1.23 | DMSO δ 8.87 (s, 2H), 8.24 (s, 1H), 6.78 (s, 2H), 4.69 (s, 2H), 4.22 (m, 4H), 3.76 (m, 4H), 3.71 (t, *J* = 5.5, 2H), 3.11 (m, 2H), 3.10 (m, 2H), 2.75 (t, *J* = 5.5, 2H), 1.04 (t, *J* = 7.1, 3H). |
| **2-39** | 5.69 | 431.3 | 3 | +++ | 0.019 | | 0.539 | DMSO δ 8.88 (s, 2H), 8.34 (s, 1H), 6.84 (s, 2H), 4.66 (s, 2H), 4.23 (m, 4H), 3.77 (m, 4H), 3.64 (t, *J* = 5.5, 2H), 3.00 (s, 3H), 2.90 (t, *J* = 5.3, 2H). |
| **2-40** | 4.31 | 507.4 | 3 | + | - | | | DMSO δ 8.85 (d, *J* = 1.3, 2H), 8.21 (d, *J* = 7.0, 1H), 6.81 (s, 2H), 4.21 (m, 4H), 4.08 (dd, *J* = 14.4, 7.2, 2H), 3.80 (m, 6H), 2.88 (m, 2H), 2.72 (m, 2H), 2.56 (m, 2H), 1.99 (m, 2H), 1.69 (m, 2H), 1.55 (m, 2H), 1.19 (m, 5H). |
| **2-41** | 4.62 | 410.2 | 2 | + | - | | | DMSO δ 9.43 (s, 2H), 9.16 (s, 1H), 8.67 (s, 1H), 4.80 (s, 2H), 4.27 (m, 4H), 4.14 (q, *J* = 7.1, 2H), 3.78 (m, 6H), 2.80 *(t, J* = 5.1, 2H), 1.24 (t, *J* = 7.1, 3H). |
| **2-42** | 3.39 | 395.2 | 2 | +++ | 0.045 | 0.294 | 0.648 | DMSO δ 8.89 (s, 2H), 8.36 (s, 1H), 6.82 (s, 1H), 4.82 (s, 2H), 4.22 (m, 4H), 3.83 (m, 2H), 3.76 (dd, *J* = 6.9, 2.3, 4H), 2.86 (t, *J* = 5.0, 1H), 2.73 (m, 1H), 2.17 (s, 3H). |
| **2-43** | 3.95 | 423.3 | 2 | ++ | 0.55 | | | DMSO δ 8.83 (s, 2H), 8.29 (s, 1H), 6.82 (s, 1H), 4.75 (s, 2H), 4.15 (m, 4H), 3.81 (t, *J* = 5.1, 2H), 3.69 (m, 4H), 2.98 (dt, *J* = 13.4, 6.7, 1H), 2.79 (m, 1H), 2.67 (m, 1H), 0.99 (t, *J* = 6.6, 6H). |
| **2-44** | 3.81 | 438.3 | 2 | +++ | - | | | DMSO δ 8.80 (s, 2H), 8.16 (s, 1H), 6.76 (s, 2H), 6.33 (d, *J* = 7.5, 1H), 4.61 (s, 2H), 4.15 (t, *J* = 4.4, 4H), 3.69 (m, 7H), 2.68 (s, 2H), 1.02 (d, *J* = 6.6, 6H). |
| **2-45** | 0.37 | 381.2 | 3 | +++ | 0.257 | | | CDCl₃ δ 8.79 (s, 2H), 7.42 (s, 1H), 5.11 (s, 2H), 4.38-4.21 (m, 4H), 3.92 - 3.76 (m, 4H), 3.74 (s, 2H), 2.91 (s, 4H), 2.74 (q, *J* = 7.2, 2H), 1.23 (t, *J* = 7.2, 3H). |
| **2-46** | 2.23 | 407.2 | 2 | ++ | - | | | DMSO δ 8.68 (s, 2H), 8.01 (s, 1H), 6.63 (s, 2H), 4.04 (m, 4H), 3.65 (s, 2H), 3.58 (m, 4H), 2.72 (m, 2H), 2.59 (m, 2H), 0.80 (m, 1H), 0.36 (m, 2H), 0.01 (m,2H). |
| **2-47** | 3.12 | 461.3 | 2 | + | - | | | DMSO δ 8.68 (s, 2H), 7.97 (s, 1H), 7.24 (s, 1H), 4.01 (m, 4H), 3.61 (s, 1H), 3.54 (m, 4H), 2.99 (t, *J* = 6.3, 1H), 2.69 (s, 1H), 2.55 (s, 1H), 0.81 (d, *J* = 28.9, 2H), 0.33 *(d, J* = 6.4, OH), 0.20 (d, *J* = 6.3, 1H), -0.01 (dd, *J* = 10.2, 5.7, 2H). |
| **2-48** | 3.77 | 411.2 | 2 | | 0.1 | | | DMSO δ 8.88 (s, 2H), 8.33 (s, 1H), 6.83 (s, 2H), 4.77 (s, 2H), 4.21 (d, *J* = 4.5, 4H), 3.77 (dd, *J* = 10.8, 6.8, 6H), 3.68 (s, 3H), 2.79 (d, *J* = 5.2, 2H). |
| **2-49** | 3.85 | 465.2 | 2 | | 0.009 | | 0.095 | DMSO δ 8.51 (s, 2H), 6.88 (s, 2H), 4.97 (s, 2H), 4.09 (s, 4H), 3.68 (d, *J* = 4.9, 4H), 3.53 (s, 2H), 2.96 (s, 3H), 2.85 (s, 2H). |
| **2-50** | 3.83 | 457.3 | 2 | | 0.025 | | 0.319 | CDCl₃ 6 8.84 (s, 2H), 7.49 (s, 1H), 5.41 (s, 2H), 4.68 (s, 2H), 4.34 (m, 4H), 3.88 (m, 4H), 3.78 (t, *J* = 5.8, 2H), 3.03 (t, *J* = 5.7, 2H), 2.35 (m, 1H), 1.26 (m, 2H), 1.01 (m, 2H). |
| **2-51** | 4.23 | 475.4 | 2 | | 0.037 | 0.120 | 0.477 | CDCl₃ δ 8.82 (s, 2H), 7.51 (m, 3H), 7.16 (t, *J* = 8.5, 2H), 5.30 (s, 2H), 4.34 (m, 4H), 3.88 (m, 6H), 2.97 (s, 2H). |
| **2-52** | 4.47 | 493.3 | 2 | | - | - | | DMSO δ 8.88 (s, 2H), 8.32 (s, 1H), 7.87 (m, 2H), 7.67 (m, 3H), 7.00 (s, 2H), 4.55 (s, 2H), 4.18 (m, 4H), 3.74 (m, 4H), 3.54 (t, *J* = 5.5, 2H), 2.78 (m, 2H). |
| **2-53** | 3.71 | 438.2 | 2 | | 0.062 | | 0.564 | MeOD δ 8.71 (s, 2H), 7.85 (s, 1H), 4.62 (s, 2H), 4.08 (m, 4H), 3.73 (m, 6H), 3.08 (m, 2H), 2.75 (t, *J* = 5.6, 2H), 1.46 (m, 2H), 0.83 (t, *J* = 7.4, 3H). |
| **2-54** | 4.11 | 472.2 | 2 | | 0.148 | | | MeOD δ 8.64 (s, 2H), 5.20 (s, 2H), 4.15 (m, 4H), 3.81 (m, 4H), 3.75 (t, *J* = 5.7, 2H), 3.15 (m, 2H), 2.86 (t, *J* = 5.4, 2H), 1.53 (m, 2H), 0.91 (t, *J* = 7.4, 3H). |
| **2-55** | 4.43 | 473.2 | 2 | | 0.033 | | | CDCl₃ δ 8.82 (s, 2H), 7.46 (s, 1H), 5.21 (s, 2H), 4.67 (s, 2H), 4.36 (m, 4H), 3.90 (m, 4H), 3.78 (t, *J* = 5.6, 2H), 3.05 (m, 4H), 1.81 (m, 2H), 1.46 (m, 2H), 0.96 (t, *J* = 7.3, 3H). |
| **2-56** | 3.79 | 501.2 | 2 | | - | | | CDCl₃ δ 8.79 (s, 2H), 7.43 (s, 1H), 5.18 (s, 2H), 4.68 (s, 2H), 4.33 (m, 4H), 4.07 (d, *J* = 11.4, 2H), 3.87 (m, 4H), 3.76 (dd, *J* = 14.2, 8.6, 2H), 3.36 (m, 2H), 3.23 (m, 1H), 2.99 *(t, J* = 5.4, 2H), 1.95 (m, 4H). |
| **2-57** | 4.21 | 491.1 | 2 | | - | | | CDCl₃ δ 8.72 (d, *J* = 5.7, 2H), 5.49 (s, 2H), 5.18 (s, 2H), 4.26 (m, 4H), 3.86 (m, 4H), 3.75 (t, J = 5.8, 2H), 3.04 (t, J = 5.7, 2H), 2.36 (m, 1H), 1.24 (m, 2H), 1.01 (m, 2H). |
| **2-58** | 4.89 | 527.2 | 2 | | 0.101 | | | CDCl₃ δ 8.67 (s, 2H), 7.85 (dd, *J* = 8.2, 1.4, 2H), 7.56 (m, 3H), 5.27 (s, 2H), 4.96 (s, 2H), 4.20 (m, 4H), 3.81 (m, 4H), 3.53 (t, *J* = 5.8, 2H), 2.92 (t, *J* = 5.7, 2H). |
| **2-59** | 3.96 | 459.4 | 2 | | 0.077 | | 0.853 | CDCl₃ δ 8.82 (s, 2H), 7.46 (s, 1H), 5.21 (s, 2H), 4.70 (s, 2H), 4.36 (m, 4H), 3.90 (m, 4H), 3.80 (t, *J* = 5.7, 2H), 3.32 (m, 1H), 3.02 (m, 2H), 1.41 (d, *J* = 6.7, 6H). |
| **2-60** | 4.36 | 493.2 | 2 | | - | | | CDCl₃ δ 8.72 (s, 2H), 5.23 (s, 2H), 5.17 (s, 2H), 4.26 (m, 4H), 3.86 (m, 4H), 3.75 (t, *J* = 5.7, 2H), 3.31 (dt, *J* = 13.6, 6.8, 1H), 3.01 (t, *J* = 5.7, 2H), 1.41 (d, *J* = 6.6, 6H). |
| **2-61** | 4.07 | 479.2 | 2 | | - | | | CDCl₃ δ 8.72 (s, 2H), 5.20 (s, 2H), 5.16 (s, 2H), 4.26 (m, 4H), 3.86 (m, 4H), 3.74 (t, *J =* 5.8, 2H), 3.10 (q, *J =* 7.4, 2H), 3.02 (t, *J* = 5.8, 2H), 1.41 (t, *J* = 7.4, 3H). |
| **2-62** | 2.77 | 461.2 | 2 | | - | | | CDCl₃ δ 8.69 (s, 2H), 7.30 (m, 3H), 6.99 (m, 2H), 5.16 (s, 2H), 4.25 (m, 4H), 3.80 (m, 4H), 3.73 (s, 2H), 3.64 (s, 2H), 2.88 (m, 4H). |
| **2-63** | 0.40 and 2.42 | 395.1 | 3 | | 0.313 | | | CDCl₃ δ 8.80 (s, 2H), 7.43 (s, 1H), 5.23 (s, 2H), 4.30 (m, 4H), 3.85 (m, 4H), 3.75 (s, 2H), 3.08 (dq, *J* = 13.0, 6.6, 1H), 2.91 (m, 4H), 1.19 (d, *J* = 6.5, 6H). |
| **2-64** | 4.67 | 511.2 | 2 | | - | | | CDCl₃ δ 8.78 (s, 2H), 7.89 (m, 2H), 7.40 (s, 1H), 7.21 (m, 2H), 5.17 (s, 2H), 4.45 (s, 2H), 4.29 (m, 4H), 3.84 (m, 4H), 3.57 *(t, J* = 5.7, 2H), 2.93 (t, *J* = 5.7, 2H). |
| **2-65** | 4.29 | 504.2 | 2 | | 0.042 | | 0.398 | CDCl₃ δ 8.74 (s, 2H), 7.41 (s, 1H), 7.27 (m, 2H), 6.97 (m, 2H), 5.31 (s, 2H), 5.15 (t, *J* = 5.5, 1H), 4.71 (s, 2H), 4.40 (d, *J* = 5.8, 2H), 4.28 (m, 4H), 3.84 (m, 4H), 3.74 (t, *J* = 5.6, 2H), 2.90 (t, *J* = 5.5, 2H). |
| **2-66** | 4.16 | 464.1 | 2 | | 0.057 | | 0.546 | CDCl₃ δ 8.76 (s, 2H), 7.48 (s, 1H), 5.18 (s, 2H), 4.68 (s, 2H), 4.56 *(d, J* = 13.6, 1H), 4.30 (m, 4H), 4.13 (m, 1H), 3.87 (m, 4H), 3.70 (t, *J* = 5.6, 2H), 2.90 (t, *J* = 5.5, 2H), 2.03 (m, 2H), 1.62 (m, 4H), 1.38 (m, 2H). |
| **2-67** | 4.40 | 478.3 | 2 | | 0.043 | | 0.500 | CDCl₃ δ 8.80 (s, 2H), 7.49 (s, 1H), 5.35 (s, 2H), 4.71 (s, 2H), 4.58 (d, *J* = 7.5, 1H), 4.32 (m, 4H), 3.88 (m, 4H), 3.70 (m, 3H), 2.93 (t, *J* = 5.5, 2H), 1.98 (m, 2H), 1.67 (m, 2H), 1.37 (m, 2H), 1.18 (m, 4H). |
| **2-68** | 3.37 | 454.1 | 2 | | - | | | CDCl₃ δ 8.81 (s, 2H), 7.46 (s, 1H), 5.28 (s, 2H), 5.19 (m, 1H), 4.72 (s, 2H), 4.33 (m, 4H), 3.91 (m, 4H), 3.77 (t, *J* = 5.7, 2H), 3.50 (m, 4H), 3.39 (s, 3H), 2.94 (t, *J* = 5.6, 2H). |
| **2-69** | 4.37 | 485.0 | 2 | | 0.023 | | 0.450 | CDCl₃ δ 8.81 (s, 2H), 7.45 (s, 1H), 5.21 (s, 2H), 4.68 (s, 2H), 4.35 (m, 4H), 3.89 (m, 4H), 3.79 *(t, J* = 5.7, 2H), 3.57 (m, 1H), 3.00 (t, *J* = 5.6, 2H), 2.03 (m, 4H), 1.71 (m, 4H). |
| **2-70** | 3.51 | 482.1 | 2 | | - | | | CDCl₃ δ 8.80 (s, 2H), 7.45 (s, 1H), 5.40 (m, 1H), 5.28 (s, 2H), 4.74 (s, 2H), 4.32 (m, 4H), 4.23 (q, *J* = 7.1, 2H), 4.06 (d, *J* = 6.6, 2H), 3.87 (m, 4H), 3.80 (t, *J* = 5.6, 2H), 2.95 (t, *J* = 5.4, 2H), 1.29 (t, *J* = 7.2, 3H). |
| **2-71** | 4.25 | 490.1 | 2 | | 0.050 | | | CDCl₃ δ 8.78 (s, 2H), 7.45 (s, 1H), 7.30 (m, 2H), 6.96 (m, 2H), 6.70 (s, 1H), 5.24 (s, 2H), 4.78 (s, 2H), 4.30 (m, 4H), 3.85 (m, 6H), 2.98 (t, *J* = 5.5, 2H). |
| **2-72** | 4.32 | 502.1 | 2 | | 0.047 | | 0.473 | CDCl₃ δ 8.83 (s, 2H), 8.10 (m, 1H), 7.54 (s, 1H), 7.29 (s, 1H), 6.97 (m, 3H), 5.25 (s, 2H), 4.86 (s, 2H), 4.35 (m, 4H), 3.94 (s, 3H), 3.90 (m, 6H), 3.05 (t, *J* = 5.6, 2H). |
| **2-73** | 3.68 | 445.2 | 2 | | 0.029 | | 0.236 | CDCl₃ δ 8.80 (s, 2H), 7.44 (s, 1H), 5.21 (s, 1H), 4.63 (s, 2H), 4.33 (m, 4H), 3.88 (m, 4H), 3.76 (m, 2H), 3.07 (q, *J =* 7.5, 2H), 3.00 (t, *J* = 5.6, 2H), 1.39 (t, *J* = 7.4, 3H). |
| **2-74** | 2.74 and 2.85 | 515.1 | 2 | | 0.074 | | 0.888 | CDCl₃ δ 8.77 (s, 2H), 7.43 (s, 1H), 7.18 (d, *J* = 8.9, 2H), 6.75 (s, 1H), 6.62 (d, *J* = 8.8, 2H), 5.36 (s, 2H), 4.75 (s, 2H), 4.30 (m, 4H), 3.85 (m, 6H), 2.95 (t, *J* = 5.3, 2H), 2.86 (s, 6H). |
| **2-75** | 4.08 | 514.1 | 2 | | - | | | DMSO δ 8.97 (s, 1H), 8.87 (s, 2H), 8.28 (s, 1H), 8.06 (d, *J* = 1.7, 1H), 7.82 (d, *J* = 7.7, 1H), 7.58 (d, *J* = 7.8, 1H), 7.41 (t, *J* = 7.9, 1H), 6.83 (s, 2H), 4.85 (s, 2H), 4.22 (m, 4H), 3.92 (t, *J =* 5.2, 2H), 3.76 (m, 4H), 2.87 (m, 2H), 2.56 (s, 3H). |
| **2-76** | 4.10 | 458.2 | 2 | | - | | | DMSO δ 8.91 (s, 2H), 8.71 (m, 2H), 8.44 (s, 1H), 7.94 (m, 1H), 7.53 (dd, *J* = 7.6, 5.0, 1H), 6.83 (s, 2H), 5.00 (s, 2H), 4.23 (m, 4H), 3.76 (m, 4H), 3.67 (m, 2H), 2.88 (m, 2H). |
| **2-77** | 3.75 | 484.0 | 2 | | 0.019 | | | DMSO δ 8.87 (s, 2H), 8.35 (s, 1H), 6.83 (s, 2H), 4.71 (s, 2H), 4.22 (m, 4H), 3.76 (m, 4H), 3.69 (t, *J* = 5.5, 2H), 3.25 (m, 2H), 2.88 (m, 2H), 2.66 (t, *J* = 7.2, 2H), 2.02 (m, 2H). |
| **2-78** | 4.78 | 529.0 | 2 | | - | | | CDCl₃ δ 8.80 (s, 2H), 8.01 (m, 1H), 7.43 (s, 1H), 7.04 (t, *J* = 8.6, 1H), 6.93 (m, 1H), 5.16 (s, 2H), 4.66 (s, 2H), 4.32 (m, 4H), 3.87 (m, 4H), 3.75 (t, *J* = 5.6, 2H), 2.92 (t, *J* = 5.7, 2H). |
| **2-79** | 4.15 | 490.1 | 2 | | - | | | CDCl₃ δ 8.80 (s, 2H), 8.00 (m, 1H), 7.50 (s, 1H), 7.02 (m, 3H), 6.85 (d, *J* = 3.5, 1H), 5.36 (s, 2H), 4.84 (s, 2H), 4.32 (m, 4H), 3.89 (m, 6H), 3.01 (dd, *J* = 13.8, 8.3, 2H). |
| **2-80** | 0.43 and 2.64 | 435.1 | 2 | | 0.340 | | | DMSO δ 8.85 (s, 2H), 8.18 (s, 1H), 6.81 (s, 2H), 4.21 (m, 4H), 3.75 (m, 6H), 2.83 (m, 2H), 2.75 (m, 2H), 2.47 (s, 2H), 2.24 (m, 1H), 1.73 (m, 2H), 1.54 (m, 4H), 1.24 (m, 2H). |
| **2-81** | 0.43 and 2.53 | 435.1 | 2 | | 0.238 | | | CDCl₃ δ 8.81 (s, 2H), 7.45 (s, 1H), 5.28 (s, 2H), 4.33 (m, 4H), 3.89 (m, 6H), 3.01 (t, *J* = 5.4, 2H), 2.90 (t, *J =* 5.1, 2H), 2.66 (m, 1H), 1.99 (m, 2H), 1.88 (m, 2H), 1.70 (m, 1H), 1.32 (m, 5H). |
| **2-82** | 3.13 and 3.19 | 550.2 | 2 | | - | | | CDCl₃ δ 8.81 (s, 2H), 7.42 (s, 1H), 5.41 (s, 2H), 4.33 (m, 4H), 4.12 (m, 2H), 3.88 (m, 4H), 3.71 (s, 2H), 2.91 (s, 4H), 2.74 (t, *J* = 12.2, 2H), 2.52 (m, 2H), 1.81 (m, 3H), 1.47 (s, 9H), 1.21 (m, 2H). |
| **2-83** | 4.21 | 504.3 | 2 | | 0.025 | | 0.497 | DMSO δ 8.86 (s, 2H), 8.24 (s, 1H), 7.36 (m, 2H), 7.07 (m, 3H), 6.83 (s, 2H), 4.75 (s, 2H), 4.31 (d, *J* = 5.6, 2H), 4.23 (m, 4H), 3.77 (m, 6H), 2.79 (m, 2H). |
| **2-84** | 2.81 | 445.3 | 2 | | 0.175 | | | DMSO δ 9.27 (s, 1H), 9.20 (dd, *J* = 5.2, 1.1, 1H), 8.82 (s, 2H), 8.15 (s, 1H), 7.70 (dd, *J* = 5.2, 2.3, 1H), 6.81 (s, 2H), 4.22 (m, 4H), 3.90 (s, 2H), 3.80 (s, 2H), 3.76 (m, 4H), 2.95 (m, 2H), 2.82 (m, 2H). |
| **2-85** | 3.72 | 475.2 | 2 | | 0.054 | | 0.344 | CDCl₃ δ 8.80 (s, 2H), 7.43 (s, 1H), 5.19 (s, 2H), 4.61 (s, 2H), 4.34 (m, 4H), 3.88 (m, 4H), 3.80 (t, *J* = 5.7, 2H), 3.74 *(t, J* = 5.7, 2H), 3.35 (m, 2H), 3.32 (s, 3H), 3.00 (t, *J* = 5.7, 2H). |
| **2-86** | 3.90 | 423.1 | 2 | | 0.094 | | | CDCl₃ δ 8.81 (s, 2H), 7.52 (s, 1H), 5.18 (s, 2H), 4.87 (s, 2H), 4.33 (m, 4H), 3.87 (m, 6H), 2.96 (m, 2H), 2.47 (m, 2H), 1.74 (m, 2H), 1.01 (t, *J* = 7.4, 3H). |
| **2-87** | 3.44 | 439.1 | 2 | | - | | | CDCl₃ δ 8.82 (s, 2H), 7.53 (s, 1H), 5.25 (s, 2H), 4.90 (s, 2H), 4.35 (m, 4H), 3.91 (m, 6H), 3.78 (t, *J* = 6.4, 2H), 3.39 (s, 3H), 2.98 (m, 2H), 2.79 (t, *J* = 6.4, 2H). |
| **2-88** | 3.30 | 522.3 | 2 | | - | | | CDCl₃ δ 8.80 (s, 2H), 7.42 (s, 1H), 5.28 (s, 2H), 4.32 (m, 4H), 3.87 (m, 4H), 3.68 (m, 4H), 3.30 (m, 2H), 2.93 (m, 4H), 2.25 (m, 1H), 1.93 (m, 2H), 1.47 (s, 9H). |
| **2-89** | 0.41 and 0.80 | 450.1 | 2 | | - | | | D₂O δ 8.79 (s, 2H), 7.98 (s, 1H), 4.78 (m, 2H), 4.04 (m, 4H), 3.83 (m, 6H), 3.43 (m, 4H), 3.17 (m, 2H), 3.05 (m, 2H), 2.40 (m, 1H), 2.08 (d, *J* = 13.8, 2H), 1.56 (m, 2H). |
| **2-90** | 4.18 | 504.3 | 2 | | 0.046 | | 0.302 | DMSO δ 8.86 (s, 2H), 8.24 (s, 1H), 7.31 (m, 3H), 7.14 (m, 2H), 6.83 (s, 2H), 4.75 (s, 2H), 4.34 (d, *J* = 5.5, 2H), 4.23 (m, 4H), 3.77 (m, 6H), 2.79 (m, 2H). |
| **2-91** | 4.35 | 490.2 | 2 | | 0.075 | | 0.443 | DMSO δ 8.91 (s, 1H), 8.81 (s, 2H), 8.23 (s, 1H), 7.38 (d, *J* = 12.2, 1H), 7.21 (m, 2H), 6.77 (s, 2H), 6.71 (m, 1H), 4.76 (s, 2H), 4.17 (m, 4H), 3.83 (m, 2H), 3.70 (m, 4H), 2.80 (m, 2H). |
| **2-92** | 2.85 | 476.3 | 2 | | - | | | DMSO 6 8.84 (s, 2H), 8.15 (s, 1H), 8.03 (s, 1H), 7.86 (d, *J* = 10.0, 1H), 6.80 (s, 2H), 4.22 (m, 4H), 3.79 (s, 2H), 3.75 (m, 6H), 2.91 (m, 2H), 2.79 (m, 2H), 2.25 (s, 3H). |
| **2-93** | 4.19 | 502.3 | 2 | | 0.064 | | 0.415 | DMSO δ 8.88 (s, 2H), 8.75 (s, 1H), 8.29 (s, 1H), 7.13 (m, 3H), 6.83 (s, 2H), 6.54 (d, *J* = 7.6, 1H), 4.82 (s, 2H), 4.23 (m, 4H), 3.89 (m, 2H), 3.76 (m, 4H), 3.71 (s, 3H), 2.86 (m, 2H). |
| **2-94** | 4.40 | 500.4 | 2 | | 0.037 | | 0.260 | DMSO δ 8.65 (s, 2H), 8.02 (s, 1H), 7.05 (t, *J* = 5.8, 1H), 6.95 (d, *J* = 8.1, 2H), 6.89 (d, *J* = 8.0, 2H), 6.62 (s, 2H), 4.52 (s, 2H), 4.03 (m, 6H), 3.55 (m, 6H), 2.56 (m, 2H), 2.05 (s, 3H). |
| **2-95** | 2.71 and 2.82 | 473.3 | 2 | | 0.240 | | | DMSO δ 8.78 (s, 2H), 8.10 (s, 1H), 7.21 (t, *J* = 8.0, 1H), 6.91 (m, 2H), 6.78 (d, *J* = 9.6, 1H), 6.73 (s, 2H), 4.15 (m, 4H), 3.70 (m, 8H), 3.68 (s, 3H), 2.82 (m, 2H), 2.72 (m, 2H). |
| **2-96** | 4.18 | 516.1 | 2 | | 0.028 | | 0.385 | CDCl₃ δ 8.80 (s, 2H), 7.46 (s, 1H), 7.27 (t, *J* = 8.6, 2H), 6.88 (t, *J* = 8.7, 2H), 5.30 (s, 2H), 5.02 *(t, J =* 5.2, 1H), 4.74 (s, 2H), 4.42 (d, *J* = 6.4, 2H), 4.33 (m, 4H), 3.88 (m, 4H), 3.81 (s, 3H), 3.76 (m, 2H), 2.93 (m, 2H). |
| **2-97** | 4.20 | 516.1 | 2 | | 0.038 | | 0.491 | CDCl₃ δ 8.80 (s, 2H), 7.47 (s, 1H), 7.27 (m, 1H), 6.91 (m, 2H), 6.82 (dd, *J* = 8.1, 2.4, 1H), 5.35 (s, 2H), 5.11 (t, *J* = 5.3, 1H), 4.75 (s, 2H), 4.46 (d, *J* = 6.3, 2H), 4.33 (m, 4H), 3.88 (m, 4H), 3.80 (s, 3H), 3.75 (m, 2H), 2.94 (t, *J* = 5.5, 2H). |
| **2-98** | 4.31 | 516.1 | 2 | | - | | | CDCl₃ δ 8.81 (s, 2H), 7.48 (s, 1H), 7.31 (m, 2H), 6.93 (m, 2H), 5.27 (m, 3H), 4.73 (s, 2H), 4.48 (d, *J* = 5.5, 2H), 4.33 (m, 4H), 3.92 (s, 3H), 3.88 (m, 4H), 3.73 (m, 2H), 2.93 (t, *J* = 5.5, 2H). |
| **2-99** | 0.39 and 2.66 | 503.1 | 2 | | - | | | CDCl₃ δ 8.79 (s, 2H), 7.37 (s, 1H), 6.98 (d, *J* = 1.6, 1H), 6.91 (dd, *J =* 8.2, 1.7, 1H), 6.85 (d, *J* = 8.1, 1H), 5.21 (s, 2H), 4.32 (m, 4H), 3.89 (s, 3H), 3.88 (s, 3H), 3.86 (m, 4H), 3.76 (s, 2H), 3.72 (s, 2H), 2.94 (m, 4H). |
| **2-100** | 3.55 | 527.1 | 2 | | 0.310 | | | CDCl₃ δ 8.78 (s, 2H), 7.44 (d, *J* = 8.6, 2H), 7.37 (s, 1H), 7.21 (d, *J* = 8.0, 2H), 5.18 (s, 2H), 4.32 (m, 4H), 3.88 (m, 4H), 3.82 (s, 2H), 3.74 (s, 2H), 2.95 (m, 4H). |
| **2-101** | 5.41 | 574.0 | 2 | | - | | | CDCl₃ δ 8.79 (s, 2H), 7.71 (d, *J* = 2.5, 1H), 7.60 (m, 1H), 7.46 (s, 1H), 7.40 (d, *J* = 8.7, 1H), 7.02 (s, 1H), 5.24 (m, 2H), 4.82 (s, 2H), 4.32 (m, 4H), 3.88 (m, 6H), 3.01 (m, 2H). |
| **2-102** | 4.13 | 502.1 | 2 | | 0.070 | | 0.541 | CDCl₃ δ 8.81 (s, 2H), 7.50 (s, 1H), 7.27 (d, *J* = 8.8, 2H), 6.86 (d, *J* = 8.9, 2H), 6.47 (s, 1H), 5.18 (s, 2H), 4.81 (s, 2H), 4.34 (m, 4H), 3.88 (m, 6H), 3.79 (s, 3H), 3.00 (m, 2H). |
| **2-103** | 0.39 and 2.09 | 422.1 | 2 | | - | | | D₂O δ 8.84 (s, 2H), 7.98 (s, 1H), 4.30 (m, 2H), 4.04 (m, 4H), 3.97 (m, 1H), 3.85 (m, 4H), 3.71 (m, 2H), 3.62 (m, 3H), 3.42 (m, 2H), 3.15 (m, 2H), 2.69 (m, 1H), 2.32 (m, 1H). |
| **2-104** | 4.07 | 545.3 | 2 | | - | | | DMSO δ 8.76 (s, 2H), 8.09 (s, 1H), 7.80 (s, 1H), 7.66 (s, 2H), 6.73 (s, 2H), 4.15 (m, 4H), 3.82 (s, 2H), 3.70 (m, 6H), 2.87 (m, 2H), 2.73 (m, 2H). |
| **2-105** | 4.39 | 486.3 | 2 | | 0.044 | | 0.345 | DMSO δ 8.88 (s, 2H), 8.66 (s, 1H), 8.29 (s, 1H), 7.35 (d, *J* = 8.4, 2H), 7.06 (d, *J* = 8.4, 2H), 6.83 (s, 2H), 4.81 (s, 2H), 4.24 (m, 4H), 3.88 (m, 2H), 3.77 (m, 4H), 2.85 (m, 2H), 2.23 (s, 3H). |
| **2-106** | 0.36 and 2.69 | 473.3 | 2 | | 0.096 | | | DMSO δ 8.78 (s, 2H), 8.09 (s, 1H), 7.24 (d, *J* = 8.6, 2H), 6.85 (d, *J* = 8.6, 2H), 6.73 (s, 2H), 4.15 (m, 4H), 3.67 (m, 11 H), 2.80 (m, 2H), 2.69 (m, 2H). |
| **2-107** | 2.32 | 444.3 | 2 | | 0.125 | | | DMSO δ 8.76 (s, 2H), 8.48 (d, *J* = 4.4, 2H), 8.09 (s, 1H), 7.35 (d, *J* = 5.9, 2H), 6.73 (s, 2H), 4.15 (m, 4H), 3.78 (s, 2H), 3.70 (m, 6H), 2.85 (m, 2H), 2.74 (m, 2H). |
| **2-108** | 2.43 | 444.3 | 2 | | - | | | DMSO δ 8.83 (s, 2H), 8.59 (s, 1H), 8.51 *(d, J* = 4.8, 1H), 8.17 (s, 1H), 7.81 (d, *J* = 7.8, 1H), 7.40 (dd, *J =* 7.8, 4.8, 1H), 6.80 (s, 2H), 4.22 (m, 4H), 3.84 (s, 2H), 3.77 (m, 6H), 2.90 (m, 2H), 2.79 (m, 2H). |
| **2-109** | 4.45 | 473.1 | 2 | | 0.095 | | 0.763 | CDCl₃ δ 8.81 (s, 2H), 7.45 (s, 1H), 5.16 (s, 2H), 4.64 (s, 2H), 4.34 (m, 4H), 3.88 (m, 4H), 3.75 (t, *J* = 5.7, 2H), 3.00 (m, 2H), 2.89 (d, *J* = 6.5, 2H), 2.34 (m, 1H), 1.13 (d, *J* = 6.7, 6H). |
| **2-110** | 0.38 and 2.41 | 472.1 | 2 | | 0.007 | | | D₂O δ 8.90 (s, 2H), 8.05 (s, 1H), 4.52 (m, 6H), 3.88 (m, 11H), 2.95 (m, 2H). |
| **2-111** | 3.10 | 450.0 | 2 | | 0.139 | | | CDCl₃ δ 8.78 (s, 2H), 7.77 (d, *J* = 3.3, 1H), 7.40 (s, 1H), 7.35 (d, *J* = 3.3, 1H), 5.14 (s, 2H), 4.33 (m, 4H), 4.22 (s, 2H), 3.93 (s, 2H), 3.88 (m, 4H), 3.10 (t, *J* = 5.6, 2H), 2.96 (t, *J* = 5.4, 2H). |
| **2-112** | 5.33 | 540.0 | 2 | | 0.090 | | | DMSO δ 9.10 (s, 1H), 8.87 (s, 2H), 8.27 (s, 1H), 7.61 (d, *J* = 1.8, 2H), 7.15 (d, *J* = 1.7, 1H), 6.83 (s, 2H), 4.83 (s, 2H), 4.23 (m, 4H), 3.89 (m, 2H), 3.76 (m, 4H), 2.86 (m, 2H). |
| **2-113** | 4.26 | 497.1 | 2 | | 0.081 | | | DMSO δ 9.12 (s, 1H), 8.88 (s, 2H), 8.30 (s, 1H), 7.94 (t, *J* = 1.7, 1H), 7.79 (d, *J* = 9.0, 1H), 7.45 (m, 2H), 6.84 (s, 2H), 4.85 (s, 2H), 4.24 (m, 4H), 3.91 (m, 2H), 3.77 (m, 4H), 2.88 (m, 2H). |
| **2-114** | 3.60 | 511.1 | 2 | | - | | | CDCl₃ δ 8.77 (s, 2H), 7.61 (d, *J* = 7.7 Hz, 2H), 7.53 (m, 2H), 7.36 (s, 1H), 5.18 (s, 2H), 4.33 (m, 4H), 3.88 (m, 6H), 3.75 (s, 2H), 2.96 (m, 4H). |
| **2-115** | 0.38 and 2.71 | 521.1 | 2 | | - | | | CDCl₃ δ 8.78 (s, 2H), 7.95 (d, *J* = 8.3, 2H), 7.64 (d, *J* = 8.3, 2H), 7.37 (s, 1H), 5.15 (s, 2H), 4.33 (m, 4H), 3.93 (s, 2H), 3.88 (m, 4H), 3.77 (s, 2H), 3.07 (s, 3H), 2.96 (m, 4H). |
| **2-116** | 2.58 and 2.75 | 521.0 | 2 | | - | | | CDCl₃ δ 8.80 (s, 2H), 8.01 (s, 1H), 7.89 (d, *J* = 7.8, 1H), 7.74 (d, *J* = 7.7, 1H), 7.58 (t, *J* = 7.7, 1H), 7.37 (s, 1H), 5.14 (s, 2H), 4.33 (m, 4H), 3.93 (s, 2H), 3.88 (m, 4H), 3.77 (s, 2H), 3.08 (s, 3H), 2.96 (m, 4H). |
| **2-117** | 2.93 and 3.06 | 521.1 | 2 | | 0.037 | | | CDCl₃ δ 8.80 (s, 2H), 8.15 (dd, *J* = 7.7, 1.3, 1H), 7.63 (td, *J* = 7.4, 1.4, 1H), 7.53 (m, 2H), 7.42 (s, 1H), 5.17 (s, 2H), 4.32 (m, 4H), 4.27 (s, 2H), 3.87 (m, 6H), 3.28 (s, 3H), 3.00 (m, 2H), 2.87 (m, 2H). |
| **5-01** | 3.51 | 355.2 | 2 | | 0.089 | 0.118 | | DMSO δ 8.86 (s, 2H), 8.14 (s, 1H), 6.80 (s, 2H), 4.22 (m, 4H), 3.75 (m, 4H), 2.78 (t, *J* = 5.0, 2H), 2.69 (t, *J =* 5.0, 1H), 1.87 (m, 4H). |
| **5-02** | 3.49 | 338.2 | 2 | | 0.117 | 0.065 | | DMSO δ 8.76 (s, 2H), 8.20 (s, 1H), 6.73 (s, 2H), 4.13 (s, 4H), 3.69 (d, *J* = 4.9, 4H), 2.85 (s, 2H), 2.67 (d, *J* = 7.3, 2H), 2.54 - 2.47 (m, 2H). |
| **5-03** | 4.09 | 424.3 | 2 | | 0.148 | 0.191 | | (700 MHz, DMSO) δ 8.88 (s, 2H), 8.24 (s, 1H), 6.80 (s, 2H), 4.21 (s, 4H), 4.14 (td, *J* = 18.0, 10.8, 2H), 3.75 (d, *J* = 4.3, 4H), 3.13 (dd, *J* = 14.2, 3.2, 1H), 2.98 (m, 2H), 2.76 (m, 2H), 2.22 (m, 1 H), 1.93 (m, 1H), 1.23 (t, *J* = 7.1, 3H). |
| **5-04** | 4.38 | 394.5 | 2 | | 0.195 | | | DMSO δ 8.77 (s, 3H), 6.96 (s, 2H), 4.23 (s, 4H), 3.78 (s, 4H), 2.87 (s, 2H), 1.09 (s, 6H) |
| **5-05** | 3.87 | 396.3 | 2 | | 0.661 | | | DMSO δ 8.79 (s, 2H), 8.28 (s, 1H), 6.84 (s, 2H), 5.50 (d, *J* = 6.4, 1H), 5.02 (d, *J* = 6.2, 1H), 4.22 (d, *J* = 4.7, 4H), 3.76 (t, *J* = 4.6, 4H), 1.97 (dd, *J* = 13.0, 5.9, 1H), 1.66 (dd, *J* = 13.2, 7.7, 1H), 1.09 (d, *J* = 12.2, 3H), 0.90 (d, *J* = 15.4, 3H). |
| **5-06** | 3.85 | 487 | 2 | | 0.045 | 0.174 | | CDCl₃ δ/ppm 8.82 (s, 2H), 7.48 (s, 1H), 7.31 (d, *J* = 8.6, 2H), 6.88 (d, *J* = 8.6, 2H), 5.30 (s, 2H), 4.76 (s, 2H), 4.47 (s, 4H), 3.98 - 3.85 (m, 4H), 3.81 (s, 3H), 3.69 (d*, J* = 6.9, 2H), 3.10 (m, 2H). |
| **5-07** | 3.93 | 380.2 | 2 | | - | | | DMSO δ 10.86 (s, 1H), 8.45 (s, 1H), 7.36 (m, 2H), 7.18 (t, *J* = 8.0, 1H), 6.74 (m, 1H), 4.03 (m, 4H), 3.63 (s, 3H), 3.57 (m, 4H), 2.78 (t, *J* = 7.6, 2H), 2.54 (t, *J =* 7.8, 2H). |
| **5-08** | 2.77 | 366.2 | 2 | | - | | | DMSO δ 8.28 (s, 1H), 7.62 (m, 2H), 7.34 (dd, *J* = 15.4, 7.5, 1H), 6.92 (dd, *J* = 8.1, 1.9, 1H), 4.34 - 4.13 (m, 4H), 3.85 (d, *J* = 7.2, 2H), 3.81 (s, 3H), 3.79 - 3.67 (m, 4H), 3.10 (t, *J* = 5.4, 2H), 2.85 (d, *J* = 5.1, 2H). |
| **5-09** | 3.7 | 381.2 | 2 | | | | | DMSO δ 8.91 (s, 2H), 8.39 (s, 1H), 6.99 (s, 2H), 4.25 (s, 4H), 3.79 (s, 4H), 3.00 (s, 3H). |
| **5-10** | 3.95 | 366.2 | 2 | | 0.409 | | | DMSO δ 10.83 (s, 1H), 9.48 (s, 1H), 8.31 (s, 1H), 7.39 (s, 1H), 7.33 (d, *J =* 7.9, 1H), 7.24 (t, *J* = 7.8, 1H), 6.75 (d, *J* = 7.9, 1H), 4.22 (m, 4H), 3.76 (m, 4H), 2.97 (t, *J* = 7.8, 2H), 2.73 (t, *J* = 7.7, 2H). |
| **5-11** | 4.92 | 367.2 | 3 | | 0.014 | | 0.475 | DMSO δ 10.73 (s, 1H), 8.70 (s, 2H), 8.19 (d, *J* = 8.7, 1H), 6.86 (s, 2H), 4.21 (m, 4H), 3.75 (m, 4H), 2.97 (t, *J* = 7.6, 2H), 2.73 (t, *J* = 7.6, 2H). |
| **5-12** | 0.31 | 339.2 | 3 | | 0.643 | | | DMSO □ 8.89 (s, 2H), 8.34 (s, 1H), 6.85 (s, 2H), 4.24 (m, 4H), 4.17 (s, 2H), 4.06 (s, 2H), 3.76 (m, 4H). |
| **6-01** | 0.36 and 1.45 | 353.2 | 3 | | 0.083 | | | DMSO δ 8.86 (s, 2H), 8.19 (s, 1H), 6.82 (s, 2H), 4.20 (m, 4H), 3.82 (s, 2H), 3.75 (m, 4H), 3.05 (m, 2H), 2.77 (m, 2H). |
| **6-02** | 0.33 and 0.41 | 367.2 | 3 | | 0.088 | 0.318 | | DMSO δ 8.79 (s, 2H), 8.13 (s, 1H), 6.74 (s, 2H), 4.13 (m, 4H), 3.69 (m, 4H), 3.46 (s, 2H), 2.80 (m, 2H), 2.73 (m, 2H), 2.36 (s, 3H). |
| **6-03** | 0.33 and 0.64 | 411.3 | 2 | | 0.050 | 0.532 | | DMSO δ 8.86 (s, 2H), 8.19 (s, 1H), 6.81 (s, 2H), 4.21 (m, 4H), 3.75 (m, 4H), 3.65 (s, 2H), 3.53 (t, *J* = 5.9, 2H), 3.27 (s, 3H), 2.90 (m, 2H), 2.86 (m, 2H), 2.75 (t, *J* = 5.7, 2H). |
| **6-04** | 0.32 and 2.39 | 424.2 | 3 | | 0.178 | | | DMSO δ 8.86 (s, 2H), 8.19 (s, 1H), 6.81 (s, 2H), 4.21 (m, 4H), 3.75 (m, 4H), 3.63 (s, 2H), 2.88 (m, 4H), 2.66 (t, *J* = 6.8, 2H), 2.43 (t, *J =* 6.5, 2H), 2.16 (s, 6H). |
| **6-05** | 2.99 | 459.2 | 3 | | - | | | DMSO δ 8.79 (s, 2H), 8.14 (s, 1H), 6.75 (s, 2H), 4.15 (m, 4H), 3.69 (m, 4H), 3.62 (s, 2H), 3.35 (t, *J* = 6.4, 2H), 2.94 (s, 3H), 2.91 (m, 2H), 2.87 (m, 2H), 2.82 (m, 2H). |
| **6-06** | 0.36 and 0.48 | 407.2 | 3 | | 0.296 | | | DMSO δ 8.70 (s, 2H), 8.03 (s, 1H), 6.65 (s, 2H), 4.05 (m, 4H), 3.59 (m, 4H), 3.51 (s, 2H), 2.73 (m, 4H), 2.30 (s, 2H), 0.78 (m, 1H), 0.36 (m, 2H), -0.00 (m, 2H). |
| **6-07** | 2.76 | 473.3 | 2 | | 0.075 | 0.278 | 0.537 | DMSO δ 8.86 (s, 2H), 8.21 (s, 1H), 7.29 (d, *J* = 8.6, 2H), 6.92 (d, *J* = 8.7, 2H), 6.82 (s, 2H), 4.20 (m, 4H), 3.76 (s, 3H), 3.74 (m, 4H), 3.69 (s, 2H), 3.54 (s, 2H), 2.88 (m, 4H). |
| **6-08** | 3.77 | 461.2 | 3 | | - | | | DMSO δ 8.79 (s, 2H), 8.13 (s, 1H), 7.34 (dd, *J* = 8.6, 5.7, 2H), 7.10 (t, *J* = 8.9, 2H), 6.75 (s, 2H), 4.12 (m, 4H), 3.66 (m, 6H), 3.49 (s, 2H), 2.82 (m, 4H). |
| **6-09** | 0.57 | 445.2 | 2 | | 0.121 | 0.180 | | DMSO δ 8.56 (s, 2H), 6.92 (s, 2H), 4.13 (m, 4H), 3.74 (m, 4H), 3.66 (s, 2H), 3.53 (t, *J* = 5.7, 2H), 3.31 (m, 2H), 3.26 (s, 3H), 2.84 (t, *J* = 5.6, 2H), 2.73 (t, *J* = 5.7, 2H). |
| **6-10** | 2.72 | 493.2 | 2 | | - | | | DMSO δ 8.57 (s, 2H), 6.93 (s, 2H), 4.14 (m, 4H), 3.75 (m, 4H), 3.70 (s, 2H), 3.41 (t, *J* = 6.3, 2H), 3.34 (m, 2H), 3.02 (s, 3H), 2.97 (t, *J* = 6.7, 2H), 2.88 (m, 2H). |
| **6-11** | 0.34 and 2.21 | 441.2 | 2 | | - | | | DMSO δ 8.41 (s, 2H), 6.76 (s, 2H), 3.97 (m, 4H), 3.57 (m, 4H), 3.52 (s, 2H), 3.17 (m, 2H), 2.68 (m, 2H), 2.28 (d, *J* = 6.5, 2H), 0.77 (m, 1H), 0.36 (m, 2H), -0.01 (m, 2H). |
| **6-12** | 2.95 | 495.3 | 2 | | 0.290 | | | DMSO δ 8.49 (s, 2H), 7.34 (dd, *J* = 8.6, 5.7, 2H), 7.10 (t, *J* = 8.9, 2H), 6.85 (s, 2H), 4.04 (m, 4H), 3.64 (m, 6H), 3.49 (s, 2H), 3.26 (m, 2H), 2.76 (m, 2H). |
| **6-13** | 3.47 | 431.2 | 2 | | 0.029 | | | DMSO δ 8.87 (s, 2H), 8.26 (s, 1H), 6.84 (s, 2H), 4.43 (s, 2H), 4.22 (m, 4H), 3.77 (m, 4H), 3.66 (m, 2H), 3.02 (m, 2H), 2.99 (s, 3H). |
| **6-14** | 4.31 | 449.2 | 2 | | 0.023 | | | DMSO δ 8.86 (s, 2H), 8.27 (s, 1H), 6.84 (s, 2H), 4.81 (s, 2H), 4.22 (m, 4H), 4.05 (m, 2H), 3.76 (m, 4H), 3.05 (m, 2H). |
| **6-15** | 4.66 | 511.2 | 2 | | - | | | DMSO δ 8.84 (s, 2H), 8.19 (s, 1H), 7.95 (dd, *J* = 8.9, 5.2, 2H), 7.45 (t, *J* = 8.8, 2H), 6.83 (s, 2H), 4.31 (s, 2H), 4.19 (m, 4H), 3.75 (m, 4H), 3.54 (m, 2H), 2.93 (m, 2H). |
| **6-16** | 3.83 | 457.2 | 2 | | 0.010 | | | DMSO δ 8.85 (s, 2H), 8.23 (s, 1H), 6.83 (s, 2H), 4.48 (s, 2H), 4.21 (m, 4H), 3.74 (m, 6H), 3.01 (m, 2H), 2.63 (m, 1H), 0.95 (m, 4H). |
| **6-17** | 3.81 | 465.3 | 2 | | 0.033 | | 0.246 | DMSO δ 8.57 (s, 2H), 6.94 (s, 2H), 4.43 (s, 2H), 4.14 (m, 4H), 3.74 (m, 4H), 3.57 (m, 2H), 3.48 (m, 2H), 3.01 (s, 3H). |
| **6-18** | 3.22 | 395.4 | 2 | | 0.014 | | | DMSO δ 8.79 (s, 2H), 8.17 (s, 1H), 6.76 (s, 2H), 4.57 (m, 2H), 4.19 (m, 4H), 3.78 (m, 2H), 3.66 (m, 4H), 2.85 (m, 2H), 2.04 (m, 3H). |
| **6-19** | 3.43 | 424.4 | 2 | | - | | | DMSO δ 8.79 (s, 2H), 8.15 (s, 1H), 6.75 (s, 2H), 6.63 (t, *J* = 5.3, 1H), 4.45 (s, 2H), 4.15 (m, 4H), 3.69 (m, 6H), 3.01 (m, 2H), 2.79 (m, 2H), 0.96 (t, *J* = 7.1, 3H). |
| **6-20** | 4.62 | 445.3 | 2 | | 0.022 | | | DMSO δ 8.93 (s, 2H), 8.31 (s, 1H), 6.90 (s, 2H), 4.54 (s, 2H), 4.28 (s, 4H), 3.81 (m, 6H), 3.23 (q, *J* = 7.3, 2H), 3.05 (m, 2H), 1.27 (t, *J* = 7.4, 3H). |
| **6-21** | 4.16 | 504.3 | 2 | | 0.001 | | 0.078 | DMSO δ 8.86 (s, 2H), 8.23 (s, 1H), 7.31 (m, 3H), 7.11 (t, *J* = 8.9, 2H), 6.83 (s, 2H), 4.57 (s, 2H), 4.24 (m, 6H), 3.78 (m, 6H), 2.88 (s, 2H). |
| **6-22** | 3.61 | 438.3 | 2 | | 0.100 | | | DMSO δ 8.86 (s, 2H), 8.22 (s, 1H), 6.82 (s, 2H), 6.39 (d, *J* = 7.5, 1H), 4.52 (s, 2H), 4.22 (s, 4H), 3.77 (m, 6H), 2.86 (m, 2H), 1.08 (s, 3H), 1.06 (s, 3H). |
| **6-23** | 4.17 | 475.3 | 2 | | - | | | DMSO δ 8.79 (s, 2H), 8.18 (s, 1H), 7.49 (m, 2H), 7.25 (t, *J* = 8.8, 2H), 6.77 (s, 2H), 4.72 (s, 2H), 4.14 (s, 4H), 3.68 (m, 4H), 2.92 (m, 2H). |
| **6-24** | 3.61 | 421.3 | 2 | | 0.029 | | 0.218 | DMSO δ 8.86 (s, 2H), 8.24 (s, 1H), 6.83 (s, 2H), 4.92 (s, 1H), 4.66 (s, 1H), 4.22 (m, 4H), 4.12 (m, 1H), 3.92 (m, 1H), 3.76 (m, 4H), 3.00 (m, 1H), 2.87 (m, 1H), 2.12 (m, 1H), 0.76 (m, 4H). |
| **6-25** | 4.29 | 478.4 | 2 | | 0.014 | | 0.092 | DMSO δ 8.79 (s, 2H), 8.15 (s, 1H), 6.76 (s, 2H), 6.31 (d, *J* = 7.6, 1H), 4.46 (s, 2H), 4.15 (m, 4H), 3.69 (m, 6H), 3.29 (m, 1H), 2.79 (m, 2H), 1.60 (m, 4H), 1.14 (m, 6H). |
| **6-26** | 3.73 | 423.3 | 2 | | 0.044 | | 0.137 | DMSO δ 8.79 (s, 2H), 8.17 (s, 1H), 6.76 (s, 2H), 4.67 (s, 1H), 4.59 (s, 1H), 4.15 (m, 4H), 3.87 (m, 2H), 3.69 (m, 4H), 2.97 (m, 1H), 2.89 (m, 1H), 2.80 (m, 1H), 0.99 (d, *J* = 6.6, 3H), 0.92 (d, *J* = 6.7, 3H). |
| **6-27** | 3.06 and 3.15 | 528.4 | 2 | | - | | | DMSO δ 8.61 (s, 1H), 8.14 (m, 1H), 7.90 (d, *J* = 8.8, 2H), 7.46 (d, *J* = 8.8, 2H), 7.29 (d, *J* = 8.6, 2H), 6.91 (d, *J* = 8.7, 2H), 6.02 (q, *J* = 4.6, 1H), 4.20 (m, 4H), 3.75 (m, 7H), 3.68 (s, 2H), 3.54 (s, 2H), 2.88 (m, 4H), 2.65 (d, *J* = 4.6, 3H). |
| **6-28** | 3.68 | 501.3 | 2 | | 0.018 | | | DMSO δ 8.86 (s, 2H), 8.24 (s, 1H), 6.84 (s, 2H), 4.52 (s, 2H), 4.22 (s, 4H), 3.91 (m, 2H), 3.76 (m, 6H), 3.58 (m, 1H), 3.32 (m, 2H), 2.97 (m, 2H), 1.87 (m, 2H), 1.65 (m, 2H). |
| **6-29** | 3.63 | 484.3 | 2 | | 0.031 | | | DMSO δ 8.87 (s, 2H), 8.26 (s, 1H), 6.84 (s, 2H), 4.49 (s, 2H), 4.22 (s, 3H), 3.76 (m, 5H), 3.25 (m, 2H), 3.00 (s, 2H), 2.64 (t, *J* = 7.3 Hz, 2H), 2.00 (dd, *J* = 14.7, 7.3 Hz, 2H). |
| **6-30** | 3.23 | 454.3 | 2 | | 0.025 | | | DMSO 8.86 (s, 2H), 8.23 (s, 1H), 6.82 (s, 2H), 6.78 (m, 1H), 4.53 (s, 2H), 4.22 (m, 4H), 3.76 (m, 6H), 3.34 (t, *J* = 5.3, 2H), 3.23 (s, 3H), 3.20 (m, 2H), 2.86 (m, 2H). |
| **6-31** | 3.62 | 488.3 | 2 | | - | | | DMSO δ 8.56 (s, 2H), 6.93 (s, 2H), 6.79 (m, 1H), 4.54 (s, 2H), 4.15 (m, 4H), 3.72 (m, 6H), 3.34 (m, 2H), 3.23 (s, 3H), 3.20 (m, 2H). |
| **6-32** | 4.21 | 490.3 | 2 | | 0.030 | | | DMSO δ 8.87 (s 2H), 8.74 (s, 1H), 8.25 (s, 1H), 7.48 (m, 2H), 7.08 *(t, J* = 8.8, 2H), 6.83 (s, 2H), 4.71 (s, 2H), 4.23 (s, 4H), 3.91 (m, 2H), 3.77 (m, 4H), 2.96 (m, 2H). |
| **6-33** | 4.28 | 502.3 | 2 | | 0.025 | | 0.188 | DMSO δ 8.87 (s, 2H), 7.93 (s, 1H), 7.57 (d, *J* = 6.5, 1H), 7.03 (m, 2H), 6.87 (m, 3H), 4.68 (s, 2H), 4.23 (m, 4H), 3.91 (m, 2H), 3.81 (s, 3H), 3.77 (m, 4H), 2.97 (m, 2H). |
| **6-34** | 3.46 | 482.3 | 2 | | - | | | DMSO δ 8.86 (s, 2H), 8.24 (s, 1H), 7.24 (m, 1H), 6.83 (s, 2H), 4.56 (s, 2H), 4.22 (m, 4H), 4.06 (q, *J* = 7.1, 2H), 3.77 (m, 6H), 2.89 (m, 2H), 1.16 (t, *J* = 7.1, 3H). |
| **6-35** | 3.33 | 439.2 | 2 | | 0.079 | | | DMSO) δ 8.86 (s, 2H), 8.24 (s, 1H), 6.83 (s, 2H), 4.68 (m, 2H), 4.22 (m, 4H), 3.90 (m, 2H), 3.76 (m, 4H), 3.59 (t, *J =* 6.7, 2H), 3.23 (s, 3H), 2.92 (m, 2H), 2.73 (m, 2H). |
| **6-36** | 4.07 | 490.3 | 2 | | 0.021 | | 0.250 | DMSO δ 8.81 (s, 2H), 8.47 (s, 1H), 8.22 (s, 1H), 7.35 (m, 1H), 7.08 (m, 3H), 6.77 (s, 2H), 4.62 (s, 2H), 4.17 (m, 4H), 3.85 (m, 2H), 3.71 (m, 4H), 2.90 (m, 2H). |
| **6-37** | 4.01 | 514.3 | 2 | | - | | | DMSO δ 8.94 (s, 1H), 8.87 (s, 2H), 8.26 (s, 1H), 8.09 (d, *J* = 1.8, 1H), 7.81 (d, *J* = 9.2, 1H), 7.57 (d, *J* = 7.7, 1H), 7.40 (t, *J* = 7.9, 1H), 6.83 (s, 2H), 4.73 (s, 2H), 4.24 (m, 4H), 3.94 (m, 2H), 3.78 (m, 4H), 2.98 (m, 2H), 2.55 (s, 3H). |
| **6-38** | 0.36 and 2.15 | 395.3 | 2 | | - | | | DMSO δ 8.79 (s, 2H), 8.12 (s, 1H), 6.74 (s, 2H), 4.14 (m, 4H), 3.69 (m, 4H), 3.58 (s, 2H), 2.92 (m, 1H), 2.79 (m, 4H), 1.02 (d, *J* = 6.5, 6H). |
| **6-39** | 2.81 | 515.4 | 2 | | 0.017 | | 0.187 | DMSO δ 8.80 (s, 2H), 8.34 (s, 1H), 8.18 (s, 1H), 7.19 (d, *J* = 8.9, 2H), 6.76 (s, 2H), 6.59 (d, *J* = 9.0, 2H), 4.60 (s, 2H), 4.17 (m, 4H), 3.82 (m, 2H), 3.70 (m, 4H), 2.88 (m, 2H), 2.75 (s, 6H). |
| **6-40** | 2.82 | 475.3 | 2 | | - | | | DMSO δ 8.86 (s, 2H), 8.20 (s, 1H), 7.31 (dd, *J* = 8.5, 5.7, 2H), 7.09 (t, *J* = 8.9, 2H), 6.81 (s, 2H), 4.21 (m, 4H), 3.76 (m, 4H), 3.67 (s, 2H), 2.87 (m, 8H). |
| **6-41** | 4.84 | 447.3 | 2 | | - | | | DMSO δ 8.86 (s, 2H), 8.24 (s, 1H), 7.09 (m, 4H), 6.82 (s, 2H), 4.36 (s, 2H), 4.23 (m, 4H), 3.77 (m, 4H), 3.71 (t, *J* = 5.5, 2H), 2.98 (m, 2H). |
| **6-42** | 0.37 | 450.1 | 3 | | 0.007 | | | DMSO δ 8.79 (s, 2H), 8.12 (s, 1H), 6.75 (s, 2H), 4.14 (m, 4H), 3.69 (m, 4H), 3.49 (s, 2H), 2.89 (d, *J* = 11.5, 2H), 2.78 (m, 4H), 2.46 (m, 2H), 2.31 (d, *J* = 6.7, 2H), 1.61 (m, 3H), 0.97 (m, 2H). |
| **6-43** | 0.38 and 2.38 | 528.4 | 2 | | - | | | DMSO δ 8.80 (s, 2H), 8.14 (s, 1H), 6.76 (s, 2H), 4.15 (m, 4H), 3.70 (m, 4H), 3.50 (m, 4H), 2.81 (s, 2H), 2.78 (s, 3H), 2.65 (m, 2H), 2.37 (m, 2H), 1.75 (m, 3H), 1.18 (m, 4H). |
| **6-44** | 4.16 | 504.3 | 2 | | 0.007 | | 0.139 | DMSO δ 8.87 (s, 2H), 8.25 (s, 1H), 7.28 (m, 3H), 7.13 (m, 2H), 6.83 (s, 2H), 4.59 (s, 2H), 4.32 (d, *J* = 5.4, 2H), 4.22 (m, 4H), 3.82 (m, 2H), 3.76 (m, 4H), 2.90 (m, 2H). |
| **6-45** | 4.37 | 490.3 | 2 | | 0.014 | | 0.101 | DMSO δ 8.91 (s, 1H), 8.87 (s, 2H), 8.25 (s, 1H), 7.46 (m, 1H), 7.27 (m, 2H), 6.83 (s, 2H), 6.77 (dd, *J* = 12.1, 5.7, 1H), 4.72 (s, 2H), 4.23 (m, 4H), 3.92 (t, *J* = 5.4, 2H), 3.77 (m, 4H), 2.97 (m, 2H). |
| **6-46** | 4.21 | 516.3 | 2 | | 0.007 | | 0.112 | DMSO δ 8.86 (s, 2H), 8.24 (s, 1H), 7.16 (m, 3H), 6.93 (d, *J* = 7.8, 1H), 6.87 (t, *J* = 7.5, 1H), 6.82 (s, 2H), 4.59 (s, 2H), 4.23 (m, 6H), 3.83 (m, 2H), 3.78 (s, 3H), 3.76 (m, 4H), 2.90 (m, 2H). |
| **6-47** | 0.32 and 0.57 | 474.3 | 3 | | - | | | DMSO δ 8.79 (s, 2H), 8.15 (s, 1H), 6.87 (m, 1H), 6.76 (s, 2H), 4.15 (m, 4H), 3.69 (m, 4H), 3.59 (s, 2H), 3.10 (m, 2H), 2.86 (s, 3H), 2.83 (m, 4H), 2.64 (m, 2H). |

## Claims

1. A compound of formula I, 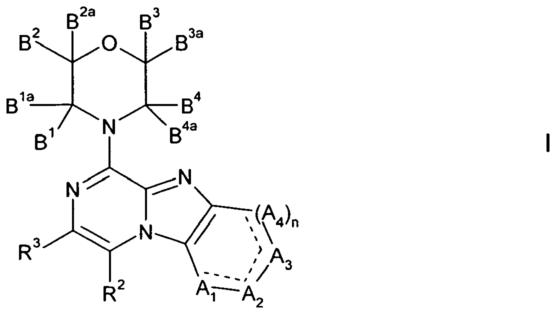 wherein:
n represents 0, 1 or 2;
A₁, A₂, A₃ and each A₄ (if present) independently represents -C(R⁴)R⁵-, -N(R⁶)-, -C(O)-, -O-, -S-, -S(O)- or -S(O)₂-;
the dotted lines represent the presence of an optional double bond, which may be present between A₁ and A₂, A₂ and A₃, A₃ and A₄ (if the latter is present, i.e. when n does not represent 0) and/or between two A₄ groups (if present, i.e. when n represents 2), provided that the A₁ to A₄-containing ring is not aromatic;
each B¹, B^{1a}, B², B^{2a}, B³, B^{3a}, B⁴ and B^{4a} independently represent hydrogen or a substituent selected from halo, -C(=Y)-R^{10a}, -C(=Y)-OR^{10a}, -C(=Y)N(R^{10a})R^{11a}, -S(O)₂N(R^{10a})R^{11a}, -SC(=Y)R^{10a}, -SC(=Y)OR^{10a}, C₁₋₁₂ alkyl, heterocycloalkyl (which latter two groups are optionally substituted by one or more substituents selected from =O and E¹), aryl and/or heteroaryl (which latter two groups are optionally substituted by one or more substituents selected from E²); or
any two B¹, B^{1a}, B², B^{2a}, B³, B^{3a}, B⁴ and B^{4a} substituents that are attached to the same carbon atom (i.e. B¹ and B^{1a}; B² and B^{2a}; B³ and B^{3a}; and/or B⁴ and B^{4a}) may together form a =O group;
or, any two B¹, B^{1a}, B², B^{2a}, B³, B^{3a}, B⁴ and B^{4a} substituents may be linked together to form a further 3- to 12- membered ring, optionally containing (in addition to the atom(s) of the morpholine ring) one or more heteroatom(s), which ring optionally contains one or more double bonds, and which ring is itself optionally substituted by one or more substituents selected from halo, =O and C₁₋₃ alkyl optionally substituted by one or more fluoro atoms;
R² represents hydrogen or a substituent selected from halo, -CN, -OR^{10b}, -N(R^{10b})R^{11b}, -C(O)N(R^{10b})R^{11b}, C₁₋₁₂ (e.g. C₁₋₆) alkyl and heterocycloalkyl (e.g. a 3- to 7-membered heterocycloalkyl), which latter two groups are optionally substituted by one or more substituents selected from E³ and =O;
R³ represents aryl or heteroaryl (both of which are optionally substituted by one or more substituents selected from E⁴);
R⁴ and R⁵ independently represent, on each occasion when used herein, hydrogen, halo, -OR^{10c}, -N(R^{10d})R^{11d}, -N(R^{10e})-C(O)-R^{10f}, -C(O)R^{10g}, -C(O)OR^{10h}, -C(O)N(R¹⁰ⁱ)R¹¹ⁱ, -N(R^{10j})-C(O)OR^{10k}, -N(R^{10m})-C(O)-N(R¹⁰ⁿ)R¹¹n, -N[-C(O)-T¹-R^{10p}]-C(O)-T²-R^{10q}, C₁₋₁₂ alkyl, heterocycloalkyl (which latter two groups are optionally substituted by one or more substituents selected from E⁵ and =O), aryl or heteroaryl (which latter two groups are optionally substituted by one or more substituents selected from E⁶); or
R⁴ and R⁵ may be linked together to form a 3- to 6-membered ring, optionally containing one or more heteroatom(s), which ring optionally contains one or more double bonds, and which ring is itself optionally substituted by one or more substituents selected from halo, =O and C₁₋₃ alkyl optionally substituted by one or more fluoro atoms;
T¹ and T² independently represent a single bond, -N(R^{10x})- or -O-;
R⁶ represents hydrogen, -C(O)-R^{10r}, -C(O)-OR^{10s}, -C(O)-N(R^{10t})R^{11t}, -S(O)₂R^{10u}, C₁₋₁₂ alkyl, heterocycloalkyl (which latter two groups are optionally substituted by one or more substituents selected from E⁷ and =O), aryl or heteroaryl (which latter two groups are optionally substituted by one or more substituents selected from E⁸);
each R^{10a}, R^{11a}, R^{10b}, R^{11b}, R^{10c}, R^{10d}, R^{11d}, R^{10e}, R^{10f}, R^{10g}, R^{10h}, R¹⁰ⁱ, R¹¹ⁱ, R^{10j}, R^{10k}, R^{10m}, R¹⁰ⁿ, R¹¹ⁿ, R^{10p}, R^{10q}, R^{10r}, R^{10s}, R^{10t}, R^{11t}, R^{10u} and R^{10x} independently represent, on each occasion when used herein, hydrogen, C₁₋₁₂ alkyl, heterocycloalkyl (which latter two groups are optionally substituted by one or more substituents selected from =O, =S, =N(R²⁰) and E¹⁰), aryl or heteroaryl (which latter two groups are optionally substituted by one or more substituents selected from E¹¹); or
any relevant pair of R^{10a} and R^{11a} and/or any pair of R^{10b} and R^{11b}, R^{10d} and R^{11d}, R¹⁰ⁱ and R¹¹ⁱ, and R¹⁰ⁿ and R¹¹ⁿ may be linked together to form a 4- to 20-membered ring, optionally containing one or more heteroatoms, optionally containing one or more unsaturations, and which ring is optionally substituted by one or more substituents selected from =O, =S, =N(R²⁰) and E¹²;
each E¹, E², E³, E⁴, E⁵, E⁶, E⁷, E⁸, E¹⁰, E¹¹ and E¹² independently represents, on each occasion when used herein:
(i) Q⁴;
(ii) C₁₋₁₂ alkyl optionally substituted by one or more substituents selected from =O and Q⁵; or
any two E¹, E², E³, E⁴, E⁵, E⁶, E⁷, E⁸, E¹⁰, E¹¹ or E¹² groups, may be linked together to form a 3- to 12-membered ring, optionally containing one or more unsaturations, and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -OC(=Y)-R²⁰, -OC(=Y)-OR²⁰, -OC(=Y)N(R²⁰)R²¹, -OS(O)₂OR²⁰, -OP(=Y)(OR²⁰)(OR²¹), -OP(OR²⁰)(OR²¹), -N(R²²)C(=Y)R²¹, -N(R²²)C(=Y)OR²¹, -N(R²²)C(=Y)N(R²⁰)R²¹, -NR²²S(O)₂R²⁰, -NR²²S(O)₂N(R²⁰)R²¹, -S(O)₂N(R²⁰)R²¹, -SC(=Y)R²⁰, -S(O)₂R²⁰, -SR²⁰, -S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
each Y independently represents, on each occasion when used herein, =O, =S, =NR²³ or =N-CN;
each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or
any relevant pair of R²⁰, R²¹ and R²², may be linked together to form a 4- to 20-membered ring, optionally containing one or more heteroatoms, optionally containing one or more unsaturations, and which ring is optionally substituted by one or more substituents selected from J⁶ and =O;
each J¹, J², J³, J⁴, J⁵ and J⁶ independently represents, on each occasion when used herein:
(i) Q⁷;
(ii) C₁₋₆ alkyl or heterocycloalkyl, both of which are optionally substituted by one or more substituents selected from =O and Q⁸;
each Q⁷ and Q⁸ independently represents, on each occasion when used herein: halo, -N(R⁵⁰)R⁵¹, -OR⁵⁰, -C(=Y^{a})-R⁵⁰ -C(=Y^{a})-OR⁵⁰, -C(=Y^{a})N(R⁵⁰)R⁵¹, -N(R⁵²)C(=Y^{a})R⁵¹, -NR⁵²S(O)₂R⁵⁰, -S(O)₂R⁵⁰, -SR⁵⁰, -S(O)R⁵⁰ or C₁₋₆ alkyl optionally substituted by one or more fluoro atoms;
each Y^{a} independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or
any relevant pair of R⁵⁰, R⁵¹ and R⁵² may be linked together to form, a 3- to 8-membered ring, optionally containing one or more heteroatoms, optionally containing one or more unsaturations, and which ring is optionally substituted by one or more substituents selected from =O and C₁₋₃ alkyl;
R⁶⁰, R⁶¹ and R⁶² independently represent hydrogen or C₁₋₆ alkyl optionally substituted by one or more fluoro atoms;
or a pharmaceutically acceptable ester, amide, solvate or salt thereof
wherein
a pharmaceutically acceptable ester is a carboxylic acid-containing compound of formula I in which one or more of said carboxylic acid groups has been derivatised to form a C₁₋₆ alkyl, C₅₋₁₀ aryl and/or C₅₋₁₀ aryl-C₁₋₆ alkyl- ester; and
a pharmaceutically acceptable amide is a carboxylic acid-containing compound of formula I in which one or more of said carboxylic acid groups has been derivatised to form an amide of formula -C(O)N(R^{z1})R^{z2}, in which R^{z1} and R^{z2} each independently represent optionally substituted C₁₋₆ alkyl, C₅₋₁₀ aryl, or C₅₋₁₀ aryl-C₁₋₆ alkylene-.

2. A compound as claimed in Claim 1, wherein: R² represents hydrogen or chloro; R³ represents optionally substituted phenyl, indazolyl, pyrimidinyl, azaindolyl, indolyl or pyridyl; A₁ represents -C(R⁴)R⁵-, -C(O)- or -N(R⁶)-; A₂ represents -N(R⁶)-, -C(R⁴)R⁵- or -C(O)-; A₃ represents -C(R⁴)R⁵-, -N(R⁶)- or -C(O)-; one of A₂ and A₃ represents -C(R⁴)R⁵- and the other represents -C(R⁴)R⁵-or -N(R⁶)-; n represents 0 or 1; A₄ represents -C(R⁴)R⁵- or -C(O)- and/or the dotted lines do not represent double bonds.

3. A compound as claimed in Claim 1 or Claim 2, wherein: each R⁴ and R⁵ independently represent hydrogen, C₁₋₆ alkyl (optionally substituted as defined herein; but preferably unsubstituted), -OR^{10c}, -C(O)OR^{10h} or -C(O)N(R¹⁰ⁱ)R¹¹ⁱ; and/or each R⁶ (when/if present) independently represents hydrogen, -C(O)R^{10r}, -C(O)OR^{10s}, -C(O)N(R^{10t})R^{11t}, -S(O)₂R^{10u}, C₁₋₆ alkyl (optionally substituted by one or more (e.g. two or, preferably, one) substituents selected from E⁷), heterocycloalkyl (e.g. a 5- or 6-membered group preferably containing one or two heteroatoms; which is optionally substituted by one or more e.g. one, E⁷ substituent(s)) or aryl (e.g. phenyl; optionally substituted by one or more substituents selected from E⁸).

4. A compound as claimed in any one of the preceding claims, wherein: each R^{10a}, R^{11a}, R^{10b}, R^{11b} R^{10c}, R^{10d}, R^{11d}, R^{10e}, R^{10f}, R^{10g}, R^{10h}, R¹⁰ⁱ, R¹¹ⁱ, R^{10j}, R^{10k}, R^{10m}, R¹⁰ⁿ, R¹¹ⁿ, R^{10p}, R^{10q}, R^{10r}, R^{10s}, R^{10t}, R^{11t}, R^{10u} and R^{10x} independently represent, on each occasion when used herein, hydrogen, C₁₋₆ alkyl (optionally substituted by one or more (e.g. one or two) substituents selected from E¹⁰), heterocycloalkyl (e.g. a 4- to 6-membered ring; which heterocycloalkyl group is optionally substituted by one or more (e.g. one or two) substituents selected from E¹⁰), aryl (optionally substituted by one or more (e.g. one or two) substituents selected from E¹¹) or heteroaryl (e.g. a 5- or preferably 6-membered group preferably containing two or one heteroatoms; which heteroaryl group is optionally substituted by one or more (e.g. one or two) substituents selected from E¹¹).

5. A compound as claimed in any one of the preceding claims, wherein: Q⁴ represents halo (e.g. chloro or fluoro), -CN, -OR²⁰, -N(R²⁰)R²¹, -C(=Y)OR²⁰, -C(=Y)-R²⁰, N(R²²)-S(O)₂R²⁰, -N(R²²)-C(=Y)-N(R²⁰)R²¹, -S(O)₂R²⁰, heterocycloalkyl (e.g. a 4- to 6-membered ring, containing preferably one heteroatom selected from nitrogen and oxygen; optionally substituted with two or, preferably, one substituent selected from J²), aryl (e.g. phenyl; optionally substituted with two or, preferably, one substituent selected from J³) or heteroaryl (e.g. a 5- or 6-membered monocyclic heteroaryl group preferably containing one or two heteroatoms preferably selected from nitrogen, oxygen and sulfur; which group may be substituted by one or more substituents selected from J³, but is preferably unsubstituted); Q⁵ represents halo (e.g. fluoro); Y represents =O; R²⁰, R²¹, R²² and R²³ independently represent hydrogen or C₁₋₆ (e.g. C₁₋₄) alkyl optionally substituted by one or more (e.g. one) substituent(s) selected from J⁴; each J¹, J², J³, J⁴, J⁵ and J⁶ independently represent Q⁷ or C₁₋₆ alkyl optionally substituted by one or more Q⁸ groups; Q⁷ represents halo (e.g. fluoro or chloro), -OR⁵⁰, -N(R⁵⁰)R⁵¹, -C(=Y^{a})-OR⁵⁰ or -S(O)₂R⁵⁰; Q⁸ represents halo (e.g. fluoro); Y^{a} represents =O; and/or R⁵⁰ represents hydrogen or C₁₋₆ (e.g. C₁₋₄) alkyl optionally substituted by one or more fluoro atoms.

6. A compound of formula I, or a pharmaceutically acceptable ester, amide, solvate or salt thereof, as defined in any one of Claims 1 to 5, for use as a pharmaceutical.

7. A pharmaceutical formulation including a compound of formula I, or a pharmaceutically acceptable ester, amide, solvate or salt thereof, as defined in any one of Claims 1 to 5, in admixture with a pharmaceutically acceptable adjuvant, diluent or carrier.

8. A compound of formula I, or a pharmaceutically acceptable ester, amide, solvate or salt thereof, as defined in any one of Claims 1 to 5, for use in the treatment of a disease selected from the group consisting of cancer, an immune disorder, a cardiovascular disease, a viral infection, inflammation, a metabolism/endocrine function disorder, a neurological disorder, an obstructive airways disease, an allergic disease, an inflammatory disease, immunosuppression, a disorder commonly connected with organ transplantation, an AIDS-related disease, benign prostate hyperplasia, familial adenomatosis, polyposis, neuro-fibromatosis, psoriasis, a bone disorder, atherosclerosis, vascular smooth cell proliferation associated with atherosclerosis, pulmonary fibrosis, arthritis glomerulonephritis and post-surgical stenosis, restenosis, stroke, diabetes, hepatomegaly, Alzheimer's disease, cystic fibrosis, a hormone-related disease, an immunodeficiency disorder, a destructive bone disorder, an infectious disease, a condition associated with cell death, thrombin-induced platelet aggregation, chronic myelogenous leukaemia, liver disease, a pathologic immune condition involving T cell activation, CNS disorders, and other associated diseases.

9. Use of a compound of formula I, or a pharmaceutically acceptable ester, amide, solvate or salt thereof, as defined in any one of Claims 1 to 5, for the manufacture of a medicament for the treatment of a disease selected from the group consisting of cancer, an immune disorder, a cardiovascular disease, a viral infection, inflammation, a metabolism/endocrine function disorder, a neurological disorder, an obstructive airways disease, an allergic disease, an inflammatory disease, immunosuppression, a disorder commonly connected with organ transplantation, an AIDS-related disease, benign prostate hyperplasia, familial adenomatosis, polyposis, neuro-fibromatosis, psoriasis, a bone disorder, atherosclerosis, vascular smooth cell proliferation associated with atherosclerosis, pulmonary fibrosis, arthritis glomerulonephritis and post-surgical stenosis, restenosis, stroke, diabetes, hepatomegaly, Alzheimer's disease, cystic fibrosis, a hormone-related disease, an immunodeficiency disorder, a destructive bone disorder, an infectious disease, a condition associated with cell death, thrombin-induced platelet aggregation, chronic myelogenous leukaemia, liver disease, a pathologic immune condition involving T cell activation, CNS disorders, and other associated diseases.

10. A combination product comprising:
(A) a compound of formula I, or a pharmaceutically-acceptable ester, amide, solvate or salt thereof as defined in any one of Claims 1 to 5; and
(B) another therapeutic agent that is useful in the treatment of in the treatment of cancer and/or a proliferative disease,
wherein each of components (A) and (B) is formulated in admixture with a pharmaceutically-acceptable adjuvant, diluent or carrier.

11. A process for the preparation of a compound of formula I as defined in Claim 1, which process comprises:
(i) reaction of a compound of formula II, 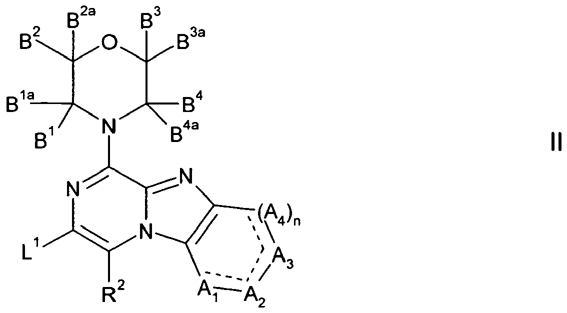 wherein L¹ represents a suitable leaving group,and A¹, A², A³, A⁴, n, the dotted lines, B¹, B^{1a}, B², B^{2a}, B³, B^{3a}, B⁴, B^{4a} and R² are as defined in Claim 1, with a compound of formula III,
R³-L² III
wherein L² represents a suitable group;
(ii) reaction of a compound of formula IV, 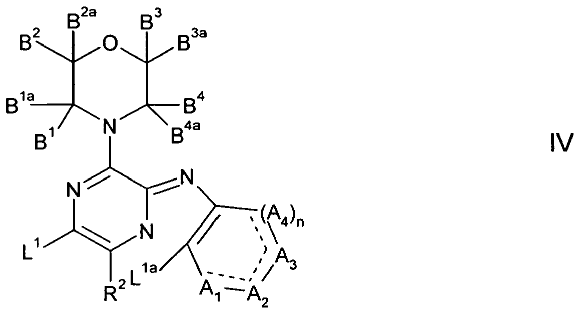 wherein L^{1a} represents a suitable leaving group, and L¹, A¹, A², A³, A⁴, n, the dotted lines, B¹, B^{1a}, B², B^{2a}, B³, B^{3a}, B⁴, B^{4a} and R² are as defined in Claim 1, with a compound of formula III as defined above;
(iii) reaction of a compound of formula V, 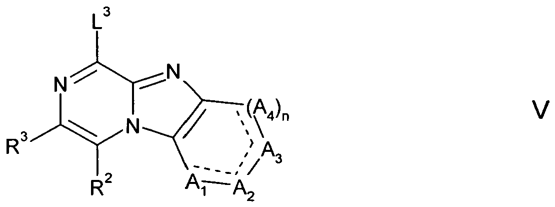 wherein L³ represents a suitable leaving group, and A¹, A², A³, A⁴, n, the dotted lines R² and R³ as defined in Claim 1, with a compound of formula VI, 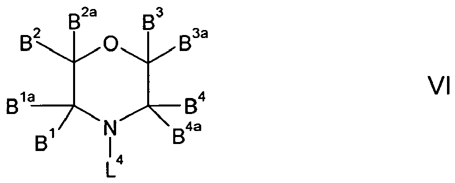 wherein L⁴ may represent hydrogen (so forming an amine group), and L¹, B¹, B^{1a}, B², B^{2a}, B³, B^{3a}, B⁴ and B^{4a} are as defined in Claim 1;
(iv) reaction of a compound of formula VII, 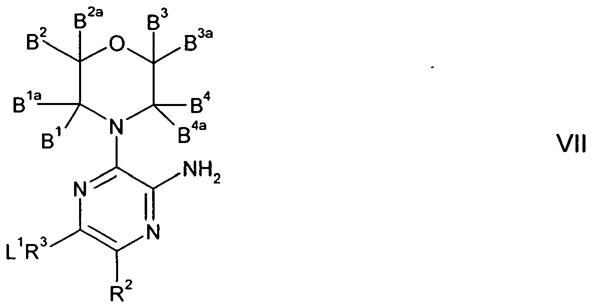 wherein L¹R³ represents either L¹ or R³, B¹, B^{1a}, B², B^{2a}, B³, B^{3a}, B⁴, B^{4a}, R², L¹ and R³ are as defined in Claim 1, with a compound of formula VIII, 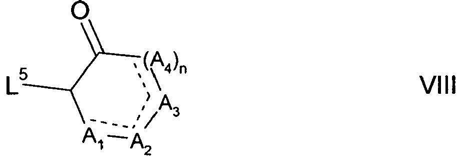 wherein L⁵ represents a suitable leaving group, and A¹, A², A³, A⁴, n, and the dotted lines are as defined in Claim 1;
(v) for compounds of formula I in which R² represents, reaction of a corresponding compound of formula I, in which R² represents hydrogen, with a reagent that is a source of halide ions;
(vi) for compounds of formula I in which R² represents a substituent other than hydrogen, or halo, reaction of a corresponding compound of formula I, in which R² represents halo, with a compound of formula IX,
R^{2a}-L⁷ IX
wherein R^{2a} represents R² as described in Claim 1 provided that it does not represent hydrogen or halo, and L⁷ represents a suitable leaving group;
(vii) for certain compounds of formula I, reaction of a compound of formula X, 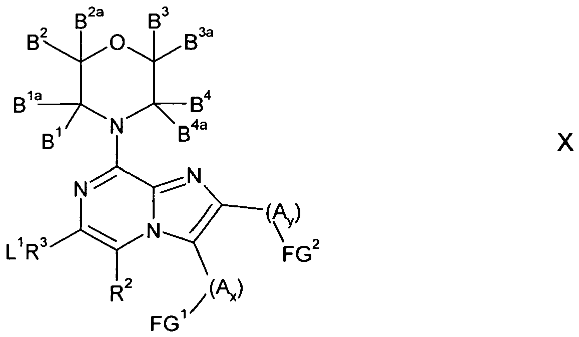 wherein (Aₓ) and (A_{y}) denotes the optional presence of the relevant A₁ to A₄ groups that are/may be present in the compound of formula I, and FG¹ and FG² independently represent mutually compatible functional groups, which may undergo an intramolecular reaction to form the requisite A₁ to A₄-containing ring of formula I (and L¹R³, B¹, B^{1a}, B², B^{2a}, B³, B^{3a}, B, B^{4a} and R² are as defined in Claim 1);
(viii) for certain compounds of formula I, reaction of a compound of formula VII as defined above, with a compound of formula XI, 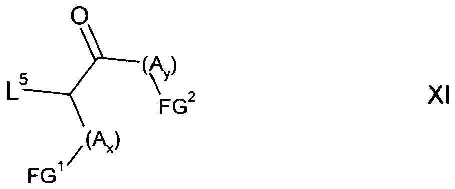 wherein (Aₓ), (A_{y}), FG¹, FG² and L⁵ are as defined above;
(ix) for compounds of formula I in which there is a -N(R⁶)- moiety present, in which R⁶ represents C₁₋₁₂ alkyl optionally substituted as hereinbefore defined (i.e. by one or more substituent(s) selected from E⁷ and =O), reductive amination of a corresponding compound of formula I in which R⁶ represents hydrogen, with a compound of formula XII,
R^{6a}-C(O)H XII
wherein R^{6a} represents C₁₋₁₁ alkyl optionally substituted by one or more substituent(s) selected from E⁷ and =O;
(x) for compounds of formula I in which there is a -N(R⁶)- moiety present, in which R⁶ represents -C(O)N(H)R^{11t}, reaction of a corresponding compound of formula I in which R⁶ represents hydrogen, with a compound of formula XIII,
R^{11t}-N=C=O XII
wherein R^{11t} is as defined in Claim 1;
(xi) for compounds of formula I in which there is a -N(R⁶)- moiety present, in which R⁶ represents -C(O)R^{10r} or -S(O)₂R^{10u}, may be prepared by reaction of a corresponding compound of formula I in which R⁶ represents hydrogen, with a compound of formula XIV,
G¹-L^{1b} XIV
wherein G¹ represents either -C(O)R^{10r} or -S(O)₂R^{10u}, and L^{1b} (attached to the -C(O)- or -S(O)₂ moieties) represents a suitable leaving group.

12. A process for the preparation of a pharmaceutical formulation as defined in Claim 7, which process comprises bringing into association a compound of formula I, or a pharmaceutically acceptable ester, amide, solvate or salt thereof as defined in any one of Claims 1 to 5, with a pharmaceutically-acceptable adjuvant, diluent or carrier.

13. A process for the preparation of a combination product as defined in Claim 10, which process comprises bringing into association a compound of formula I, or a pharmaceutically acceptable ester, amide, solvate or salt thereof as defined in any one of Claims 1 to 5, with the other therapeutic agent that is useful in the treatment of cancer and/or a proliferative disease, and at least one pharmaceutically-acceptable adjuvant, diluent or carrier.

## Patentansprüche

1. Verbindung der Formel I, 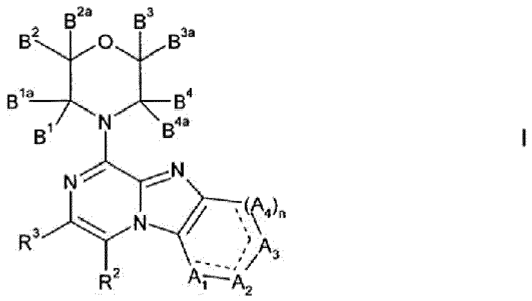 wobei:
n gleich 0, 1 oder 2 darstellt;
A₁, A₂, A₃ und jedes A₄ (falls vorhanden) unabhängig voneinander -C(R⁴)R⁵-, -N(R⁶)-, -C(O)-, -O-, -S-, -S(O)- oder -S(O)₂- darstellen;
die gestrichelten Linien die Gegenwart einer optionalen Doppelbindung darstellen, die zwischen A₁ und A₂, A₂ und A₃, A₃ und A₄ (falls das Letztere vorhanden ist, das heißt wenn n nicht gleich 0 darstellt) und/oder zwischen zwei A₄-Gruppen (falls vorhanden, das heißt wenn n gleich 2 darstellt) vorliegen kann, sofern der A₁ bis A₄ enthaltende Ring nicht aromatisch ist;
jedes B¹, B^{1a}, B², B^{2a}, B³, B^{3a}, B⁴ und B^{4a} unabhängig voneinander Wasserstoff oder einen Substituenten darstellen, der ausgewählt ist aus Halogen, -C(=Y)-R^{10a}, -C(=Y)-OR^{10a}, -C(=Y)N(R^{10a})R^{11a}, -S(O)₂N(R^{10a})-R^{11a}, -SC(=Y)R^{10a}, -SC(=Y)OR^{10a}, C₁₋₁₂-Alkyl, Heterocycloalkyl (wobei die letzten beiden Gruppen gegebenenfalls durch einen oder mehrere Substituenten substituiert sind, die ausgewählt sind aus =O und E¹), Aryl und/oder Heteroaryl (wobei die letzten beiden Gruppen gegebenenfalls durch einen oder mehrere Substituenten substituiert sind, die ausgewählt sind aus E²); oder
beliebige zwei B¹, B^{1a}, B², B^{2a}, B³, B^{3a}, B⁴ und B^{4a}-Substituenten, die an das gleiche Kohlenstoffatom gebunden sind (das heißt B¹ und B^{1a}; B² und B^{2a}; B³ und B^{3a}; und/oder B⁴ und B^{4a}) gemeinsam eine =O-Gruppe bilden können; oder
beliebige zwei B¹, B^{1a}, B², B^{2a}, B³, B^{3a}, B⁴ und B^{4a}-Substituenten miteinander verbunden sein können, um einen weiteren 3- bis 12-gliedrigen Ring zu bilden, der gegebenenfalls (zusätzlich zu dem/den Atom(en) des Morpholin-Ringes) ein oder mehrere Heteroatom(e) enthält, wobei der Ring gegebenenfalls eine oder mehrere Doppelbindungen enthält und wobei der Ring selbst gegebenenfalls durch einen oder mehrere Substituenten substituiert ist, die ausgewählt sind aus Halogen, =O und C₁₋₃-Alkyl, das gegebenenfalls durch ein oder mehrere Fluoratome substituiert ist;
R² Wasserstoff oder einen Substituenten darstellt, der ausgewählt ist aus Halogen, -CN, -OR^{10b}, -N(R^{10b})R^{11b}, -C(O)N(R^{10b})R^{11b}, C₁₋₁₂ (zum Beispiel C₁₋₆) -Alkyl und Heterocycloalkyl (zum Beispiel ein 3- bis 7-gliedriges Heterocycloalkyl), wobei die letzten beiden Gruppen gegebenenfalls durch einen oder mehrere Substituenten substituiert sind, die ausgewählt sind aus E³ und =O;
R³ Aryl oder Heteroaryl darstellt (von denen beide gegebenenfalls durch einen oder mehrere Substituenten substituiert sind, die ausgewählt sind aus E⁴);
R⁴ und R⁵ unabhängig voneinander jedes Mal, wenn sie hierin verwendet werden, Wasserstoff, Halogen, -OR^{10b}, -N(R^{10d})R^{11d}, -N(R^{10e})-C(O)-R^{10f}, -C(O)R^{10g}, -C(O)OR^{10h}, -C(O)N(R¹⁰ⁱ)R¹¹ⁱ, -N(R^{10j})-C(O)OR^{10k}, -N(R^{10m})-C(O)-N(R¹⁰ⁿ)R¹¹ⁿ, -N[-C(O)-T¹-R^{10p}]-C(O)-T²-R^{10q}, C₁₋₁₂-Alkyl, Heterocycloalkyl (wobei die letzten beiden Gruppen gegebenenfalls durch einen oder mehrere Substituenten substituiert sind, die ausgewählt sind aus E⁵ und =O), Aryl oder Heteroaryl (wobei die beiden letzteren Gruppen gegebenenfalls durch einen oder mehrere Substituenten substituiert sind, die ausgewählt sind aus E⁶) darstellen; oder
R⁴ und R⁵ miteinander verbunden sein können, um einen 3- bis 6-gliedrigen Ring zu bilden, der gegebenenfalls ein oder mehrere Heteroatom(e) enthält, wobei der Ring gegebenenfalls eine oder mehrere Doppelbindungen enthält und wobei der Ring selbst gegebenenfalls durch einen oder mehrere Substituenten substituiert ist, die ausgewählt sind aus Halogen, =O und C₁₋₃-Alkyl, das gegebenenfalls durch ein oder mehrere Fluoratome substituiert ist;
T¹ und T² unabhängig voneinander eine Einfachbindung, -N(R^{10x})-oder -O- darstellen;
R⁶ Wasserstoff, -C(O)-R^{10r}, -C(O)-OR^{10s}, -C(O)-N(R^{10t})R^{11t}, -S(O)₂R^{10u}, C₁₋₁₂-Alkyl, Heterocycloalkyl (wobei die letzten beiden Gruppen gegebenenfalls durch einen oder mehrere Substituenten substituiert sind, die ausgewählt sind aus E⁷ und =O), Aryl oder Heteroaryl (wobei die letzten beiden Gruppen gegebenenfalls durch einen oder mehrere Substituenten substituiert sind, die ausgewählt sind aus E⁸) darstellt;
jedes R^{10a}, R^{11a}, R^{10b}, R^{11b}, R^{10c}, R^{10d}, R^{11d}, R^{10e}, R^{10f}, R^{10g}, R^{10h}, R¹⁰ⁱ, R¹¹ⁱ, R^{10j}, R^{10k}, R^{10m}, R¹⁰ⁿ, R¹¹ⁿ, R^{10p}, R^{10q}, R^{10r}, R^{10s}, R^{10t}, R^{11t}, R^{10u} und R^{10x} unabhängig voneinander jedes Mal, wenn sie hierin verwendet werden, Wasserstoff, C₁₋₁₂-Alkyl, Heterocycloalkyl (wobei die letzten beiden Gruppen gegebenenfalls durch einen oder mehrere Substituenten substituiert sind, die ausgewählt sind aus =O, =S, =N(R²⁰) und E¹⁰), Aryl oder Heteroaryl (wobei die letzten beiden Gruppen gegebenenfalls durch einen oder mehrere Substituenten substituiert sind, die ausgewählt sind aus E¹¹) darstellen; oder
jedes relevante Paar von R^{10a} und R^{11a} und/oder jedes Paar von R^{10b} und R^{11b}, R^{10d} und R^{11d} R¹⁰ⁱ und R¹¹ⁱ und R¹⁰ⁿ und R¹¹ⁿ miteinander verbunden sein können, um einen 4- bis 20-gliedrigen Ring zu bilden, der gegebenenfalls ein oder mehrere Heteroatome enthält, gegebenenfalls eine oder mehrere Ungesättigtheiten enthält, und wobei der Ring gegebenenfalls durch einen oder mehrere Substituenten substituiert ist, die ausgewählt sind aus =O, =S, =N(R²⁰) und E¹²;
jedes E¹, E², E³, E⁴, E⁵, E⁶, E⁷, E⁸, E¹⁰, E¹¹ und E¹² unabhängig voneinander jedes Mal, wenn sie hierin verwendet werden, darstellen:
(i) Q⁴;
(ii) C₁₋₁₂-Alkyl, das gegebenenfalls durch einen oder mehrere Substituenten substituiert ist, die ausgewählt sind aus =O und Q⁵; oder
beliebige zwei E¹, E², E³, E⁴, E⁵, E⁶, E⁷, E⁸, E¹⁰, E¹¹ und E¹²-Gruppen miteinander verbunden sein können, um einen 3- bis 12-gliedrigen Ring zu bilden, der gegebenenfalls eine oder mehrere Ungesättigtheiten enthält, und wobei der Ring gegebenenfalls durch einen oder mehrere Substituenten substituiert ist, die ausgewählt sind aus =O und J¹;
jedes Q⁴ und Q⁵ unabhängig voneinander jedes Mal, wenn sie hierin verwendet werden, Halogen, -CN, -NO₂, -N(R²⁰)R²¹, -OR²⁰, -C(=Y)-R²⁰, -C(=Y)-OR²⁰, -C(=Y)N(R²⁰)R²¹, -OC(=Y)-R²⁰, -OC(=Y)-OR²⁰, -OC(=Y)N(R²⁰)R²¹, -OS(O)₂OR²⁰, -OP(=Y)(OR²⁰)(OR²¹), -OP(OR²⁰)(OR²¹), -N(R²²)-C(=Y)R²¹, -N(R²²)C(=Y)OR²¹, -N(R²²)-C(=Y)N(R²⁰)R²¹, -NR²²S(O)₂R²⁰, -NR²²S(O)₂N(R²⁰)R²¹, -S(O)₂N(R²⁰)R²¹, -SC(Y)R²⁰, -S(O)₂R²⁰, -SR²⁰, -S(O)R²⁰, C₁₋₆-Alkyl, Heterocycloalkyl (wobei letzten beiden Gruppen gegebenenfalls durch einen oder mehrere Substituenten substituiert sind, die ausgewählt sind aus =O und J²), Aryl oder Heteroaryl (wobei die letzten beiden Gruppen gegebenenfalls durch einen oder mehrere Substituenten substituiert sind, die ausgewählt sind aus J³) darstellen;
jedes Y unabhängig voneinander jedes Mal, wenn es hierin verwendet wird, =O, =S, =NR²³ oder =N-CN darstellt;
jedes R²⁰, R²¹, R²² und R²³ unabhängig voneinander jedes Mal, wenn sie hierin verwendet werden, Wasserstoff, C₁₋₆-Alkyl, Heterocycloalkyl (wobei die letzten beiden Gruppen gegebenenfalls durch einen oder mehrere Substituenten substituiert sind, die ausgewählt sind aus J⁴ und =O), Aryl oder Heteroaryl (wobei die letzten beiden Gruppen gegebenenfalls durch einen oder mehrere Substituenten substituiert sind, die ausgewählt sind aus J⁵) darstellen; oder
jedes relevante Paar von R²⁰, R²¹ und R²² miteinander verbunden sein können, um einen 4- bis 20-gliedrigen Ring zu bilden, der gegebenenfalls ein oder mehrere Heteroatome enthält, gegebenenfalls eine oder mehrere Ungesättigtheiten enthält und wobei der Ring gegebenenfalls durch einen oder mehrere Substituenten substituiert ist, die ausgewählt sind aus J⁶ und =O;
jedes J¹, J², J³, J⁴, J⁵ und J⁶ unabhängig voneinander jedes Mal,
wenn sie hierin verwendet werden, darstellen:
(i) Q⁷;
(ii) C₁₋₆-Alkyl oder Heterocycloalkyl, die beide gegebenenfalls mit einem oder mehreren Substituenten substituiert sind, die ausgewählt sind aus =O und Q⁸;
jedes Q⁷ und Q⁸ unabhängig voneinander jedes Mal, wenn sie hierin verwendet werden, Halogen, -N(R⁵⁰)R⁵¹, -OR⁵⁰, -C(=Y^{a})-R⁵⁰, -C(=Y)-OR⁵⁰, -C(=Y)N(R⁵⁰)R⁵¹, -N(R⁵²)-C(=Y)R⁵¹, -NR⁵²S(O)₂R⁵⁰, -S(O)₂R⁵⁰, -SR⁵⁰, -S(O)R⁵⁰ oder C₁₋₆-Alkyl, das gegebenenfalls durch ein oder mehrere Fluoratome substituiert ist, darstellen;
jedes Y^{a} unabhängig voneinander jedes Mal, wenn es hierin verwendet wird, =O, =S, =NR⁵³ oder =N-CN darstellt;
jedes R⁵⁰, R⁵¹, R⁵² und R⁵³ unabhängig voneinander jedes Mal, wenn sie hierin verwendet werden, Wasserstoff oder C₁₋₆-Alkyl, das gegebenenfalls durch einen oder mehrere Substituenten substituiert ist, die ausgewählt sind aus Fluor, -OR⁶⁰ und -N(R⁶¹)R⁶² darstellen; oder
jedes relevante Paar von R⁵⁰, R⁵¹ und R⁵² miteinander verbunden sein können, um einen 3- bis 8-gliedrigen Ring zu bilden, der gegebenenfalls ein oder mehrere Heteroatome enthält, gegebenenfalls eine oder mehrere Ungesättigtheiten enthält und wobei der Ring gegebenenfalls durch einen oder mehrere Substituenten substituiert ist, die ausgewählt sind aus =O und C₁₋₃-Alkyl;
R⁶⁰, R⁶¹ und R⁶² unabhängig voneinander Wasserstoff oder C₁₋₆-Alkyl, das gegebenenfalls durch ein oder mehrere Fluoratome substituiert ist, darstellen;
oder ein pharmazeutisch verträglicher Ester, Amid, Solvat oder Salz davon,
wobei
ein pharmazeutisch verträglicher Ester eine eine Carbonsäure enthaltende Verbindung der Formel I ist, wobei eine oder mehrere der Carbonsäure-Gruppen derivatisiert worden sind, um ein C₁₋₆-Alkyl, C₅₋₁₀-Aryl und/oder C₅₋₁₀-Aryl-C₁₋₆-alkyl-ester zu bilden und
ein pharmazeutisch verträgliches Amid eine eine Carbonsäure enthaltende Verbindung der Formel I ist, wobei eine oder mehrere der Carbonsäure-Gruppen derivatisiert worden sind, um ein Amid der Formel -C(O)N(R^{z1})R^{z2} zu bilden, wobei R^{z1} und R^{z2} unabhängig voneinander gegebenenfalls substituiertes C₁₋₆-Alkyl, C₅₋₁₀-Aryl oder C₅₋₁₀-Aryl-C₁₋₆-alkylen darstellen.

2. Verbindung nach Anspruch 1, wobei: R² Wasserstoff oder Chlor darstellt; R³ gegebenenfalls substituiertes Phenyl, Indazolyl, Pyrimidinyl, Azaindolyl, Indolyl oder Pyridyl darstellt; A₁ -C(R⁴)R⁵-, -C(O)- oder -N(R⁶)- darstellt; A₂ -N(R⁶)-, -C(R⁴)R⁵- oder -C(O)-darstellt; A₃ -C(R⁴)R⁵-, -N(R⁶)- oder -C(O)- darstellt; eines der A₂ und A₃ -C(R⁴)R⁵- darstellt und das andere -C(R⁴)R⁵- oder -N(R⁶)-darstellt; n gleich 0 oder 1 darstellt; A₄ -C(R⁴)R⁵- oder -C(O)- darstellt und/oder die gestrichelten Linien keine Doppelbindungen darstellen.

3. Verbindung nach Anspruch 1 oder 2, wobei: jedes R⁴ und R⁵ unabhängig voneinander Wasserstoff, C₁₋₆-Alkyl (gegebenenfalls substituiert wie hierin definiert, aber bevorzugt unsubstituiert), -OR^{10c}, -C(O)OR^{10h} oder -C(O)N(R¹⁰ⁱ)R¹¹ⁱ darstellen; und/oder R⁶ (falls vorhanden) unabhängig voneinander Wasserstoff, -C(O)R^{10r}, -C(O)OR^{10s}, -C(O)N(R^{10t})R^{11t}, -S(O)₂R^{10u}, C₁₋₆-Alkyl (gegebenenfalls durch einen oder mehrere (zum Beispiel zwei oder vorzugsweise einen) Substituenten substituiert, die ausgewählt sind aus E⁷), Heterocycloalkyl (zum Beispiel eine 5- oder 6-gliedrige Gruppe, die vorzugsweise ein oder zwei Heteroatome enthält und die gegebenenfalls durch einen oder mehrere, zum Beispiel einen, E⁷-Substituenten substituiert ist) oder Aryl (zum Beispiel Phenyl, gegebenenfalls durch einen oder mehrere Substituenten substituiert, die ausgewählt sind aus E⁸) darstellt.

4. Verbindung nach einem der vorhergehenden Ansprüche, wobei: jedes R^{10a}, R^{11a}, R^{10b}, R^{11b}, R^{10c}, R^{10d}, R^{11d}, R^{10e}, R^{10f}, R^{10g}, R^{10h}, R¹⁰ⁱ, R¹¹ⁱ, R^{10j}, R^{10k}, R^{10m}, R¹⁰ⁿ, R¹¹ⁿ, R^{10p}, R^{10q}, R^{10r}, R^{10s}, R^{10t}, R^{11t}, R^{10u} und R^{10x} unabhängig voneinander jedes Mal, wenn sie hierin verwendet werden, Wasserstoff, C₁₋₆-Alkyl (gegebenenfalls durch einen oder mehrere (zum Beispiel einen oder zwei) Substituenten substituiert, die ausgewählt sind aus E¹⁰), Heterocycloalkyl (zum Beispiel einen 4- bis 6-gliedrigen Ring, wobei die Heterocycloalkyl-Gruppe gegebenenfalls durch einen oder mehrere (zum Beispiel einen oder zwei) Substituenten substituiert ist, die ausgewählt sind aus E¹⁰), Aryl (gegebenenfalls durch einen oder mehrere (zum Beispiel einen oder zwei) Substituenten substituiert, die ausgewählt sind aus E¹¹) oder Heteroaryl (zum Beispiel eine 5- oder vorzugsweise 6-gliedrigen Gruppe, die bevorzugt zwei oder ein Heteroatom enthält, wobei die Heteroaryl-Gruppe gegebenenfalls durch einen oder mehrere (zum Beispiel einen oder zwei) Substituenten substituiert ist, die ausgewählt sind aus E¹¹) darstellen.

5. Verbindung nach einem der vorhergehenden Ansprüche, wobei: Q⁴ Halogen (zum Beispiel Chlor oder Fluor), -CN, -OR²⁰, -N(R²⁰)R²¹, -C(=Y)OR²⁰, -C(=Y)-R²⁰, -N(R²²)-S(O)₂R₂₀, -N(R²²)-C(=Y)-N(R²⁰)R²¹, -S(O)₂R²⁰, Heterocycloalkyl (zum Beispiel einen 4- bis 6-gliedrigen Ring, der bevorzugt ein Heteroatom enthält, das ausgewählt ist aus Stickstoff und Sauerstoff, und gegebenenfalls mit zwei oder insbesondere einem Substituenten substituiert ist, der ausgewählt ist aus J²), Aryl (zum Beispiel Phenyl; gegebenenfalls substituiert mit zwei oder vorzugsweise einem Substituenten, der ausgewählt ist aus J³) oder Heteroaryl (zum Beispiel eine 5- oder 6-gliedrige monocyclische Heteroaryl-Gruppe, die vorzugsweise ein oder zwei Heteroatome enthält, die bevorzugt ausgewählt sind aus Stickstoff, Sauerstoff und Schwefel, wobei die Gruppe durch einen oder mehrere Substituenten substituiert sein kann, die ausgewählt sind aus J³, die aber vorzugsweise unsubstituiert ist) darstellt; Q⁵ Halogen (zum Beispiel Fluor) darstellt; Y =O darstellt; R²⁰, R²¹, R²² und R²³ unabhängig voneinander Wasserstoff oder C₁₋₆ (zum Beispiel C₁₋₄) -Alkyl darstellen, das gegebenenfalls durch einen oder mehrere (zum Beispiel einen) Substituenten substituiert ist, die ausgewählt sind aus J⁴; jedes J¹, J², J³, J⁴, J⁵ und J⁶ unabhängig voneinander Q⁷ oder C₁₋₆-Alkyl darstellen, das gegebenenfalls durch eine oder mehrere Q⁸-Gruppen substituiert ist; Q⁷ Halogen (zum Beispiel Fluor oder Chlor), -OR⁵⁰, -N(R⁵⁰)R⁵¹, -C(=Y^{a})-OR⁵⁰ oder -S(O)₂R⁵⁰ darstellt; Q⁸ Halogen (zum Beispiel Fluor) darstellt; Y^{a} =O darstellt; und/oder R⁵⁰ Wasserstoff oder C₁₋₆ (zum Beispiel C₁₋₄) -Alkyl darstellt, das gegebenenfalls durch ein oder mehrere Fluoratome substituiert ist.

6. Verbindung der Formel I oder ein pharmazeutisch verträglicher Ester, Amid, Solvat oder Salz davon, wie in einem der Ansprüche 1 bis 5 definiert, zur Verwendung als ein Arzneimittel.

7. Pharmazeutische Formulierung, umfassend eine Verbindung der Formel I oder ein pharmazeutisch verträglicher Ester, Amid, Solvat oder Salz davon, wie in einem der Ansprüche 1 bis 5 definiert, in Beimischung mit einem pharmazeutisch verträglichen Adjuvans, Verdünnungsmittel oder Träger.

8. Verbindung der Formel I oder ein pharmazeutisch verträglicher Ester, Amid, Solvat oder Salz davon, wie in einem der Ansprüche 1 bis 5 definiert, zur Verwendung bei der Behandlung einer Erkrankung, die ausgewählt ist aus der Gruppe bestehend aus Krebs, einer Immunstörung, einer kardiovaskulären Erkrankung, einer viralen Infektion, Entzündung, einer Metabolismus-/endokrine Funktion-Störung, einer neurologischen Störung, einer obstruktiven Atemwegserkrankung, einer allergischen Erkrankung, einer entzündlichen Erkrankung, Immunosupression, einer Störung, die gewöhnlich mit einer Organtransplantation verbunden ist, einer mit AIDS verbundenen Erkrankung, gutartiger Prostatahyperplasie, familiärer Adenomatose, Polypose, Neurofibromatose, Psoriasis, einer Knochenstörung, Atherosklerose, vaskulärer glatter Zellproliferation, die mit Atherosklerose verbunden ist, pulmonaler Fibrose, Arthritis, Glomerulonephritis und post-operativer Stenose, Restenose, Schlaganfall, Diabetes, Hepatomegalie, Alzheimer-Krankheit, zystischer Fibrose, einer hormonbedingten Erkrankung, einer Immunopathie, einer destruktiven Knochenstörung, einer infektiösen Erkrankung, einem Zustand, der mit Zelltod verbunden ist, Thrombin-induzierter Thrombozytenaggregation, chronischer myeloischer Leukämie, Lebererkrankung, einem pathologischen Immunzustand, an dem T-Zellaktivierung beteiligt ist, ZNS-Störungen und anderen verbundenen Erkrankungen.

9. Verwendung einer Verbindung der Formel I oder ein pharmazeutisch verträglicher Ester, Amid, Solvat oder Salz davon, wie in einem der Ansprüche 1 bis 5 definiert, zur Herstellung eines Medikaments zur Behandlung einer Erkrankung, die ausgewählt ist aus der Gruppe bestehend aus Krebs, einer Immunstörung, einer kardiovaskulären Erkrankung, einer viralen Infektion, Entzündung, einer Metabolismus/endokrine Funktion-Störung, einer neurologischen Störung, einer obstruktiven Atemwegserkrankung, einer allergischen Erkrankung, einer entzündlichen Erkrankung, Immunosupression, einer Störung, die gewöhnlich mit einer Organtransplantation verbunden ist, einer mit AIDS verbundenen Erkrankung, gutartiger Prostatahyperplasie, familiärer Adenomatose, Polypose, Neurofibromatose, Psoriasis, einer Knochenstörung, Atherosklerose, vaskulärer glatter Zellproliferation, die mit Atherosklerose verbunden ist, pulmonaler Fibrose, Arthritis, Glomerulonephritis und post-operativer Stenose, Restenose, Schlaganfall, Diabetes, Hepatomegalie, Alzheimer-Krankheit, zystischer Fibrose, einer hormonbedingten Erkrankung, einer Immunopathie, einer destruktiven Knochenstörung, einer infektiösen Erkrankung, einem Zustand, der mit Zelltod verbunden ist, Thrombin-induzierter Thrombozytenaggregation, chronischer myeloischer Leukämie, Lebererkrankung, einem pathologischen Immunzustand, an dem T-Zellaktivierung beteiligt ist, ZNS-Störungen und anderen verbundenen Erkrankungen.

10. Kombinationsprodukt, aufweisend:
(A) eine Verbindung der Formel I oder ein pharmazeutisch verträglicher Ester, Amid, Solvat oder Salz davon, wie in einem der Ansprüche 1 bis 5 definiert; und
(B) ein anderes therapeutisches Mittel, das bei der Behandlung von Krebs und/oder einer proliferativen Erkrankung nützlich ist,
wobei jede der Komponenten (A) und (B) in Beimischung mit einem pharmazeutisch verträglichen Adjuvans, Verdünnungsmittel oder Träger formuliert ist.

11. Verfahren zur Zubereitung einer Verbindung der Formel I, wie in Anspruch 1 definiert, wobei das Verfahren aufweist:
(i) Reaktion einer Verbindung der Formel II, 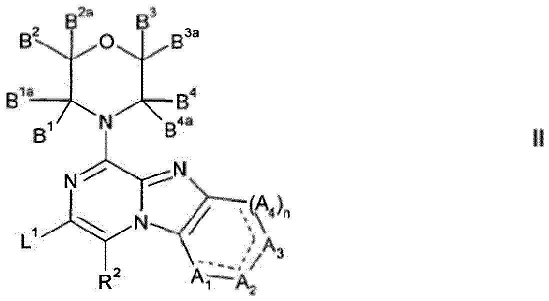 wobei L¹ eine geeignete Abgangsgruppe darstellt und A¹, A², A³, A⁴, n, die gestrichelten Linien, B¹, B^{1a}, B², B^{2a}, B³, B^{3a}, B⁴, B^{4a} und R² wie in Anspruch 1 definiert sind, mit einer Verbindung der Formel III,
R³-L² III
wobei L² eine geeignete Gruppe darstellt;
(ii) Reaktion einer Verbindung der Formel IV, 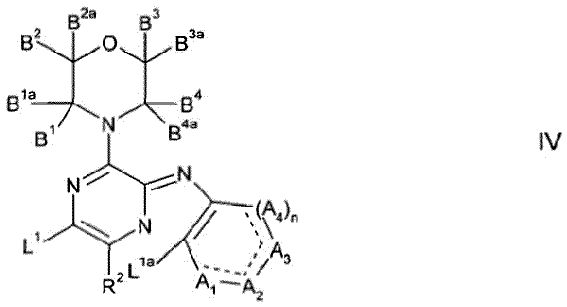 wobei L^{1a} eine geeignete Abgangsgruppe darstellt und L¹, A¹, A², A³, A⁴, n, die gestrichelten Linien, B¹, B^{1a}, B², B^{2a}, B³, B^{3a}, B⁴, B^{4a} und R² wie in Anspruch 1 definiert sind, mit einer Verbindung der Formel III, wie oben definiert;
(iii) Reaktion einer Verbindung der Formel V, 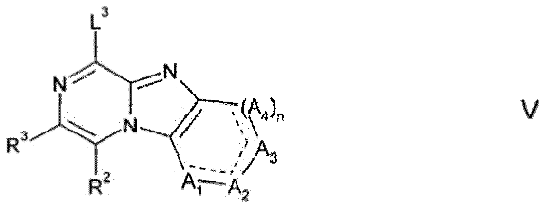 wobei L³ eine geeignete Abgangsgruppe darstellt und A¹, A², A³, A⁴, n, die gestrichelten Linien, R² und R³ wie in Anspruch 1 definiert sind, mit einer Verbindung der Formel VI, 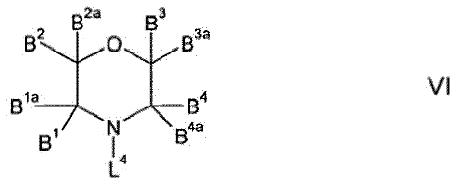 wobei L⁴ Wasserstoff darstellen kann (und so eine Amin-Gruppe bildet) und L¹, B¹, B^{1a}, B², B^{2a}, B³, B^{3a}, B⁴ und B^{4a} wie in Anspruch 1 definiert sind;
(iv) Reaktion einer Verbindung der Formel VII, 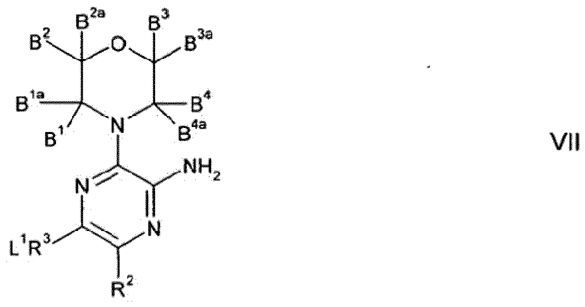 wobei L¹R³ entweder L¹ oder R³ darstellt und B¹, B^{1a}, B², B^{2a}, B³, B^{3a}, B⁴, B^{4a}, R², L¹ und R³ wie in Anspruch 1 definiert sind, mit einer Verbindung der Formel VIII, 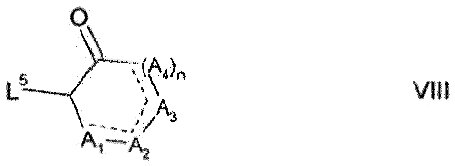 wobei L⁵ eine geeignete Abgangsgruppe darstellt und A¹, A², A³, A⁴, n und die gestrichelten Linien wie in Anspruch 1 definiert sind;
(v) für Verbindungen der Formel I, in denen R² darstellt, Reaktion einer entsprechenden Verbindung der Formel I, in der R² Wasserstoff darstellt, mit einem Reagens, das eine Quelle für Halogenid-Ionen ist;
(vi) für Verbindungen der Formel I, in denen R² einen von Wasserstoff oder Halogen verschiedenen Substituenten darstellt, Reaktion einer entsprechenden Verbindung der Formel I, in der R² Halogen darstellt, mit einer Verbindung der Formel IX,
R^{2a}-L⁷ IX
wobei R^{2a} R², wie in Anspruch 1 beschrieben, darstellt, sofern es nicht Wasserstoff oder Halogen darstellt, und L⁷ eine geeignete Abgangsgruppe darstellt;
(vii) für bestimmte Verbindungen der Formel I, Reaktion einer Verbindung der Formel X, 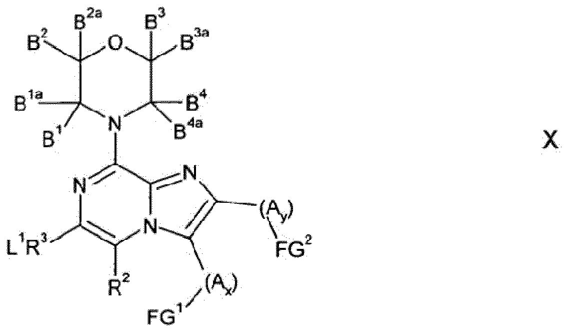 wobei (Aₓ) und (Ay) die optionale Gegenwart der relevanten A¹ bis A⁴-Gruppen bezeichnen, die in der Verbindung der Formel I vorhanden sind oder sein können, und FG¹ und FG² unabhängig voneinander gegenseitig kompatible funktionellen Gruppen darstellen, die eine intramolekulare Reaktion erfahren können, um den erforderlichen A¹ bis A⁴-enthaltenden Ring der Formel I zu bilden (und L¹R³, B¹, B^{1a}, B², B^{2a}, B³, B^{3a}, B⁴, B^{4a} und R² sind wie in Anspruch 1 definiert);
(viii) für bestimmte Verbindungen der Formel I, Reaktion einer Verbindung der Formel VII, wie oben definiert, mit einer Verbindung der Formel XI, 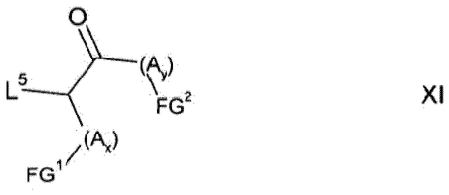 wobei (Aₓ), (Ay), FG¹, FG² und L⁵ wie oben definiert sind;
(ix) für Verbindungen der Formel I, in denen eine -N(R⁶)-Einheit vorhanden ist, in der R⁶ ein C₁₋₁₂-Alkyl darstellt, das gegebenenfalls wie vorstehend definiert substituiert ist (das heißt durch einen oder mehrere Substituenten, die ausgewählt sind aus E⁷ und =O), reduktive Aminierung einer entsprechenden Verbindung der Formel I, in der R⁶ Wasserstoff darstellt, mit einer Verbindung der Formel XII,
R^{6a}-C(O)H XII
wobei R^{6a} ein C₁₋₁₁-Alkyl darstellt, das gegebenenfalls durch einen oder mehrere Substituenten substituiert ist, die ausgewählt sind aus E⁷ und =O;
(x) für Verbindungen der Formel I, in denen eine -N(R⁶)-Einheit vorhanden ist, in der R⁶ -C(O)N(H)R^{11t} darstellt, Reaktion einer entsprechenden Verbindung der Formel I, in der R⁶ Wasserstoff darstellt, mit einer Verbindung der Formel XIII,
R^{11t}-N=C=O XIII
wobei R^{11t} wie in Anspruch 1 definiert ist;
(xi) für Verbindungen der Formel I, in denen eine -N(R⁶)-Einheit vorhanden ist, in der R⁶ -C(O)R^{10r} oder -S(O)₂R^{10u} darstellt, kann zubereitet werden durch Reaktion einer entsprechenden Verbindung der Formel I, in der R⁶ Wasserstoff darstellt, mit einer Verbindung der Formel XIV,
G¹-L^{1b} XIV
wobei G¹ entweder -C(O)R^{10r} oder -S(O)₂R^{10u} darstellt und L^{1b} (an die -C(O)- oder -S(O)₂-Einheiten gebunden) eine geeignete Abgangsgruppe darstellt.

12. Verfahren zur Zubereitung einer pharmazeutischen Formulierung, wie in Anspruch 7 definiert, wobei das Verfahren ein Zusammenbringen einer Verbindung der Formel I oder eines pharmazeutisch verträglichen Esters, Amids, Solvats oder Salzes davon, wie in einem der Ansprüche 1 bis 5 definiert, mit einem pharmazeutisch verträglichen Adjuvans, Verdünnungsmittel oder Träger aufweist.

13. Verfahren zur Zubereitung eines Kombinationsproduktes, wie in Anspruch 10 definiert, wobei das Verfahren ein Zusammenbringen einer Verbindung der Formel I oder eines pharmazeutisch verträglichen Esters, Amids, Solvats oder Salzes davon, wie in einem der Ansprüche 1 bis 5 definiert, mit dem anderen therapeutischen Mittel, das bei der Behandlung von Krebs und/oder einer proliferativen Erkrankung nützlich ist, und mindestens einem pharmazeutisch verträglichen Adjuvans, Verdünnungsmittel oder Träger aufweist.

## Revendications

1. Composé de formule I, 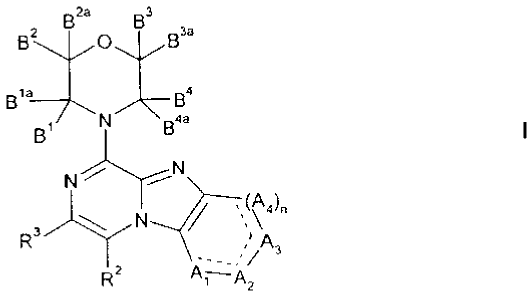 dans laquelle :
n représente 0, 1 ou 2 ;
A₁, A₂, A₃ et chaque A₄ (si présent) représentent indépendamment -C(R⁴)R⁵-, -N(R⁶)-, -C(O)-, -O-, -S-, -S(O)- ou -S(O)₂- ;
les lignes en pointillés représentent la présence d'une double liaison facultative, qui peut être présente entre A₁ et A₂, A₂ et A₃, A₃ et A₄ (si ce dernier est présent, c'est-à-dire lorsque n ne représente pas 0) et/ou entre deux groupes A₄ (si présents, c'est-à-dire lorsque n représente 2), à condition que le cycle contenant A₁ à A₄ ne soit pas aromatique ;
chaque B¹, B^{1a}, B², B^{2a}, B³, B^{3a}, B⁴ et B^{4a} représente indépendamment un hydrogène ou un substituant choisi parmi halogéno, -C(=Y)-R^{10a}, -C(=Y)-OR^{10a}, -C(=Y)N(R^{10a})R^{11a}, -S(O)₂N(R^{10a})R^{11a}, -SC(=Y)R^{10a}, -SC (=Y) OR^{10a}, alkyle en C₁ à ₁₂, hétérocycloalkyle (lesquels deux derniers groupes étant facultativement substitués par un ou plusieurs substituants choisis parmi =0 et E¹), aryle et/ou hétéroaryle (lesquels deux derniers groupes étant facultativement substitués par un ou plusieurs substituants choisis parmi E²) ; ou
deux substituants B¹, B^{1a}, B², B^{2a}, B³, B^{3a}, B⁴ et B^{4a} quelconques qui sont attachés au même atome de carbone (c'est-à-dire B¹ et B^{1a} ; B² et B^{2a} ; B³ et B^{3a} ; et/ou B⁴ et B^{4a}) peuvent former ensemble un groupe =0 ;
ou deux substituants B¹, B^{1a}, B², B^{2a}, B³, B^{3a}, B⁴ et B^{4a} quelconques peuvent être liés ensemble pour former un cycle à 3 à 12 chaînons supplémentaire, contenant facultativement (en plus de l'atome ou des atomes du cycle morpholine) un ou plusieurs hétéroatome(s), lequel cycle contient facultativement une ou plusieurs doubles liaisons, et lequel cycle est lui-même facultativement substitué par un ou plusieurs substituants choisis parmi halogéno, =0 et alkyle en C_{1 à 3} facultativement substitué par un ou plusieurs atomes fluoro ;
R² représente un hydrogène ou un substituant choisi parmi halogéno, -CN, -OR^{10b}, -N(R^{10b})R^{11b}, -C(O)N(R^{10b})R^{11b}, alkyle en C₁ à ₁₂ (par exemple, en C₁ à ₆) et hétérocycloalkyle (par exemple, un hétérocycloalkyle à 3 à 7 chaînons), ces deux derniers groupes étant facultativement substitués par un ou plusieurs substituants choisis parmi E³ et =O ;
R³ représente un aryle ou hétéroaryle (tous deux étant facultativement substitués par un ou plusieurs substituants choisis parmi E⁴) ;
R⁴ et R⁵ représentent indépendamment, chaque fois qu'ils sont utilisés ici, un hydrogène, halogéno, -OR^{10c}, -N(R^{10d})R^{11d}, -N(R^{10e})-C(O)-R^{10f}, -C(O)R^{10g}, -C(O)OR^{10h}, -C(O)N(R¹⁰ⁱ)R¹¹ⁱ, -N(R^{10j})-C(O)OR^{10k}, -N(R^{10m})-C(O)-N(R¹⁰ⁿ)R¹¹ⁿ, -N[-C(O)-T¹-R^{10p}]-C(O)-T²-R^{10q}, alkyle en C_{1 à 12,} hétérocycloalkyle (lesquels deux derniers groupes étant facultativement substitués par un ou plusieurs substituants choisis parmi E⁵ et =0), aryle ou hétéroaryle (lesquels deux derniers groupes étant facultativement substitués par un ou plusieurs substituants choisis parmi E⁶) ; ou
R⁴ et R⁵ peuvent être liés ensemble pour former un cycle à 3 à 6 chaînons, contenant facultativement un ou plusieurs hétéroatome(s), lequel cycle contient facultativement une ou plusieurs doubles liaisons, et lequel cycle est lui-même facultativement substitué par un ou plusieurs substituants choisis parmi halogéno, =0 et alkyle en C_{1 à 3} facultativement substitué par un ou plusieurs atomes fluoro ;
T¹ et T² représentent indépendamment une simple liaison, -N(R^{10x}) - ou -O- ;
R⁶ représente un hydrogène, -C(O)-R^{10r}, -C(O)-OR^{10s}, -C(O)-N(R^{10t})R^{11t}, -S(O)₂R^{10u}, alkyle en C₁ à ₁₂, hétérocycloalkyle (lesquels deux derniers groupes étant facultativement substitués par un ou plusieurs substituants choisis parmi E⁷ et =O), aryle ou hétéroaryle (lesquels deux derniers groupes étant facultativement substitués par un ou plusieurs substituants choisis parmi E⁸) ;
chaque R^{10a}, R^{11a}, R^{10b}, R^{11b}, R^{10c}, R^{10d}, R^{11d}, R^{10e}, R^{10f}, R^{10g}, R^{10h}, R¹⁰ⁱ, R¹¹ⁱ, R^{10j}, R^{10k}, R^{10m}, R¹⁰ⁿ, R¹¹ⁿ, R^{10p}, R^{10q}, R^{10r}, R^{10s}, R^{10t}, R^{11t}, R^{10u} et R^{10x} représente indépendamment, chaque fois qu'ils sont utilisés ici, un hydrogène, alkyle en C_{1 à 12}, hétérocycloalkyle (lesquels deux derniers groupes étant facultativement substitués par un ou plusieurs substituants choisis parmi =O, =S, =N(R²⁰) et E¹⁰), aryle ou hétéroaryle (lesquels deux derniers groupes étant facultativement substitués par un ou plusieurs substituants choisis parmi E¹¹) ; ou
toutes les paires pertinentes de R^{10a} et R^{11a} et/ou toutes les paires de R^{10b} et R^{11b}, R^{10d} et R^{11d}, R¹⁰ⁱ et R¹¹ⁱ, et R¹⁰ⁿ et R¹¹ⁿ peuvent être liées ensemble pour former un cycle à 4 à 20 chaînons, contenant facultativement un ou plusieurs hétéroatomes, contenant facultativement une ou plusieurs insaturations, et lequel cycle est facultativement substitué par un ou plusieurs substituants choisis parmi =O, =S, =N(R²⁰) et E¹² ;
chaque E¹, E², E³, E⁴, E⁵, E⁶, E⁷, E⁸, E¹⁰, E¹¹ et E¹² représente indépendamment, chaque fois qu'ils sont utilisés ici :
(i) Q₄ ;
(ii) alkyle en C₁ à ₁₂ facultativement substitué par un ou plusieurs substituants choisis parmi =O et Q⁵ ; ou
deux groupes E¹, E², E³, E⁴, E⁵, E⁶, E⁷, E⁸, E¹⁰, E¹¹ ou E¹² quelconques peuvent être liés ensemble pour former un cycle à 3 à 12 chaînons contenant facultativement une ou plusieurs insaturations, et lequel cycle est facultativement substitué par un ou plusieurs substituants choisis parmi =O et J¹ ;
chaque Q⁴ et Q⁵ représente indépendamment, chaque fois qu'ils sont utilisés ici : un halogéno, -CN, -NO₂, -N(R²⁰)R²¹, -OR²⁰, -C(=Y)-R²⁰, -C(=Y)-OR²⁰, -C(=Y)-OR²⁰, -C(=Y)N(R²⁰)R²¹, -OC(=Y)-R²⁰, -OC (=Y)N(R²⁰)R²¹, -OS(O)₂OR²⁰, -OP(=Y)(OR²⁰)(OR²¹), -OP(OR²⁰)(OR²¹), -N(R²²)C(=Y)R²¹; -N(R²²)C(=Y)OR²¹; -N(R²²)C(=Y)N(R²⁰)R²¹; -NR²²S(O)₂R²⁰ ; -NR²²S(O)₂N(R²⁰)R²¹ ; -S(O)₂N(R²⁰)R²¹ ; -SC(=Y)R²⁰ ; -S(O)₂R²⁰ ; -SR²⁰, -S(O)R²⁰, alkyle en C_{1 à 6}, hétérocycloalkyle (lesquels deux derniers groupes étant facultativement substitués par un ou plusieurs substituants choisis parmi =0 et J²), aryle ou hétéroaryle (lesquels deux derniers groupes étant facultativement substitués par un ou plusieurs substituants choisis parmi J³) ;
chaque Y représente indépendamment, chaque fois qu'il est utilisé ici, =O, =S, =NR²³ ou =N-CN ;
chaque R²⁰, R²¹, R²² et R²³ représente indépendamment, chaque fois qu'ils sont utilisés ici, un hydrogène, alkyle en C_{1 à 6}, hétérocycloalkyle (lesquels deux derniers groupes étant facultativement substitués par un ou plusieurs substituants choisis parmi J⁴ et =0), aryle ou hétéroaryle (lesquels deux derniers groupes étant facultativement substitués par un ou plusieurs substituants choisis parmi J⁵) ; ou
toutes les paires pertinentes de R²⁰, R²¹ et R²² peuvent être liées ensemble pour former un cycle à 4 à 20 chaînons contenant facultativement un ou plusieurs hétéroatomes, contenant facultativement une ou plusieurs insaturations, et lequel cycle est facultativement substitué par un ou plusieurs substituants choisis parmi J⁶ et =O ;
chaque J¹, J², J³, J⁴, J⁵ et J⁶ représente indépendamment, chaque fois qu'ils sont utilisés ici :
(i) Q⁷ ;
(ii) alkyle en C_{1 à 6} ou hétérocycloalkyle, tous deux étant facultativement substitués par un ou plusieurs substituants choisis parmi =O et Q⁸ ;
chaque Q⁷ et Q⁸ représente indépendamment, chaque fois qu'ils sont utilisés ici : un halogéno, -N(R⁵⁰)R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹ ; -NR⁵²S(O)₂R⁵⁰ ; -S(O)₂R⁵⁰, -SR⁵⁰, -S(O)R⁵⁰ ou alkyle en C_{1 à 6} facultativement substitué par un ou plusieurs atomes fluoro ;
chaque Y^{a} représente indépendamment, chaque fois qu'il est utilisé ici, =O, =S, =NR⁵³ ou =N-CN ;
chaque R⁵⁰, R⁵¹, R⁵² et R⁵³ représente indépendamment, chaque fois qu'ils sont utilisés ici, un hydrogène ou alkyle en C_{1 à 6} facultativement substitué par un ou plusieurs substituants choisis parmi fluoro, -OR⁶⁰ et -N(R⁶¹)R⁶² ; ou
toutes les paires pertinentes de R⁵⁰, R⁵¹ et R⁵² peuvent être liées ensemble pour former un cycle à 3 à 8 chaînons, contenant facultativement un ou plusieurs hétéroatomes, contenant facultativement une ou plusieurs insaturations, et lequel cycle est facultativement substitué par un ou plusieurs substituants choisis parmi =0 et alkyle en C_{1 à 3} ;
R⁶⁰, R⁶¹ et R⁶² représentent indépendamment un hydrogène ou alkyle en C_{1 à 6} facultativement substitué par un ou plusieurs atomes fluoro ;
ou un ester, amide, solvate ou sel pharmaceutiquement acceptable de celui-ci
dans lequel
un ester pharmaceutiquement acceptable est un composé contenant un acide carboxylique de formule I dans lequel un ou plusieurs groupes acide carboxylique ont été dérivatisés pour former un ester d'alkyle en C₁ à ₆, aryle en C_{5 à 10} et/ou aryle en C_{5 à 10}-alkyle en C₁ à ₆ ; et
un amide pharmaceutiquement acceptable est un composé contenant un acide carboxylique de formule I dans lequel un ou plusieurs desdits groupes acide carboxylique ont été dérivatisés pour former un amide de formule -C(O)N(R^{z1})R^{z2}, où chaque R^{z1} et R^{z2} représente indépendamment un alkyle en C_{1 à 6} facultativement substitué, aryle en C_{5 à 10}, ou aryle en C_{5 à 10}-alkylène en C_{1 à 6}-.

2. Composé selon la revendication 1, dans lequel R² représente un hydrogène ou chloro ; R³ représente un phényle, indazolyle, pyrimidinyle, azaindolyle, indolyle ou pyridyle facultativement substitué ; A₁ représente -C(R⁴)R⁵-, -C(O)- ou -N(R⁶)- ; A₂ représente -N(R⁶)-, -C(R⁴)R⁵- ou -C(O)- ; A₃ représente -C(R⁴)R⁵-, -N(R⁶)- ou -C(O)- ; l'un de A₂ et A₃ représente -C(R⁴)R⁵- et l'autre représente -C(R⁴)R⁵- ou -N(R⁶)- ; n représente 0 ou 1 ; A₄ représente -C(R⁴)R⁵- ou -C(O)-et/ou les lignes en pointillés ne représentent pas des doubles liaisons.

3. Composé selon la revendication 1 ou la revendication 2, dans lequel : chaque R⁴ et R⁵ représente indépendamment un hydrogène, alkyle en C_{1 à 6} (facultativement substitué comme défini ici ; mais de préférence non substitué), -OR^{10c}, -C(O)OR^{10h} ou -C(O)N(R¹⁰ⁱ)R¹¹ⁱ ; et/ou chaque R⁶ (lorsque/si présent) représente indépendamment un hydrogène, -C(O)R^{10r}, -C (0) OR^{10s}, -C (0) N (R^{10t})R^{11t}, -S(O)₂R^{10u}, alkyle en C₁ à ₆ (facultativement substitué par un ou plusieurs (par exemple, deux ou, de préférence un) substituants choisis parmi E⁷), hétérocycloalkyle (par exemple, un groupe à 5 ou 6 chaînons contenant un ou deux hétéroatomes ; qui est facultativement substitué par un ou plusieurs, par exemple, un, substituant(s) E⁷) ou aryle (par exemple, phényle ; facultativement substitué par un ou plusieurs substituants choisis parmi E⁸).

4. Composé selon l'une quelconque des revendications précédentes, dans lequel : chaque R^{10a}, R^{11a}, R^{10b}, R^{11b}, R^{10c}, R^{10d}, R^{11d}, R^{10e}, R^{10f}, R^{10g}, R^{10h}, R¹⁰ⁱ, R¹¹ⁱ, R^{10j}, R^{10k}, R^{10m}, R¹⁰ⁿ, R¹¹ⁿ, R^{10p}, R^{10q}, R^{10r}, R^{10s}, R^{10t}, R^{11t}, R^{10u} et R^{10x} représente indépendamment, chaque fois qu'ils sont utilisés ici, un hydrogène, alkyle en C₁ à ₆ (facultativement substitué par un ou plusieurs (par exemple, un ou deux) substituants choisis parmi E¹⁰), hétérocycloalkyle (par exemple, un cycle à 4 à 6 chaînons ; lequel groupe hétérocycloalkyle étant facultativement substitué par un ou plusieurs (par exemple, un ou deux) substituants choisis parmi E¹⁰), aryle (facultativement substitué par un ou plusieurs (par exemple, un ou deux) substituants choisis parmi E¹¹) ou hétéroaryle (par exemple, un groupe à 5 ou de préférence 6 chaînons contenant de préférence deux ou un hétéroatomes ; lequel groupe hétéroaryle est facultativement substitué par un ou plusieurs (par exemple, un ou deux) substituants choisis parmi E¹¹).

5. Composé selon l'une quelconque des revendications précédentes, dans lequel : Q⁴ représente un halogéno (par exemple, chloro ou fluoro), -CN, -OR²⁰, -N(R²⁰)R²¹, -C(=Y)OR²⁰, -C(=Y)-R²⁰, N(R²²)-S(O)₂R²⁰, -N(R²²)-C(=Y)-N(R²⁰)R²¹, -S(O)₂R²⁰, hétérocycloalkyle (par exemple, un cycle à 4 à 6 chaînons, contenant de préférence un hétéroatome choisi parmi l'azote et l'oxygène ; facultativement substitué par deux ou, de préférence un, substituants choisis parmi J²), aryle (par exemple, phényle ; facultativement substitué par deux ou, de préférence un, substituants choisis parmi J³) ou hétéroaryle (par exemple, un groupe hétéroaryle monocyclique à 5 ou 6 chaînons contenant de préférence un ou plusieurs hétéroatomes de préférence choisis parmi l'azote, l'oxygène et le soufre ; lequel groupe peut être substitué par un ou plusieurs substituants choisis parmi J³, mais est de préférence non substitué) ; Q⁵ représente un halogéno (par exemple, fluoro) ; Y représente =O ; R²⁰, R²¹, R²² et R²³ représentent indépendamment un hydrogène ou alkyle en C_{1 à 6} (par exemple, en C_{1 à 4}) facultativement substitué par un ou plusieurs (par exemple, un) substituant(s) choisi (s) parmi J⁴ ; chaque J¹, J², J³, J⁴, J⁵ et J⁶ représente indépendamment Q⁷ ou un alkyle en C_{1 à 6} facultativement substitué par un ou plusieurs groupes Q⁸; Q⁷ représente un halogéno (par exemple, fluoro ou chloro), -OR⁵⁰, -N(R⁵⁰)R⁵¹, -C(=Y^{a})-OR⁵⁰ ou -S(O)₂R⁵⁰ ; Q⁸ représente un halogéno (par exemple, fluoro) ; Y^{a} représente =O ; et/ou R⁵⁰ représente un hydrogène ou alkyle en C₁ à ₆ (de préférence, en C₁ à ₄) facultativement substitué par un ou plusieurs atomes fluoro.

6. Composé de formule I, ou ester, amide, solvate ou sel pharmaceutiquement acceptable de celui-ci, tel que défini dans l'une quelconque des revendications 1 à 5, pour une utilisation en tant que produit pharmaceutique.

7. Formulation pharmaceutique comprenant un composé de formule I, ou un ester, amide, solvate ou sel pharmaceutiquement acceptable de celui-ci, tel que défini dans l'une quelconque des revendications 1 à 5, en mélange avec un adjuvant, diluant ou véhicule pharmaceutiquement acceptable.

8. Composé de formule I, ou ester, amide, solvate ou sel pharmaceutiquement acceptable de celui-ci, tel que défini dans l'une quelconque des revendications 1 à 5, pour une utilisation dans le traitement d'une maladie choisie dans le groupe consistant en un cancer, un trouble immunitaire, une maladie cardiovasculaire, une infection virale, une inflammation, un trouble fonctionnel du métabolisme/de la fonction endocrinienne, un trouble neurologique, une maladie obstructive des voies aériennes, une maladie allergique, une maladie inflammatoire, une immunosuppression, un trouble communément lié à la transplantation d'organe, une maladie liée au SIDA, une hyperplasie bénigne de la prostate, une adénomatose familiale, une polypose, une neurofibromatose, un psoriasis, un trouble osseux, une athérosclérose, une prolifération de cellules lisses vasculaires associée à une athérosclérose, une fibrose pulmonaire, une arthrite, une glomérulonéphrite et une sténose post-chirurgicale, une resténose, un accident vasculaire cérébral, un diabète, une hépatomégalie, une maladie d'Alzheimer, une fibrose kystique, une maladie liée aux hormones, un trouble d'immunodéficience, un trouble destructeur des os, une maladie infectieuse, une affection associée à la mort cellulaire, une agrégation plaquettaire induite par la thrombine, une leucémie myélogène chronique, une maladie du foie, une affection immunitaire pathologique impliquant l'activation des cellules T, des troubles du SNC, et d'autres maladies associées.

9. Utilisation d'un composé de formule I, ou d'un ester, amide, solvate ou sel pharmaceutiquement acceptable de celui-ci, tel que défini dans l'une quelconque des revendications 1 à 5, pour la fabrication d'un médicament destiné au traitement d'une maladie choisie dans le groupe consistant en un cancer, un trouble immunitaire, une maladie cardiovasculaire, une infection virale, une inflammation, un trouble fonctionnel du métabolisme/de la fonction endocrinienne, un trouble neurologique, une maladie obstructive des voies aériennes, une maladie allergique, une maladie inflammatoire, une immunosuppression, un trouble communément lié à la transplantation d'organe, une maladie liée au SIDA, une hyperplasie bénigne de la prostate, une adénomatose familiale, une polypose, une neurofibromatose, un psoriasis, un trouble osseux, une athérosclérose, une prolifération de cellules lisses vasculaires associée à une athérosclérose, une fibrose pulmonaire, une arthrite, une glomérulonéphrite et une sténose post-chirurgicale, une resténose, un accident vasculaire cérébral, un diabète, une hépatomégalie, une maladie d'Alzheimer, une fibrose kystique, une maladie liée aux hormones, un trouble d'immunodéficience, un trouble destructeur des os, une maladie infectieuse, une affection associée à la mort cellulaire, une agrégation plaquettaire induite par la thrombine, une leucémie myélogène chronique, une maladie du foie, une affection immunitaire pathologique impliquant l'activation des cellules T, des troubles du SNC, et d'autres maladies associées.

10. Produit combiné comprenant :
(A) un composé de formule I, ou un ester, amide, solvate ou sel pharmaceutiquement acceptable de celui-ci, tel que défini dans l'une quelconque des revendications 1 à 5 ; et
(B) un autre agent thérapeutique qui est utile dans le traitement d'un cancer et/ou d'une maladie proliférative,
dans lequel chacun des composants (A) et (B) est formulé en mélange avec un adjuvant, diluant ou véhicule pharmaceutiquement acceptable.

11. Procédé de préparation d'un composé de formule I tel que défini dans la revendication 1, lequel procédé comprend :
(i) la réaction d'un composé de formule II, 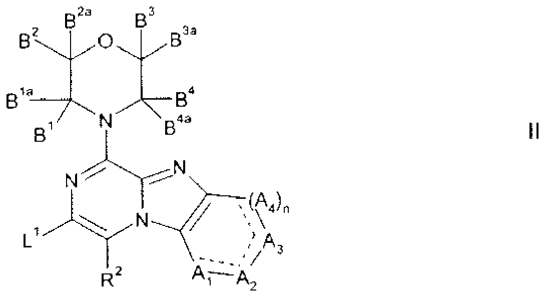 dans laquelle L¹ représente un groupe partant convenable, et A¹, A², A³, A⁴, n, les lignes en pointillés, B¹, B^{1a}, B², B^{2a}, B³, B^{3a}, B⁴, B^{4a} et R² sont tels que définis dans la revendication 1, avec un composé de formule III,
R³-L² III
dans laquelle L² représente un groupe convenable ;
(ii) la réaction d'un composé de formule IV, 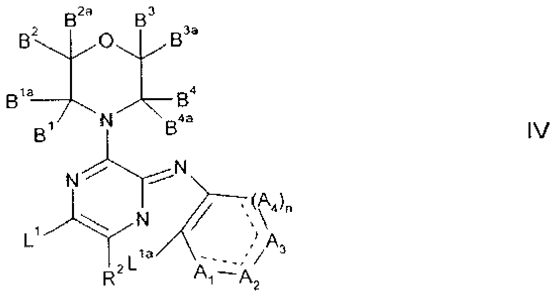 dans laquelle L^{1a} représente un groupe partant convenable, et L¹, A¹, A², A³, A⁴, n, les lignes en pointillés, B¹, B^{1a}, B², B^{2a}, B³, B^{3a}, B⁴, B^{4a} et R² sont tels que définis dans la revendication 1, avec un composé de formule III tel que défini ci-dessus ;
(iii) la réaction d'un composé de formule V, 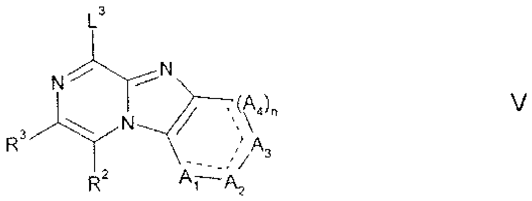 dans laquelle L³ représente un groupe partant convenable, et A¹, A², A³, A⁴, n, les lignes en pointillés, R² et R³ sont tels que définis dans la revendication 1, avec un composé de formule VI, 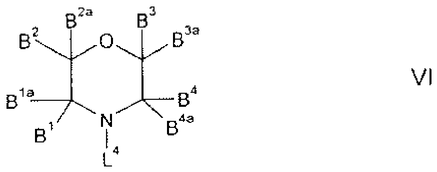 dans laquelle L⁴ peut représenter un hydrogène (formant ainsi un groupe amine), et L₁, B¹, B^{1a}, B², B^{2a}, B³, B^{3a}, B⁴, B^{4a} et R² sont tels que définis dans la revendication 1 ;
(iv) la réaction d'un composé de formule VII, 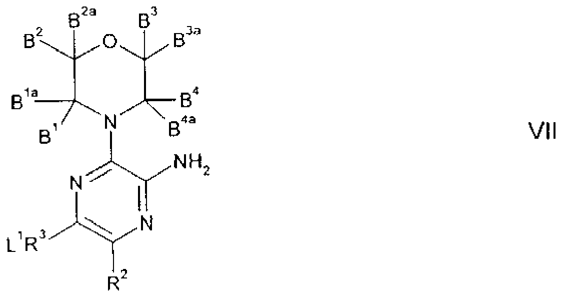 dans laquelle L¹R³ représente soit L¹ soit R³, B¹, B^{1a}, B², B^{2a}, B³, B^{3a}, B⁴, B^{4a}, R², L¹ et R³ sont tels que définis dans la revendication 1, avec un composé de formule VIII, 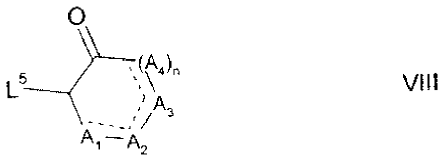 dans laquelle L⁵ représente un groupe partant convenable, et A¹, A², A³, A⁴, n, et les lignes en pointillés sont tels que définis dans la revendication 1 ;
(v) pour les composés de formule I où R² représente, la réaction d'un composé correspondant de formule I, où R² représente un hydrogène, avec un réactif qui est une source d'ions halogénure ;
(vi) pour les composés de formule I où R² représente un substituant autre qu'un hydrogène, ou halogéno, la réaction d'un composé correspondant de formule I, dans laquelle R² représente un halogéno, avec un composé de formule IX,
R^{2a}-L⁷ IX
dans laquelle R^{2a} représente R² comme décrit dans la revendication 1 à condition qu'il ne représente pas un hydrogène ou halogéno, et L⁷ représente un groupe partant convenable ;
(vii) pour certains composés de formule I, la réaction d'un composé de formule X, 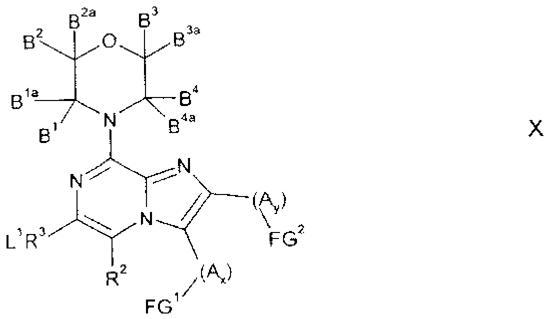 dans laquelle (Aₓ) et (Ay) désignent la présence facultative des groupes pertinents A₁ et A₄ qui sont/peuvent être présents dans le composé de formule I, et FG¹ et FG² représentent indépendamment des groupes fonctionnels mutuellement compatibles, qui peuvent subir une réaction intramoléculaire pour former le cycle contenant A₁ à A₄ requis de formule I (et L¹R³, B¹, B^{1a}, B², B^{2a}, B³, B^{3a}, B⁴, B^{4a} et R² sont tels que définis dans la revendication 1) ;
(viii) pour certains composés de formule I, la réaction d'un composé de formule VII comme décrit ci-dessus, avec un composé de formule XI, 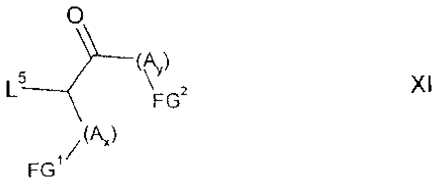 dans laquelle (Aₓ), (A_{y}), FG¹, FG² et L⁵ sont tels que définis ci-dessus ;
(ix) pour les composés de formule I où une fraction -N(R⁶)- est présente, dans laquelle R⁶ représente un alkyle en C_{1 à 12} facultativement substitué comme défini ci-avant (c'est-à-dire par un ou plusieurs substituant(s) choisi(s) parmi E⁷ et =0), l'amination réductrice d'un composé correspondant de formule I dans laquelle R⁶ représente un hydrogène, avec un composé de formule XII,
R⁶-C(O)H XII
dans laquelle R^{6a} représente un alkyle en C_{1 à 11} facultativement substitué par un ou plusieurs substituant (s) choisi(s) parmi E⁷ et =O ;
(x) pour les composés de formule I où un groupe fonctionnel -N(R⁶)- est présent, dans lequel R⁶ représente -C(O)N(H)R^{11t} ; la réaction d'un composé correspondant de formule I dans laquelle R⁶ représente un hydrogène, avec un composé de formule XIII,
R^{11t}-N=C=O XIII
dans laquelle R^{11t} est tel que défini dans la revendication 1 ;
(xi) les composés de formule I où un groupe fonctionnel -N(R⁶)- est présent, dans lequel R⁶ représente -C(O)R^{10r} ou -S(O)₂R^{10u}, peuvent être préparés par réaction d'un composé correspondant de formule I dans laquelle R⁶ représente hydrogène, avec un composé de formule XIV,
G¹-L^{1b} XIV
dans laquelle G¹ représente soit -C(O)R^{10r} soit -S(O)₂R^{10u}, et L^{1b} (attaché aux groupes fonctionnels -C(O)- ou -S(O)₂) représente un groupe partant convenable.

12. Procédé pour la préparation d'une formulation pharmaceutique telle que définie dans la revendication 7, lequel procédé comprend l'apport en association d'un composé de formule I, ou d'un ester, amide, solvate ou sel pharmaceutiquement acceptable de celui-ci, tel que défini dans l'une quelconque des revendications 1 à 5, avec un adjuvant, diluant ou véhicule pharmaceutiquement acceptable.

13. Procédé pour la préparation d'un produit combiné tel que défini dans la revendication 10, lequel procédé comprend le fait d'amener en association un composé de formule I, ou un ester, amide, solvate ou sel pharmaceutiquement acceptable de celui-ci, tel que défini dans l'une quelconque des revendications 1 à 5, avec l'autre agent thérapeutique qui est utile dans le traitement du cancer et/ou d'une maladie proliférative, et au moins un adjuvant, diluant ou véhicule pharmaceutiquement acceptable.
